(19) Europäisches Patentamt
European Patent Office
Office européen des brevets

(11) EP 3 772 517 B1

(12) **EUROPEAN PATENT SPECIFICATION**

(45) Date of publication and mention of the grant of the patent:
**02.11.2022 Bulletin 2022/44**

(21) Application number: **20190113.9**

(22) Date of filing: **07.08.2020**

(51) International Patent Classification (IPC):
***C07F 15/00*** (2006.01) ***C09K 11/06*** (2006.01)
***H01L 51/00*** (2006.01) ***H01L 51/50*** (2006.01)

(52) Cooperative Patent Classification (CPC):
**C07F 15/0086; C09K 11/06; H01L 51/0087;**
C09K 2211/1007; C09K 2211/1029;
C09K 2211/104; C09K 2211/1048; C09K 2211/185;
H01L 51/0081; H01L 51/5016

(54) **ORGANOMETALLIC COMPOUND, ORGANIC LIGHT-EMITTING DEVICE INCLUDING SAME, AND DIAGNOSTIC COMPOSITION INCLUDING SAME**

ORGANOMETALLISCHE VERBINDUNG, ORGANISCHE LICHTEMITTIERENDE VORRICHTUNG DAMIT UND DIAGNOSEZUSAMMENSETZUNG DAMIT

COMPOSÉ ORGANOMÉTALLIQUE, DISPOSITIF ÉLECTROLUMINESCENT ORGANIQUE LE COMPRENANT ET COMPOSITION DE DIAGNOSTIC LE COMPRENANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.08.2019 KR 20190097638**

(43) Date of publication of application:
**10.02.2021 Bulletin 2021/06**

(73) Proprietors:
- **Samsung Electronics Co., Ltd.**
  **Gyeonggi-do 16677 (KR)**
- **Samsung SDI Co., Ltd.**
  **Gyeonggi-do 17084 (KR)**

(72) Inventors:
- **MIN, Minsik**
  **16678 Gyeonggi-do (KR)**
- **KIM, Wook**
  **17084 Gyeonggi-do (KR)**
- **KIM, Sangmo**
  **16678 Gyeonggi-do (KR)**
- **KIM, Jongsoo**
  **16678 Gyeonggi-do (KR)**
- **KIM, Joonghyuk**
  **16678 Gyeonggi-do (KR)**
- **BAE, Hyejin**
  **16678 Gyeonggi-do (KR)**
- **SON, Jhunmo**
  **16678 Gyeonggi-do (KR)**
- **LEE, Hasup**
  **16678 Gyeonggi-do (KR)**
- **JUNG, Yongsik**
  **16678 Gyeonggi-do (KR)**

(74) Representative: **Elkington and Fife LLP**
**Prospect House**
**8 Pembroke Road**
**Sevenoaks, Kent TN13 1XR (GB)**

(56) References cited:
**US-A1- 2018 337 350** **US-A1- 2019 036 042**
**US-A1- 2019 074 455** **US-A1- 2019 214 584**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

FIELD OF THE INVENTION

One or more embodiments of the present disclosure relate to an organometallic compound, an organic light-emitting device including the organometallic compound, and a diagnostic composition that includes the organometallic compound.

BACKGROUND OF THE INVENTION

Organic light-emitting devices (OLEDs) are self-emission devices which produce full-color images. In addition, OLEDs have wide viewing angles and exhibit excellent driving voltage and response speed characteristics.

OLEDs include an anode, a cathode, and an organic layer between the anode and the cathode and including an emission layer. A hole transport region may be disposed between the anode and the em ission layer, and an electron transport region may be disposed between the emission layer and the cathode. Holes provided from the anode may move toward the emission layer through the hole transport region, and electrons provided from the cathode may move toward the emission layer through the electron transport region. The holes and the electrons recombine in the emission layer to produce excitons. These excitons transit from an excited state to a ground state to thereby generate light.

Further, light-emitting compounds, e.g., phosphorescence-emitting compounds, can also be used to monitor, sense, or detect biological materials, including a variety of cells and proteins. US2019/036042A1 discloses organometallic compounds used in OLEDs.

SUMMARY OF THE INVENTION

Provided are an organometallic compound, an organic light-emitting device including the organometallic compound, and a diagnostic composition including the organometallic compound.

Additional aspects will be set forth in part in the description which follows and, in part, will be apparent from the description, or may be learned by practice of the presented embodiments.

According to an aspect of an embodiment, an organometallic compound may be represented by Formula 1:

Formula 1

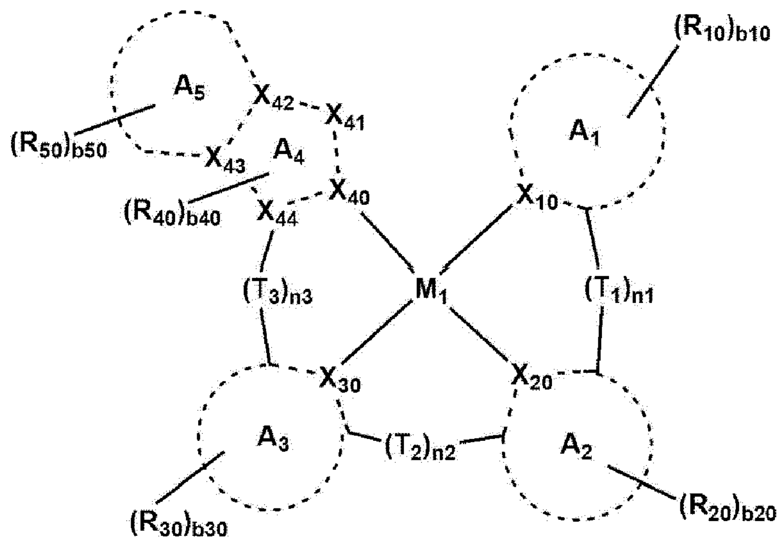

wherein, in Formula 1,

$M_1$ may be beryllium (Be), magnesium (Mg), aluminum (Al), calcium (Ca), titanium (Ti), manganese (Mn), cobalt (Co), copper (Cu), zinc (Zn), gallium (Ga), germanium (Ge), zirconium (Zr), ruthenium (Ru), rhodium (Rh), palladium (Pd), silver (Ag), rhenium (Re), platinum (Pt), or gold (Au),

$A_1$ to $A_3$ may each independently be a $C_5$-$C_{30}$ carbocyclic group or a $C_1$-$C_{30}$ heterocyclic group,

$A_4$ may be a 5-membered heterocyclic group,

$A_5$ may be a $C_7$-$C_{30}$ carbocyclic group of at least two rings including a 6-membered carbocyclic group, or $A_5$ may

be a $C_1$-$C_{30}$ heterocyclic group of at least two rings including a 6-membered carbocyclic group or a 6-membered heterocyclic group,

$X_{10}$, $X_{20}$, $X_{30}$ and $X_{40}$ to $X_{44}$ may each independently be C or N,

$T_1$ and $T_3$ may each independently be a single bond, *-N[$(L_1)_{a1}$-$(R_1)_{b1}$]-*', *-B($R_1$)-*', *-P($R_1$)-*', *-C($R_1$)($R_2$)-*', *-Si($R_1$)($R_2$)-*', *-Ge($R_1$)($R_2$)-*', *-S-*', *-Se-*', *-O-*', *-C(=O)-*', *-S(=O)-*', *-S(=O)$_2$-*', *-C($R_1$)=C($R_2$)-*', *-C(=S)-*', or *-C≡C-*',

$T_2$ is a single bond, *-N[$(L_1)a_1$-$(R_1)b_1$]-*', *-P($R_1$)-*', *-C($R_1$) ($R_2$)-*', *-Si($R_1$)($R_2$)-*', *-Ge($R_1$)($R_2$)-*', *-S-*', *-Se-*', *-O-*', *-C(=O)-*', *-S(=O)-*', *-S(=O)$_2$-*', *- C($R_1$)=C($R_2$)-*', *-C(=S)-*', or *-C≡C-*',

wherein * and *' may each indicate a binding site to an adjacent atom n1 may be an integer from 1 to 3,

$L_1$ may be a single bond, a substituted or unsubstituted $C_5$-$C_{30}$ carbocyclic group, or a substituted or unsubstituted $C_1$-$C_{30}$ heterocyclic group,

a1 may be an integer from 1 to 3, and when a1 is 2 or greater, at least two $L_1$ groups may be identical to or different from each other,

$R_1$, $R_2$, $R_{10}$, $R_{20}$, $R_{30}$, $R_{40}$, and $R_{50}$ may each independently be hydrogen, deuterium, -F, -Cl, -Br, -I, -$SF_5$, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_6$-$C_{60}$ aryloxy group, a substituted or unsubstituted $C_6$-$C_{60}$ arylthio group, a substituted or unsubstituted $C_7$-$C_{60}$ arylalkyl group, a substituted or unsubstituted $C_1$-$C_{60}$ heteroaryl group, a substituted or unsubstituted $C_1$-$C_{60}$ heteroaryloxy group, a substituted or unsubstituted $C_1$-$C_{60}$ heteroarylthio group, a substituted or unsubstituted $C_2$-$C_{60}$ heteroarylalkyl group, a substituted or unsubstituted monovalent non-aromatic condensed polycyclic group, a substituted or unsubstituted monovalent non-aromatic condensed heteropolycyclic group, -N($Q_1$)($Q_2$), -Si($Q_3$)($Q_4$)($Q_5$), -Ge($Q_3$)($Q_4$)($Q_5$), -B($Q_6$)($Q_7$), -P($Q_8$)($Q_9$), or -P(=O)($Q_8$)($Q_9$),

at least two adjacent $R_1$, $R_2$, $R_{10}$, $R_{20}$, $R_{30}$, $R_{40}$, or $R_{50}$ groups may optionally be bound together to form a substituted or unsubstituted $C_5$-$C_{30}$ carbocyclic group or a substituted or unsubstituted $C_1$-$C_{30}$ heterocyclic group,

b1 may be an integer from 1 to 5, and when b1 is 2 or greater, at least two $R_1$ groups may be identical to or different from each other,

b10, b20, b30, and b50 may each independently be an integer from 1 to 10, b40 may be an integer from 1 to 3,

when b10 is 2 or greater, at least two $R_{10}$ groups may be identical to or different from each other, when b20 is 2 or greater, at least two $R_{20}$ groups may be identical to or different from each other, when b30 is 2 or greater, at least two $R_{30}$ groups may be identical to or different from each other, when b40 is 2 or greater, at least two $R_{40}$ groups may be identical to or different from each other, when b50 is 2 or greater, at least two $R_{50}$ groups may be identical to or different from each other, and

at least one substituent of the substituted $C_5$-$C_{30}$ carbocyclic group, the substituted $C_1$-$C_{30}$ heterocyclic group, the substituted $C_1$-$C_{60}$ alkyl group, the substituted $C_2$-$C_{60}$ alkenyl group, the substituted $C_2$-$C_{60}$ alkynyl group, the substituted $C_1$-$C_{60}$ alkoxy group, the substituted $C_3$-$C_{10}$ cycloalkyl group, the substituted $C_1$-$C_{10}$ heterocycloalkyl group, the substituted $C_3$-$C_{10}$ cycloalkenyl group, the substituted $C_1$-$C_{10}$ heterocycloalkenyl group, the substituted $C_6$-$C_{60}$ aryl group, the substituted $C_6$-$C_{60}$ aryloxy group, the substituted $C_6$-$C_{60}$ arylthio group, the substituted $C_7$-$C_{60}$ arylalkyl group, the substituted $C_1$-$C_{60}$ heteroaryl group, the substituted $C_1$-$C_{60}$ heteroaryloxy group, the substituted $C_1$-$C_{60}$ heteroarylthio group, the substituted $C_2$-$C_{60}$ heteroarylalkyl group, the substituted monovalent non-aromatic condensed polycyclic group, or the substituted monovalent non-aromatic condensed heteropolycyclic group may be:

deuterium, -F, -Cl, -Br, -I, -$CD_3$, -$CD_2H$, -$CDH_2$, -$CF_3$, -$CF_2H$, -$CFH_2$, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, or a $C_1$-$C_{60}$ alkoxy group;

a $C_1$-$C_{60}$ alkyl group, a $C_2$-$C_{60}$ alkenyl group, a $C_2$-$C_{60}$ alkynyl group, and a $C_1$-$C_{60}$ alkoxy group, each substituted with at least one selected from deuterium, -F, -Cl, -Br, -I, -$CD_3$, -$CD_2H$, -$CDH_2$, -$CF_3$, -$CF_2H$, -$CFH_2$, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_7$-$C_{60}$ arylalkyl group, a $C_1$-$C_{60}$ heteroaryl group, a $C_1$-$C_{60}$ heteroaryloxy group, a $C_1$-$C_{60}$ heteroarylthio group, a $C_2$-$C_{60}$ heteroarylalkyl group, a monovalent non-aromatic condensed polycyclic group, a monovalent non-aromatic condensed heteropolycyclic group, -N($Q_{11}$)($Q_{12}$), -Si($Q_{13}$)($Q_{14}$)($Q_{15}$), -Ge($Q_{13}$)($Q_{14}$)($Q_{15}$), -B($Q_{16}$)($Q_{17}$), -P($Q_{18}$)($Q_{19}$), or

-P(=O)($Q_{18}$)($Q_{19}$);

a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, or a monovalent non-aromatic condensed heteropolycyclic group;

a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, or a monovalent non-aromatic condensed heteropolycyclic group, each substituted with at least one selected from deuterium, - F, -Cl, -Br, -I, -$CD_3$, -$CD_2H$, -$CDH_2$, -$CF_3$, -$CF_2H$, -$CFH_2$, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_7$-$C_{60}$ arylalkyl group, a $C_1$-$C_{60}$ heteroaryl group, a $C_1$-$C_{60}$ heteroaryloxy group, a $C_1$-$C_{60}$ heteroarylthio group, a $C_2$-$C_{60}$ heteroarylalkyl group, a monovalent non-aromatic condensed polycyclic group, a monovalent non-aromatic condensed heteropolycyclic group, -N($Q_{21}$)($Q_{22}$), -Si($Q_{23}$)($Q_{24}$)($Q_{25}$), -Ge($Q_{23}$)($Q_{24}$)($Q_{25}$), -B($Q_{26}$)($Q_{27}$), -P($Q_{28}$)($Q_{29}$), or -P(=O)($Q_{28}$)($Q_{29}$); or

-N(Q31)(Q32), -Si($Q_{33}$)($Q_{34}$)($Q_{35}$), -Ge($Q_{33}$)($Q_{34}$)($Q_{35}$), -B($Q_{36}$)($Q_{37}$), -P($Q_{38}$)($Q_{39}$), or -P(=O)($Q_{38}$)($Q_{39}$),

wherein Q1 to $Q_9$, Q11 to Q19, Q21 to Q29, and Q31 to Q39 may each independently be hydrogen, a $C_1$-$C_{60}$ alkyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, or a monovalent non-aromatic condensed heteropolycyclic group, each except hydrogen substituted with at least one of deuterium, -F, -Cl, -Br, -I, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group substituted with a $C_1$-$C_{60}$ alkyl group, a $C_6$-$C_{60}$ aryl group, or a combination thereof, a $C_6$-$C_{60}$ aryloxy group; a $C_6$-$C_{60}$ arylthio group; a $C_7$-$C_{60}$ arylalkyl group; a $C_1$-$C_{60}$ heteroaryl group; a $C_1$-$C_{60}$ heteroaryloxy group; a $C_1$-$C_{60}$ heteroarylthio group; a $C_2$-$C_{60}$ heteroarylalkyl group; a monovalent non-aromatic condensed polycyclic group; or a monovalent non-aromatic condensed heteropolycyclic group.

According to an aspect of another embodiment, an organic light-emitting device may include: a first electrode; a second electrode; and an organic layer disposed between the first electrode and the second electrode, the organic layer including an emission layer and at least one of the organometallic compounds.

According to an aspect of still another embodiment, a diagnostic composition may include at least one organometallic compound represented by Formula 1.

BRIEF DESCRIPTION OF THE DRAWINGS

These and/or other aspects will become apparent and more readily appreciated from the following description of the embodiments, taken in conjunction with the accompanying drawings in which:

The FIGURE 1 is a schematic cross-sectional view of an organic light-emitting device according to an embodiment.

DETAILED DESCRIPTION OF THE EMBODIMENTS

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects. As used herein, the term "and/or" includes any and all combinations of one or more of the associated listed items. Expressions such as "at least one of," when preceding a list of elements, modify the entire list of elements and do not modify the individual elements of the list.

It will be understood that when an element is referred to as being "on" another element, it can be directly in contact with the other element or intervening elements may be present therebetween. In contrast, when an element is referred to as being "directly on" another element, there are no intervening elements present.

It will be understood that, although the terms first, second, third etc. may be used herein to describe various elements, components, regions, layers, and/or sections, these elements, components, regions, layers, and/or sections

should not be limited by these terms. These terms are only used to distinguish one element, component, region, layer, or section from another element, component, region, layer, or section. Thus, a first element, component, region, layer, or section discussed below could be termed a second element, component, region, layer, or section without departing from the teachings of the present embodiments.

The terminology used herein is for the purpose of describing particular embodiments only and is not intended to be limiting. As used herein, the singular forms "a," "an," and "the" are intended to include the plural forms as well, unless the context clearly indicates otherwise.

The term "or" means "and/or." It will be further understood that the terms "comprises" and/or "comprising," or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.

Unless otherwise defined, all terms (including technical and scientific terms) used herein have the same meaning as commonly understood by one of ordinary skill in the art to which this general inventive concept belongs. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

Exemplary embodiments are described herein with reference to a cross section illustration that is a schematic illustration of one or more idealized embodiments. As such, variations from the shapes of the illustration as a result, for example, of manufacturing techniques and/or tolerances, are to be expected. Thus, embodiments described herein should not be construed as limited to the particular shapes of regions as illustrated herein but are to include deviations in shapes that result, for example, from manufacturing. For example, a region illustrated or described as flat may, typically, have rough and/or nonlinear features. Moreover, sharp angles that are illustrated may be rounded. Thus, the regions illustrated in the figure are schematic in nature and their shapes are not intended to illustrate the precise shape of a region and are not intended to limit the scope of the present claims.

"About" or "approximately" as used herein is inclusive of the stated value and means within an acceptable range of deviation for the particular value as determined by one of ordinary skill in the art, considering the measurement in question and the error associated with measurement of the particular quantity (i.e., the limitations of the measurement system). For example, "about" can mean within one or more standard deviations, or within ± 30%, 20%, 10%, 5% of the stated value.

An aspect of the present disclosure provides an organometallic compound that may be represented by Formula 1:

Formula 1

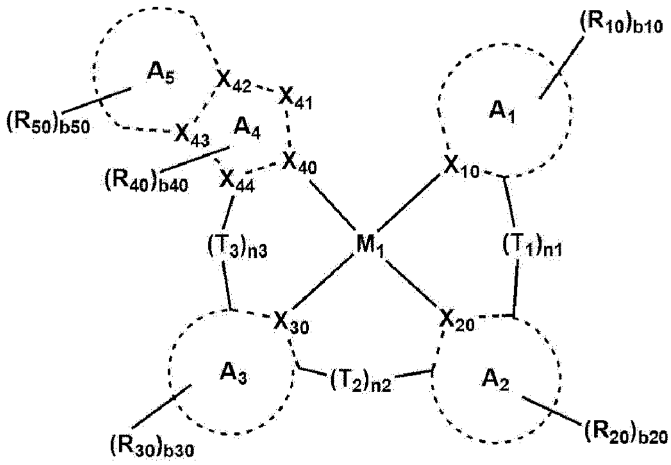

wherein, in Formula 1, $M_1$ may be beryllium (Be), magnesium (Mg), aluminum (Al), calcium (Ca), titanium (Ti), manganese (Mn), cobalt (Co), copper (Cu), zinc (Zn), gallium (Ga), germanium (Ge), zirconium (Zr), ruthenium (Ru), rhodium (Rh), palladium (Pd), silver (Ag), rhenium (Re), platinum (Pt), or gold (Au).

In some embodiments, $M_1$ may be Pd, Pt, or Au, but embodiments are not limited thereto.

In Formula 1, $A_1$ to $A_3$ may each independently be a $C_5$-$C_{30}$ carbocyclic group or a $C_1$-$C_{30}$ heterocyclic group.

In some embodiments, $A_1$ to $A_3$ may each independently be a phenyl group, a naphthyl group, an anthracenyl group, a phenanthrenyl group, a triphenylenyl group, a pyrenyl group, a chrysenyl group, a cyclopentadiene group, a 1,2,3,4-tetrahydronaphthyl group, a furanyl group, a thiophenyl group, a silolyl group, an indenyl group, a fluorenyl group,

an indolyl group, an isoindolyl group, a carbazolyl group, benzocarbazolyl group, a dibenzocarbazolyl group, a benzofuranyl group, a dibenzofuranyl group, a benzothiophenyl group, a benzosilolyl group, a dibenzosilolyl group, an azafluorenyl group, an azacarbazolyl group, an azadibenzofuranyl group, an azadibenzothiophenyl group, an azadibenzosilolyl group, a pyridinyl group, an imidazopyridinyl group, a pyrimidinyl group, an imidazopyrimidinyl group, a pyrazinyl group, a pyridazinyl group, a triazinyl group, a purinyl group, a quinolinyl group, a benzoquinolinyl group, an isoquinolinyl group, a quinoxalinyl group, a quinazolinyl group, a cinnolinyl group, a phenanthrolinyl group, a pyrrolyl group, a pyrazolyl group, an imidazolyl group, a triazolyl group, a tetrazolyl group, an oxazolyl group, an isoxazolyl group, a thiazolyl group, an isothiazolyl group, an oxadiazolyl group, a thiadiazolyl group, a benzopyrazolyl group, a benzimidazolyl group, an indazolyl group, a benzoxazolyl group, an isobenzoxazolyl group, a benzothiazolyl group, a benzoxadiazolyl group, a benzothiadiazolyl group, a benzotriazolyl group, a diazaindenyl group, a triazaindenyl group, a 5,6,7,8-tetrahydroisoquinolinyl group, or a 5,6,7,8-tetrahydroquinolinyl group.

In some embodiments, ring $A_1$ and ring $A_3$ may each independently be a phenyl group, a naphthyl group, a 1,2,3,4-tetrahydronaphthyl group, a fluorenyl group, a carbazolyl group, a dibenzofuranyl group, a dibenzothiophenyl group, or a dibenzosilolyl group.

In Formula 1, $A_4$ may be a 5-membered heterocyclic group.

In some embodiments, $A_4$ may be a furanyl group, a thiophenyl group, a pyrrolyl group, a silolyl group, an oxazolyl group, an isoxazolyl group, an oxadiazolyl group, an isooxadiazolyl group, an oxatriazolyl group, an isooxatriazolyl group, a thiazolyl group, an isothiazolyl group, a thiadiazolyl group, an isothiadiazolyl group, a thiatriazolyl group, an isothiatriazolyl group, a pyrazolyl group, an imidazolyl group, a triazolyl group, a tetrazolyl group, an azasilolyl group, a diazasilolyl group, or a triazasilolyl group.

In some embodiments, $A_4$ may be a pyrrolyl group, a pyrazolyl group, an imidazolyl group, a triazolyl group, or a tetrazolyl group.

In Formula 1, $A_5$ may be a $C_7$-$C_{30}$ carbocyclic group of at least two rings including a 6-membered carbocyclic group, or $A_5$ is a $C_1$-$C_{30}$ heterocyclic group of at least two rings including a 6-membered carbocyclic group or a 6-membered heterocyclic group.

In some embodiments, the at least two rings in $A_5$ may be condensed.

In some embodiments, the 6-membered carbocyclic group may be a cyclohexane group or a benzene group. In some embodiments, the 6-membered carbocyclic group may be a benzene group. It is to be understood that a "6-membered carbocyclic group" refers to a 6-membered carbocyclic ring in the structure of $A_5$.

In some embodiments, the 6-membered heterocyclic group may be selected from a pyridine group, a pyrimidine group, a pyrazine group, a pyridazine group, and a triazine group. In some embodiments, the 6-membered heterocyclic group may be a pyridine group. It is to be understood that a "6-membered heterocyclic group" refers to a 6-membered heterocyclic ring in the structure of $A_5$.

In some embodiments, $A_5$ may be a group represented by any one of Formulae A5-1 to A5-6:

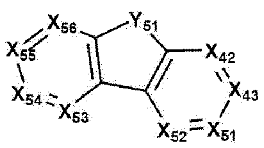
A5-1

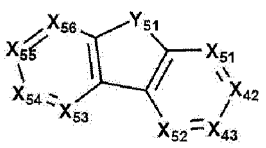
A5-2

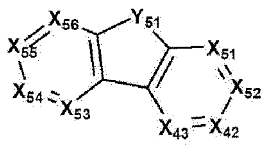
A5-3

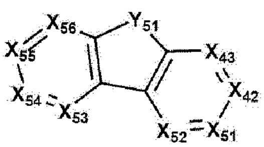
A5-4

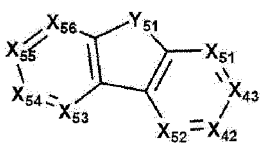
A5-5

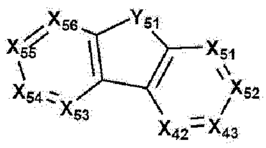
A5-6

wherein, in Formulae A5-1 to A5-6,

$Y_{51}$ may be *-O-*',*-S-*', *-N($R_5$)-*', *-C($R_5$)($R_6$)-*', *-Si($R_5$)($R_6$)-*', *-B($R_5$)-*', *-P($R_5$)-*',or *-P(=O)($R_5$)-*', wherein * and *' each indicate a binding site to an adjacent atom,

$R_5$ and $R_6$ may respectively be understood by referring to the descriptions of $R_1$ and $R_2$ provided herein,

$X_{42}$ and $X_{43}$ may respectively be understood by referring to the descriptions of $X_{42}$ and $X_{43}$ provided herein, and $X_{51}$ to $X_{56}$ may each independently be C or N,

$X_{51}$ may be C($R_{51}$) or N, $X_{52}$ may be C($R_{52}$) or N, $X_{53}$ may be C($R_{53}$) or N, $X_{54}$ may be C($R_{54}$) or N, $X_{55}$ may be

$C(R_{55})$ or N, $X_{56}$ may be $C(R_{56})$ or N, and
$R_{51}$ to $R_{56}$ may each independently be understood by referring to the description of $R_{50}$ provided herein.

In some embodiments, $X_{51}$ to $X_{56}$ may each be C.
In some embodiments, at least one of $X_{51}$ to $X_{56}$ may be N.
In Formula 1, $X_{10}$, $X_{20}$, $X_{30}$, and $X_{40}$ to $X_{44}$ may each independently be C or N.
In some embodiments, $X_{10}$ may be N, and $X_{20}$, $X_{30}$, and $X_{40}$ may each be C.
In some embodiments, $X_{40}$ in $A_4$ may be C, and $X_{41}$ and $X_{44}$ may each be N.
In some embodiments, $X_{42}$ and $X_{43}$ in $A_4$ may each be C.
In some embodiments, a bond between $M_1$ and $X_{10}$, a bond between $M_1$ and $X_{20}$, a bond between $M_1$ and $X_{30}$, and a bond between $M_1$ and $X_{40}$ may each independently be a coordinate bond or a covalent bond.
In Formula 1, two of a bond between $M_1$ and $X_{10}$, a bond between $M_1$ and $X_{20}$, a bond between $M_1$ and $X_{30}$, or a bond between $M_1$ and $X_{40}$ may each be a covalent bond, and the other two bonds may each be a coordinate bond. The organometallic compound represented by Formula 1 may be electrically neutral.
In some embodiments, a bond between $M_1$ and $X_{10}$ may be a coordinate bond, a bond between $M_1$ and $X_{20}$ may be a covalent bond, a bond between $M_1$ and $X_{30}$ may be a covalent bond, and a bond between $M_1$ and $X_{40}$ may be a coordinate bond.
In Formula 1, $T_1$ to $T_3$ may each independently be a single bond, *-N[$(L_1)_{a1}$-$(R_1)_{b1}$]-*', *-B($R_1$)-*', *-P($R_1$)-*', *-C($R_1$)($R_2$)-*', *-Si($R_1$)($R_2$)-*', *-Ge($R_1$)($R_2$)-*', *-S-*', *-Se-*', *-O-*', *-C(=O)-*', *-S(=O)-*', *-S(=O)$_2$-*', *-C($R_1$)=*', *=C($R_1$)-*', *-C($R_1$)=C($R_2$)-*', *-C(=S)-*', or *-C≡C-*', wherein * and *' each indicate a binding site to an adjacent atom. $R_1$ and $R_2$ may respectively be understood by referring to the descriptions of $R_1$ and $R_2$ provided herein.
In some embodiments, $T_1$ to $T_3$ may each independently be *-N[$(L_1)_{a1}$-$(R_1)_{b1}$]-*', *-C($R_1$)($R_2$)-*', *-Si($R_1$)($R_2$)-*', *-O-*',or *-S-*'. In some embodiments, $T_1$ may be *-N[$(L_1)_{a1}$-$(R_1)_{b1}$]-*', *-O-*',or *-S-*', wherein * and *' each indicate a binding site to an adjacent atom.
In Formula 1, n1 to n3 may each independently be an integer from 1 to 3.
In some embodiments, n1 to n3 may each independently be 1 or 2.
In some embodiments, n1 to n3 may each be 1.
In Formula 1, $L_1$ may be a single bond, a substituted or unsubstituted $C_5$-$C_{30}$ carbocyclic group, or a substituted or unsubstituted $C_1$-$C_{30}$ heterocyclic group.
In some embodiments, $L_1$ may be:

a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylenylene group, a naphthacenylene group, a picenylene group, a perylenylene group, or a pentacenylene group; or
a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylenylene group, a naphthacenylene group, a picenylene group, a perylenylene group, or a pentacenylene group, each substituted with at least one of deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_3$-$C_{10}$ cycloalkenyl group, a $C_1$-$C_{10}$ heterocycloalkyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, or a monovalent non-aromatic condensed heteropolycyclic group.

In Formula 1, a1 may be an integer from 1 to 3, and when a1 is 2 or greater, at least two $L_1$ groups may be identical to or different from each other. In some embodiments, a1 may be 1 or 2.
In Formula 1, $R_1$, $R_2$, $R_{10}$, $R_{20}$, $R_{30}$, $R_{40}$, and $R_{50}$ may each independently be hydrogen, deuterium, -F, -Cl, -Br, -I, -$SF_5$, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_6$-$C_{60}$ aryloxy group, a substituted or unsubstituted $C_6$-$C_{60}$ arylthio group, a substituted or unsubstituted $C_7$-$C_{60}$ arylalkyl group, a substituted or unsubstituted $C_1$-$C_{60}$ heteroaryl group, a

substituted or unsubstituted $C_1$-$C_{60}$ heteroaryloxy group, a substituted or unsubstituted $C_1$-$C_{60}$ heteroarylthio group, a substituted or unsubstituted $C_2$-$C_{60}$ heteroarylalkyl group, a substituted or unsubstituted monovalent non-aromatic condensed polycyclic group, a substituted or unsubstituted monovalent non-aromatic condensed heteropolycyclic group, $-N(Q_1)(Q_2)$, $-Si(Q_3)(Q_4)(Q_5)$, $-Ge(Q_3)(Q_4)(Q_5)$, $-B(Q_6)(Q_7)$, $-P(Q_8)(Q_9)$, or $-P(=O)(Q_8)(Q_9)$.

At least two adjacent groups R1, $R_2$, $R_{10}$, $R_{20}$, $R_{30}$, $R_{40}$, or $R_{50}$ may optionally be bound together to form a substituted or unsubstituted $C_5$-$C_{30}$ carbocyclic group or a substituted or unsubstituted $C_1$-$C_{30}$ heterocyclic group, for example any two or more adjacent groups $R_1$, $R_2$, $R_{10}$, $R_{20}$, $R_{30}$, $R_{40}$, or $R_{50}$ may optionally be bound together through a single bond, a double bond, or a first linking group to form a substituted or unsubstituted $C_5$-$C_{30}$ carbocyclic group or a substituted or unsubstituted $C_1$-$C_{30}$ heterocyclic group. In some embodiments, the substituted $C_5$-$C_{30}$ carbocyclic group and the substituted $C_1$-$C_{30}$ heterocyclic group may each independently be substituted with at least one $R_{10a}$. $R_{10a}$ may be understood by referring to the description of $R_1$ provided herein. For example, two adjacent groups $R_1$, $R_2$, $R_{10}$, $R_{20}$, $R_{30}$, $R_{40}$, and $R_{50}$ optionally may together form a fluorene group, a xanthene group, or an acridine group, each unsubstituted or substituted with at least one $R_{10a}$.

The first linking group may be $*-N(R_3)-*'$, $*-B(R_3)-*'$, $*-P(R_3)-*'$, $*-C(R_3)(R_4)-*'$, $*-Si(R_3)(R_4)-*'$, $*-Ge(R_3)(R_4)-*'$, $*-S-*'$, $*-Se-*'$, $*-O-*'$, $*-C(=O)-*'$, $*-S(=O)-*'$, $*-S(=O)_2-*'$, $*-C(R_3)=*'$, $*=C(R_3)-*'$, $*-C(R_3)=C(R_4)-*'$, $*-C(=S)-*'$, or $*-C\equiv C-*'$, $R_3$ and $R_4$ may each be understood by referring to the description of $R_1$ provided herein, and * and *' may each indicate a binding site to an adjacent atom.

In Formula 1, b1 may be an integer from 1 to 5, and when b1 is 2 or greater, at least two $R_1$ groups may be identical to or different from each other. In some embodiments, b1 may be 1, 2, or 3.

In Formula 1, b10, b20, b30, and b50 may each independently be an integer from 1 to 10, b40 may be an integer from 1 to 3, and when b10 is 2 or greater, at least two $R_{10}$ groups may be identical to or different from each other, when b20 is 2 or greater, at least two $R_{20}$ groups may be identical to or different from each other, when b30 is 2 or greater, at least two $R_{30}$ groups may be identical to or different from each other, when b40 is 2 or greater, at least two $R_{40}$ groups may be identical to or different from each other, when b50 is 2 or greater, at least two $R_{50}$ groups may be identical to or different from each other.

In some embodiments, $R_1$, $R_2$, $R_{10}$, $R_{20}$, $R_{30}$, $R_{40}$, and $R_{50}$ may each independently be:

hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, $-SF_5$, a $C_1$-$C_{20}$ alkyl group, or a $C_1$-$C_{20}$ alkoxy group;

a $C_1$-$C_{20}$ alkyl group or a $C_1$-$C_{20}$ alkoxy group, each substituted with at least one of deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C1-$C_{10}$ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, or a pyrimidinyl group;

a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, or an imidazopyrimidinyl group;

a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl

group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, or an imidazopyrimidinyl group, each substituted with at least one of deuterium, -F, -Cl, -Br, -I, $-CD_3$, $-CD_2H$, $-CDH_2$, $-CF_3$, $-CF_2H$, - $CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{20}$ alkyl group, a $C_1$-$C_{20}$ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, or an imidazopyrimidinyl group; or

$-N(Q_1)(Q_2)$, $-Si(Q_3)(Q_4)(Q_5)$, $-Ge(Q_3)(Q_4)(Q_5)$, $-B(Q_6)(Q_7)$, $-P(Q_8)(Q_9)$, or $-P(=O)(Q_8)(Q_9)$,

wherein $Q_1$ to $Q_9$ may each independently be:

$-CH_3$, $-CD_3$, $-CD_2H$, $-CDH_2$, $-CH_2CH_3$, $-CH_2CD_3$, $-CH_2CD_2H$, $-CH_2CDH_2$, - $CHDCH_3$, $-CHDCD_2H$, $-CHDCDH_2$, $-CHDCD_3$, $-CD_2CD_3$, $-CD_2CD_2H$, or $-CD_2CDH_2$;

an n-propyl group, an iso-propyl group, an n-butyl group, an iso-butyl group, a sec-butyl group, a tert-butyl group, an n-pentyl group, an iso-pentyl group, a sec-pentyl group, a tert-pentyl group, a phenyl group, or a naphthyl group; or

an n-propyl group, an iso-propyl group, an n-butyl group, an isobutyl group, a sec-butyl group, a tert-butyl group, an n-pentyl group, an isopentyl group, a sec-pentyl group, a tert-pentyl group, a phenyl group, or a naphthyl group, each substituted with at least one of deuterium, a $C_1$-$C_{10}$ alkyl group, or a phenyl group.

In some embodiments, $R_1$ and $R_2$ may each independently be:

a $C_1$-$C_{30}$ alkyl group;

a $C_1$-$C_{30}$ alkyl group substituted with at least one of deuterium, -F, -Cl, -Br, -I, - $CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{10}$ alkyl group, a $C_1$-$C_{10}$ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, or a pyrimidinyl group;

a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzoimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, or an imidazopyrimidinyl group; or

a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl

group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, or an imidazopyrimidinyl group, each substituted with at least one of deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{10}$ alkyl group, a $C_1$-$C_{10}$ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, or a pyrimidinyl group.

In some embodiments, $R_1$, $R_2$, $R_{10}$, $R_{20}$, $R_{30}$, $R_{40}$, and $R_{50}$ may each independently be:

hydrogen, deuterium, -F, a cyano group, a nitro group, $-SF_5$, a methyl group, an ethyl group, an n-propyl group, an iso-propyl group, an n-butyl group, an isobutyl group, a sec-butyl group, a tert-butyl group, an n-pentyl group, an isopentyl group, a sec-pentyl group, a tert-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, a tert-decyl group, a methoxy group, an ethoxy group, a propoxy group, a butoxy group, a pentoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, a pyrimidinyl group, a carbazolyl group, a dibenzofuranyl group, or a dibenzothiophenyl group;

a methyl group, an ethyl group, an n-propyl group, an iso-propyl group, an n-butyl group, an iso-butyl group, a sec-butyl group, a tert-butyl group, an n-pentyl group, an iso-pentyl group, a sec-pentyl group, a tert-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an iso-decyl group, a sec-decyl group, a tert-decyl group, a methoxy group, an ethoxy group, a propoxy group, a butoxy group, a pentoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, a pyrimidinyl group, a carbazolyl group, a dibenzofuranyl group, or a dibenzothiophenyl group, each substituted with at least one of deuterium, -F, $-CD_3$, $-CD_2H$, $-CDH_2$, $-CF_3$, $-CF_2H$, $-CFH_2$, a cyano group, a nitro group, a $C_1$-$C_{10}$ alkyl group, a $C_1$-$C_{10}$ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, a pyrimidinyl group, a carbazolyl group, a dibenzofuranyl group, or a dibenzothiophenyl group; or

$-N(Q_1)(Q_2)$, $-Si(Q_3)(Q_4)(Q_5)$, $-Ge(Q_3)(Q_4)(Q_5)$, $-B(Q_6)(Q_7)$, $-P(Q_8)(Q_9)$, or $-P(=O)(Q_8)(Q_9)$,

wherein $Q_1$ to $Q_9$ may each independently be:

$-CH_3$, $-CD_3$, $-CD_2H$, $-CDH_2$, $-CH_2CH_3$, $-CH_2CD_3$, $-CH_2CD_2H$, $-CH_2CDH_2$, $-CHDCH_3$, $-CHDCD_2H$, $-CHDCDH_2$, $-CHDCD_3$, $-CD_2CD_3$, $-CD_2CD_2H$, or $-CD_2CDH_2$;

an n-propyl group, an iso-propyl group, an n-butyl group, an iso-butyl group, a sec-butyl group, a tert-butyl group, an n-pentyl group, an iso-pentyl group, a sec-pentyl group, a tert-pentyl group, a phenyl group, or a naphthyl group; or

an n-propyl group, an iso-propyl group, an n-butyl group, an isobutyl group, a sec-butyl group, a tert-butyl group, an n-pentyl group, an isopentyl group, a sec-pentyl group, a tert-pentyl group, a phenyl group, or a naphthyl group, each substituted with at least one of deuterium, a $C_1$-$C_{10}$ alkyl group, or a phenyl group.

In some embodiments, $R_1$, $R_2$, $R_{10}$, $R_{20}$, $R_{30}$, $R_{40}$, and $R_{50}$ may each independently be: hydrogen, deuterium, -F, a cyano group, a nitro group, $-SF_5$, $-CH_3$, $-CD_3$, $-CD_2H$, $-CDH_2$, $-CF_3$, $-CF_2H$, $-CFH_2$, a group represented by any one of Formulae 9-1 to 9-19, or a group represented by any one of Formulae 10-1 to 10-194:

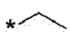
9-1

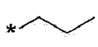
9-2

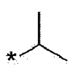
9-3

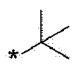
9-4

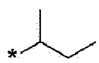
9-5

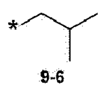
9-6

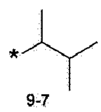
9-7

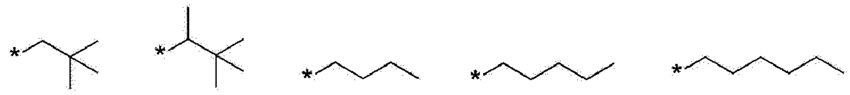

9-8 9-9 9-10 9-11 9-12

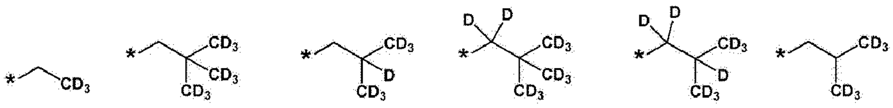

9-13 9-14 9-15 9-16 9-17 9-18

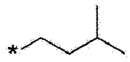

9-19

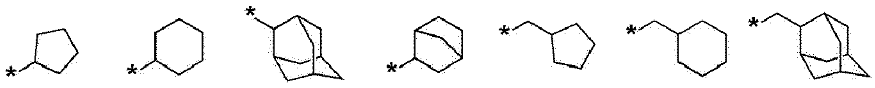

10-1 10-2 10-3 10-4 10-5 10-6 10-7

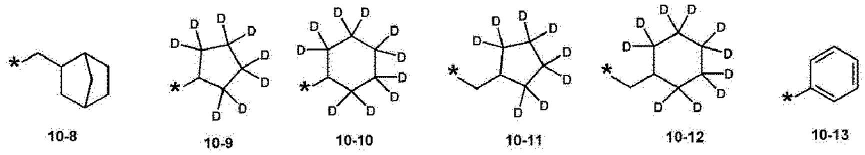

10-8 10-9 10-10 10-11 10-12 10-13

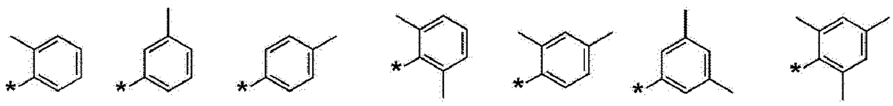

10-14 10-15 10-16 10-17 10-18 10-19 10-20

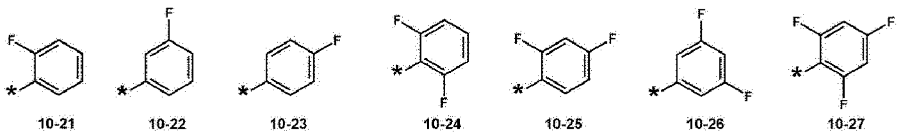

10-21 10-22 10-23 10-24 10-25 10-26 10-27

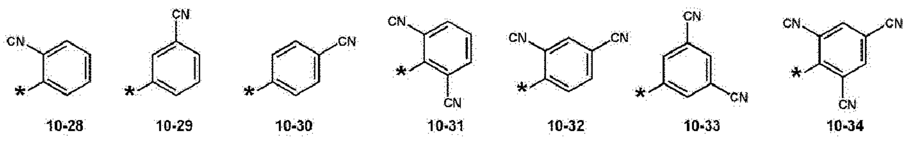

10-28 10-29 10-30 10-31 10-32 10-33 10-34

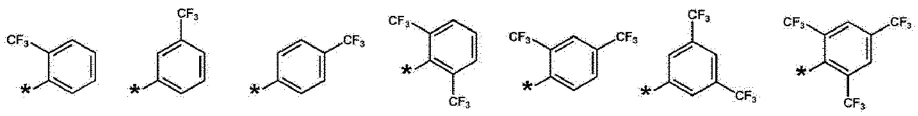

10-35 10-36 10-37 10-38 10-39 10-40 10-41

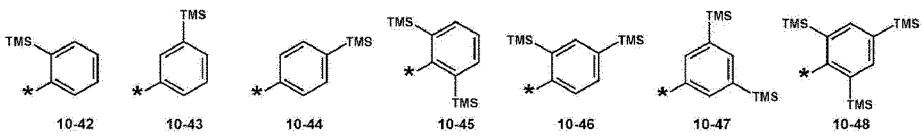

10-42 10-43 10-44 10-45 10-46 10-47 10-48

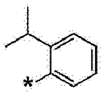 10-49 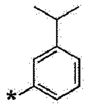 10-50 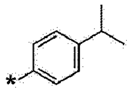 10-51 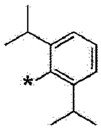 10-52 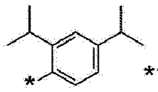 10-53 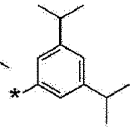 10-54 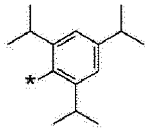 10-55

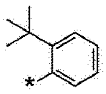 10-56 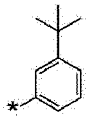 10-57 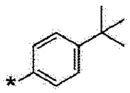 10-58 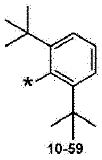 10-59 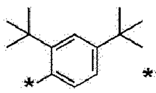 10-60 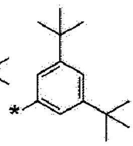 10-61 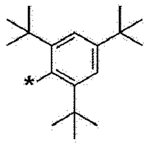 10-62

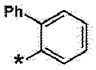 10-63 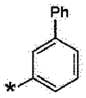 10-64 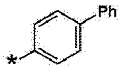 10-65 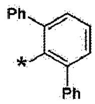 10-66 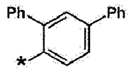 10-67 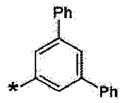 10-68 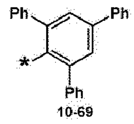 10-69

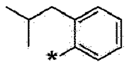 10-70 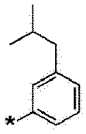 10-71 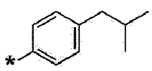 10-72 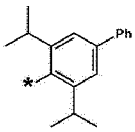 10-73 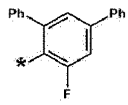 10-74 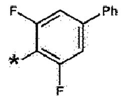 10-75

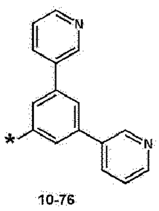 10-76 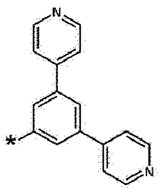 10-77 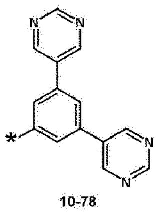 10-78 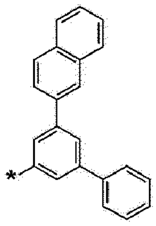 10-79 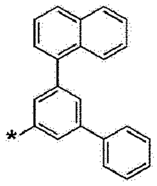 10-80

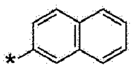 10-81 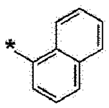 10-82 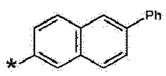 10-83 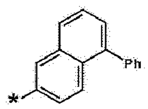 10-84 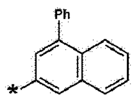 10-85

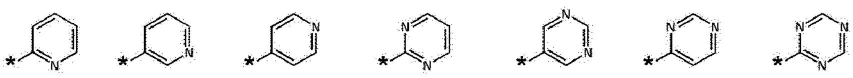

10-86 10-87 10-88 10-89 10-90 10-91 10-92

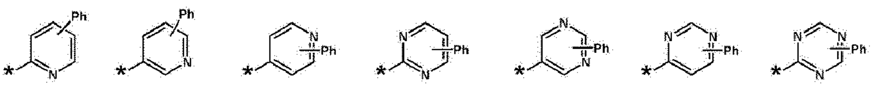

10-93 10-94 10-95 10-96 10-97 10-98 10-99

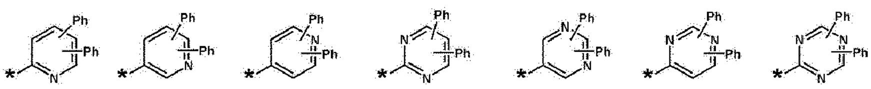

10-100 10-101 10-102 10-103 10-104 10-105 10-106

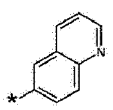 10-107 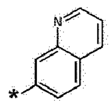 10-108 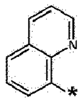 10-109 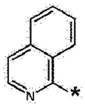 10-110 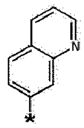 10-111 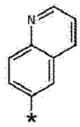 10-112

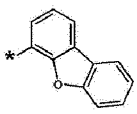 10-113 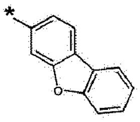 10-114 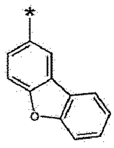 10-115 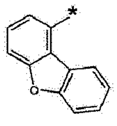 10-116 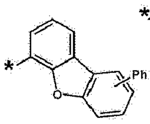 10-117 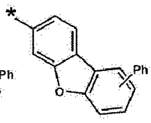 10-118

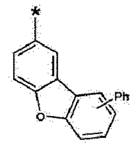 10-119 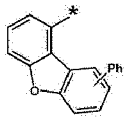 10-120 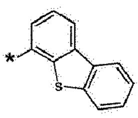 10-121 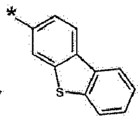 10-122 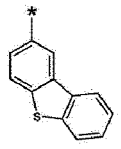 10-123 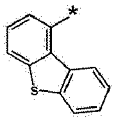 10-124

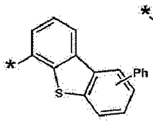 10-125 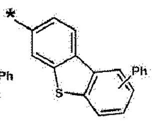 10-126 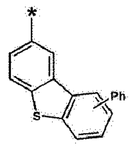 10-127 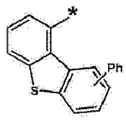 10-128 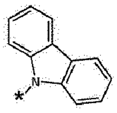 10-129 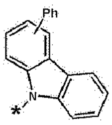 10-130

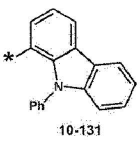 10-131 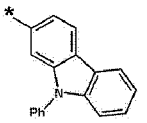 10-132 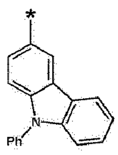 10-133 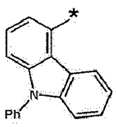 10-134 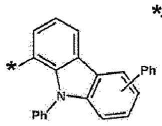 10-135 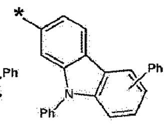 10-136

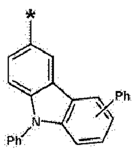 10-137 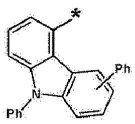 10-138 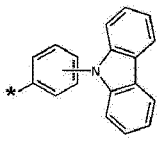 10-139 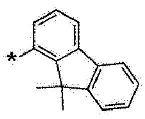 10-140 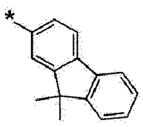 10-141 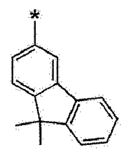 10-142

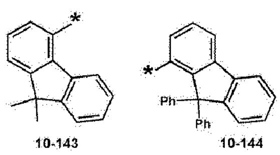 10-143 10-144 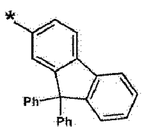 10-145 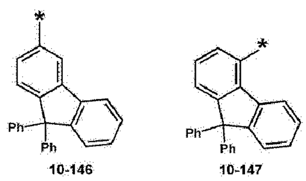 10-146 10-147

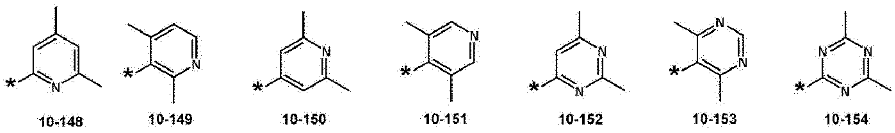 10-148 10-149 10-150 10-151 10-152 10-153 10-154

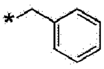 10-155 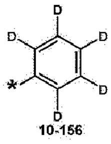 10-156 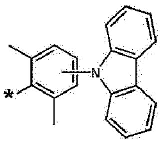 10-157 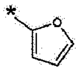 10-158 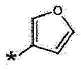 10-159 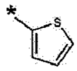 10-160 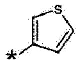 10-161

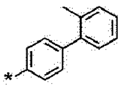 10-162 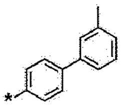 10-163 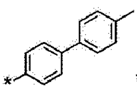 10-164 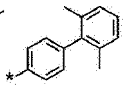 10-165 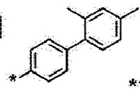 10-166 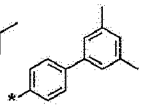 10-167 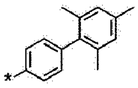 10-168

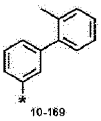 10-169 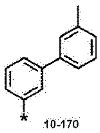 10-170 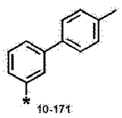 10-171 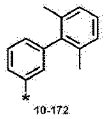 10-172 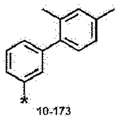 10-173 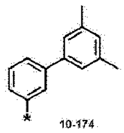 10-174 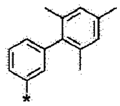 10-175

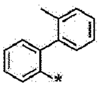 10-176 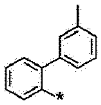 10-177 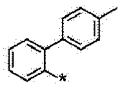 10-178 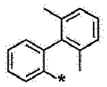 10-179 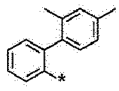 10-180 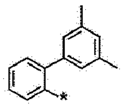 10-181 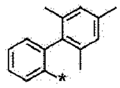 10-182

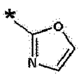 10-183 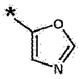 10-184 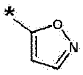 10-185 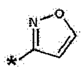 10-186 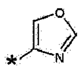 10-187 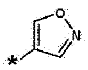 10-188 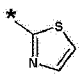 10-189 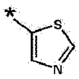 10-190 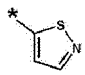 10-191

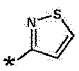 10-192 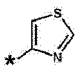 10-193 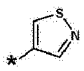 10-194

wherein, in Formulae 9-1 to 9-19 and 10-1 to 10-194, * indicates a binding site to an adjacent atom, "Ph" indicates a phenyl group, and "TMS" indicates a trimethylsilyl group.

In some embodiments, at least one $R_{10}$ may be any one of Formulae 9-1 to 9-19.

In some embodiments, at least one $R_{40}$ may be any one of Formulae 9-1 to 9-19 or Formulae 10-1 to 10-194.

In some embodiments, at least one of $R_1$, $R_2$, $R_{10}$, $R_{20}$, $R_{30}$, $R_{40}$, or $R_{50}$ may be:

a $C_1$-$C_{30}$ alkyl group; or

a $C_1$-$C_{30}$ alkyl group substituted with at least one of deuterium, -F, -Cl, -Br, -I, - $CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{10}$ alkyl group, a $C_1$-$C_{10}$ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, or a pyrimidinyl group;

a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, or an imidazopyrimidinyl group; or

a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, or an imidazopyrimidinyl group, each substituted with at least one of deuterium, -F, -Cl, -Br, -I, $-CD_3$, $-CD_2H$, $-CDH_2$, $-CF_3$, $-CF_2H$, $- CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{20}$ alkyl group, a $C_1$-$C_{20}$ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, or an imidazopyrimidinyl group.

In Formula 1, b1, b10, b20, b30, b40, and b50 may each respectively indicate the number of $R_1$ groups, $R_{10}$ groups, $R_{20}$ groups, $R_{30}$ groups, $R_{40}$ groups, and $R_{50}$ groups, wherein b1 may be an integer from 1 to 5, when b1 is 2 or greater, at least two $R_1$(s) may be identical to or different from each other, b10, b20, b30, and b50 are each independently an integer from 1 to 10, b40 is an integer from 1 to 3, when b10 is 2 or greater, at least two $R_{10}$ groups may be identical to or different from each other, when b20 is 2 or greater, at least two $R_{20}$ groups may be identical to or different from each other, when b30 is 2 or greater, at least two $R_{30}$ groups may be identical to or different from each other, when b40 is 2 or greater, at least two $R_{40}$ groups are identical to or different from each other, when b50 is 2 or greater, at least two $R_{50}$ groups may be identical to or different from each other.

In some embodiments, b10 and b40 may each independently be an integer from 1 to 4, and b20 and b30 may each independently be an integer from 1 to 3.

In some embodiments, at least two selected from $R_1$, $R_2$, $R_{10}$, $R_{20}$, $R_{30}$, $R_{40}$, and $R_{50}$ may optionally be bound together to form a substituted or unsubstituted $C_5$-$C_{30}$ carbocyclic group or a substituted or unsubstituted $C_1$-$C_{30}$ heterocyclic group.

In some embodiments, in Formula 1, when b10 is 2 or greater, at least two $R_{10}$ groups may optionally be bound together to form a cyclopentane group, a cyclopentadiene group, a furan group, a thiophene group, a pyrrole group, a silole group, an adamantane group, a norbornane group, a norbornene group, a cyclohexane group, a cyclohexene group, a benzene group, a naphthalene group, an indene group, an indole group, a benzofuran group, a benzothiophene group, a benzosilole group, a fluorene group, a carbazole group, a dibenzofuran group, a dibenzothiophene group, or a dibenzosilole group, each unsubstituted or substituted with at least one $R_{10a}$.

In some embodiments, in Formula 1, when b20 is 2 or greater, at least two $R_{20}$ groups may optionally be bound together to form a cyclopentane group, a cyclopentadiene group, a furan group, a thiophene group, a pyrrole group, a silole group, an adamantane group, a norbornane group, a norbornene group, a cyclohexane group, a cyclohexene group, a benzene group, a naphthalene group, an indene group, an indole group, a benzofuran group, a benzothiophene group, a benzosilole group, a fluorene group, a carbazole group, a dibenzofuran group, a dibenzothiophene group, or a dibenzosilole group, each unsubstituted or substituted with at least one $R_{10a}$.

In some embodiments, in Formula 1, when b30 is 2 or greater, at least two $R_{30}$ groups may optionally be bound together to form a cyclopentane group, a cyclopentadiene group, a furan group, a thiophene group, a pyrrole group, a silole group, an adamantane group, a norbornane group, a norbornene group, a cyclohexane group, a cyclohexene group, a benzene group, a naphthalene group, an indene group, an indole group, a benzofuran group, a benzothiophene

group, a benzosilole group, a fluorene group, a carbazole group, a dibenzofuran group, a dibenzothiophene group, or a dibenzosilole group, each unsubstituted or substituted with at least one $R_{10a}$.

In some embodiments, in Formula 1, when b40 is 2 or greater, at least two $R_{40}$ groups may optionally be bound together to form a cyclopentane group, a cyclopentadiene group, a furan group, a thiophene group, a pyrrole group, a silole group, an adamantane group, a norbornane group, a norbornene group, a cyclohexane group, a cyclohexene group, a benzene group, a naphthalene group, an indene group, an indole group, a benzofuran group, a benzothiophene group, a benzosilole group, a fluorene group, a carbazole group, a dibenzofuran group, a dibenzothiophene group, or a dibenzosilole group, each unsubstituted or substituted with at least one $R_{10a}$.

In some embodiments, in Formula 1, when b50 is 2 or greater, at least two $R_{50}$ groups may optionally be bound together to form a cyclopentane group, a cyclopentadiene group, a furan group, a thiophene group, a pyrrole group, a silole group, an adamantane group, a norbornane group, a norbornene group, a cyclohexane group, a cyclohexene group, a benzene group, a naphthalene group, an indene group, an indole group, a benzofuran group, a benzothiophene group, a benzosilole group, a fluorene group, a carbazole group, a dibenzofuran group, a dibenzothiophene group, or a dibenzosilole group, each unsubstituted or substituted with at least one $R_{10a}$.

In some embodiments, in Formula 1, $R_1$ and $R_2$ may optionally be bound together to form a cyclopentane group, a cyclopentadiene group, a furan group, a thiophene group, a pyrrole group, a silole group, an adamantane group, a norbornane group, a norbornene group, a cyclohexane group, a cyclohexene group, a benzene group, a naphthalene group, an indene group, an indole group, a benzofuran group, a benzothiophene group, a benzosilole group, a fluorene group, a carbazole group, a dibenzofuran group, a dibenzothiophene group, or a dibenzosilole group, each unsubstituted or substituted with at least one $R_{10a}$.

In some embodiments, in Formula 1, any one of $R_1$ or $R_2$ and any one of $R_{10}$, $R_{20}$, $R_{30}$, $R_{40}$, or $R_{50}$ may optionally be bound together to form a cyclopentane group, a cyclopentadiene group, a furan group, a thiophene group, a pyrrole group, a silole group, an adamantane group, a norbornane group, a norbornene group, a cyclohexane group, a cyclohexene group, a benzene group, a naphthalene group, an indene group, an indole group, a benzofuran group, a benzothiophene group, a benzosilole group, a fluorene group, a carbazole group, a dibenzofuran group, a dibenzothiophene group, or a dibenzosilole group, each unsubstituted or substituted with at least one $R_{10a}$.

In some embodiments, the organometallic compound represented by Formula 1 may be represented by any one of Formulae 11-1 to 11-6:

Formula 11-1

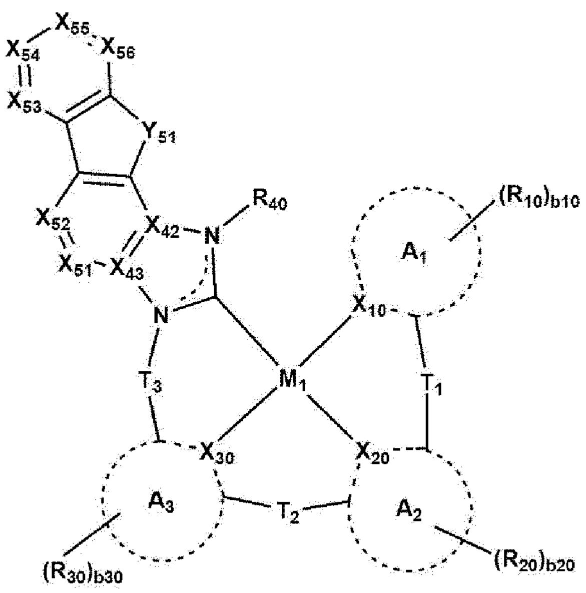

Formula 11-2

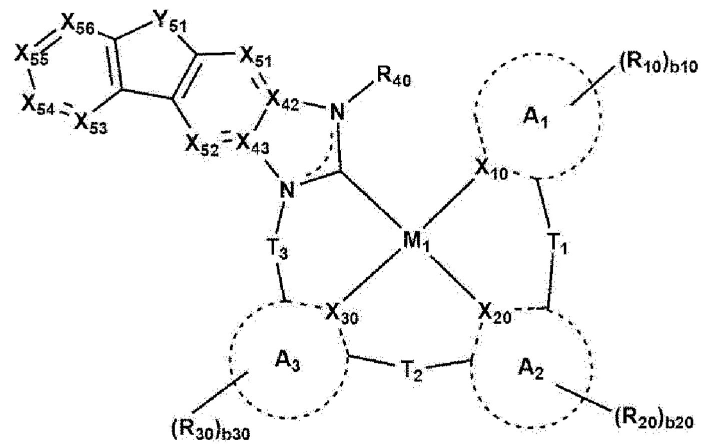

Formula 11-3

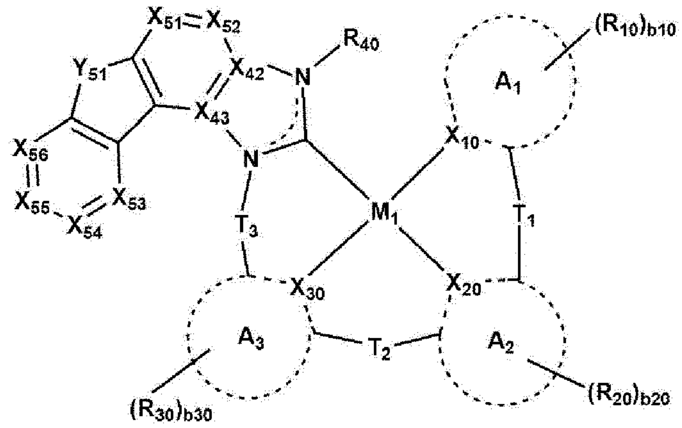

Formula 11-4

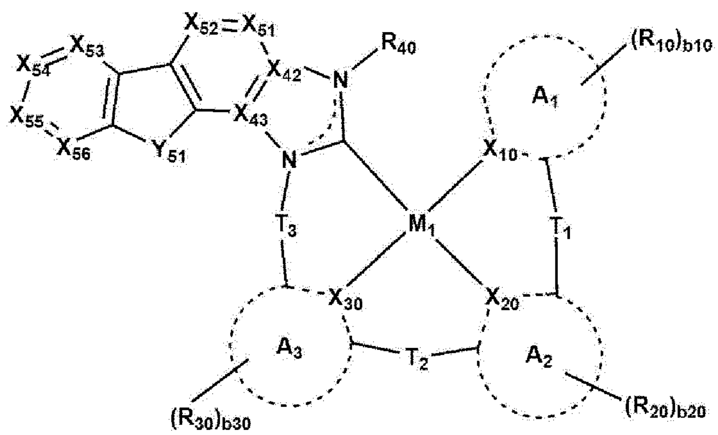

Formula 11-5

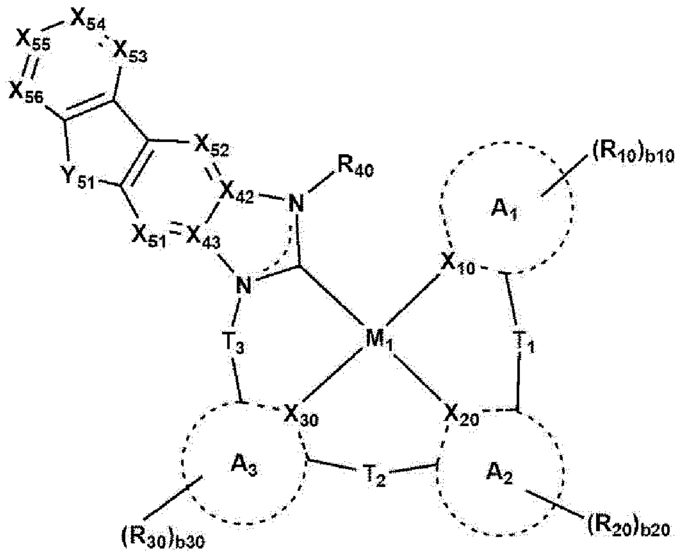

Formula 11-6

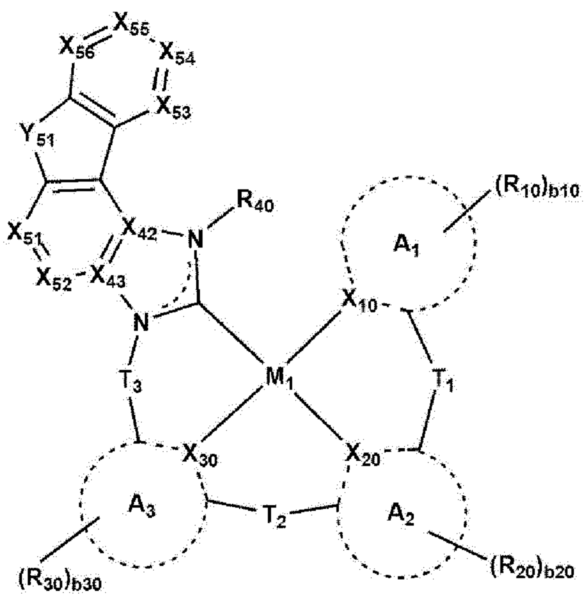

wherein, in Formulae 11-1 to 11-6,

$M_1$, $A_1$ to $A_3$, $X_{10}$, $X_{20}$, $X_{30}$, $X_{42}$, $X_{43}$, $T_1$ to $T_3$, $R_{10}$, $R_{20}$, $R_{30}$, $R_{40}$, $R_{50}$, b10, b20, and b30 may respectively be understood by referring to the descriptions of $M_1$, $A_1$ to $A_3$, $X_{10}$, $X_{20}$, $X_{30}$, $X_{42}$, $X_{43}$, $T_1$ to $T_3$, $R_{10}$, $R_{20}$, $R_{30}$, $R_{40}$, $R_{50}$, b10, b20, and b30 provided herein,

$Y_{51}$ may be *-O-*',*-S-*', *-N($R_5$)-*', *-C($R_5$)($R_6$)-*', *-Si($R_5$)($R_6$)-*', *-B($R_5$)-*', *-P($R_5$)-*', or *-P(=O)($R_5$)-*', wherein * and *' may each indicate a binding site to an adjacent atom,

$R_5$ and $R_6$ may respectively be understood by referring to the descriptions of $R_1$ and $R_2$ provided herein,

$X_{51}$ may be C($R_{51}$) or N, $X_{52}$ may be C($R_{52}$) or N, $X_{53}$ may be C($R_{53}$) or N, $X_{54}$ may be C($R_{54}$) or N, $X_{55}$ may be C($R_{55}$) or N, $X_{56}$ may be C($R_{56}$) or N, and

$R_{51}$ to $R_{56}$ may each independently be understood by referring to the description of $R_{50}$ provided herein.

In some embodiments, the organometallic compound represented by Formula 1 may be represented by any one of Formulae 12-1 to 12-6:

Formula 12-1

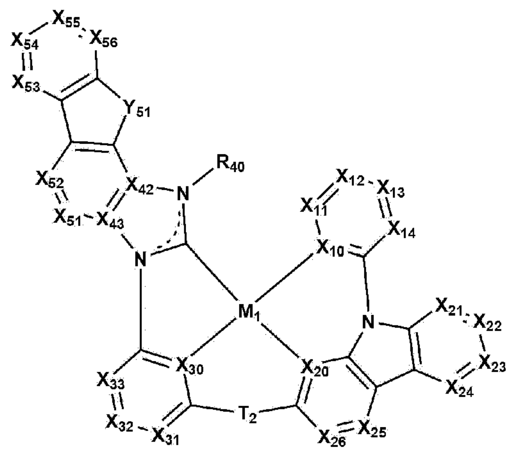

Formula 12-2

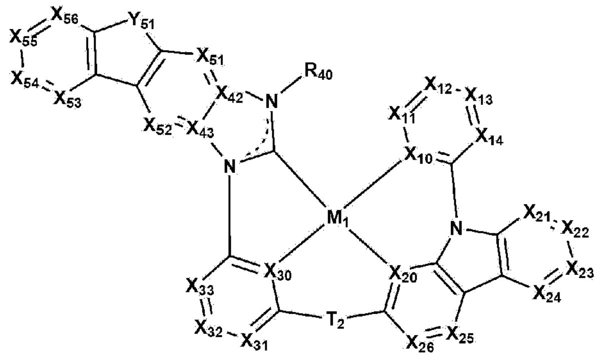

Formula 12-3

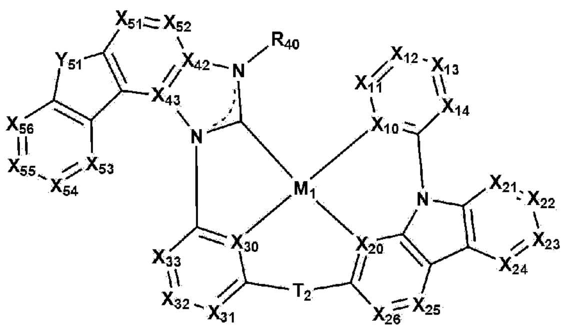

Formula 12-4

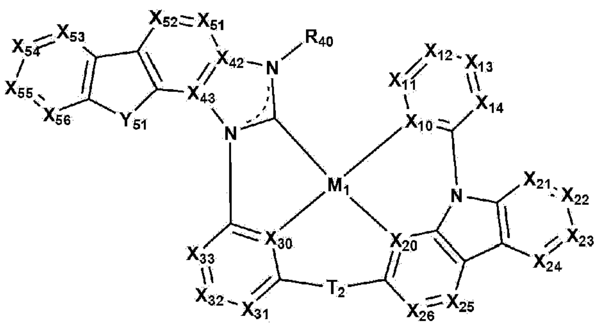

Formula 12-5

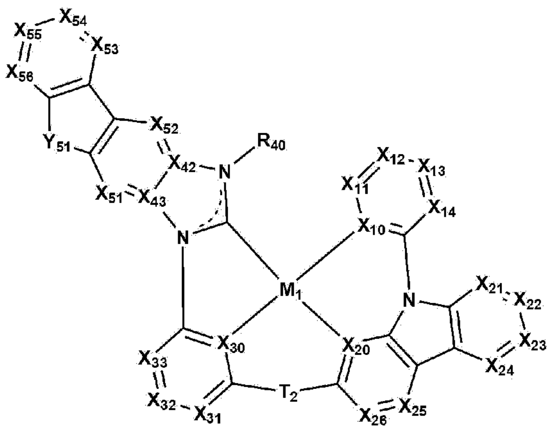

Formula 12-6

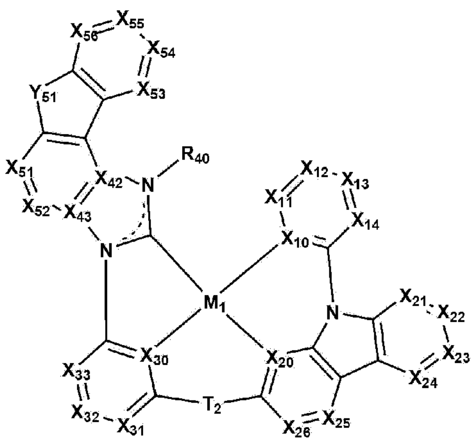

wherein, in Formulae 12-1 to 12-6,

$M_1$, $X_{10}$, $X_{20}$, $X_{30}$, $X_{42}$, $X_{43}$, $R_{40}$, and $T_2$ may respectively be understood by referring to the descriptions of $M_1$, $X_{10}$, $X_{20}$, $X_{30}$, $X_{42}$, $X_{43}$, $R_{40}$, and $T_2$ provided herein,

$Y_{51}$ may be *-O-*',*-S-*', *-N($R_5$)-*', *-C($R_5$)($R_6$)-*', *-Si($R_5$)($R_6$)-*', *-B($R_5$)-*', *-P($R_5$)-*', or *-P(=O)($R_5$)-*', wherein * and *' may each indicate a binding site to an adjacent atom,

$R_5$ and $R_6$ may respectively be understood by referring to the descriptions of $R_1$ and $R_2$ provided herein,

$X_{11}$ may be C($R_{11}$) or N, $X_{12}$ may be C($R_{12}$) or N, $X_{13}$ may be C($R_{13}$) or N, $X_{14}$ may be C($R_{14}$) or N,

$X_{21}$ may be C($R_{21}$) or N, $X_{22}$ may be C($R_{22}$) or N, $X_{23}$ may be C($R_{23}$) or N, $X_{24}$ may be C($R_{24}$) or N, $X_{25}$ may be C($R_{25}$) or N, $X_{26}$ may be C($R_{26}$) or N, and

$X_{31}$ may be $C(R_{31})$ or N, $X_{32}$ may be $C(R_{32})$ or N, $X_{33}$ may be $C(R_{33})$ or N,

$X_{51}$ may be $C(R_{51})$ or N, $X_{52}$ may be $C(R_{52})$ or N, $X_{53}$ may be $C(R_{53})$ or N, $X_{54}$ may be $C(R_{54})$ or N, $X_{55}$ may be $C(R_{55})$ or N, $X_{56}$ may be $C(R_{56})$ or N,

$R_{11}$ to $R_{14}$ may each independently be understood by referring to the description of $R_{10}$ provided herein,

$R_{21}$ to $R_{26}$ may each independently be understood by referring to the description of $R_{20}$ provided herein,

$R_{31}$ to $R_{33}$ may each independently be understood by referring to the description of $R_{30}$ provided herein, and

$R_{51}$ to $R_{56}$ may each independently be understood by referring to the description of $R_{50}$ provided herein.

In one or more embodiments, the organometallic compound may be any one of Compounds 1 to 72, but embodiments are not limited thereto:

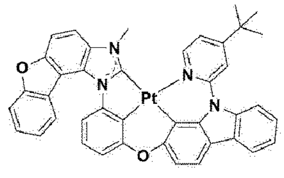

1

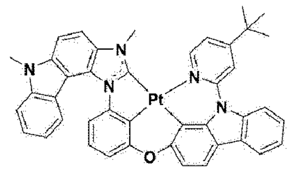

2

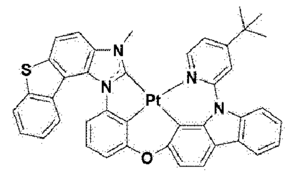

3

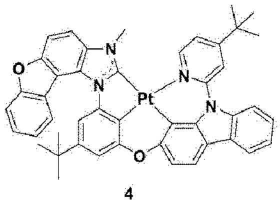

4

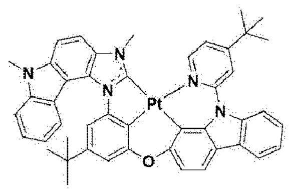

5

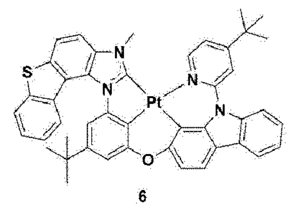

6

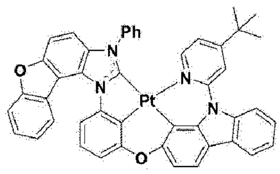

7

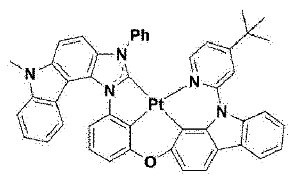

8

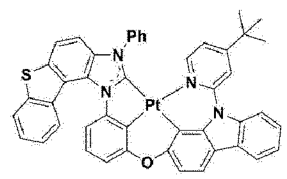

9

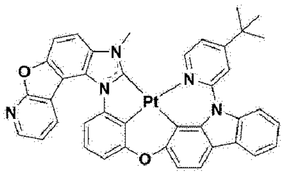

10

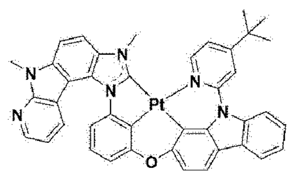

11

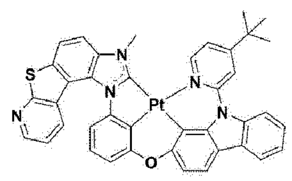

12

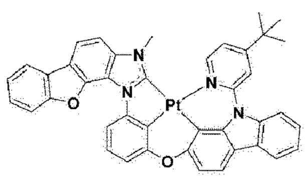

13

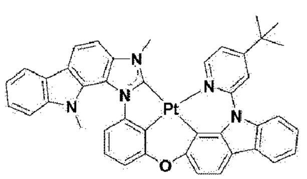

14

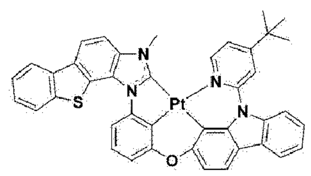

15

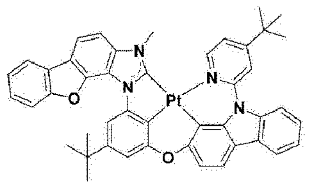
16

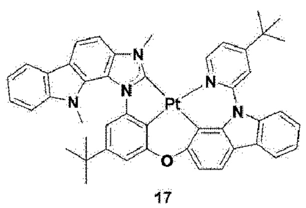
17

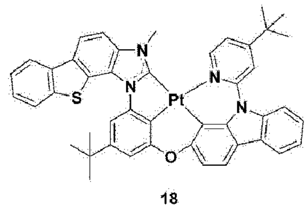
18

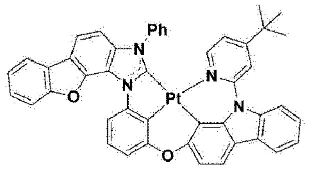
19

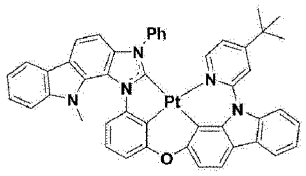
20

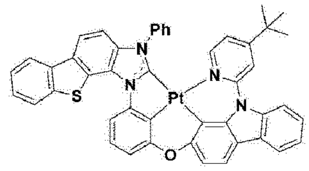
21

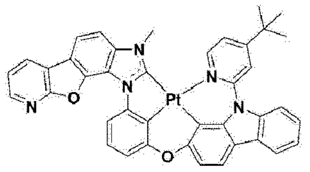
22

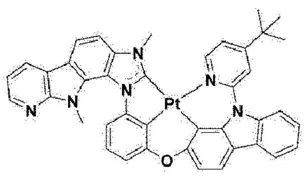
23

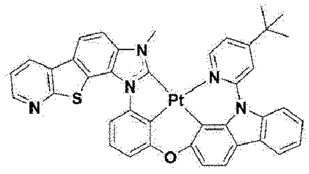
24

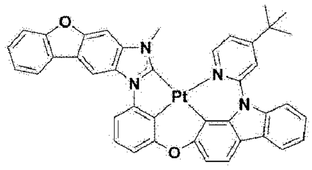
25

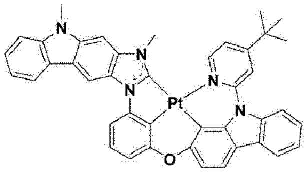
26

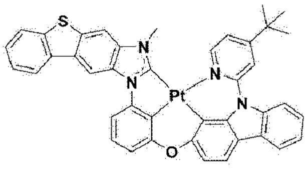
27

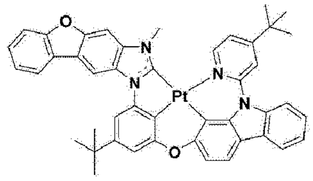
28

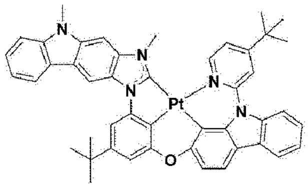
29

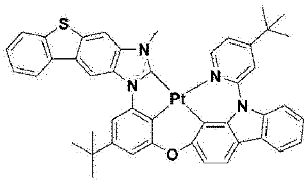
30

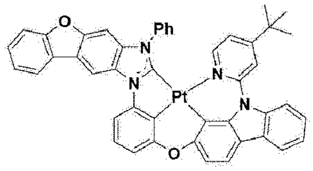
31

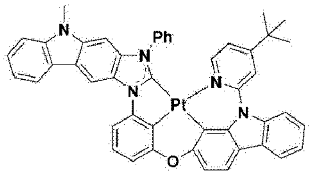
32

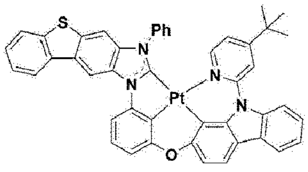
33

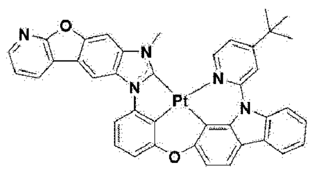
34

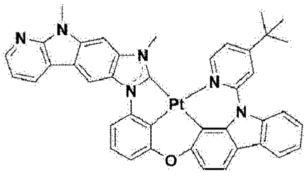
35

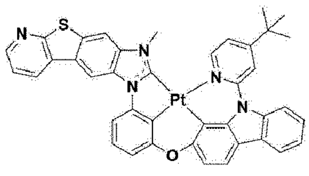
36

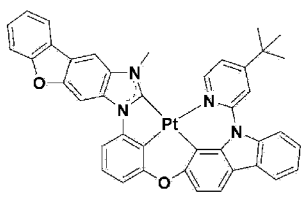

37

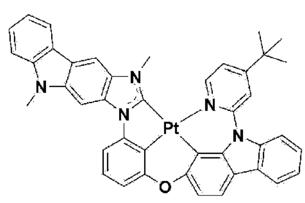

38

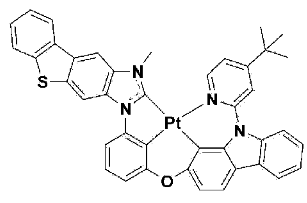

39

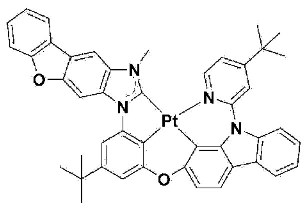

40

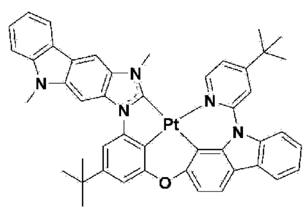

41

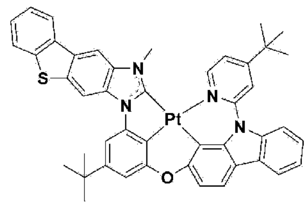

42

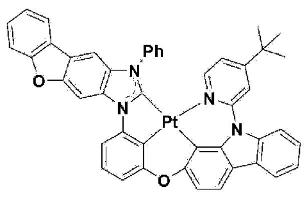

43

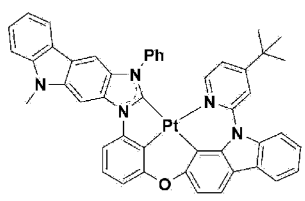

44

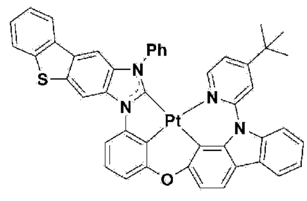

45

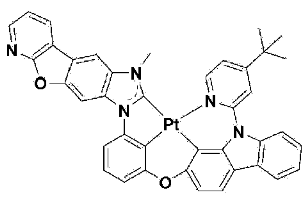

46

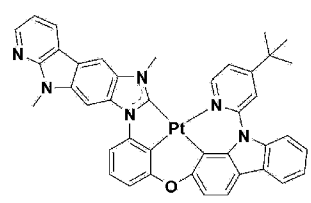

47

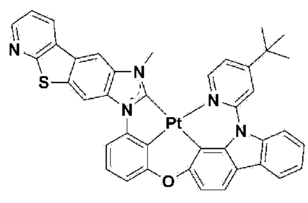

48

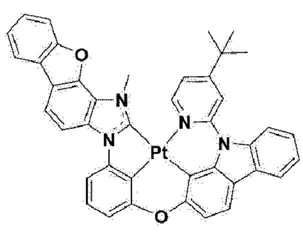

49

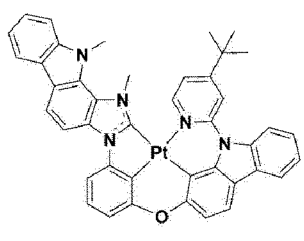

50

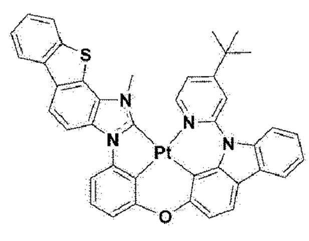

51

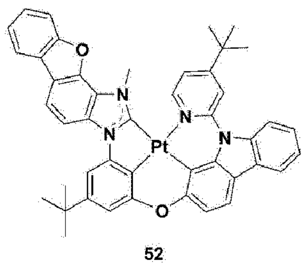

52

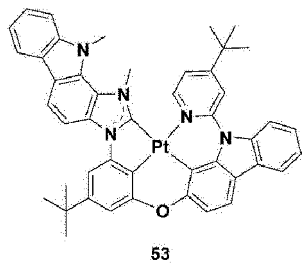

53

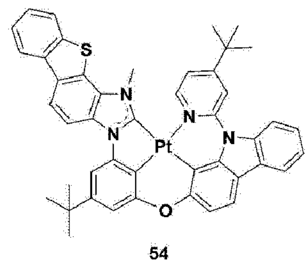

54

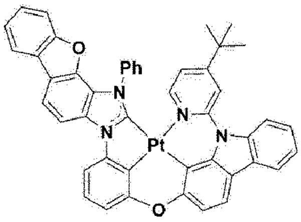

55

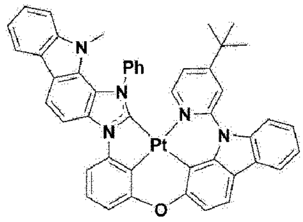

56

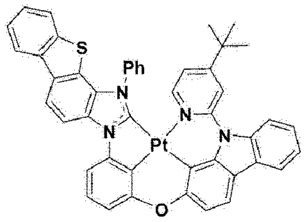

57

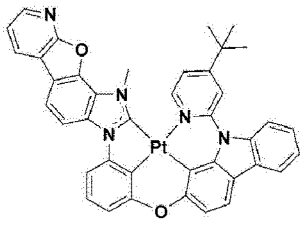

58

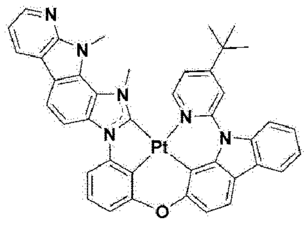

59

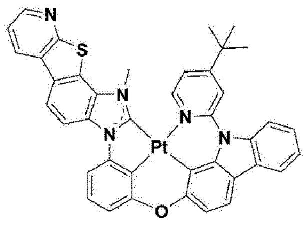

60

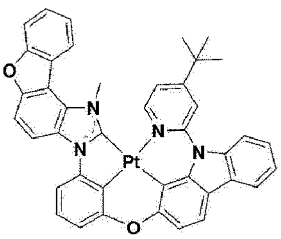

61

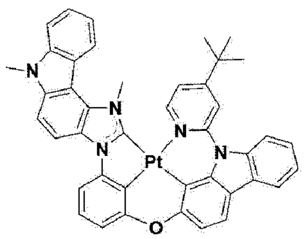

62

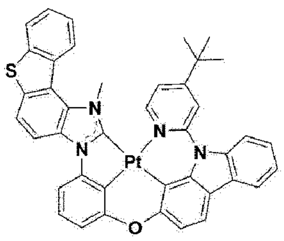

63

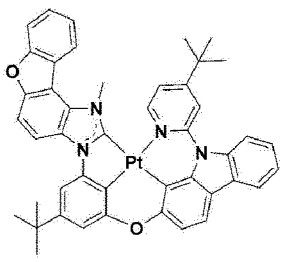

64

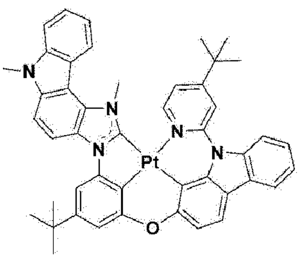

65

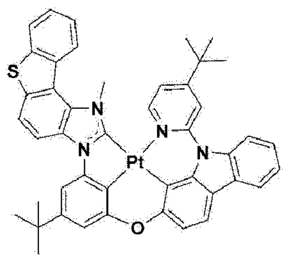

66

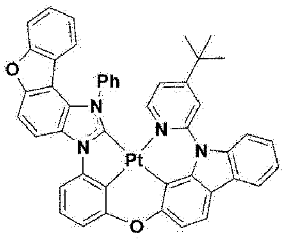

67

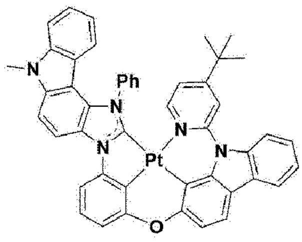

68

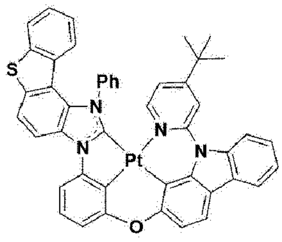

69

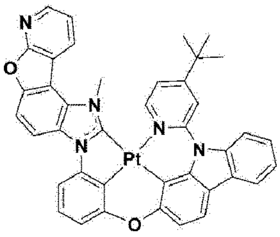

70

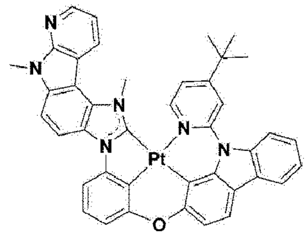

71

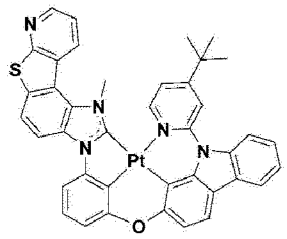

72

The organometallic compound represented by Formula 1 may satisfy the structure of Formula 1, and due to a structure in which $A_5$, i.e., at least two rings that essentially include a 6-membered ring, is condensed to $A_4$, i.e., a 5-membered heterocyclic group, the organometallic compound is suitable for deep blue light emission. Thus, while not wishing to be bound by theory, an electronic device, e.g., an organic light-emitting device, including the organometallic compound represented by Formula 1 may have excellent luminescent efficiency, excellent color-coordinate, and a low driving voltage.

For example, the highest occupied molecular orbital (HOMO), lowest unoccupied molecular orbital (LUMO), singlet ($S_1$), and triplet ($T_1$) energy levels of the organometallic Compounds 1 to 12, 49, A, and B were evaluated by using a Gaussian program according to a density functional theory (DFT) method (the molecular structure optimization was performed at a degree of B3LYP, and 6-31G(d,p)). The results thereof are shown in Table 1, where the values are reported as electron volts (eV).

Table 1

| Compound No. | HOMO (eV) | LUMO (eV) | $T_1$ (eV) | $S_1$ (eV) |
|---|---|---|---|---|
| Compound 1 | -4.72 | -1.37 | 2.66 | 2.81 |
| Compound 2 | -4.59 | -1.24 | 2.67 | 2.80 |
| Compound 3 | -4.71 | -1.42 | 2.64 | 2.77 |
| Compound 4 | -4.69 | -1.37 | 2.65 | 2.79 |
| Compound 5 | -4.57 | -1.23 | 2.65 | 2.79 |
| Compound 6 | -4.69 | -1.42 | 2.62 | 2.75 |
| Compound 7 | -4.69 | -1.34 | 2.65 | 2.83 |
| Compound 8 | -4.58 | -1.16 | 2.66 | 2.86 |
| Compound 9 | -4.69 | -1.40 | 2.62 | 2.76 |
| Compound 10 | -4.80 | -1.59 | 2.64 | 2.74 |
| Compound 11 | -4.68 | -1.37 | 2.66 | 2.79 |
| Compound 12 | -4.80 | -1.61 | 2.62 | 2.70 |
| Compound 49 | -4.69 | -1.31 | 2.66 | 2.83 |
| Compound A | -4.75 | -1.43 | 2.60 | 2.76 |
| Compound B | -4.84 | -1.42 | 2.60 | 2.84 |

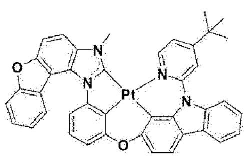

1

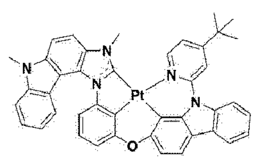

2

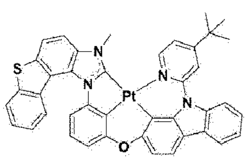

3

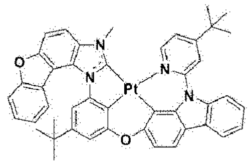

4

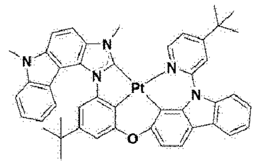

5

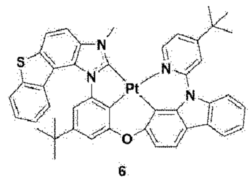

6

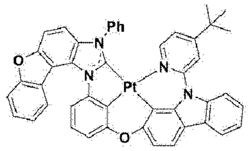

7

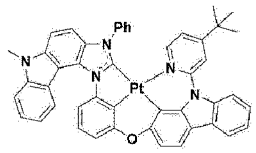

8

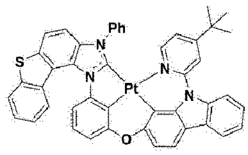

9

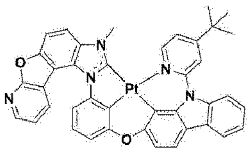

10

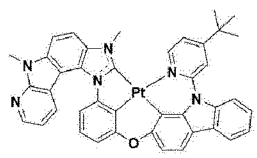

11

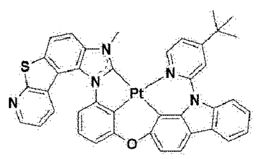

12

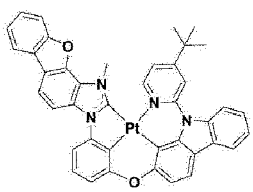

49

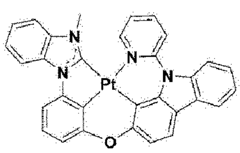

A

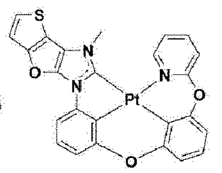

B

Referring to the results of Table 1, due to the high $T_1$ energy level, the organometallic compound represented by Formula 1 was found to have suitable electrical characteristics for use as an emission layer material in an electronic device, e.g., an organic light-emitting device.

A method of synthesizing the organometallic compound represented by Formula 1 may be apparent to one of ordinary skill in the art by referring to Synthesis Examples provided herein.

The organometallic compound represented by Formula 1 may be suitable for use in an organic layer of an organic light-emitting device, for example, as an emission layer material. Thus, according to another aspect, there is provided an organic light-emitting device that may include a first electrode; a second electrode; and an organic layer disposed between the first electrode and the second electrode, wherein the organic layer includes: an emission layer, and at least one of the organometallic compounds represented by Formula 1.

Since the organic light-emitting device has an organic layer including the organometallic compound represented by Formula 1, the organic light-emitting device may have a low driving voltage, high efficiency, high power efficiency, high quantum efficiency, long lifespan, low roll-off ratio, and excellent color purity.

For example, in the organic light-emitting device, the first electrode may be an anode, the second electrode may be a cathode, and the organic layer may further include a hole transport region disposed between the first electrode and the emission layer and an electron transport region disposed between the emission layer and the second electrode, wherein the hole transport region may include a hole injection layer, a hole transport layer, an electron blocking layer, or a combination thereof, and the electron transport region may include a hole blocking layer, an electron transport layer, an electron injection layer, or a combination thereof.

In some embodiments, the organometallic compound represented by Formula 1 may be included in the emission layer.

In the emission layer, the organometallic compound may serve as an emitter. In some embodiments, an emission layer including the organometallic compound represented by Formula 1 may emit phosphorescence produced upon transition of triplet excitons to a ground state of the organometallic compound.

In some embodiments, an emission layer including the organometallic compound represented by Formula 1 may further include a host. The host may be selected from any suitable hosts, and the host may be understood by

referring to the description of the host provided herein. In some embodiments, a content of a host in the emission layer may be greater than a content of the organometallic compound represented by Formula 1.

In one or more embodiments, the emission layer may include a host and a dopant, the host may be any suitable hosts, and the dopant may include the organometallic compound represented by Formula 1. The emission layer may emit phosphorescence produced upon transition of triplet excitons to a ground state of the organometallic compound that serve as a dopant.

In some embodiments, the emission layer may emit blue light having a maximum emission wavelength in a range of about 430 nanometers (nm) to about 480 nm.

As used herein, a layer (such as an organic layer) including the organometallic compound of Formula 1 refers to a layer that includes at least one of the organometallic compounds of Formula 1. For example, a layer may include two or more different organometallic compounds of Formula 1.

For example, in an exemplary embodiment, Compound 1 in Table 1 may only be included in the organic layer as an organometallic compound. In this embodiment, Compound 1 may be included in the emission layer of the organic light-emitting device.

In some embodiments, Compounds 1 and 2 may be included in the organic layer as organometallic compounds. In this embodiment, Compounds 1 and 2 may both be included in the same layer. For example, both Compounds 1 and 2 may be included in an emission layer.

The term "organic layer" as used herein refers to a single layer or a plurality of layers that are disposed between the first electrode and the second electrode in an organic light-emitting device. The "organic layer" may include organic compounds and organometallic complexes including metals.

The FIGURE 1 illustrates a schematic cross-sectional view of an exemplary organic light-emitting device 10 according to one or more embodiments. Hereinafter, a structure of an organic light-emitting device according to one or more embodiments and a method of manufacturing the organic light-emitting device will be described with reference to the FIGURE 1. The organic light-emitting device 10 may include a first electrode 11, an organic layer 15, and a second electrode 19, which in some embodiments may be sequentially layered in this stated order.

A substrate may be additionally disposed under the first electrode 11 (i.e., the first electrode is disposed on a substrate) or a substrate may be disposed on the second electrode 19. The substrate may be any substrate used in organic light-emitting devices, e.g., a glass substrate or a transparent plastic substrate, each having excellent mechanical strength, thermal stability, transparency, surface smoothness, ease of handling, and water repellency.

The first electrode 11 may be formed by depositing or sputtering, onto the substrate, a material for forming the first electrode 11. The first electrode 11 may be an anode. The material for forming the first electrode 11 may be selected from materials with a high work function for easy hole injection. The first electrode 11 may be a reflective electrode, a semi-transmissive electrode, or a transmissive electrode. The material for forming the first electrode 11 may be indium tin oxide (ITO), indium zinc oxide (IZO), tin oxide ($SnO_2$), or zinc oxide (ZnO). In some embodiments, the material for forming the first electrode 11 may be a metal, such as magnesium (Mg), aluminum (Al), aluminum-lithium (Al-Li), calcium (Ca), magnesium-indium (Mg-In), or magnesium-silver (Mg-Ag).

The first electrode 11 may have a single-layered structure or a multi-layered structure including a plurality of layers. In some embodiments, the first electrode 11 may have a triple-layered structure of ITO/Ag/ITO, but in other embodiments the structure of the first electrode 11 are not limited thereto.

The organic layer 15 may be disposed on the first electrode 11.

The organic layer 15 may include a hole transport region, an emission layer, and an electron transport region.

The hole transport region may be disposed between the first electrode 11 and the emission layer.

The hole transport region may include a hole injection layer, a hole transport layer, an electron blocking layer, a buffer layer, or a combination thereof.

The hole transport region may include a hole injection layer only or a hole transport layer only. In some embodiments, the hole transport region may include a hole injection layer and a hole transport layer which are sequentially stacked on the first electrode 11. In some embodiments, the hole transport region may include a hole injection layer, a hole transport layer, and an electron blocking layer, which are sequentially stacked on the first electrode 11.

When the hole transport region includes a hole injection layer, the hole injection layer may be formed on the first electrode 11 by using one or more suitable methods, such as vacuum deposition, spin coating, casting, and Langmuir-Blodgett (LB) deposition.

When a hole injection layer is formed by vacuum-deposition, for example, the vacuum deposition may be performed at a temperature in a range of about 100°C to about 500°C, at a vacuum degree in a range of about $10^{-8}$ torr to about $10^{-3}$ torr (wherein 1 torr =133.322 Pa), and at a rate in a range of about 0.01 Angstroms per second (Å/sec) to about 100 Å/sec, though the conditions may vary depending on a compound used as a hole injection material and a structure and thermal properties of a desired hole injection layer, but embodiments are not limited thereto.

When a hole injection layer is formed by spin coating, the spin coating may be performed at a rate in a range of about 2,000 revolutions per minute (rpm) to about 5,000 rpm (wherein 1 rpm = 1/60 Hz) and at a temperature in a

range of about 80°C to 200°C to facilitate removal of a solvent after the spin coating, though the conditions may vary depending on a compound used as a hole injection material and a structure and thermal properties of a desired hole injection layer, but embodiments are not limited thereto.

The conditions for forming a hole transport layer and an electron blocking layer may be inferred from the conditions for forming the hole injection layer.

The hole transport region may include at least one selected from m-MTDATA, TDATA, 2-TNATA, NPB, β-NPB, TPD, spiro-TPD, spiro-NPB, methylated-NPB, TAPC, HMTPD, 4,4',4"-tris(N-carbazolyl)triphenylamine (TCTA), polyaniline/dodecylbenzene sulfonic acid (PANI/DBSA), poly(3,4-ethylenedioxythiophene)/poly(4-styrene sulfonate) (PEDOT/PSS), polyaniline/camphor-sulfonic acid (PANI/CSA), polyaniline/poly(4-styrene sulfonate (PANI/PSS), a compound represented by Formula 201, and a compound represented by Formula 202:

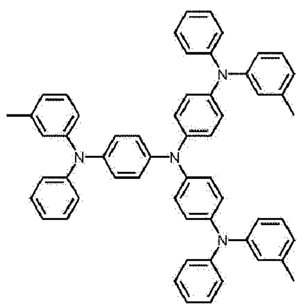

m-MTDATA

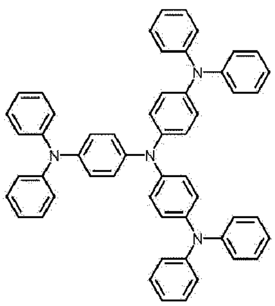

TDATA

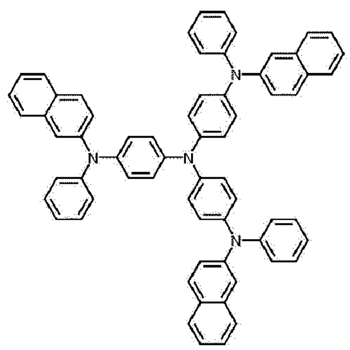

2-TNATA

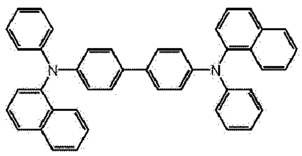

NPB

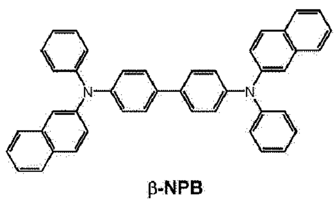

β-NPB

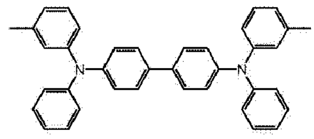

TPD

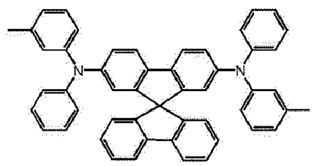

Spiro-TPD

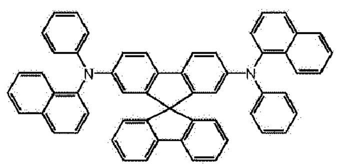

Spiro-NPB

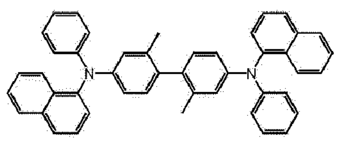

methylated NPB

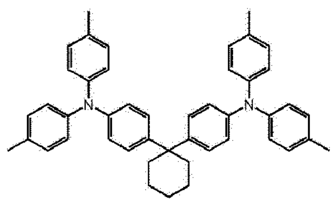

TAPC

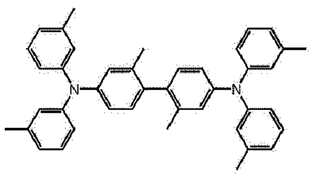

HMTPD

Formula 201

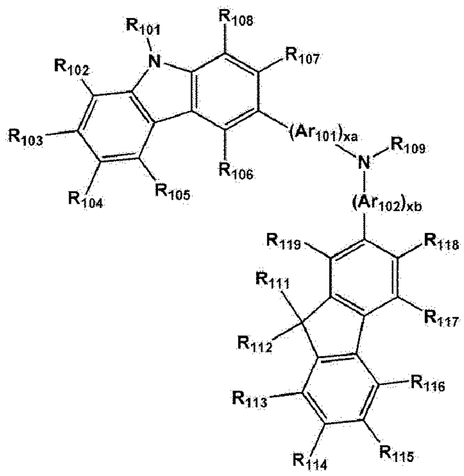

Formula 202

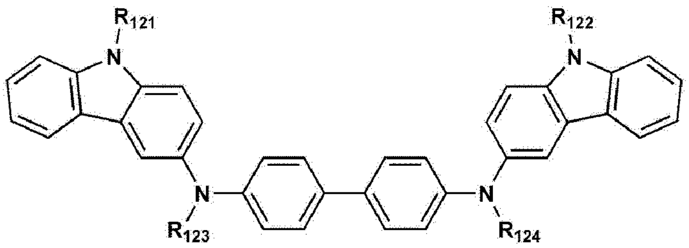

wherein, in Formula 201, $Ar_{101}$ and $Ar_{102}$ may each independently be:

a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylenylene group, a naphthacenylene group, a picenylene group, a perylenylene group, or a pentacenylene group; or a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylenylene group, a naphthacenylene group, a picenylene group, a perylenylene group, or a pentacenylene group, each substituted with at least one of deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_3$-$C_{10}$ cycloalkenyl group, a $C_1$-$C_{10}$ heterocycloalkyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_7$-$C_{60}$ arylalkyl group, a $C_1$-$C_{60}$ heteroaryl group, a $C_1$-$C_{60}$ heteroaryloxy group, a $C_1$-$C_{60}$ heteroarylthio group, a $C_2$-$C_{60}$ heteroarylalkyl group, a monovalent non-aromatic condensed polycyclic group, or a monovalent non-aromatic condensed heteropolycyclic group.

In Formula 201, xa and xb may each independently be an integer from 0 to 5. In some embodiments, xa and xb may each independently be 0, 1, or 2. In some embodiments, xa may be 1, and xb may be 0, but embodiments are not limited thereto.

In Formulae 201 and 202, $R_{101}$ to $R_{108}$, $R_{111}$ to $R_{119}$, and $R_{121}$ to $R_{124}$ may each independently be:

hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{10}$ alkyl group (e.g., a methyl group, an ethyl group, a propyl group, a butyl group, pentyl group, or a hexyl group), or a $C_1$-$C_{10}$ alkoxy group (e.g., a methoxy group, an ethoxy group, a propoxy group, a butoxy group, or a pentoxy group);

a $C_1$-$C_{10}$ alkyl group or a $C_1$-$C_{10}$ alkoxy group, each substituted with at least one of deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, or a phosphoric acid group or a salt thereof;

a phenyl group, a naphthyl group, an anthracenyl group, a fluorenyl group, or a pyrenyl group; or

a phenyl group, a naphthyl group, an anthracenyl group, a fluorenyl group, or a pyrenyl group, each substituted with at least one of deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{10}$ alkyl group, or a $C_1$-$C_{10}$ alkoxy group, but embodiments are not limited thereto.

In Formula 201, $R_{109}$ may be:

a phenyl group, a naphthyl group, an anthracenyl group, or a pyridinyl group; or

a phenyl group, a naphthyl group, an anthracenyl group, or a pyridinyl group, each substituted with at least one of deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{20}$ alkyl group, a $C_1$-$C_{20}$ alkoxy group, a phenyl group, a naphthyl group, an anthracenyl group, or a pyridinyl group.

In some embodiments, the compound represented by Formula 201 may be represented by Formula 201A, but embodiments are not limited thereto:

Formula 201A

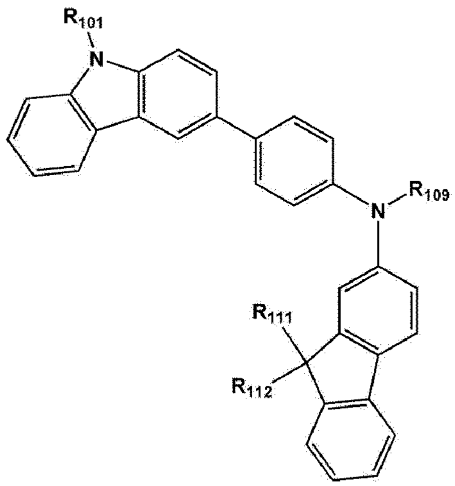

wherein, in Formula 201A, $R_{101}$, $R_{111}$, $R_{112}$, and $R_{109}$ may respectively be understood by referring to the descriptions of $R_{101}$, $R_{111}$, $R_{112}$, and $R_{109}$ provided herein.

In some embodiments, the compounds represented by Formulae 201 and 202 may include Compounds HT1 to HT20, but embodiments are not limited thereto:

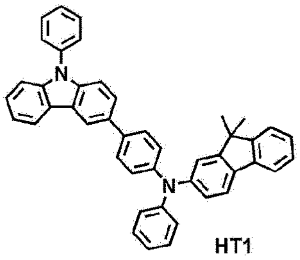
HT1

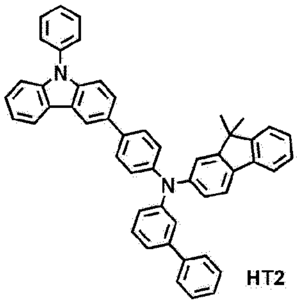
HT2

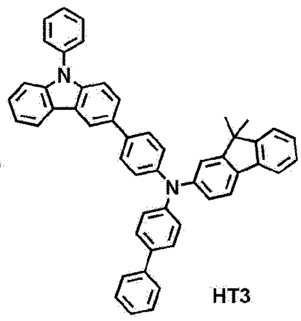
HT3

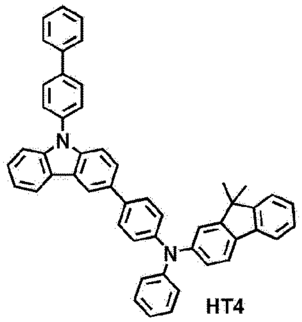
HT4

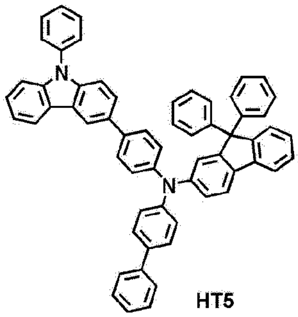
HT5

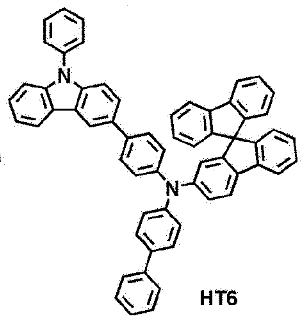
HT6

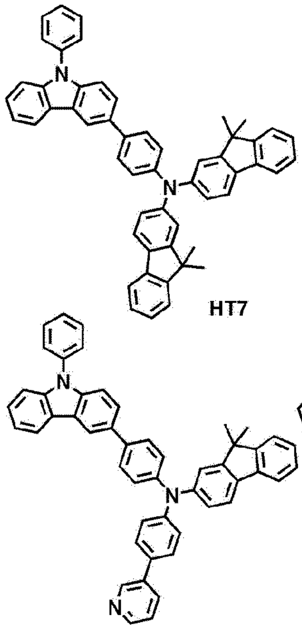
HT7

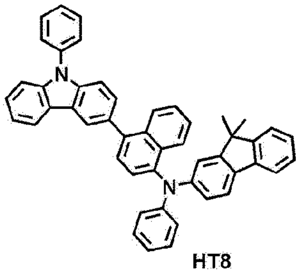
HT8

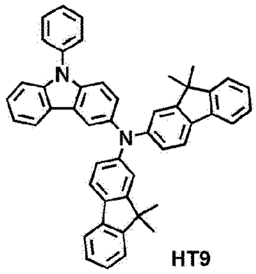
HT9

HT10

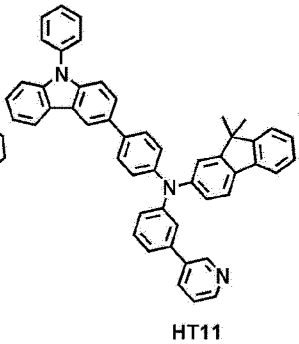
HT11

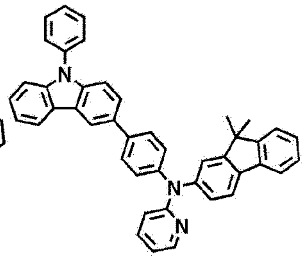
HT12

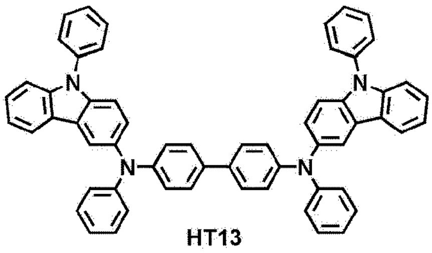
HT13

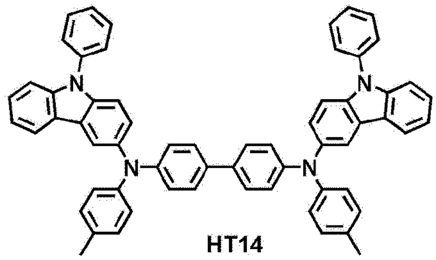
HT14

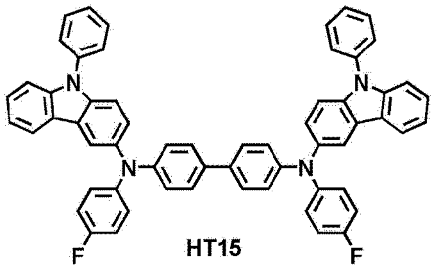
HT15

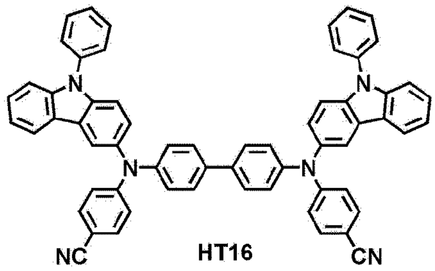
HT16

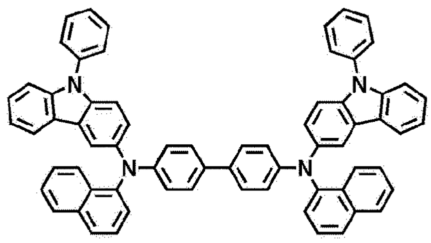

HT17

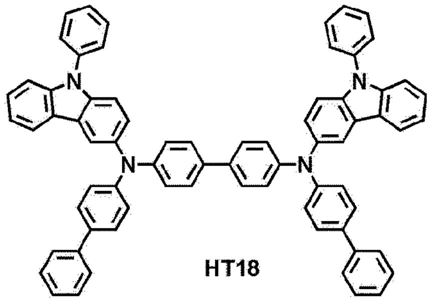

HT18

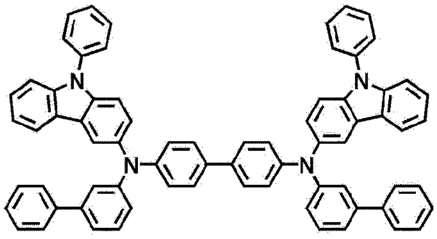

HT19

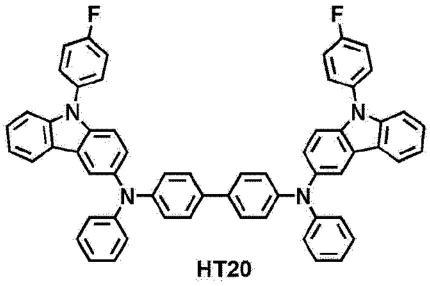

HT20

The thickness of the hole transport region may be in a range of about 100 Angstroms (Å) to about 10,000 Å, for example, about 100 Å to about 1,000 Å. When the hole transport region includes at least one selected from a hole injection layer and a hole transport layer, the thickness of the hole injection layer may be in a range of about 100 Å to about 10,000 Å, for example, about 100 Å to about 1,000 Å, the thickness of the hole transport layer may be in a range of about 50 Å to about 2,000 Å, for example, about 100 Å to about 1,500 Å. While not wishing to be bound by theory, it is understood that when the thicknesses of the hole transport region, the hole injection layer, and the hole transport layer are within any of these ranges, excellent hole transport characteristics may be obtained without a substantial increase in driving voltage.

The hole transport region may include a charge generating material as well as the aforementioned materials, to improve conductive properties of the hole transport region. The charge generating material may be substantially homogeneously or non-homogeneously dispersed in the hole transport region.

The charge generating material may include, for example, a p-dopant. The p-dopant may include one of a quinone derivative, a metal oxide, and a compound containing a cyano group, but embodiments are not limited thereto. For example, non-limiting examples of the p-dopant include a quinone derivative, such as tetracyanoquinodimethane (TCNQ) or 2,3,5,6-tetrafluoro-tetracyano-1,4-benzoquinonedimethane (F4-TCNQ); a metal oxide, such as a tungsten oxide or a molybdenum oxide; and a compound containing a cyano group, such as Compound HT-D1, but embodiments are not limited thereto:

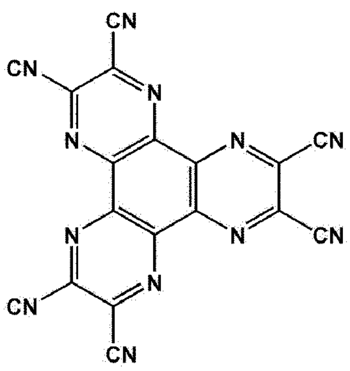

HT-D1

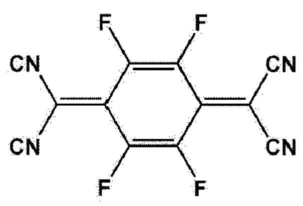

F4-TCNQ

The hole transport region may further include a buffer layer.

The buffer layer may compensate for an optical resonance distance depending on a wavelength of light emitted from the emission layer to improve the efficiency of an organic light-emitting device.

An emission layer may be formed on the hole transport region by using one or more suitable methods, such

as vacuum deposition, spin coating, casting, LB deposition, or the like. When the emission layer is formed by vacuum deposition or spin coating, vacuum deposition and coating conditions for forming the emission layer may be generally similar to those conditions for forming a hole injection layer, though the conditions may vary depending on a compound that is used.

When the hole transport region includes an electron blocking layer, a material for forming the electron blocking layer may be selected from the materials for forming a hole transport region and host materials described herein, but embodiments are not limited thereto. In some embodiments, when the hole transport region includes an electron blocking layer, mCP described herein may be used for forming the electron blocking layer.

The emission layer may include a host and a dopant, and the dopant may include the organometallic compound represented by Formula 1.

The host may include at least one selected from TPBi, TBADN, ADN (also known as "DNA"), CBP, CDBP, TCP, mCP, and Compounds H50 and H51:

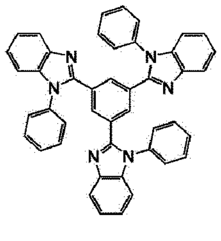

TPBi

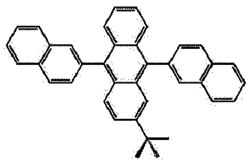

TBADN

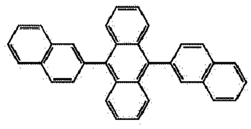

ADN

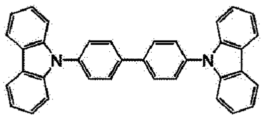

CBP

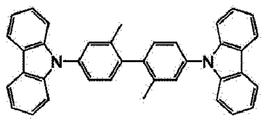

CDBP

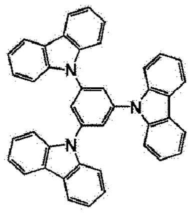

TCP

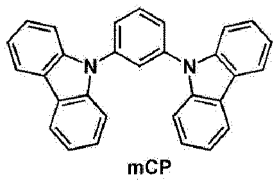

mCP

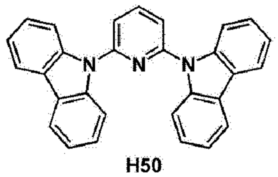

H50

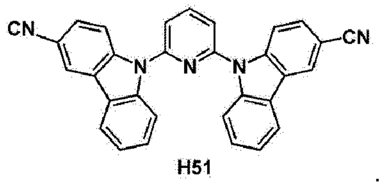

H51

.

In some embodiments, the host may further include a compound represented by Formula 301:

Formula 301

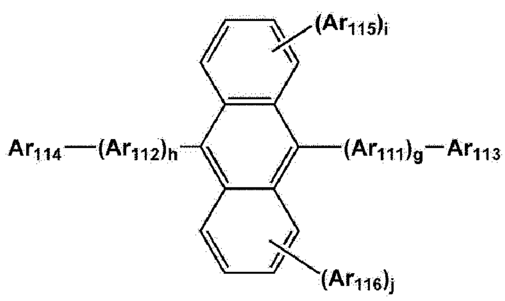

wherein, in Formula 301, $Ar_{111}$ and $Ar_{112}$ may each independently be:

a phenylene group, a naphthylene group, a phenanthrenylene group, or a pyrenylene group; or

a phenylene group, a naphthylene group, a phenanthrenylene group, or a pyrenylene group, each substituted with at least one of a phenyl group, a naphthyl group, or an anthracenyl group.

In Formula 301, $Ar_{113}$ to $Ar_{116}$ may each independently be:

a $C_1$-$C_{10}$ alkyl group, a phenyl group, a naphthyl group, a phenanthrenyl group, or a pyrenyl group; or
a phenyl group, a naphthyl group, a phenanthrenyl group, or a pyrenyl group, each substituted with at least one of a phenyl group, a naphthyl group, or an anthracenyl group.

In Formula 301, g, h, i, and j may each independently be an integer from 0 to 4. In some embodiments, g, h, i, and j may each independently be 0, 1, or 2.

In Formula 301, $Ar_{113}$ to $Ar_{116}$ may each independently be:

a $C_1$-$C_{10}$ alkyl group substituted with at least one of a phenyl group, a naphthyl group, or an anthracenyl group;
a phenyl group, a naphthyl group, an anthracenyl group, a pyrenyl group, a phenanthrenyl group, or a fluorenyl group;
a phenyl group, a naphthyl group, an anthracenyl group, a pyrenyl group, a phenanthrenyl group, or a fluorenyl group, each substituted with at least one of deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a phenyl group, a naphthyl group, an anthracenyl group, a pyrenyl group, a phenanthrenyl group, or a fluorenyl group; or
a group of the formula

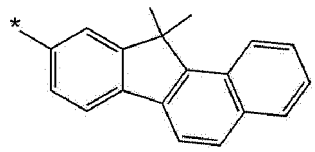

,

but embodiments are not limited thereto.

In some embodiments, the host may include a compound represented by Formula 302:

Formula 302

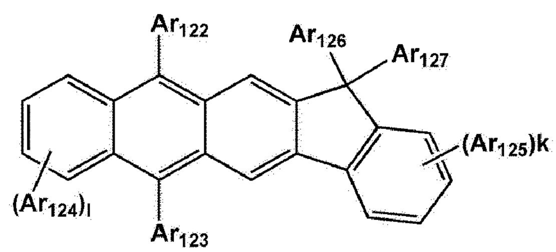

In Formula 302, $Ar_{122}$ to $Ar_{125}$ may each independently be understood by referring to the descriptions for $Ar_{113}$ in Formula 301 provided herein.

In Formula 302, $Ar_{126}$ and $Ar_{127}$ may each independently be a $C_1$-$C_{10}$ alkyl group (e.g., a methyl group, an ethyl group, or a propyl group).

In Formula 302, k and l may each independently be an integer from 0 to 4. In some embodiments, k and l may each be 0, 1, or 2.

When the organic light-emitting device 10 is a full-color organic light-emitting device, the emission layer may be patterned into a red emission layer, a green emission layer, and a blue emission layer. In some embodiments, the emission layer may have a structure in which the red emission layer, the green emission layer, and/or the blue emission layer are layered to emit white light. In some embodiments, the structure of the emission layer may vary.

When the emission layer includes the host and the dopant, an amount of the dopant may be in a range of about 0.01 parts to about 15 parts by weight based on about 100 parts by weight of the host, but embodiments are not limited thereto.

The thickness of the emission layer may be in a range of about 100 Å to about 1,000 Å, and in some embodiments, about 200 Å to about 600 Å. While not wishing to be bound by theory, when the thickness of the emission layer is within any of these ranges, improved luminescence characteristics may be obtained without a substantial increase in driving voltage.

Next, an electron transport region may be formed on the emission layer.

The electron transport region may include a hole blocking layer, an electron transport layer, an electron injection layer, or a combination thereof.

In some embodiments, the electron transport region may have a hole blocking layer/an electron transport layer/an electron injection layer structure or an electron transport layer/an electron injection layer structure, but embodiments are not limited thereto. The electron transport layer may have a single-layered structure or a multi-layered structure including two or more different materials.

The conditions for forming a hole blocking layer, an electron transport layer, and an electron injection layer may be inferred based on the conditions for forming the hole injection layer.

When the electron transport region includes a hole blocking layer, the hole blocking layer may include, for example, at least one selected from BCP, Bphen, and BAlq, but embodiments are not limited thereto:

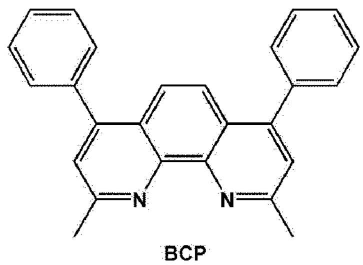

BCP

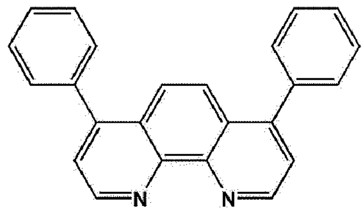

Bphen

.

The thickness of the hole blocking layer may be in a range of about 20 Å to about 1,000 Å, for example, about 30 Å to about 300 Å. While not wishing to be bound by theory, when the thickness of the hole blocking layer is within any of these ranges, excellent hole blocking characteristics may be obtained without a substantial increase in driving voltage.

The electron transport layer may include at least one selected from BCP, Bphen, $Alq_3$, BAlq, TAZ, and NTAZ:

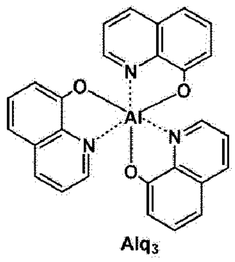

$Alq_3$

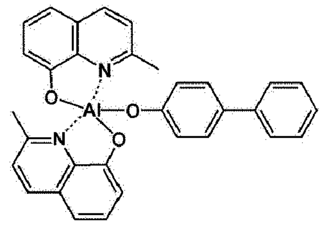

BAlq

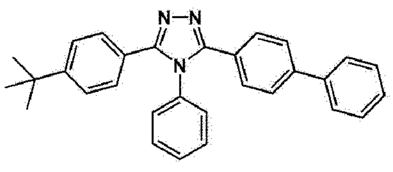

TAZ

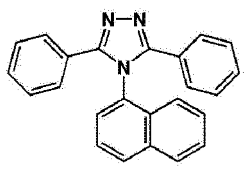

NTAZ

.

In some embodiments, the electron transport layer may include at least one selected from Compounds ET1 to ET25, but embodiments are not limited thereto:

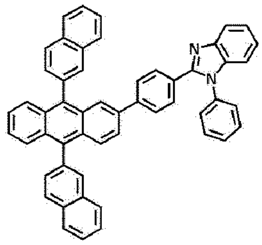
ET1

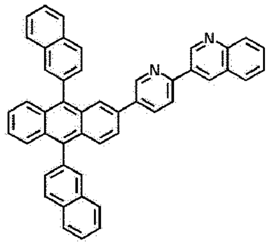
ET2

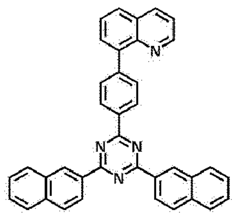
ET3

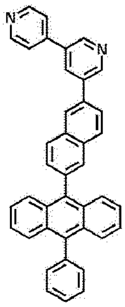
ET4

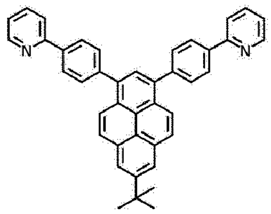
ET5

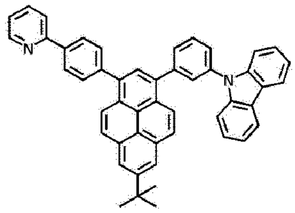
ET6

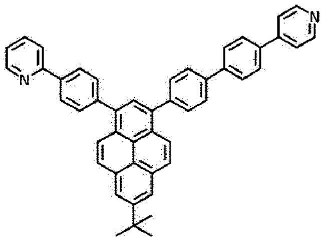
ET7

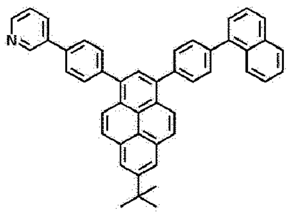
ET8

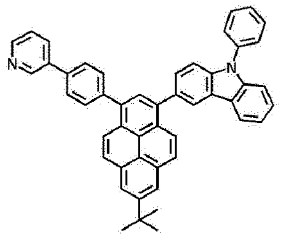
ET9

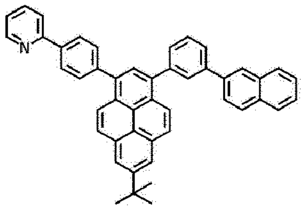
ET10

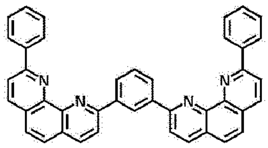
ET11

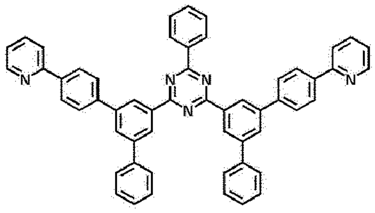
ET12

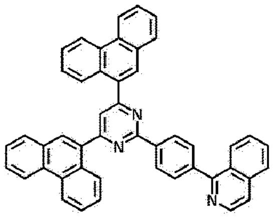
ET13

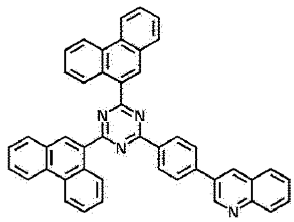
ET14

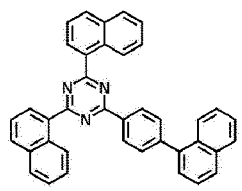
ET15

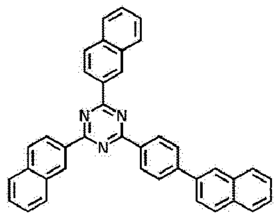
ET16

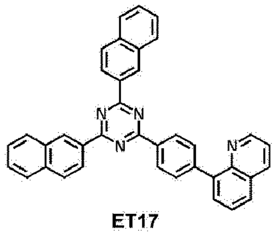
ET17

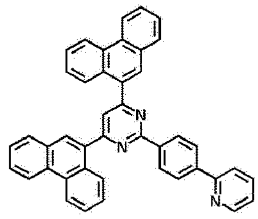
ET18

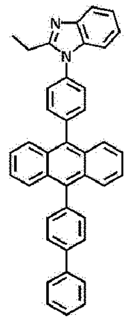
ET19

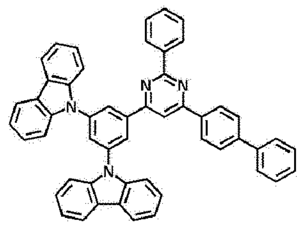
ET20

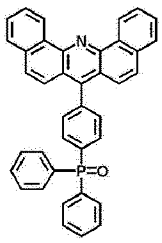
ET21

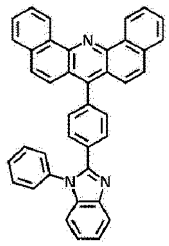
ET22

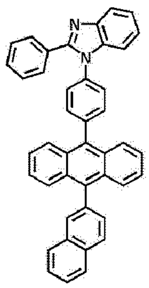
ET23

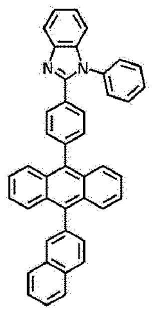
ET24

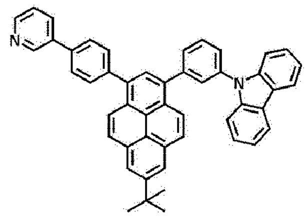
ET25

.

The thickness of the electron transport layer may be in a range of about 100 Å to about 1,000 Å, and in some embodiments, about 150 Å to about 500 Å. While not wishing to be bound by theory, when the thickness of the electron transport layer is within any of these ranges, excellent electron transport characteristics may be obtained without a substantial increase in driving voltage.

The electron transport layer may further include a metal-containing material, in addition to the materials described above.

The metal-containing material may include a Li complex. The Li complex may include, e.g., Compound ET-D1 (LiQ) or Compound ET-D2:

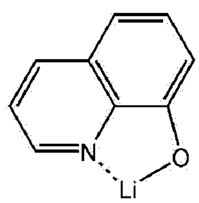
ET-D1

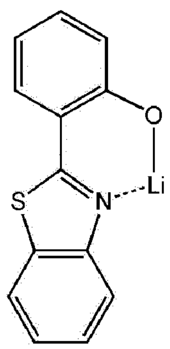
ET-D2

.

The electron transport region may include an electron injection layer that facilitates electron injection from the second electrode 19.

The electron injection layer may include at least one selected from, LiF, NaCl, CsF, $Li_2O$, and BaO.

The thickness of the electron injection layer may be in a range of about 1 Å to about 100 Å, and in some embodiments, about 3 Å to about 90 Å. While not wishing to be bound by theory, when the thickness of the electron injection layer is within any of these ranges, excellent electron injection characteristics may be obtained without a substantial increase in driving voltage.

The second electrode 19 may be on the organic layer 15. The second electrode 19 may be a cathode. A material for forming the second electrode 19 may be a material with a relatively low work function, such as a metal, an alloy, an electrically conductive compound, and a mixture thereof. Examples of the material for forming the second electrode 19 may include lithium (Li), magnesium (Mg), aluminum (Al), aluminum-lithium (Al-Li), calcium (Ca), magnesium-indium (Mg-In), and magnesium-silver (Mg-Ag). In some embodiments, ITO or IZO may be used to form a transmissive second electrode 19 to manufacture a top emission light-emitting device. In some embodiments, the material for forming the second electrode 19 may vary.

Hereinbefore the organic light-emitting device 10 has been described with reference to the FIGURE 1, but embodiments are not limited thereto.

According to an aspect of still another embodiment, a diagnostic composition may include at least one organometallic compound represented by Formula 1.

Since the organometallic compound represented by Formula 1 provides high luminescence efficiency, the diagnostic efficiency of the diagnostic composition that includes the organometallic compound represented by Formula 1 may be excellent.

The diagnostic composition may be applied in various ways, such as in a diagnostic kit, a diagnostic reagent, a biosensor, or a biomarker.

The term "$C_1$-$C_{60}$ alkyl group" as used herein refers to a linear or branched saturated aliphatic hydrocarbon monovalent group having 1 to 60 carbon atoms. Examples thereof include a methyl group, an ethyl group, a propyl group, an iso-butyl group, a sec-butyl group, a tert-butyl group, a pentyl group, an iso-amyl group, and a hexyl group. The term "$C_1$-$C_{60}$ alkylene group" as used herein refers to a divalent group having the same structure as the $C_1$-$C_{60}$ alkyl group.

The term "$C_1$-$C_{60}$ alkoxy group" as used herein refers to a monovalent group represented by $-OA_{101}$ (wherein $A_{101}$ is a $C_1$-$C_{60}$ alkyl group). Examples thereof include a methoxy group, an ethoxy group, and an isopropyloxy group.

The term "$C_2$-$C_{60}$ alkenyl group" as used herein refers to a group formed by placing at least one carbon-carbon double bond in the middle or at the terminus of the $C_2$-$C_{60}$ alkyl group. Examples thereof include an ethenyl group, a propenyl group, and a butenyl group. The term "$C_2$-$C_{60}$ alkenylene group" as used herein refers to a divalent group having the same structure as the $C_2$-$C_{60}$ alkenyl group.

The term "$C_2$-$C_{60}$ alkynyl group" as used herein refers to a group formed by placing at least one carbon-carbon triple bond in the middle or at the terminus of the $C_2$-$C_{60}$ alkyl group. Examples thereof include an ethenyl group and a propenyl group. The term "$C_2$-$C_{60}$ alkynylene group" as used herein refers to a divalent group having the same structure as the $C_2$-$C_{60}$ alkynyl group.

The term "$C_3$-$C_{10}$ cycloalkyl group" as used herein refers to a monovalent monocyclic saturated hydrocarbon group including 3 to 10 carbon atoms. Examples thereof include a cyclopropyl group, a cyclobutyl group, a cyclopentyl group, a cyclohexyl group, and a cycloheptyl group. The term "$C_3$-$C_{10}$ cycloalkylene group" as used herein refers to a divalent group having the same structure as the $C_3$-$C_{10}$ cycloalkyl group.

The term "$C_1$-$C_{10}$ heterocycloalkyl group" as used herein refers to a monovalent monocyclic group including at least one heteroatom selected from N, O, P, Si, and S as a ring-forming atom and 1 to 10 carbon atoms. Examples thereof include a tetrahydrofuranyl group and a tetrahydrothiophenyl group. The term "$C_1$-$C_{10}$ heterocycloalkylene group" as used herein refers to a divalent group having the same structure as the $C_1$-$C_{10}$ heterocycloalkyl group.

The term "$C_3$-$C_{10}$ cycloalkenyl group" as used herein refers to a monovalent monocyclic group that has 3 to 10 carbon atoms and at least one carbon-carbon double bond in its ring, wherein the molecular structure as a whole is non-aromatic. Examples thereof include a cyclopentenyl group, a cyclohexenyl group, and a cycloheptenyl group. The term "$C_3$-$C_{10}$ cycloalkenylene group" as used herein refers to a divalent group having the same structure as the $C_3$-$C_{10}$ cycloalkenyl group.

The term "$C_1$-$C_{10}$ heterocycloalkenyl group" as used herein refers to a monovalent monocyclic group including at least one heteroatom selected from N, O, P, Si, and S as a ring-forming atom, 1 to 10 carbon atoms, and at least one double bond in its ring. Examples of the $C_1$-$C_{10}$ heterocycloalkenyl group include a 2,3-dihydrofuranyl group and a 2,3-dihydrothiophenyl group. The term "$C_1$-$C_{10}$ heterocycloalkylene group" as used herein refers to a divalent group having the same structure as the $C_1$-$C_{10}$ heterocycloalkenyl group.

The term "$C_6$-$C_{60}$ aryl group" as used herein refers to a monovalent group having a carbocyclic aromatic system having 6 to 60 carbon atoms. The term "$C_6$-$C_{60}$ arylene group" as used herein refers to a divalent group having a

carbocyclic aromatic system having 6 to 60 carbon atoms. Examples of the $C_6$-$C_{60}$ aryl group include a phenyl group, a naphthyl group, an anthracenyl group, a phenanthrenyl group, a pyrenyl group, and a chrysenyl group. When the $C_6$-$C_{60}$ aryl group and the $C_6$-$C_{60}$ arylene group each include a plurality of rings, the plurality of rings may be fused to each other. The term "$C_7$-$C_{60}$ alkylaryl group" as used herein refers to a $C_6$-$C_{60}$ aryl group substituted with at least one $C_1$-$C_{60}$ alkyl group.

The term "$C_1$-$C_{60}$ heteroaryl group" as used herein refers to a monovalent group having a heterocyclic aromatic system having at least one heteroatom selected from N, O, P, Si, and S as a ring-forming atom and 1 to 60 carbon atoms. The term "$C_1$-$C_{60}$ heteroarylene group" as used herein refers to a divalent group having a heterocyclic aromatic system having at least one heteroatom selected from N, O, P, Si, and S as a ring-forming atom and 1 to 60 carbon atoms. Examples of the $C_1$-$C_{60}$ heteroaryl group include a pyridinyl group, a pyrimidinyl group, a pyrazinyl group, a pyridazinyl group, a triazinyl group, a quinolinyl group, and an isoquinolinyl group. When the $C_1$-$C_{60}$ heteroaryl group and the $C_1$-$C_{60}$ heteroarylene group each include a plurality of rings, the plurality of rings may be fused to each other. The term "$C_2$-$C_{60}$ alkylheteroaryl group" as used herein refers to a $C_1$-$C_{60}$ heteroaryl group substituted with at least one $C_1$-$C_{60}$ alkyl group.

The term "$C_6$-$C_{60}$ aryloxy group" as used herein indicates $-OA_{102}$ (wherein $A_{102}$ is a $C_6$-$C_{60}$ aryl group), the term "$C_6$-$C_{60}$ arylthio group" as used herein indicates - $SA_{103}$ (wherein $A_{103}$ is a $C_6$-$C_{60}$ aryl group), and the term "$C_7$-$C_{60}$ arylalkyl group" as used herein indicates $-A_{104}A_{105}$ (wherein $A_{105}$ is the $C_6$-$C_{59}$ aryl group and $A_{104}$ is the $C_1$-$C_{54}$ alkylene group).

The term "$C_1$-$C_{60}$ heteroaryloxy group" as used herein refers to $-OA_{106}$ (wherein $A_{106}$ is the $C_1$-$C_{60}$ heteroaryl group), the term "$C_1$-$C_{60}$ heteroarylthio group" as used herein indicates $-SA_{107}$ (wherein $A_{107}$ is the $C_1$-$C_{60}$ heteroaryl group), and the term "$C_2$-$C_{60}$ heteroarylalkyl group" as used herein refers to $-A_{108}A_{109}$ ($A_{109}$ is a $C_1$-$C_{59}$ heteroaryl group, and $A_{108}$ is a $C_1$-$C_{59}$ alkylene group).

The term "monovalent non-aromatic condensed polycyclic group" as used herein refers to a monovalent group that has two or more condensed rings and only carbon atoms (e.g., the number of carbon atoms may be in a range of 8 to 60) as ring-forming atoms, wherein the molecular structure as a whole is non-aromatic. Examples of the monovalent non-aromatic condensed polycyclic group include a fluorenyl group. The term "divalent non-aromatic condensed polycyclic group" as used herein refers to a divalent group having substantially the same structure as the monovalent non-aromatic condensed polycyclic group.

The term "monovalent non-aromatic condensed heteropolycyclic group" as used herein refers to a monovalent group that has two or more condensed rings and a heteroatom selected from N, O, P, Si, and S and carbon atoms (e.g., the number of carbon atoms may be in a range of 1 to 60) as ring-forming atoms, wherein the molecular structure as a whole is non-aromatic. Examples of the monovalent non-aromatic condensed heteropolycyclic group include a carbazolyl group. The term "divalent non-aromatic condensed heteropolycyclic group" as used herein refers to a divalent group having substantially the same structure as the monovalent non-aromatic condensed heteropolycyclic group.

The term "$C_5$-$C_{30}$ carbocyclic group" as used herein refers to a saturated or unsaturated cyclic group including 5 to 30 carbon atoms only as ring-forming atoms. The $C_5$-$C_{30}$ carbocyclic group may be a monocyclic group or a polycyclic group.

The term "$C_1$-$C_{30}$ heterocyclic group" as used herein refers to saturated or unsaturated cyclic group including 1 to 30 carbon atoms and at least one heteroatom selected from N, O, P, Si, and S as ring-forming atoms. The $C_1$-$C_{30}$ heterocyclic group may be a monocyclic group or a polycyclic group.

At least one substituent of the substituted $C_5$-$C_{30}$ carbocyclic group, the substituted $C_1$-$C_{30}$ heterocyclic group, the substituted $C_1$-$C_{60}$ alkyl group, the substituted $C_2$-$C_{60}$ alkenyl group, the substituted $C_2$-$C_{60}$ alkynyl group, the substituted $C_1$-$C_{60}$ alkoxy group, the substituted $C_3$-$C_{10}$ cycloalkyl group, the substituted $C_1$-$C_{10}$ heterocycloalkyl group, the substituted $C_3$-$C_{10}$ cycloalkenyl group, the substituted $C_1$-$C_{10}$ heterocycloalkenyl group, the substituted $C_6$-$C_{60}$ aryl group, the substituted $C_7$-$C_{60}$ alkyl aryl group, the substituted $C_6$-$C_{60}$ aryloxy group, the substituted $C_6$-$C_{60}$ arylthio group, the substituted $C_7$-$C_{60}$ arylalkyl group, the substituted $C_1$-$C_{60}$ heteroaryl group, the substituted $C_1$-$C_{60}$ heteroaryloxy group, the substituted $C_1$-$C_{60}$ heteroarylthio group, the substituted $C_2$-$C_{60}$ heteroarylalkyl group, the substituted $C_2$-$C_{60}$ alkylheteroaryl group, the substituted monovalent non-aromatic condensed polycyclic group, or the substituted monovalent non-aromatic condensed heteropolycyclic group may be:

deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, or a $C_1$-$C_{60}$ alkoxy group;

a $C_1$-$C_{60}$ alkyl group, a $C_2$-$C_{60}$ alkenyl group, a $C_2$-$C_{60}$ alkynyl group, or a $C_1$-$C_{60}$ alkoxy group, each substituted with at least one of deuterium, -F, -Cl, -Br, -I, - $CD_3$, $-CD_2H$, $-CDH_2$, $-CF_3$, $-CF_2H$, $-CFH_2$, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a $C_3$-$C_{10}$ cycloalkyl group,

a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_7$-$C_{60}$ alkyl aryl group, a $C_6$-$C_{60}$ aryloxy group, a $C_6$-$C_{60}$ arylthio group, a $C_7$-$C_{60}$ arylalkyl group, a $C_1$-$C_{60}$ heteroaryl group, a $C_1$-$C_{60}$ heteroaryloxy group, a $C_1$-$C_{60}$ heteroarylthio group, a $C_2$-$C_{60}$ heteroarylalkyl group, a $C_2$-$C_{60}$ alkylheteroaryl group, a monovalent non-aromatic condensed polycyclic group, a monovalent non-aromatic condensed heteropolycyclic group, -N($Q_{11}$)($Q_{12}$), -Si($Q_{13}$)($Q_{14}$)($Q_{15}$), - Ge($Q_{13}$)($Q_{14}$)($Q_{15}$), -B($Q_{16}$)($Q_{17}$), -P($Q_{18}$)($Q_{19}$), or -P(=O)($Q_{18}$)($Q_{19}$);

a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_7$-$C_{60}$ alkyl aryl group, a $C_6$-$C_{60}$ aryloxy group, a $C_6$-$C_{60}$ arylthio group, a $C_7$-$C_{60}$ arylalkyl group, a $C_1$-$C_{60}$ heteroaryl group, a $C_1$-$C_{60}$ heteroaryloxy group, a $C_1$-$C_{60}$ heteroarylthio group, a $C_2$-$C_{60}$ heteroarylalkyl group, a $C_2$-$C_{60}$ alkylheteroaryl group, a monovalent non-aromatic condensed polycyclic group, or a monovalent non-aromatic condensed heteropolycyclic group;

a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_7$-$C_{60}$ alkyl aryl group, a $C_6$-$C_{60}$ aryloxy group, a $C_6$-$C_{60}$ arylthio group, a $C_7$-$C_{60}$ arylalkyl group, a $C_1$-$C_{60}$ heteroaryl group, a $C_1$-$C_{60}$ heteroaryloxy group, a $C_1$-$C_{60}$ heteroarylthio group, a $C_2$-$C_{60}$ heteroarylalkyl group, a $C_2$-$C_{60}$ alkylheteroaryl group, a monovalent non-aromatic condensed polycyclic group, or a monovalent non-aromatic condensed heteropolycyclic group, each substituted with at least one of deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_7$-$C_{60}$ alkyl aryl group, a $C_6$-$C_{60}$ aryloxy group, a $C_6$-$C_{60}$ arylthio group, a $C_7$-$C_{60}$ arylalkyl group, a $C_1$-$C_{60}$ heteroaryl group, a $C_1$-$C_{60}$ heteroaryloxy group, a $C_1$-$C_{60}$ heteroarylthio group, a $C_2$-$C_{60}$ heteroarylalkyl group, a $C_2$-$C_{60}$ alkylheteroaryl group, a monovalent non-aromatic condensed polycyclic group, a monovalent non-aromatic condensed heteropolycyclic group, -N($Q_{21}$)($Q_{22}$), -Si($Q_{23}$)($Q_{24}$)($Q_{25}$), -Ge($Q_{23}$)($Q_{24}$)($Q_{25}$), -B($Q_{26}$)($Q_{27}$), -P($Q_{28}$)($Q_{29}$), or -P(=O)($Q_{28}$)($Q_{29}$); or

-N(Q31)(Q32), -Si($Q_{33}$)($Q_{34}$)($Q_{35}$), -Ge($Q_{33}$)($Q_{34}$)($Q_{35}$), -B($Q_{36}$)($Q_{37}$), -P($Q_{38}$)($Q_{39}$), or -P(=O)($Q_{38}$)($Q_{39}$),

wherein $Q_1$ to $Q_9$, $Q_{11}$ to $Q_{19}$, $Q_{21}$ to $Q_{29}$, and $Q_{31}$ to $Q_{39}$ may each independently be:

hydrogen, deuterium, -F, -Cl, -Br, -I, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_1$-$C_{60}$ alkyl group substituted with at least one of deuterium, a $C_1$-$C_{60}$ alkyl group, or a $C_6$-$C_{60}$ aryl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryl group substituted with at least one of deuterium, a $C_1$-$C_{60}$ alkyl group, or a $C_6$-$C_{60}$ aryl group, a $C_6$-$C_{60}$ aryloxy group, a $C_6$-$C_{60}$ arylthio group, a $C_7$-$C_{60}$ arylalkyl group, a $C_1$-$C_{60}$ heteroaryl group, a $C_1$-$C_{60}$ heteroaryloxy group; a $C_1$-$C_{60}$ heteroarylthio group; a $C_2$-$C_{60}$ heteroarylalkyl group; a $C_2$-$C_{60}$ alkylheteroaryl group, a monovalent non-aromatic condensed polycyclic group, or a monovalent non-aromatic condensed heteropolycyclic group.

As used herein, a "coordinate bond" refers to a bond in which the bonding electrons are from one of the bonded atoms (i.e., a dative bond such as the Fe-CO bond in $Fe(CO)_5$).

As used herein, a "covalent bond" refers to a bond in which the bonding electrons are from each of the bonded atoms.

Hereinafter, a compound and an organic light-emitting device according to an embodiment will be described in detail with reference to Synthesis Examples and Examples, however, the present disclosure is not limited thereto. The wording "B was used instead of A" used in describing Synthesis Examples means that an identical molar equivalent of B was used in place of A.

EXAMPLES

Synthesis Example 1: Synthesis of Compound 1

(1) Synthesis of Intermediate (A)

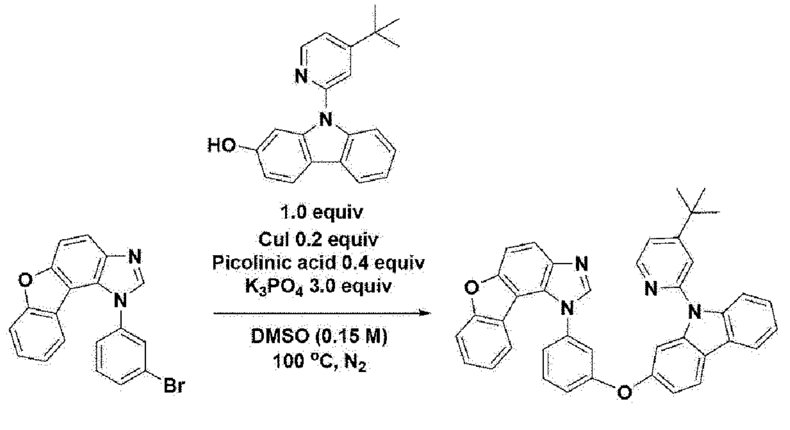


(A)

5.0 grams (g) (13.76 millimoles, mmol) of 1-(3-bromophenyl)-1H-benzo[2,3]benzofuro[4,5-d]imidazole, 4.35 g (13.76 mmol) of 9-(4-(tert-butyl)pyridin-2-yl)-9H-carbazol-2-ol, 0.52 g (2.75 mmol) of copper(I) iodide, 0.68 g (5.5 mmol) of picolinic acid, and 8.76 g (41.28 mmol) of potassium phosphate tribasic were mixed with 90 milliliters (mL) of dimethyl sulfoxide (DMSO), followed by stirring at a temperature of 100°C for 18 hours. Once the reaction was complete, the mixture was cooled to room temperature, and then, an organic layer was extracted using saturated ammonium chloride ($NH_4Cl$) and ethyl acetate (EA). The organic layer was dried with anhydrous magnesium sulfate ($MgSO_4$) and then filtered, followed by concentration under reduced pressure. The resulting product was purified by silica gel column chromatography to thereby obtain 4.04 g (8.26 mmol) of Intermediate (A) (yield: 60 %).

LC-Mass (calculated value (m/z): 598.24 grams per mole (g/mol), measured value: $M^{+1}$ = 599 g/mol).

(2) Synthesis of Intermediate (B)

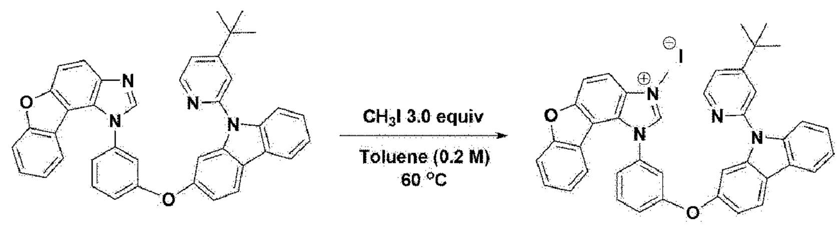


(A) (B)

4.04 g (8.26 mmol) of Intermediate (A) and 3.52 g (24.78 mmol) of methyl iodide were mixed with 40 mL of toluene, followed by stirring at a temperature of 60°C for 12 hours. Once the reaction was complete, the mixture was cooled to room temperature, and the resulting product was purified by silica gel column chromatography to thereby obtain 4.89 g (6.61 mmol) of Intermediate (B) (yield: 80 %).

(3) Synthesis of Compound 1

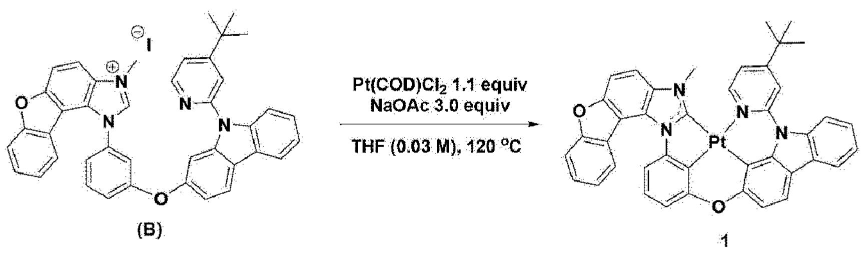


(B) 1

4.89 g (6.61 mmol) of Intermediate (B), 2.72 g (7.27 mmol) of Pt(COD)$Cl_2$ (dichloro(1,5-cyclooctadiene)platinum(II)), and 1.63 g (19.83 mmol) of sodium acetate (NaOAc) were mixed with 220 mL of tetrahydrofuran (THF), followed by stirring at a temperature of 120°C for 48 hours. Once the reaction was complete, the mixture was cooled to room temperature, and then, an organic layer was extracted using saturated ammonium chloride ($NH_4Cl$) and dichloromethane (DCM). The organic layer was dried with anhydrous magnesium sulfate ($MgSO_4$) and filtered, followed by concentration under reduced pressure. The resulting product was purified by silica gel column chromatography to thereby obtain 1.87 g (2.31 mmol) of Compound 1 (yield: 35 %).
LC-Mass (calculated value (m/z): 805.20 g/mol, measured value: $M^{+1}$ = 806 g/mol).

Synthesis Example 2: Synthesis of Compound 49

(1) Synthesis of Intermediate (C)

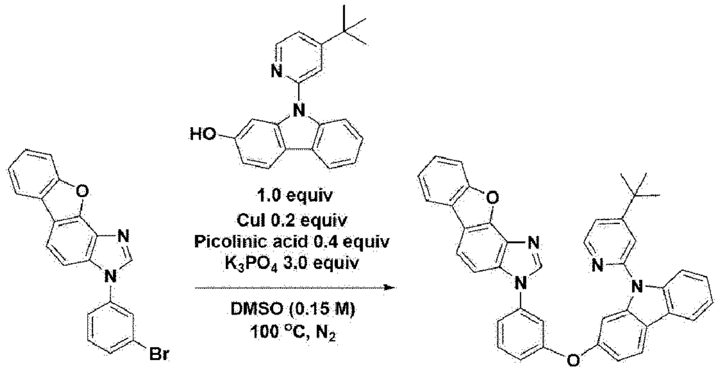


(C)

5.0 g (13.76 mmol) of 3-(3-bromophenyl)-3H-benzo[2,3]benzofuro[6,7-d]imidazole, 4.35 g (13.76 mmol) of 9-(4-(tert-butyl)pyridin-2-yl)-9H-carbazol-2-ol, 0.52 g (2.75 mmol) of copper(I) iodide, 0.68 g (5.5 mmol) of picolinic acid, and 8.76 g (41.28 mmol) of potassium phosphate tribasic were mixed with 90 mL of DMSO, followed by stirring at a temperature of 100°C for 18 hours. Once the reaction was complete, the mixture was cooled to room temperature, and then, an organic layer was extracted using saturated ammonium chloride ($NH_4Cl$) and ethyl acetate (EA). The organic layer was dried with anhydrous magnesium sulfate ($MgSO_4$) and filtered, followed by concentration under reduced pressure. The resulting product was purified by silica gel column chromatography to thereby obtain 4.38 g (8.94 mmol) of Intermediate (C) (yield: 65 %).
LC-Mass (calculated value (m/z): 598.24 g/mol, measured value: $M^{+1}$ = 599 g/mol).

(2) Synthesis of Intermediate (D)

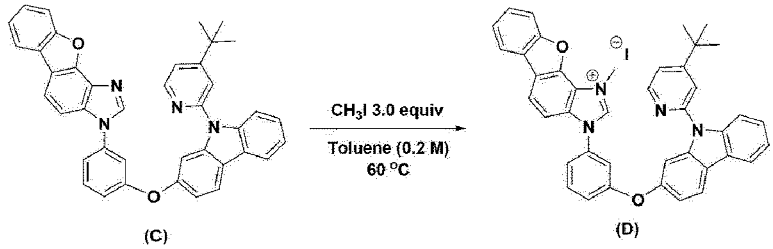


(C) (D)

4.38 g (8.94 mmol) of Intermediate (C) and 3.81 g (26.82 mmol) of methyl iodide were mixed with 40 mL of toluene, followed by stirring at a temperature of 60°C for 12 hours. Once the reaction was complete, the mixture was cooled to room temperature, and the resulting product was purified by silica gel column chromatography to thereby obtain 5.36 g (7.24 mmol) of Intermediate (D) (yield: 81 %).

(3) Synthesis of Compound 49

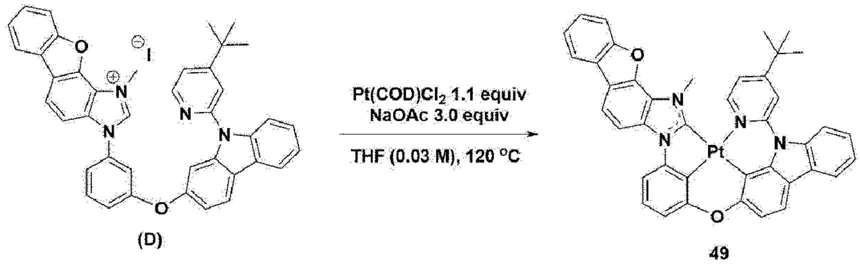


5.36 g (7.24 mmol) of Intermediate (D), 2.98 g (7.96 mmol) of $Pt(COD)Cl_2$, and 1.76 g (21.72 mmol) of sodium acetate were mixed with 230 mL of tetrahydrofuran (THF), followed by stirring at a temperature of 120°C for 48 hours. Once the reaction was complete, the mixture was cooled to room temperature, and then, an organic layer was extracted using saturated ammonium chloride ($NH_4Cl$) and dichloromethane (DCM). The organic layer was dried with anhydrous magnesium sulfate ($MgSO_4$) and filtered, followed by concentration under reduced pressure. The resulting product was purified by silica gel column chromatography to thereby obtain 2.04 g (2.53 mmol) of Compound 49 (yield: 35 %).
LC-Mass (calculated value (m/z): 805.20 g/mol, measured value: $M^{+1}$ = 806 g/mol).

Evaluation Example 1: Evaluation of maximum emission wavelength (color-coordinate) and full width at half maximum (FWHM)

Compound 1 was diluted in toluene at a concentration of 10 millimoles per liter (millimolar, mM), and a PL spectrum of Compound 1 was measured at room temperature by using an ISC PC1 spectrofluorometer, in which a xenon lamp is mounted. From the results, the maximum emission wavelength, color-coordinate, and FWHM of Compound 1 were evaluated. This process was performed on Compounds 49 and A. The results thereof are also shown in Table 2.

Evaluation Example 2: Measurement of decay time

Compound 1 was co-vacuum-deposited at a vacuum degree of $10^{-7}$ torr (wherein 1 torr =133.322 Pa) at a weight ratio of 2 wt% with the hosts used in the Examples on a quartz substrate to form a film having a thickness of 40 nm.
The PL spectrum of each film was evaluated at room temperature by using a time-resolved photoluminescence (TRPL) measurement system, FluoTime 300 (available from PicoQuant), and a pumping source, PLS340 (available from PicoQuant, excitation wavelength = 340 nm, spectral width = 20 nm). Then, a wavelength of the main peak in the PL spectrum was determined, and upon photon pulses (pulse width = 500 picoseconds, ps) applied to the film by PLS340, the number of photons emitted at the wavelength of the main peak for each film was repeatedly measured over time by time-correlated single photon counting (TCSPC), thereby obtaining TRPL curves available for the sufficient fitting. Based on the results obtained therefrom, one or more exponential decay functions were set forth for the fitting, thereby obtaining $T_{decay}$(Ex), i.e., a decay time, for each film. The results thereof are shown in Table 2. The function used for the fitting is as described in Equation 1, and the average value of $T_{decay}$ values for each of the exponential decay functions used for the fitting was taken as $T_{decay}$(Ex), i.e., a decay time. Here, during the same measurement time as the measurement time for obtaining TRPL curves, the same measurement was repeated once more in a dark state (i.e., a state where a pumping signal incident on each of the films was blocked), thereby obtaining a baseline or a background signal curve available as a baseline for the fitting: This same process was performed on Compounds 49 and A. The results thereof are shown in Table 2.

Equation 1

$$f(t) = \sum_{i=1}^{n} A_i \, exp(-t/T_{decay,i})$$

Evaluation Example 3: Evaluation of PL quantum yield (PLQY)

$CH_2Cl_2$ solution of PMMA, 5 wt% of CBP, and Compound 1 were mixed together. The resultant obtained therefrom was coated on a quartz substrate by using a spin coater, heat-treated in an oven at a temperature of 80°C , and cooled to room temperature, thereby obtaining a film.

The photoluminescence quantum yield (PLQY) of the film was evaluated by using Hamamatsu Photonics absolute PL quantum yield measurement system employing PLQY measurement software (Hamamatsu Photonics, Ltd., Shizuoka, Japan), in which a xenon light source, a monochromator, a photonic multichannel analyzer, and an integrating sphere are mounted. Thus, the PLQY of Compound 1 was evaluated. This process was also performed on Compounds 49 and A. The results thereof are shown in Table 2.

Table 2

| Compound | Maximum emission wavelength (nm) | Color coordinate (CIEx,y) | FWHM (nm) | Decay time (μs) | PLQY |
|---|---|---|---|---|---|
| Compound A | 465 nm | (0.14, 0.20) | 43 | 2.65 | 0.599 |
| Compound 1 | 459 nm | (0.14, 0.12) | 22 | 2.16 | 0.621 |
| Compound 49 | 457 nm | (0.14, 0.12) | 21 | 2.55 | 0.696 |

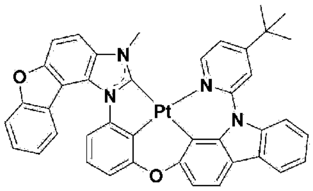

1

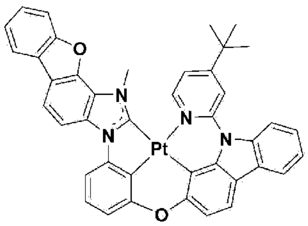

49

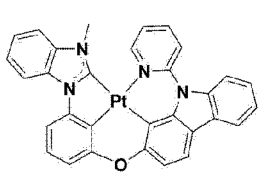

A

Referring to the results of Table 2, it was found that Compounds 1 and 49 each had excellent characteristics such as a narrow FHWM, a short decay time, and a high emission quantum efficiency, as compared with Compound A.

Example 1

An ITO glass substrate was cut to a size of 50 millimeters (mm) × 50 mm × 0.5 mm. Then the glass substrate was sonicated separately in acetone, isopropyl alcohol, and then pure water for about 15 minutes in each solvent and cleaned by exposure to ultraviolet irradiation with ozone for 30 minutes.

Thereafter, a hole injection layer was formed to have a thickness of 600 Å on the ITO electrode (anode) on the glass substrate by depositing m-MTDATA at a rate of about 1 Å/sec. A hole transport layer was formed to have a thickness of 250 Å on the hole injection layer by depositing α-NPD at a rate of 1 Å/sec.

An emission layer was formed to have a thickness of 400 Å on the hole transport layer by co-depositing Compound 1 (as a dopant) and CBP (as a host) at a deposition rate of 0.1 Å/sec and 1 Å/sec, respectively.

A hole blocking layer was formed on the emission layer by depositing BAlq at a rate of 1 Å/sec to have a thickness of 50 Å. Then, an electron transport layer was formed on the hole blocking layer by depositing $Alq_3$ to have a thickness of 300 Å. An electron injection layer was formed on the electron transport layer by depositing LiF to have a thickness of 10 Å. A second electrode (cathode) was formed on the electron injection layer by vacuum-depositing Al to have a thickness of 1,200 Å. Therefore, the manufacture of an organic light-emitting device was completed, in which the organic light-emitting device included an ITO/m-MTDATA (600 Å)/α-NPD (250 Å)/CBP+10% Compound 1 (400 Å)/BAlq (50 Å)/$Alq_3$ (300 Å)/LiF (10 Å)/Al (1,200 Å) structure.

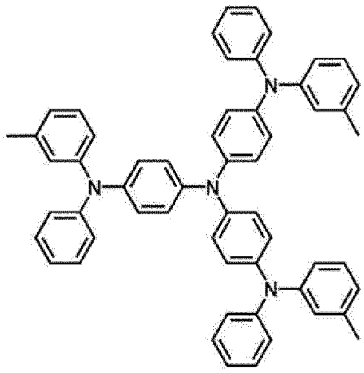

m-MTDATA

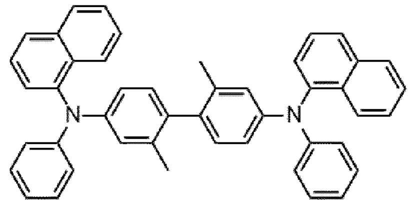

α-NPD

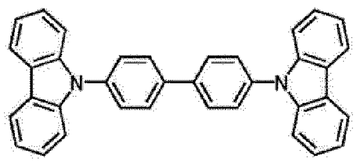

CBP

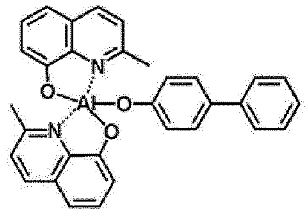

BAlq

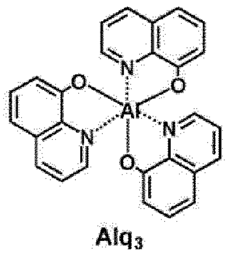

$Alq_3$

Example 2 and Comparative Examples 1 and 2

Organic light-emitting devices were manufactured in substantially the same manner as in Example 1, except that the compounds listed in Table 3 were used instead of Compound 1 as a dopant in the formation of an emission layer.

Evaluation Example 4: Evaluation of characteristics of organic light-emitting device

The EL spectrum, driving voltage, and external quantum emission efficiency of the organic light-emitting devices manufactured in Examples 1 and 2 and Comparative Examples 1 and 2 were measured. The measurement method is as follows. The results thereof are shown in Table 3. The values of the driving voltage and the external quantum efficiency in Table 3 are shown in a relative value (%), as compared with the driving voltage and the external quantum efficiency of the organic light-emitting device in Comparative Example 2.

(1) Measurement of current density depending on applied voltages

The current of the prepared organic light-emitting devices were measured as values of current in a unit device thereof using a current voltmeter (Keithley 2400) while increasing the applied voltage from 0 volts (V) to 10 V. The result was obtained by dividing a current value by an area.

(2) Measurement of luminance depending on applied voltages

The luminance of the prepared organic light-emitting devices were measured by using a luminance meter (Minolta Cs-1000A) while increasing the applied voltage from 0 V to 10 V.

(3) Measurement of EL spectra

The EL spectra of the manufactured organic light-emitting devices at a luminance of about 500 candelas per square meter ($cd/m^2$) were measured by using a luminance meter (Minolta Cs-1000A). Then, the maximum emission wavelength was evaluated.

(4) Measurement of external quantum efficiency

A Keithley 2400 current voltmeter and a luminance meter (Minolta Cs-1000A) were used in evaluation.

The EL spectrum, driving voltage, and external quantum emission efficiency of the organic light-emitting devices manufactured in Examples 1 and 2 and Comparative Examples 1 and 2 are shown in Table 3.

Table 3

| No. | Dopant compound | Driving voltage (V) (relative %) | External quantum efficiency (relative %) | Maximum emission wavelength (nm) |
|---|---|---|---|---|
| Comparative Example 1 | Compound A | 98 % | 200 % | 460 |
| Example 1 | Compound 1 | 89 % | 324% | 459 |
| Example 2 | Compound 49 | 87 % | 284% | 459 |
| Comparative Example 2 | Compound D | 100 % | 100% | 442 |

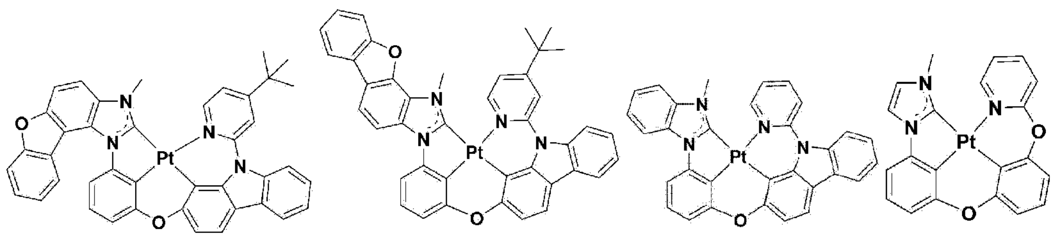

1 49 A D

Referring to the results of Table 3, the organic light-emitting devices manufactured in Examples 1 and 2 were found to have excellent external quantum efficiency and a low or equal level of driving voltage, as compared with the organic light-emitting devices manufactured in Comparative Examples 1 and 2.

As apparent from the foregoing description, the organometallic compound has a narrow FWHM and a short decay time, thus having improved quantum efficiency. Thus, an organic light-emitting device including the organometallic compound may have improved luminescent external quantum efficiency and a low driving voltage. Further, a diagnostic composition that includes the organometallic compound may have a high diagnostic efficiency, because the organometallic compound is excellent in phosphorescent emission characteristics.

It should be understood that embodiments described herein should be considered in a descriptive sense only and not for purposes of limitation. Descriptions of features or aspects within each embodiment should typically be considered as available for other similar features or aspects in other embodiments.

While one or more embodiments have been described with reference to the figures, it will be understood by those of ordinary skill in the art that various changes in form and details may be made therein without departing from the scope of the present disclosure as defined by the following claims.

## Claims

**1.** An organometallic compound represented by Formula 1:

Formula 1

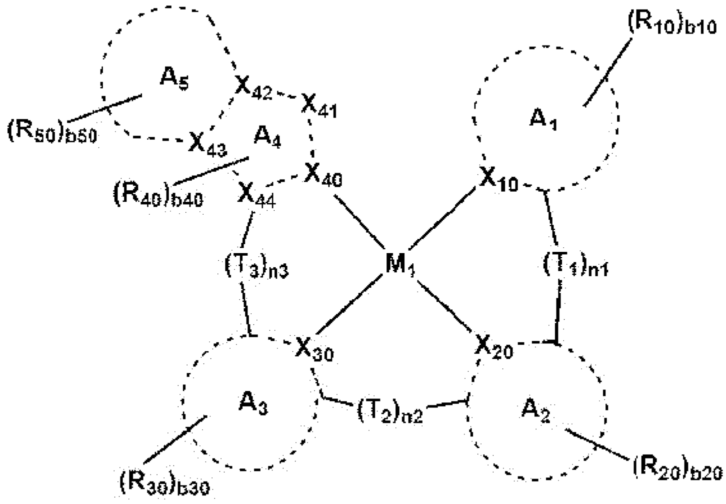

wherein, in Formula 1,

$M_1$ is beryllium, magnesium, aluminum, calcium, titanium, manganese, cobalt, copper, zinc, gallium, germanium, zirconium, ruthenium, rhodium, palladium, silver, rhenium, platinum, or gold,
$A_1$ to $A_3$ are each independently a $C_5$-$C_{30}$ carbocyclic group or a $C_1$-$C_{30}$ heterocyclic group,
$A_4$ is a 5-membered heterocyclic group,
$A_5$ is a $C_7$-$C_{30}$ carbocyclic group of at least two rings comprising a 6-membered carbocyclic group, or $A_5$ is a $C_1$-$C_{30}$ heterocyclic group of at least two rings comprising a 6-membered carbocyclic group or a 6-membered heterocyclic group,
$X_{10}$, $X_{20}$, $X_{30}$, and $X_{40}$ to $X_{44}$ are each independently C or N,
$T_1$ and $T_3$ are each independently a single bond, *-N[$(L_1)_{a1}$-$(R_1)_{b1}$]-*', *-B($R_1$)-*', *-P($R_1$)-*', *-C($R_1$)($R_2$)-*', *-Si($R_1$)($R_2$)-*', *-Ge($R_1$)($R_2$)-*', *-S-*', *-Se-*', *-O-*', *-C(=O)-*', *-S(=O)-*', *-S(=O)$_2$-*', *-C($R_1$)=C($R_2$)-*', *-C(=S)-*', or *-C≡C-*',
$T_2$ is a single bond, *-N[$(L_1)_{a1}$-$(R_1)_{b1}$]-*', *-P($R_1$)-*', *-C($R_1$)($R_2$)-*', *-Si($R_1$)($R_2$)-*', *-Ge($R_1$)($R_2$)-*', *-S-*', *-Se-*', *-O-*', *-C(=O)-*', *-S(=O)-*', *-S(=O)$_2$-*', *-C($R_1$)=C($R_2$)-*', *-C(=S)-*', or *-C≡C-*',
* and *' each indicate a binding site to an adjacent atom,
n1 to n3 are each independently an integer from 1 to 3,
$L_1$ is a single bond, a substituted or unsubstituted $C_5$-$C_{30}$ carbocyclic group, or a substituted or unsubstituted $C_1$-$C_{30}$ heterocyclic group,
a1 is an integer from 1 to 3, and when a1 is 2 or greater, at least two $L_1$ groups are identical to or different from each other,
$R_1$, $R_2$, $R_{10}$, $R_{20}$, $R_{30}$, $R_{40}$, and $R_{50}$ are each independently hydrogen, deuterium, -F, -Cl, -Br, -I, -$SF_5$, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_6$-$C_{60}$ aryloxy group, a substituted or unsubstituted $C_6$-$C_{60}$ arylthio group, a substituted or unsubstituted $C_7$-$C_{60}$ arylalkyl group, a substituted or unsubstituted $C_1$-$C_{60}$ heteroaryl group, a substituted or unsubstituted $C_1$-$C_{60}$ heteroaryloxy group, a substituted or unsubstituted $C_1$-$C_{60}$ heteroarylthio group, a substituted or unsubstituted $C_2$-$C_{60}$ heteroarylalkyl group, a substituted or unsubstituted monovalent non-aromatic condensed polycyclic group, a substituted or unsubstituted monovalent non-aromatic condensed heteropolycyclic group, -N($Q_1$)($Q_2$), -Si($Q_3$)($Q_4$)($Q_5$), -Ge($Q_3$)($Q_4$)($Q_5$), - B($Q_6$)($Q_7$), -P($Q_8$)($Q_9$), or -P(=O)($Q_8$)($Q_9$),
at least two adjacent $R_1$, $R_2$, $R_{10}$, $R_{20}$, $R_{30}$, $R_{40}$, or $R_{50}$ groups are optionally bound together to form a substituted or unsubstituted $C_5$-$C_{30}$ carbocyclic group or a substituted or unsubstituted $C_1$-$C_{30}$ heterocyclic group,
b1 is an integer from 1 to 5, and when b1 is 2 or greater, at least two $R_1$ groups are identical to or different from each other,

b10, b20, b30, and b50 are each independently an integer from 1 to 10,
b40 is an integer from 1 to 3,
when b10 is 2 or greater, at least two $R_{10}$ groups are identical to or different from each other, when b20 is 2 or greater, at least two $R_{20}$ groups are identical to or different from each other, when b30 is 2 or greater, at least two $R_{30}$ groups are identical to or different from each other, when b40 is 2 or greater, at least two $R_{40}$ groups are identical to or different from each other, when b50 is 2 or greater, at least two $R_{50}$ groups are identical to or different from each other, and
at least one substituent of the substituted $C_5$-$C_{30}$ carbocyclic group, the substituted $C_1$-$C_{30}$ heterocyclic group, the substituted $C_1$-$C_{60}$ alkyl group, the substituted $C_2$-$C_{60}$ alkenyl group, the substituted $C_2$-$C_{60}$ alkynyl group, the substituted $C_1$-$C_{60}$ alkoxy group, the substituted $C_3$-$C_{10}$ cycloalkyl group, the substituted $C_1$-$C_{10}$ heterocycloalkyl group, the substituted $C_3$-$C_{10}$ cycloalkenyl group, the substituted $C_1$-$C_{10}$ heterocycloalkenyl group, the substituted $C_6$-$C_{60}$ aryl group, the substituted $C_6$-$C_{60}$ aryloxy group, the substituted $C_6$-$C_{60}$ arylthio group, the substituted $C_7$-$C_{60}$ arylalkyl group, the substituted $C_1$-$C_{60}$ heteroaryl group, the substituted $C_1$-$C_{60}$ heteroaryloxy group, the substituted $C_1$-$C_{60}$ heteroarylthio group, the substituted $C_2$-$C_{60}$ heteroarylalkyl group, the substituted monovalent non-aromatic condensed polycyclic group, or the substituted monovalent non-aromatic condensed heteropolycyclic group is:

deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, or a $C_1$-$C_{60}$ alkoxy group;
a $C_1$-$C_{60}$ alkyl group, a $C_2$-$C_{60}$ alkenyl group, a $C_2$-$C_{60}$ alkynyl group, or a $C_1$-$C_{60}$ alkoxy group, each substituted with at least one selected from deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_7$-$C_{60}$ arylalkyl group, a $C_1$-$C_{60}$ heteroaryl group, a $C_1$-$C_{60}$ heteroaryloxy group, a $C_1$-$C_{60}$ heteroarylthio group, a $C_2$-$C_{60}$ heteroarylalkyl group, a monovalent non-aromatic condensed polycyclic group, a monovalent non-aromatic condensed heteropolycyclic group, $-N(Q_{11})(Q_{12})$, $-Si(Q_{13})(Q_{14})(Q_{15})$, $-Ge(Q_{13})(Q_{14})(Q_{18})$, $-B(Q_{16})(Q_{17})$, $-P(Q_{18})(Q_{19})$, or $-P(=O)(Q_{18})(Q_{19})$;
a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, or a monovalent non-aromatic condensed heteropolycyclic group;
a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, or a monovalent non-aromatic condensed heteropolycyclic group, each substituted with at least one of deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_7$-$C_{60}$ arylalkyl group, a $C_1$-$C_{60}$ heteroaryl group, a $C_1$-$C_{60}$ heteroaryloxy group, a $C_1$-$C_{60}$ heteroarylthio group, a $C_2$-$C_{60}$ heteroarylalkyl group, a monovalent non-aromatic condensed polycyclic group, a monovalent non-aromatic condensed heteropolycyclic group, $-N(Q_{21})(Q_{22})$, $-Si(Q_{23})(Q_{24})(Q_{25})$, $-Ge(Q_{23})(Q_{24})(Q_{25})$, $-B(Q_{26})(Q_{27})$, $-P(Q_{28})(Q_{29})$, or $-P(=O)(Q_{28})(Q_{29})$; or
$-N(Q_{31})(Q_{32})$, $-Si(Q_{33})(Q_{34})(Q_{35})$, $-Ge(Q_{33})(Q_{34})(Q_{35})$, $-B(Q_{36})(Q_{37})$, $-P(Q_{38})(Q_{39})$, or $-P(=O)(Q_{38})(Q_{39})$,
wherein $Q_1$ to $Q_9$, $Q_{11}$ to $Q_{19}$, $Q_{21}$ to $Q_{29}$, and $Q_{31}$ to $Q_{39}$ are each independently hydrogen; -F; -Cl; -Br; -I; a hydroxyl group; a cyano group; a nitro group; an amidino group; a hydrazine group; a hydrazone group; a carboxylic acid group or a salt thereof; a sulfonic acid group or a salt thereof; a phosphoric acid group or a salt thereof; a $C_1$-$C_{60}$ alkyl group; a $C_2$-$C_{60}$ alkenyl group; a $C_2$-$C_{60}$ alkynyl group; a $C_1$-$C_{60}$ alkoxy group; a $C_3$-$C_{10}$ cycloalkyl group; a $C_1$-$C_{10}$ heterocycloalkyl group; a $C_3$-$C_{10}$ cycloalkenyl group; a $C_1$-$C_{10}$ heterocycloalkenyl group a $C_6$-$C_{60}$ aryl group; a $C_6$-$C_{60}$ aryl group substituted with a $C_1$-$C_{60}$ alkyl group, a $C_6$-$C_{60}$ aryl group, or a combination thereof; a $C_6$-$C_{60}$ aryloxy group; a $C_6$-$C_{60}$ arylthio group; a $C_7$-$C_{60}$ arylalkyl group; a $C_1$-$C_{60}$ heteroaryl group; a $C_1$-$C_{60}$ heteroaryloxy group; a $C_1$-$C_{60}$ heteroarylthio group; a $C_2$-$C_{60}$ heteroarylalkyl group; a monovalent non-aromatic condensed polycyclic group; or a monovalent

non-aromatic condensed heteropolycyclic group.

**2.** The organometallic compound of claim 1, wherein $M_1$ is Pt, Pd, or Au; and/or
wherein $A_1$ to $A_3$ are each independently a phenyl group, a naphthyl group, an anthracenyl group, a phenanthrenyl group, a triphenylenyl group, a pyrenyl group, a chrysenyl group, a cyclopentadiene group, a 1,2,3,4-tetrahydronaphthyl group, a furanyl group, a thiophenyl group, a silolyl group, an indenyl group, a fluorenyl group, an indolyl group, an isoindolyl group, a carbazolyl group, benzocarbazolyl group, a dibenzocarbazolyl group, a benzofuranyl group, a dibenzofuranyl group, a benzothiophenyl group, a benzosilolyl group, a dibenzosilolyl group, an azafluorenyl group, an azacarbazolyl group, an azadibenzofuranyl group, an azadibenzothiophenyl group, an azadibenzosilolyl group, a pyridinyl group, an imidazopyridinyl group, a pyrimidinyl group, an imidazopyrimidinyl group, a pyrazinyl group, a pyridazinyl group, a triazinyl group, a purinyl group, a quinolinyl group, a benzoquinolinyl group, an isoquinolinyl group, a quinoxalinyl group, a quinazolinyl group, a cinnolinyl group, a phenanthrolinyl group, a pyrrolyl group, a pyrazolyl group, an imidazolyl group, a triazolyl group, a tetrazolyl group, an oxazolyl group, an isoxazolyl group, a thiazolyl group, an isothiazolyl group, an oxadiazolyl group, a thiadiazolyl group, a benzopyrazolyl group, a benzimidazolyl group, an indazolyl group, a benzoxazolyl group, an isobenzoxazolyl group, a benzothiazolyl group, a benzoxadiazolyl group, a benzothiadiazolyl group, a benzotriazolyl group, a diazaindenyl group, a triazaindenyl group, a 5,6,7,8-tetrahydroisoquinolinyl group, or a 5,6,7,8-tetrahydroquinolinyl group.

**3.** The organometallic compound of claim 1 or 2, wherein $X_{40}$ in $A_4$ is C, and $X_{41}$ and $X_{44}$ are each N; and/or
wherein $A_5$ is represented by any one of Formulae A5-1 to A5-6:

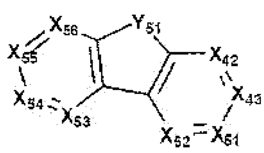

A5-1

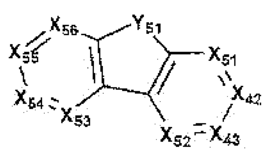

A5-2

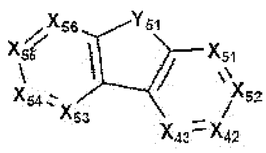

A5-3

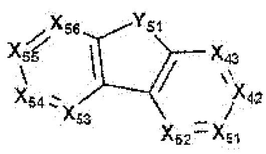

A5-4

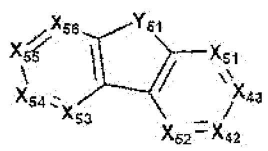

A5-5

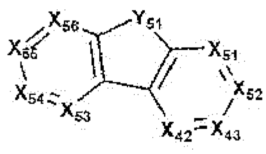

A5-6

wherein, in Formulae A5-1 to A5-6,

$Y_{51}$ is *-O-*', *-S-*', *-N($R_5$)-*', *-C($R_5$)($R_6$)-*', *-Si($R_5$)($R_6$)-*', *-B($R_5$)-*', *-P($R_5$)-*', or *-P(=O)($R_5$)-*', wherein * and *' each indicate a binding site to an adjacent atom, $R_5$ and $R_6$ are respectively understood by referring to $R_1$ and $R_2$ in claim 1, $X_{42}$ and $X_{43}$ are respectively understood by referring to $X_{42}$ and $X_{43}$ in claim 1, and $X_{51}$ to $X_{56}$ are each independently C or N.

**4.** The organometallic compound of any of claims 1 to 3, wherein $X_{10}$ is N, and $X_{20}$, $X_{30}$, and $X_{40}$ are each C; and/or

wherein
a bond between $M_1$ and $X_{10}$ is a coordinate bond,
a bond between $M_1$ and $X_{20}$ is a covalent bond,
a bond between $M_1$ and $X_{30}$ is a covalent bond, and
a bond between $M_1$ and $X_{40}$ is a coordinate bond; and/or
wherein $T_1$ to $T_3$ are each independently a single bond, *-N[($L_1$)$_{a1}$-($R_1$)$_{b1}$]-*', *-C($R_1$)($R_2$)-*', *-Si($R_1$)($R_2$)-*', *-O-*', or *-S-*', wherein * and *' each indicate a binding site to an adjacent atom.

**5.** The organometallic compound of any of claims 1 to 4, wherein $L_1$ is:

a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylenylene group, a naphthacenylene group, a picenylene group, a perylenylene group, or a pentacenylene group; or

a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylenylene group, a naphthacenylene group, a picenylene group, a perylenylene group, or a pentacenylene group, each substituted with at least one of deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_3$-$C_{10}$ cycloalkenyl group, a $C_1$-$C_{10}$ heterocycloalkyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, or a monovalent non-aromatic condensed heteropolycyclic group.

**6.** The organometallic compound of any of claims 1 to 5, wherein $R_1$, $R_2$, $R_{10}$, $R_{20}$, $R_{30}$, $R_{40}$, and $R_{50}$ are each independently:

hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, $-SF_5$, a $C_1$-$C_{20}$ alkyl group, or a $C_1$-$C_{20}$ alkoxy group;

a $C_1$-$C_{20}$ alkyl group or a $C_1$-$C_{20}$ alkoxy group, each substituted with at least one of deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{10}$ alkyl group, a $C_1$-$C_{10}$ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, or a pyrimidinyl group;

a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, or an imidazopyrimidinyl group;

a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, or an imidazopyrimidinyl group, each substituted with at least one of deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{10}$ alkyl group, a $C_1$-$C_{10}$ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, or a pyrimidinyl group; or

$-N(Q_1)(Q_2)$, $-Si(Q_3)(Q_4)(Q_5)$, $-Ge(Q_3)(Q_4)(Q_5)$, $-B(Q_6)(Q_7)$, $-P(Q_8)(Q_9)$, or $-P(=O)(Q_8)(Q_9)$, wherein $Q_1$ to $Q_9$ are each independently:

$-CH_3$, $-CD_3$, $-CD_2H$, $-CDH_2$, $-CH_2CH_3$, $-CH_2CD_3$, $-CH_2CD_2H$, $-CH_2CDH_2$, - $CHDCH_3$, $-CHDCD_2H$, $-CHDCDH_2$, $-CHDCD_3$, $-CD_2CD_3$, $-CD_2CD_2H$, or $-CD_2CDH_2$;
an n-propyl group, an iso-propyl group, an n-butyl group, an iso-butyl group, a sec-butyl group, a tert-butyl group, an n-pentyl group, an iso-pentyl group, a sec-pentyl group, a tert-pentyl group, a phenyl group, or a naphthyl group; or
an n-propyl group, an iso-propyl group, an n-butyl group, an isobutyl group, a sec-butyl group, a tert-butyl group, an n-pentyl group, an isopentyl group, a sec-pentyl group, a tert-pentyl group, a phenyl group, and a naphthyl group, each substituted with at least one selected from deuterium, a $C_1$-$C_{10}$ alkyl group, or a phenyl group.

**7.** The organometallic compound of any of claims 1 to 6, wherein $R_1$, $R_2$, $R_{10}$, $R_{20}$, $R_{30}$, $R_{40}$, and $R_{50}$ are each independently hydrogen, deuterium, -F, a cyano group, a nitro group, $-SF_5$, $-CH_3$, $-CD_3$, $-CD_2H$, $-CDH_2$, $-CF_3$, $-CF_2H$, $-CFH_2$, a group represented by any one of Formulae 9-1 to 9-19, or a group represented by any one of Formulae 10-1 to 10-194:

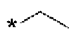
9-1
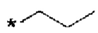
9-2
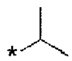
9-3
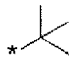
9-4
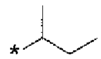
9-5
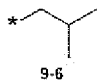
9-6
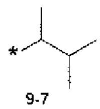
9-7

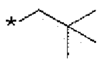
9-8
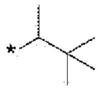
9-9
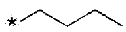
9-10
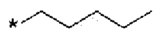
9-11
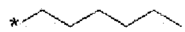
9-12

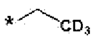
9-13
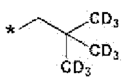
9-14
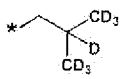
9-15
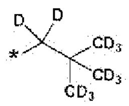
9-16
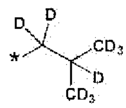
9-17
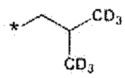
9-18

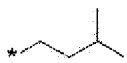
9-19

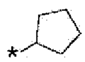
10-1
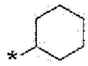
10-2
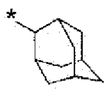
10-3
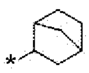
10-4
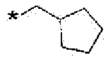
10-5
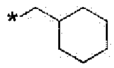
10-6
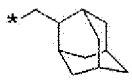
10-7

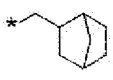
10-8
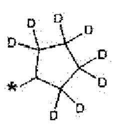
10-9
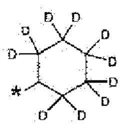
10-10
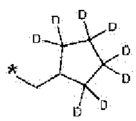
10-11
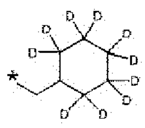
10-12
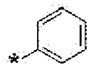
10-13

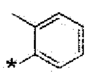
10-14
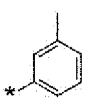
10-15
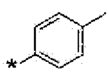
10-16
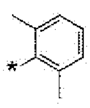
10-17
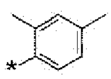
10-18
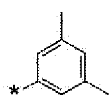
10-19
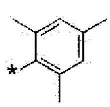
10-20

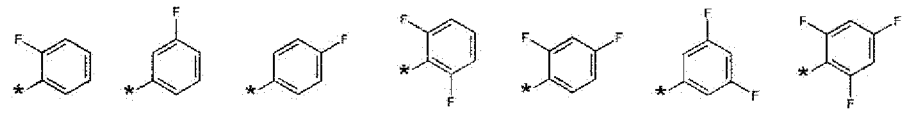

10-21 10-22 10-23 10-24 10-25 10-26 10-27

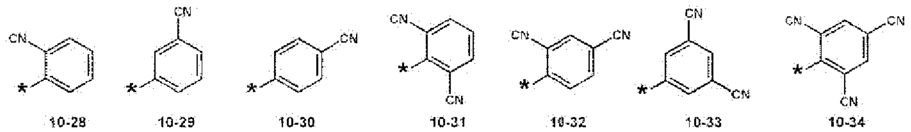

10-28 10-29 10-30 10-31 10-32 10-33 10-34

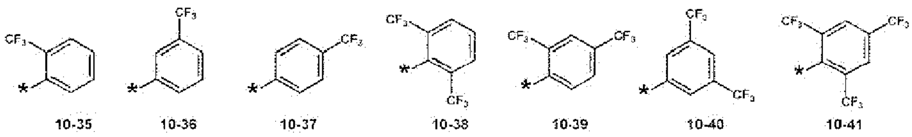

10-35 10-36 10-37 10-38 10-39 10-40 10-41

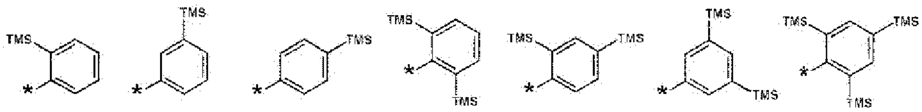

10-42 10-43 10-44 10-45 10-46 10-47 10-48

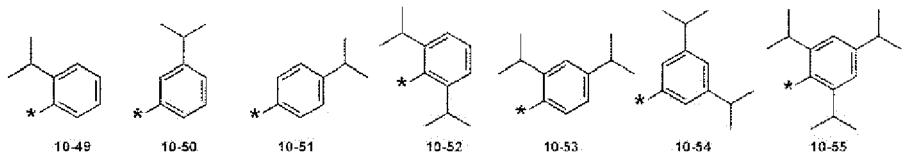

10-49 10-50 10-51 10-52 10-53 10-54 10-55

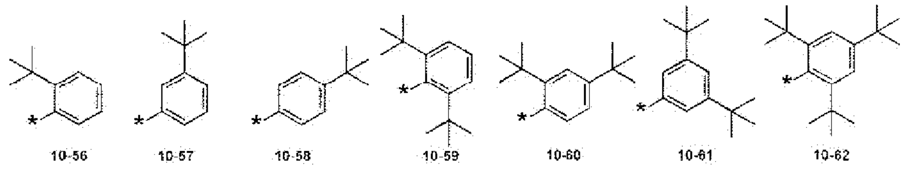

10-56 10-57 10-58 10-59 10-60 10-61 10-62

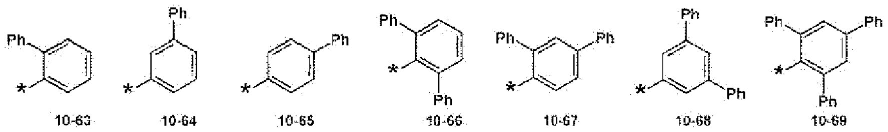

10-63 10-64 10-65 10-66 10-67 10-68 10-69

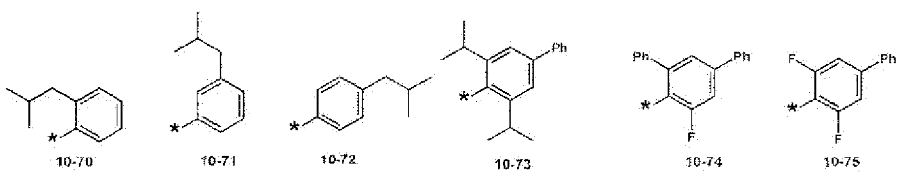

10-70 10-71 10-72 10-73 10-74 10-75

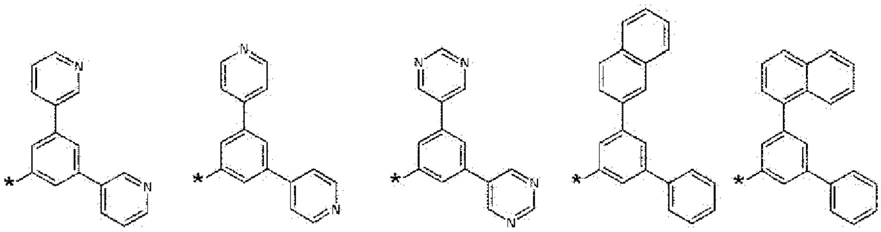

10-76 10-77 10-78 10-79 10-80

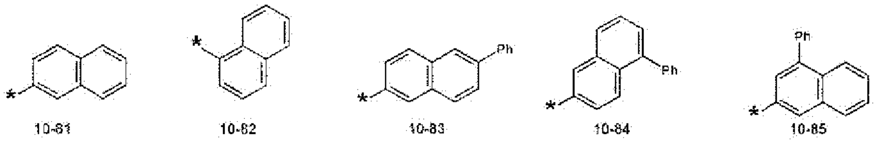

10-81 10-82 10-83 10-84 10-85

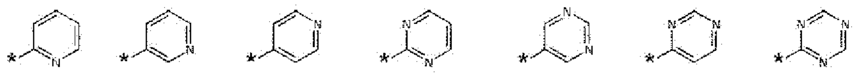

10-86 10-87 10-88 10-89 10-90 10-91 10-92

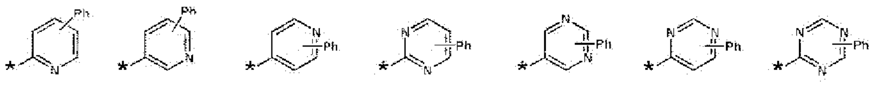

10-93 10-94 10-95 10-96 10-97 10-98 10-99

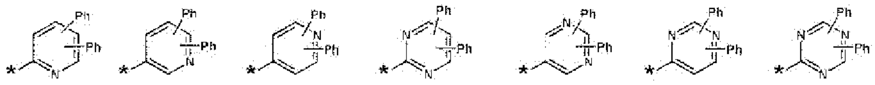

10-100 10-101 10-102 10-103 10-104 10-105 10-106

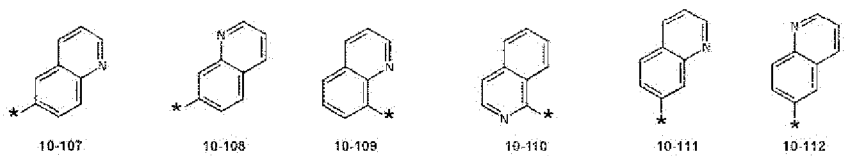

10-107 10-108 10-109 10-110 10-111 10-112

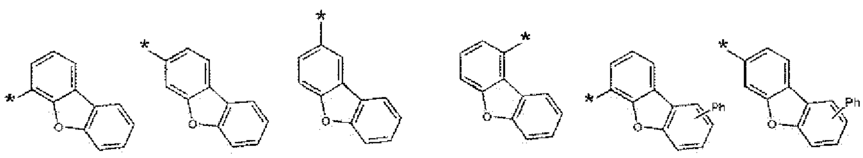

10-113 10-114 10-115 10-116 10-117 10-118

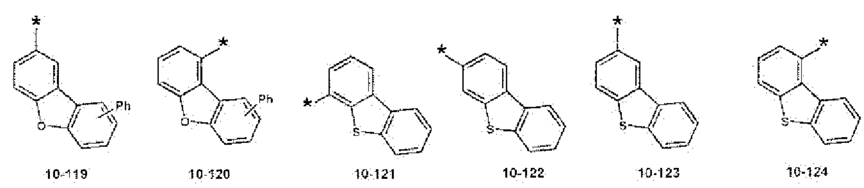

10-119 10-120 10-121 10-122 10-123 10-124

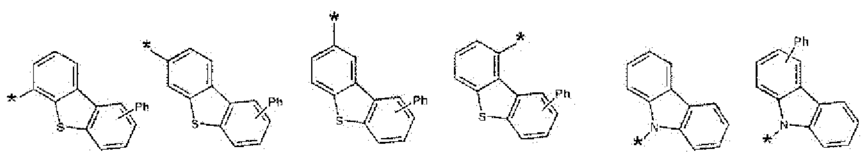

10-125 10-126 10-127 10-128 10-129 10-130

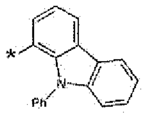
10-131
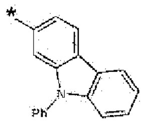
10-132
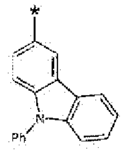
10-133
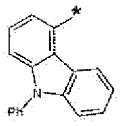
10-134
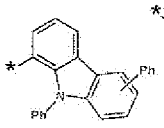
10-135
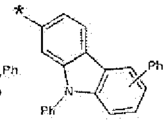
10-136

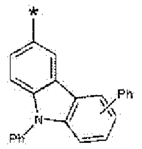
10-137
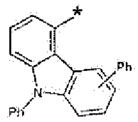
10-138
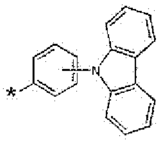
10-139
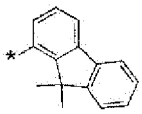
10-140
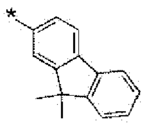
10-141
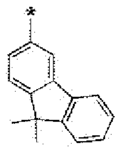
10-142

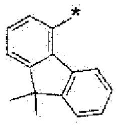
10-143
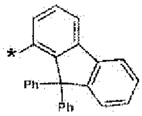
10-144
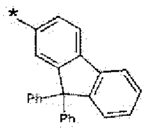
10-145
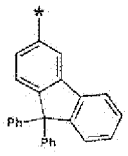
10-146
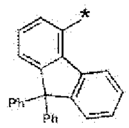
10-147

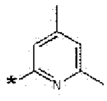
10-148
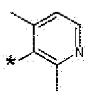
10-149
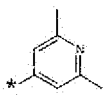
10-150
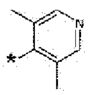
10-151
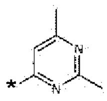
10-152
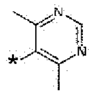
10-153
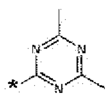
10-154

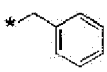
10-155
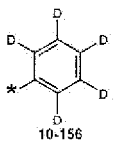
10-156
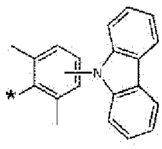
10-157
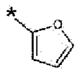
10-158
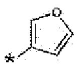
10-159
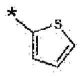
10-160
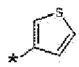
10-161

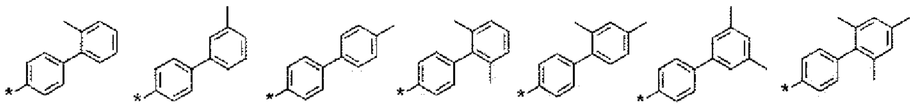
10-162 10-163 10-164 10-165 10-166 10-167 10-168

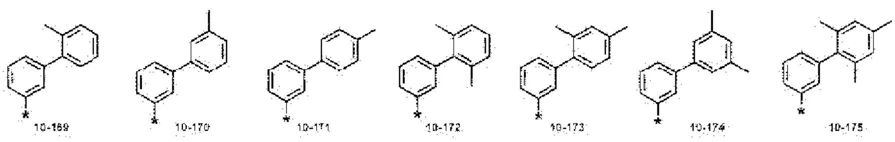
10-169 10-170 10-171 10-172 10-173 10-174 10-175

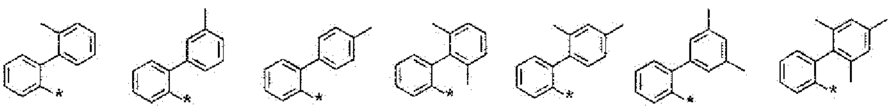
10-176 10-177 10-178 10-179 10-180 10-181 10-182

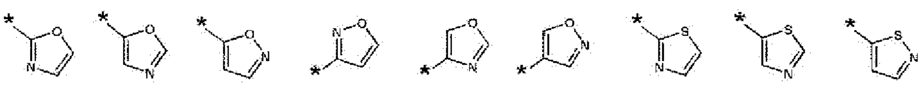
10-183 10-184 10-185 10-186 10-187 10-188 10-189 10-190 10-191

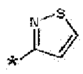
10-192

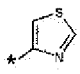
10-193

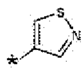
10-194

wherein, in Formulae 9-1 to 9-19 and 10-1 to 10-194, * indicates a binding site to an adjacent atom, "Ph" indicates a phenyl group, and "TMS" indicates a trimethylsilyl group.

**8.** The organometallic compound of any of claims 1 to 7, wherein the organometallic compound represented by Formula 1 is represented by any one of Formulae 11-1 to 11-6:

Formula 11-1

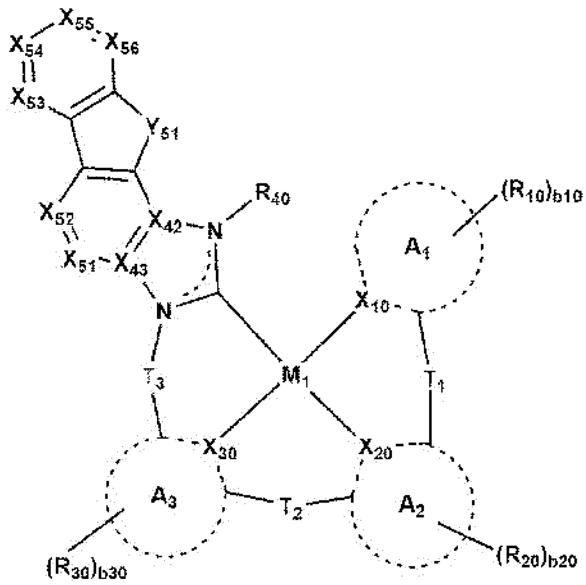

Formula 11-2

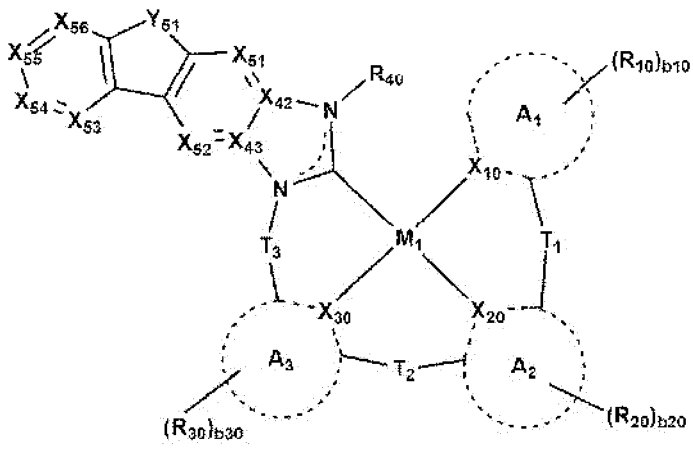

Formula 11-3

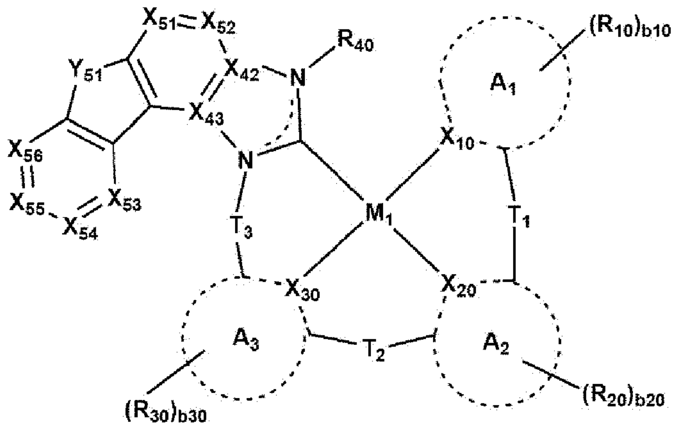

Formula 11-4

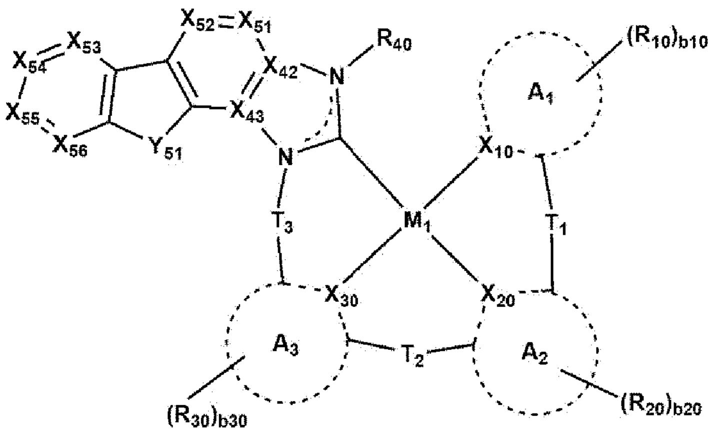

Formula 11-5

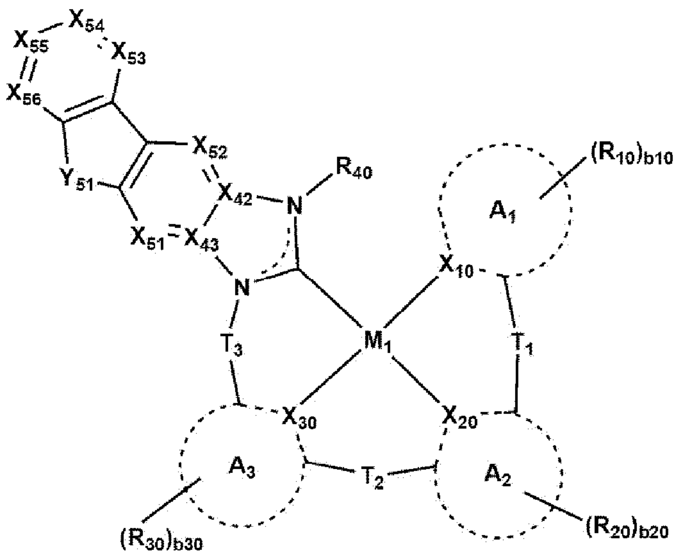

Formula 11-6

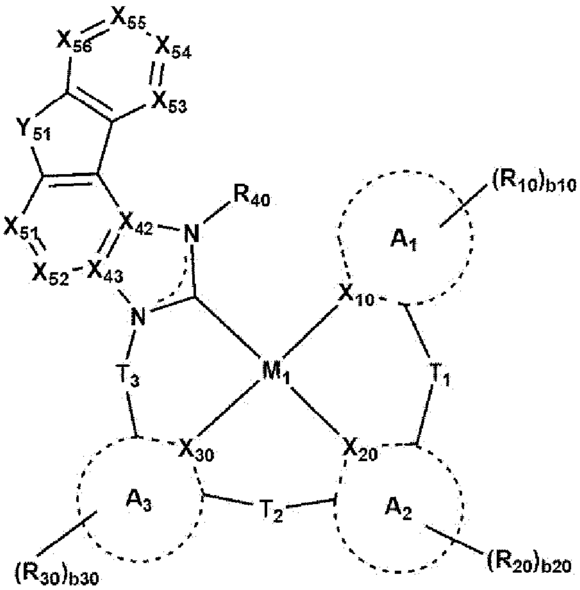

wherein, in Formulae 11-1 to 11-6,

$M_1$, $A_1$ to $A_3$, $X_{10}$, $X_{20}$, $X_{30}$, $X_{42}$, $X_{43}$, $T_1$ to $T_3$, $R_{10}$, $R_{20}$, $R_{30}$, $R_{40}$, $R_{50}$, b10, b20, and b30 are respectively understood by referring to the descriptions of $M_1$, $A_1$ to $A_3$, $X_{10}$, $X_{20}$, $X_{30}$, $X_{42}$, $X_{43}$, $T_1$ to $T_3$, $R_{10}$, $R_{20}$, $R_{30}$, $R_{40}$, $R_{50}$, b10, b20, and b30 in claim 1,

$Y_{51}$ is *-O-*', *-S-*', *-N($R_5$)-*', *-C($R_5$)($R_6$)-*', *-Si($R_5$)($R_6$)-*', *-B($R_5$)-*', *-P($R_5$)-*', or *-P(=O)($R_5$)-*', wherein * and *' each indicate a binding site to an adjacent atom,

$R_5$ and $R_6$ are respectively understood by referring to the descriptions of $R_1$ and $R_2$ in claim 1,

X51 is C($R_{51}$) or N, $X_{52}$ is C($R_{52}$) or N, $X_{53}$ is C($R_{53}$) or N, $X_{54}$ is C($R_{54}$) or N, $X_{55}$ is C($R_{55}$) or N, $X_{56}$ is C($R_{56}$) or N, and

$R_{51}$ to $R_{56}$ are each independently understood by referring to the description of $R_{50}$ in claim 1.

**9.** The organometallic compound of any of claims 1 to 8, wherein the organometallic compound represented by Formula 1 is represented by any one of Formulae 12-1 to 12-6:

Formula 12-1

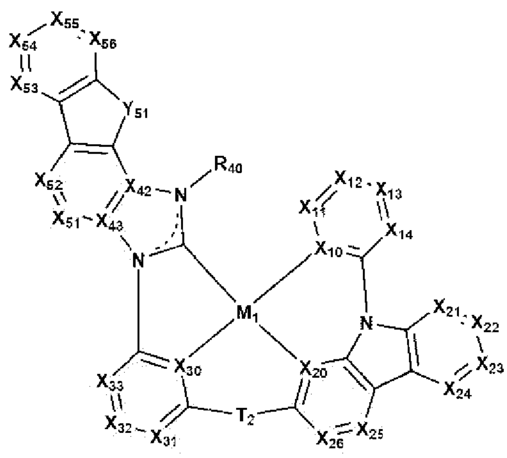

Formula 12-2

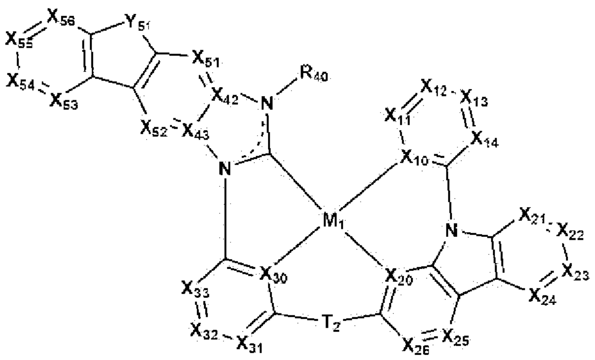

Formula 12-3

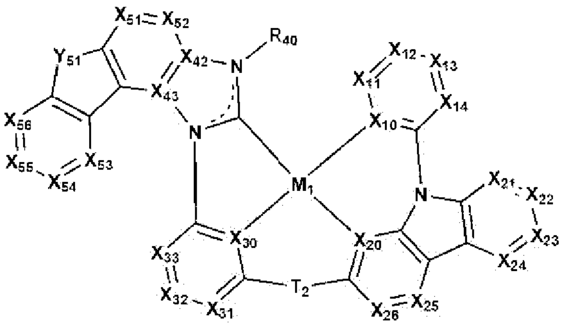

Formula 12-4

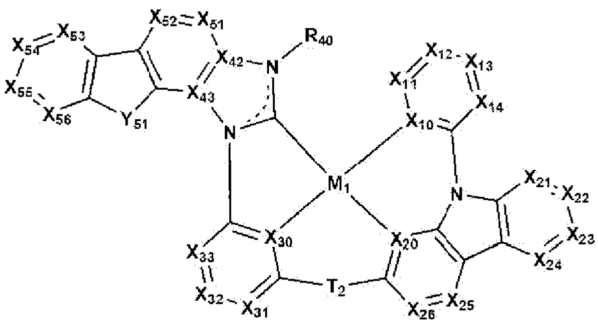

Formula 12-5

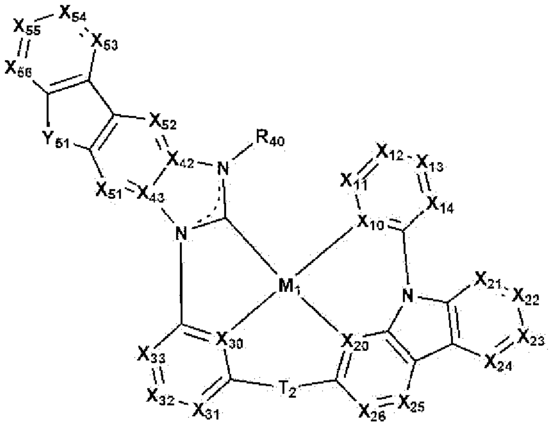

Formula 12-6

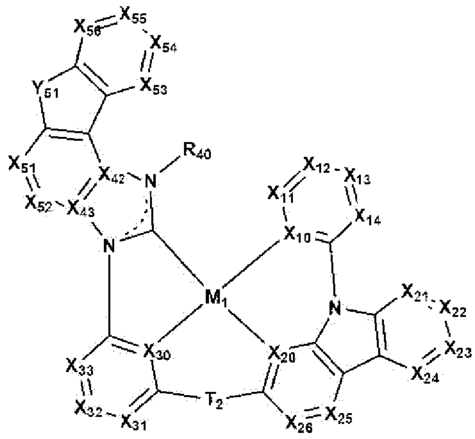

wherein, in Formulae 12-1 to 12-6,

$M_1$, $X_{10}$, $X_{20}$, $X_{30}$, $X_{42}$, $X_{43}$, $R_{40}$, and $T_2$ are respectively understood by referring to $M_1$, $X_{10}$, $X_{20}$, $X_{30}$, $X_{42}$, $X_{43}$, $R_{40}$, and $T_2$ in claim 1,
$Y_{51}$ is *-O-*', *-S-*', *-N($R_5$)-*', *-C($R_5$)($R_6$)-*', *-Si($R_5$)($R_6$)-*', *-B($R_5$)-*', *-P($R_5$)-*', or *-P(=O)($R_5$)-*', wherein * and *' each indicate a binding site to an adjacent atom,
$R_5$ and $R_6$ are respectively understood by referring to $R_1$ and $R_2$ in claim 1,
$X_{11}$ is C($R_{11}$) or N, $X_{12}$ is C($R_{12}$) or N, $X_{13}$ is C($R_{13}$) or N, and $X_{14}$ is C($R_{14}$) or N,
$X_{21}$ is C($R_{21}$) or N, $X_{22}$ is C($R_{22}$) or N, $X_{23}$ is C($R_{23}$) or N, $X_{24}$ is C($R_{24}$) or N, $X_{25}$ is C($R_{25}$) or N, and $X_{26}$ is C($R_{26}$) or N,
$X_{31}$ is C($R_{31}$) or N, $X_{32}$ is C($R_{32}$) or N, and $X_{33}$ is C($R_{33}$) or N,
$X_{51}$ is C($R_{51}$) or N, $X_{52}$ is C($R_{52}$) or N, $X_{53}$ is C($R_{53}$) or N, $X_{54}$ is C($R_{54}$) or N, $X_{55}$ is C($R_{55}$) or N, and $X_{56}$ is C($R_{56}$) or N,
$R_{11}$ to $R_{14}$ are each independently understood by referring to $R_{10}$ in claim 1,
$R_{21}$ to $R_{26}$ are each independently understood by referring to $R_{20}$ in claim 1,
$R_{31}$ to $R_{33}$ are each independently understood by referring to $R_{30}$ in claim 1, and
$R_{51}$ to $R_{56}$ are each independently understood by referring to $R_{50}$ in claim 1.

**10.** The organometallic compound of any of claims 1 to 9, wherein the organometallic compound is any one of Compounds 1 to 72:

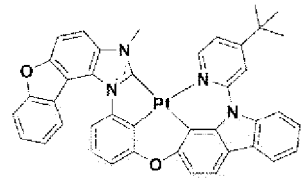

1

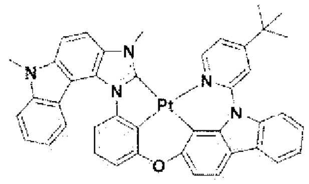

2

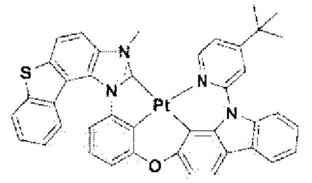

3

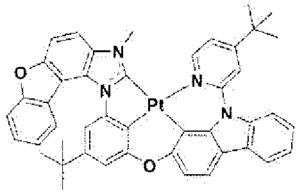

4

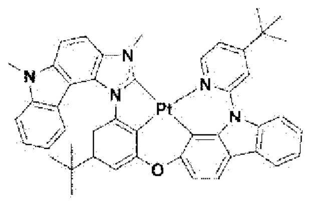

5

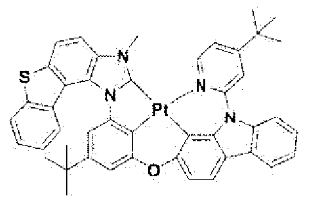

6

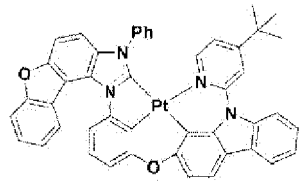
7

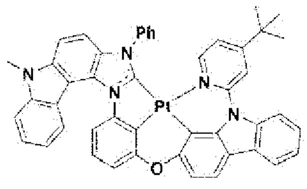
8

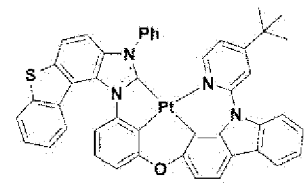
9

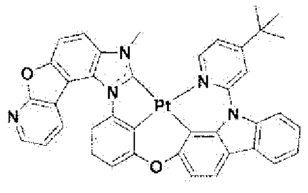
10

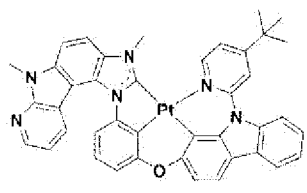
11

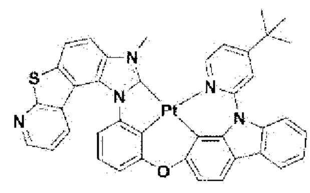
12

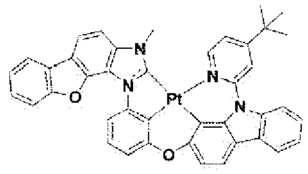
13

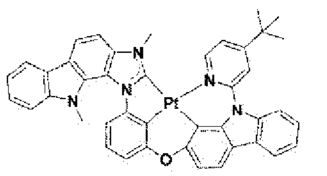
14

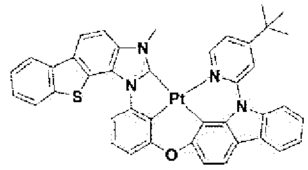
15

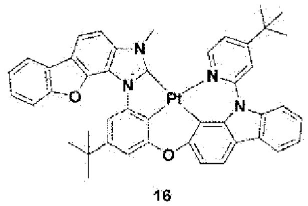
16

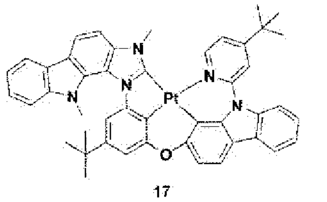
17

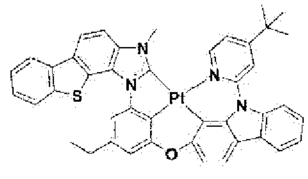
18

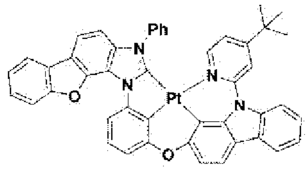
19

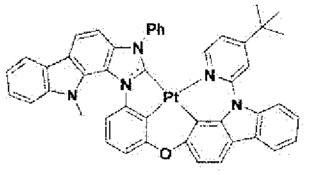
20

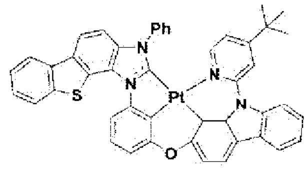
21

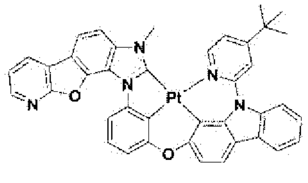
22

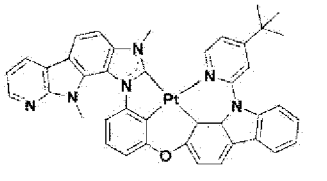
23

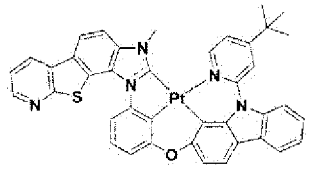
24

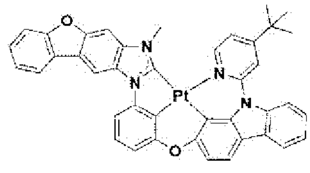
25

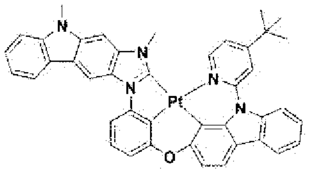
26

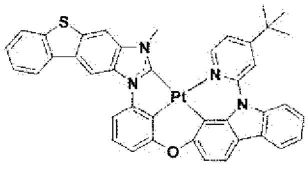
27

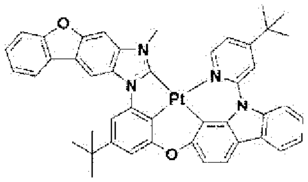
28
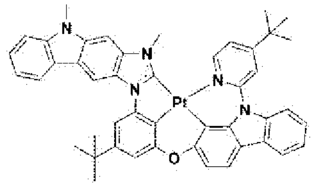
29
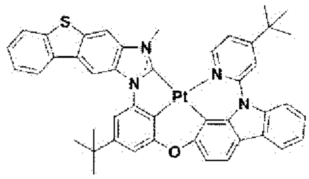
30

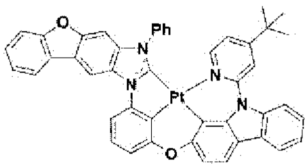
31
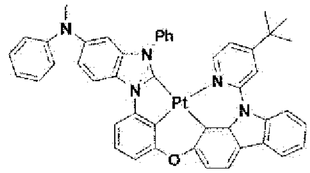
32
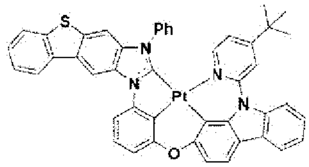
33

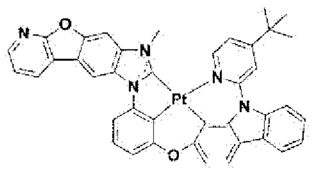
34
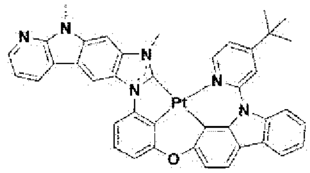
35
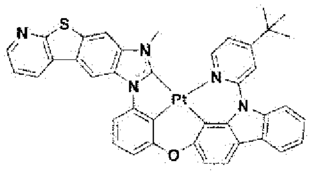
36

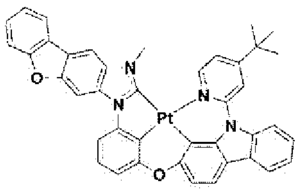
37
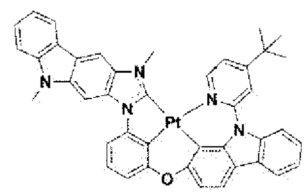
38
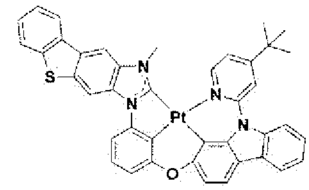
39

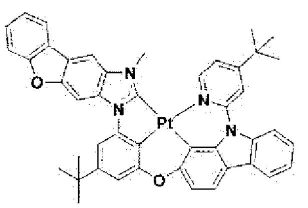
40
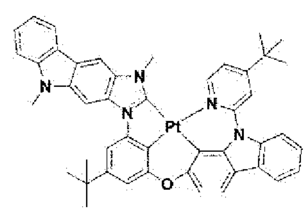
41
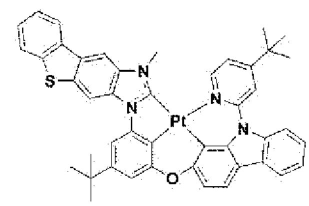
42

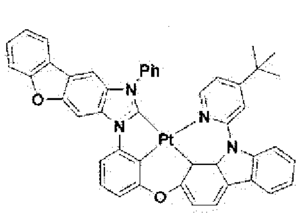
43
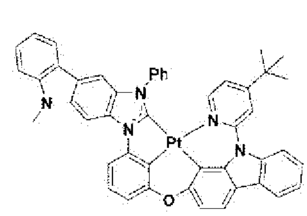
44
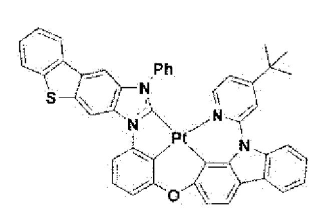
45

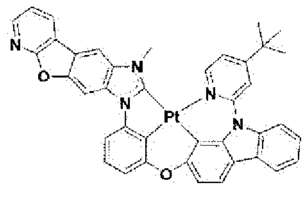
46

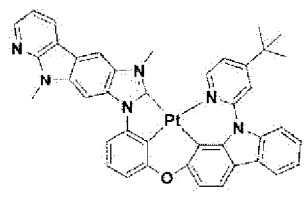
47

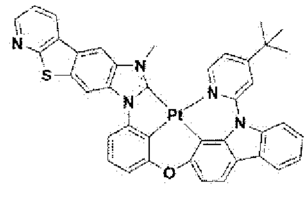
48

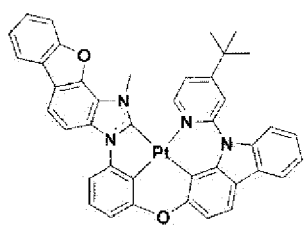
49

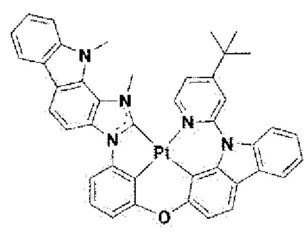
50

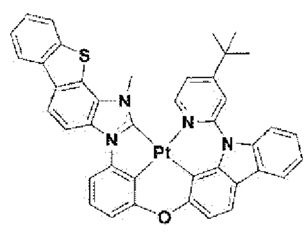
51

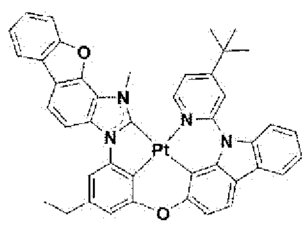
52

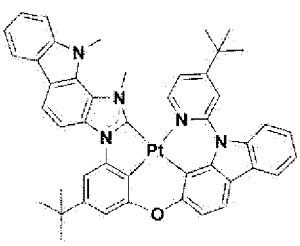
53

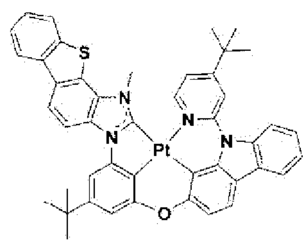
54

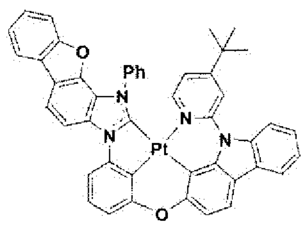
55

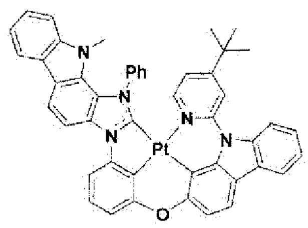
56

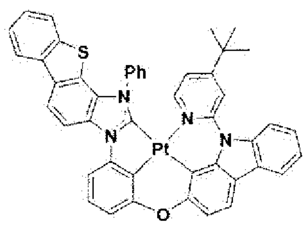
57

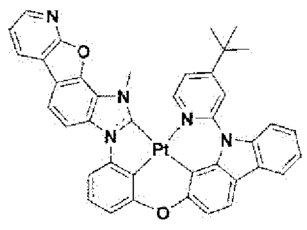
58

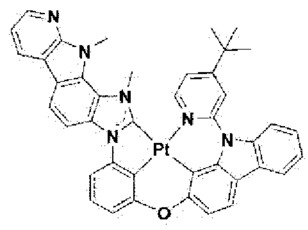
59

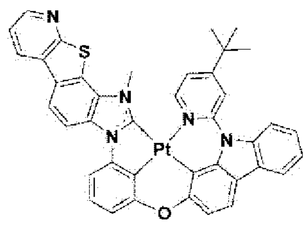
60

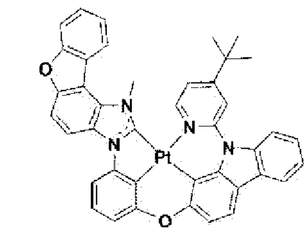
61

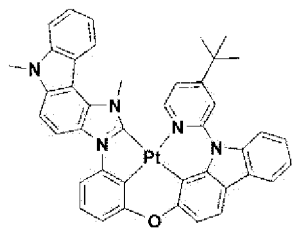
62

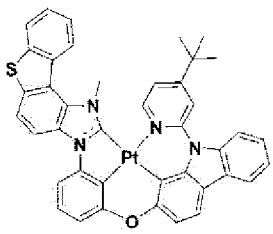
63

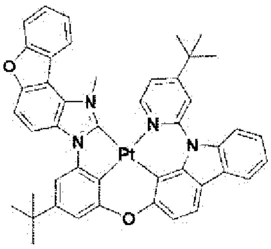

64

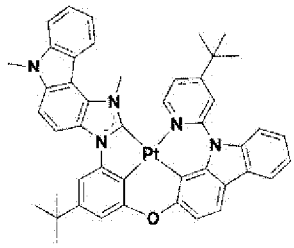

65

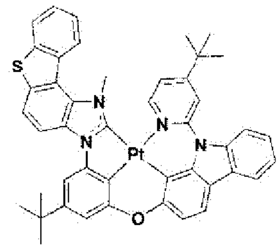

66

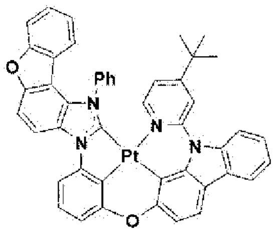

67

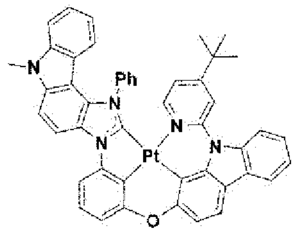

68

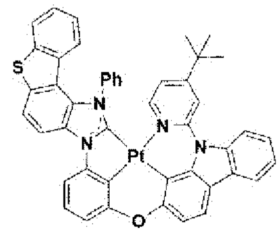

69

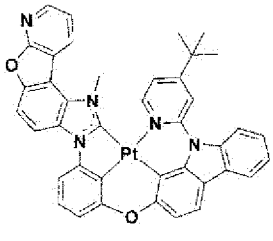

70

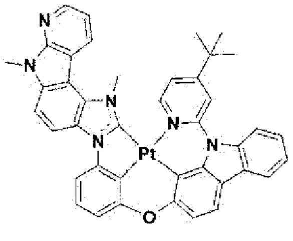

71

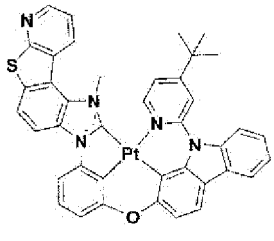

72

**11.** An organic light-emitting device comprising:

a first electrode;
a second electrode; and
an organic layer disposed between the first electrode and the second electrode, wherein the organic layer comprises:

an emission layer, and
at least one organometallic compound of any of claims 1 to 10.

**12.** The organic light-emitting device of claim 11, wherein

the first electrode is an anode,
the second electrode is a cathode, and
the organic layer comprises a hole transport region disposed between the first electrode and the emission layer and an electron transport region disposed between the emission layer and the second electrode,
the hole transport region comprises a hole injection layer, a hole transport layer, an electron blocking layer, or any combination thereof, and
the electron transport region comprises a hole blocking layer, an electron transport layer, an electron injection layer, or any combination thereof.

**13.** The organic light-emitting device of claim 11 or 12, wherein the emission layer comprises the organometallic compound.

**14.** The organic light-emitting device of claim 13, wherein the emission layer further comprises a host, wherein the host is present in the emission layer in an amount greater than an amount of the organometallic compound in the emission layer; and/or
wherein the emission layer emits blue light having a maximum emission wavelength in a range of 430 nanometers

to 480 nanometers.

**15.** A diagnostic composition comprising at least one organometallic compound of any of claims 1 to 10.

**Patentansprüche**

**1.** Metallorganische Verbindung, dargestellt durch Formel 1:

Formel 1

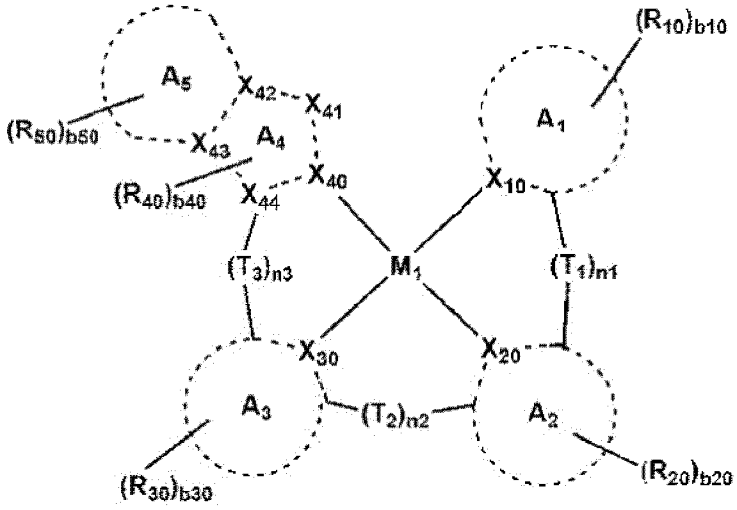

wobei, in Formel 1,

$M_1$ Beryllium, Magnesium, Aluminium, Calcium, Titan, Mangan, Kobalt, Kupfer, Zink, Gallium, Germanium, Zirkonium, Ruthenium, Rhodium, Palladium, Silber, Rhenium, Platin oder Gold ist,
$A_1$ bis $A_3$ jeweils unabhängig eine carbocyclische $C_5$-$C_{30}$-Gruppe oder eine heterocyclische $C_1$-$C_{30}$-Gruppe sind,
$A_4$ eine 5-gliedrige heterocyclische Gruppe ist,
$A_5$ eine carbocyclische $C_7$-$C_{30}$-Gruppe aus mindestens zwei Ringen ist, umfassend eine 6-gliedrige carbocyclische Gruppe, oder $A_5$ eine heterocyclische $C_1$-$C_{30}$-Gruppe aus mindestens zwei Ringen ist, umfassend eine 6-gliedrige carbocyclische Gruppe oder eine 6-gliedrige heterocyclische Gruppe,
$X_{10}$, $X_{20}$, $X_{30}$ und $X_{40}$ bis $X_{44}$ jeweils unabhängig C oder N sind,
$T_1$ und $T_3$ jeweils unabhängig eine Einfachbindung, *-N[$(L_1)_{a1}$-$(R_1)_{b1}$]-*', *-B($R_1$)-*', *-P($R_1$)-*', *-C($R_1$)($R_2$)-*', *-Si($R_1$)($R_2$)-*', *-Ge($R_1$)($R_2$)-*', *-S-*', *-Se-*', *-O-*1, *-C(=O)-*', *-S(=O)-*', *-S$(=O)_2$-*', *-C($R_1$)=C($R_2$)-*', *-C(=S)-*' oder *-C≡C-*' sind,
$T_2$ eine Einfachbindung, *-N[$(L_1)_{a1}$-$(R_1)_{b1}$]-*', *-P($R_1$)-*', *-C($R_1$)($R_2$)-*', *-Si($R_1$)($R_2$)-*', *-Ge($R_1$)($R_2$)-*', *-S-*', *-Se-*', *-O-*', *-C(=O)-*', *-S(=O)-*', *-S$(=O)_2$-*', *-C($R_1$)=C($R_2$)-*', *-C(=S)-*' oder *-C≡C-*' ist,
* und *' jeweils eine Bindungsstelle an einem benachbarten Atom angeben,
n1 bis n3 jeweils unabhängig eine ganze Zahl von 1 bis 3 sind,
$L_1$ eine Einfachbindung, eine substituierte oder unsubstituierte carbocyclische $C_5$-$C_{30}$-Gruppe oder eine substituierte oder unsubstituierte heterocyclische $C_1$-$C_{30}$-Gruppe ist,
a1 eine ganze Zahl von 1 bis 3 ist, und wenn a1 2 oder größer ist, mindestens zwei $L_1$-Gruppen identisch oder voneinander verschieden sind,
$R_1$, $R_2$, $R_{10}$, $R_{20}$, $R_{30}$, $R_{40}$ und $R_{50}$ jeweils unabhängig Wasserstoff, Deuterium, -F, -Cl, -Br, -I, -$SF_5$, eine Hydroxylgruppe, eine Cyanogruppe, eine Nitrogruppe, einer Amidinogruppe, eine Hydrazingruppe, eine Hydrazongruppe, eine Carbonsäuregruppe oder ein Salz davon, eine Sulfonsäuregruppe oder ein Salz davon, eine Phosphorsäuregruppe oder ein Salz davon, eine substituierte oder unsubstituierte $C_1$-$C_{60}$-Alkylgruppe, eine substituierte oder unsubstituierte $C_2$-$C_{60}$-Alkenylgruppe, eine substituierte oder unsubstituierte $C_2$-$C_{60}$-Alkinylgruppe, eine substituierte oder unsubstituierte $C_1$-$C_{60}$-Alkoxygruppe, eine substituierte oder unsubstituierte $C_3$-$C_{10}$-Cycloalkylgruppe, eine substituierte oder unsubstituierte $C_1$-$C_{10}$-Heterocycloalkylgruppe, eine substituierte oder unsubstituierte $C_3$-$C_{10}$-Cycloalkenylgruppe, eine substituierte oder unsubstituierte $C_1$-$C_{10}$-Hetero-

cycloalkenylgruppe, eine substituierte oder unsubstituierte $C_6$-$C_{60}$-Arylgruppe, eine substituierte oder unsubstituierte $C_6$-$C_{60}$-Aryloxygruppe, eine substituierte oder unsubstituierte $C_6$-$C_{60}$-Arylthiogruppe, eine substituierte oder unsubstituierte $C_7$-$C_{60}$-Arylalkylgruppe, eine substituierte oder unsubstituierte $C_1$-$C_{60}$-Heteroarylgruppe, eine substituierte oder unsubstituierte $C_1$-$C_{60}$-Heteroaryloxygruppe, eine substituierte oder unsubstituierte $C_1$-$C_{60}$-Heteroarylthiogruppe, eine substituierte oder unsubstituierte $C_2$-$C_{60}$-Heteroarylalkylgruppe, eine substituierte oder unsubstituierte einwertige nicht-aromatische kondensierte polycyclische Gruppe, eine substituierte oder unsubstituierte einwertige nicht-aromatische kondensierte heteropolycyclische Gruppe, $-N(Q_1)(Q_2)$, $-Si(Q_3)(Q_4)(Q_5)$, - $Ge(Q_3)(Q_4)(Q_5)$, $-B(Q_6)(Q_7)$, $-P(Q_8)(Q_9)$ oder $-P(=O)(Q_8)(Q_9)$ sind,

mindestens zwei benachbarte $R_1$-, $R_2$-, $R_{10}$-, $R_{20}$-, $R_{30}$-, $R_{40}$ oder $R_{50}$ -Gruppen optional miteinander verbunden sind, um eine substituierte oder unsubstituierte carbocyclische $C_5$-$C_{30}$-Gruppe oder eine substituierte oder unsubstituierte heterocyclische $C_1$-$C_{30}$-Gruppe zu bilden,

b1 eine ganze Zahl von 1 bis 5 ist, und wenn b1 2 oder größer ist, mindestens zwei $R_1$-Gruppen identisch oder voneinander verschieden sind,

b10, b20, b30 und b50 jeweils unabhängig eine ganze Zahl von 1 bis 10 sind,

b40 eine ganze Zahl von 1 bis 3 ist,

wenn b10 2 oder größer ist, mindestens zwei $R_{10}$-Gruppen gleich oder voneinander verschieden sind, wenn b20 2 oder größer ist, mindestens zwei $R_{20}$-Gruppen gleich oder voneinander verschieden sind, wenn b30 2 oder größer ist, mindestens zwei $R_{30}$-Gruppen gleich oder voneinander verschieden sind, wenn b40 2 oder größer ist, mindestens zwei $R_{40}$-Gruppen gleich oder voneinander verschieden sind, wenn b50 2 oder größer ist, mindestens zwei $R_{50}$-Gruppen gleich oder voneinander verschieden sind, und

mindestens ein Substituent der substituierten carbocyclischen $C_5$-$C_{30}$-Gruppe, der substituierten heterocyclischen $C_1$-$C_{30}$-Gruppe, der substituierten $C_1$-$C_{60}$-Alkylgruppe, der substituierten $C_2$-$C_{60}$-Alkenylgruppe, der substituierten $C_2$-$C_{60}$-Alkinylgruppe, der substituierten $C_1$-$C_{60}$-Alkoxygruppe, der substituierten $C_3$-$C_{10}$-Cycloalkylgruppe, der substituierten $C_1$-$C_{10}$-Heterocycloalkylgruppe, der substituierten $C_3$-$C_{10}$-Cycloalkenylgruppe, der substituierten $C_1$-$C_{10}$-Heterocycloalkenylgruppe, der substituierten $C_6$-$C_{60}$-Arylgruppe, der substituierten $C_6$-$C_{60}$-Aryloxygruppe, der substituierten $C_6$-$C_{60}$-Arylthiogruppe, der substituierten $C_7$-$C_{60}$-Arylalkylgruppe, der substituierten $C_1$-$C_{60}$-Heteroarylgruppe, der substituierten $C_1$-$C_{60}$-Heteroaryloxygruppe, der substituierten $C_1$-$C_{60}$-Heteroarylthiogruppe, der substituierten $C_2$-$C_{60}$-Heteroarylalkylgruppe, der substituierten monovalenten nicht-aromatischen kondensierten polycyclischen Gruppe und der substituierten monovalenten nicht-aromatischen kondensierten heteropolycyclischen Gruppe wie folgt ist:

Deuterium, -F, -Cl, -Br, -I, $-CD_3$, $-CD_2H$, $-CDH_2$, $-CF_3$, $-CF_2H$, $-CFH_2$, eine Hydroxylgruppe, eine Cyanogruppe, eine Nitrogruppe, eine Amidinogruppe, eine Hydrazingruppe, eine Hydrazongruppe, eine Carbonsäuregruppe oder ein Salz davon, eine Sulfonsäuregruppe oder ein Salz davon, eine Phosphorsäuregruppe oder ein Salz davon, eine $C_1$-$C_{60}$-Alkylgruppe, eine $C_2$-$C_{60}$-Alkenylgruppe, eine $C_2$-$C_{60}$-Alkinylgruppe oder eine $C_1$-$C_{60}$-Alkoxygruppe;

eine $C_1$-$C_{60}$-Alkylgruppe, eine $C_2$-$C_{60}$-Alkenylgruppe, eine $C_2$-$C_{60}$-Alkinylgruppe oder eine $C_1$-$C_{60}$-Alkoxygruppe, jeweils substituiert mit mindestens einem, ausgewählt aus Deuterium, -F, -Cl, -Br, -I, $-CD_3$, $-CD_2H$, $-CDH_2$, $-CF_3$, - $CF_2H$, $-CFH_2$, einer Hydroxylgruppe, einer Cyanogruppe, einer Nitrogruppe, einer Amidinogruppe, einer Hydrazingruppe, einer Hydrazongruppe, einer Carbonsäuregruppe oder einem Salz davon, einer Sulfonsäuregruppe oder einem Salz davon, einer Phosphorsäuregruppe oder einem Salz davon, einer $C_3$-$C_{10}$-Cycloalkylgruppe, einer $C_1$-$C_{10}$-Heterocycloalkylgruppe, einer $C_3$-$C_{10}$-Cycloalkenylgruppe, einer $C_1$-$C_{10}$-Heterocycloalkenylgruppe, einer $C_6$-$C_{60}$-Arylgruppe, einer $C_6$-$C_{60}$-Aryloxygruppe, einer $C_6$-$C_{60}$-Arylthiogruppe, einer $C_7$-$C_{60}$-Arylalkylgruppe, einer $C_1$-$C_{60}$-Heteroarylgruppe, einer $C_1$-$C_{60}$-Heteroaryloxygruppe, einer $C_1$-$C_{60}$-Heteroarylthiogruppe, einer $C_2$-$C_{60}$-Heteroarylalkylgruppe, einer monovalenten nicht-aromatischen kondensierten polycyclischen Gruppe, einer monovalenten nicht-aromatischen kondensierten polycyclischen heteropolycyclischen Gruppe, $-N(Q_{11})(Q_{12})$, $-Si(Q_{13})(Q_{14})(Q_{15})$, $-Ge(Q_{13})(Q_{14})(Q_{15})$, - $B(Q_{16})(Q_{17})$, $-P(Q_{18})(Q_{19})$ oder $-P(=O)(Q_{18})(Q_{19})$;

eine $C_3$-$C_{10}$-Cycloalkylgruppe, eine $C_1$-$C_{10}$-Heterocycloalkylgruppe, eine $C_3$-$C_{10}$-Cycloalkenylgruppe, eine $C_1$-$C_{10}$-Heterocycloalkenylgruppe, eine $C_6$-$C_{60}$-Arylgruppe, eine $C_6$-$C_{60}$-Aryloxygruppe, eine $C_6$-$C_{60}$-Arylthiogruppe, eine $C_1$-$C_{60}$-Heteroarylgruppe, eine monovalente nicht-aromatische kondensierte polycyclische Gruppe oder eine monovalente nicht-aromatische kondensierte heteropolycyclische Gruppe;

eine $C_3$-$C_{10}$-Cycloalkylgruppe, eine $C_1$-$C_{10}$-Heterocycloalkylgruppe, eine $C_3$-$C_{10}$-Cycloalkenylgruppe, eine $C_1$-$C_{10}$-Heterocycloalkenylgruppe, eine $C_6$-$C_{60}$-Arylgruppe, eine $C_6$-$C_{60}$-Aryloxygruppe, eine $C_6$-$C_{60}$-Arylthiogruppe, eine $C_1$-$C_{60}$-Heteroarylgruppe, eine monovalente nicht-aromatische kondensierte polycyclische Gruppe, oder eine monovalente nicht-aromatische kondensierte heteropolycyclische Gruppe, jeweils substituiert mit mindestens einem von Deuterium, -F, -Cl, -Br, -I, $-CD_3$, - $CD_2H$, $-CDH_2$, $-CF_3$, $-CF_2H$, $-CFH_2$, einer Hydroxylgruppe, einer Cyanogruppe, einer Nitrogruppe, einer Amidinogruppe, einer Hydrazingruppe, einer Hydrazongruppe, einer Carbonsäuregruppe oder einem Salz davon, einer Sulfonsäuregruppe oder einem Salz davon, einer Phosphorsäuregruppe oder einem Salz davon, einer $C_1$-$C_{60}$-Alkylgruppe, einer $C_2$-$C_{60}$-Alkenylgruppe, einer $C_2$-$C_{60}$-Alkinylgruppe, einer $C_1$-$C_{60}$-Alkoxygruppe, einer $C_3$-$C_{10}$-Cycloalkylgruppe, einer $C_1$-$C_{10}$-Heterocycloalkylgruppe, einer $C_3$-$C_{10}$-Cycloalkenylgruppe, einer $C_1$-$C_{10}$-Heterocycloalkenylgruppe, einer $C_6$-$C_{60}$-Arylgruppe, einer $C_6$-$C_{60}$-Aryloxygruppe, einer $C_6$-$C_{60}$-Arylthiogruppe, einer $C_7$-$C_{60}$-Arylalkylgruppe, einer $C_1$-$C_{60}$-Heteroarylgruppe, einer $C_1$-$C_{60}$-Heteroaryloxygruppe, einer $C_1$-$C_{60}$-Heteroarylthiogruppe, einer $C_2$-$C_{60}$-Heteroarylalkylgruppe, einer monovalenten nicht-aromatischen kondensierten polycyclischen Gruppe, einer monovalenten nicht-aromatischen kondensierten heteropolycyclischen Gruppe, - $N(Q_{21})(Q_{22})$, $-Si(Q_{23})(Q_{24})(Q_{25})$, $-Ge(Q_{23})(Q_{24})(Q_{25})$, $-B(Q_{26})(Q_{27})$, $-P(Q_{28})(Q_{29})$ oder - $P(=O)(Q_{28})(Q_{29})$; oder

$-N(Q_{31})(Q_{32})$, $-Si(Q_{33})(Q_{34})(Q_{35})$, $-Ge(Q_{33})(Q_{34})(Q_{35})$, $-B(Q_{36})(Q_{37})$, $-P(Q_{38})(Q_{39})$ oder - $P(=O)(Q_{38})(Q_{39})$, wobei $Q_1$ bis $Q_9$, $Q_{11}$ bis $Q_{19}$, $Q_{21}$ bis $Q_{29}$ und $Q_{31}$ bis $Q_{39}$ jeweils unabhängig Wasserstoff; -F; -Cl; -Br; -I; eine Hydroxylgruppe; eine Cyanogruppe; eine Nitrogruppe; eine Amidinogruppe; eine Hydrazingruppe; eine Hydrazongruppe; eine Carbonsäuregruppe oder ein Salz davon; eine Sulfonsäuregruppe oder ein Salz davon; eine Phosphorsäuregruppe oder ein Salz davon; eine $C_1$-$C_{60}$-Alkylgruppe; eine $C_2$-$C_{60}$-Alkenylgruppe; eine $C_2$-$C_{60}$-Alkinylgruppe; eine $C_1$-$C_{60}$-Alkoxygruppe; eine $C_3$-$C_{10}$-Cycloalkylgruppe; eine $C_1$-$C_{10}$-Heterocycloalkylgruppe; eine $C_3$-$C_{10}$-Cycloalkenylgruppe; eine $C_1$-$C_{10}$-Heterocycloalkenylgruppe; eine $C_6$-$C_{60}$-Arylgruppe; eine $C_6$-$C_{60}$-Arylgruppe substituiert mit einer $C_1$-$C_{60}$-Alkylgruppe, eine $C_6$-$C_{60}$-Arylgruppe oder eine Kombination davon; eine $C_6$-$C_{60}$-Aryloxygruppe; eine $C_6$-$C_{60}$-Arylthiogruppe; eine $C_7$-$C_{60}$-Arylalkylgruppe; eine $C_1$-$C_{60}$-Heteroarylgruppe, eine $C_1$-$C_{60}$-Heteroaryloxygruppe; eine $C_1$-$C_{60}$-Heteroarylthiogruppe; eine $C_2$-$C_{60}$-Heteroarylalkylgruppe; eine monovalente nicht-aromatische kondensierte polycyclische Gruppe; oder eine monovalente nicht-aromatische kondensierte heteropolycyclische Gruppe sind.

**2.** Metallorganische Verbindung nach Anspruch 1, wobei $M_1$ Pt, Pd oder Au ist; und/oder wobei $A_1$ bis $A_3$ jeweils unabhängig voneinander eine Phenylgruppe, eine Naphthylgruppe, eine Anthracenylgruppe, eine Phenanthrenylgruppe, eine Triphenylenylgruppe, eine Pyrenylgruppe, eine Chrysenylgruppe, eine Cyclopentadiengruppe, eine 1,2,3,4-Tetrahydronaphthylgruppe, eine Furanylgruppe, eine Thiophenylgruppe, eine Silolylgruppe, eine Indenylgruppe, eine Fluorenylgruppe, eine Indolylgruppe, eine Isoindolylgruppe, eine Carbazolylgruppe, eine Benzocarbazolylgruppe, eine Dibenzocarbazolylgruppe, eine Benzofuranylgruppe, eine Dibenzofuranylgruppe, eine Benzothiophenylgruppe, eine Benzosilolylgruppe, eine Dibenzosilolylgruppe, eine Azafluorenylgruppe, eine Azacarbazolylgruppe, eine Azadibenzofuranylgruppe, eine Azadibenzothiophenylgruppe, eine Azadibenzosilolylgruppe, eine Pyridinylgruppe, eine Imidazopyridinylgruppe, eine Pyrimidinylgruppe, eine Imidazopyrimidinylgruppe, eine Pyrazinylgruppe, eine Pyridazinylgruppe, eine Triazinylgruppe, eine Purinylgruppe, eine Chinolinylgruppe, eine Benzochinolinylgruppe, eine Isochinolinylgruppe, eine Chinoxalinylgruppe, eine Chinazolinylgruppe, eine Cinnolinylgruppe, eine Phenanthrolinylgruppe, eine Pyrrolylgruppe, eine Pyrazolylgruppe, eine Imidazolylgruppe, eine Triazolylgruppe, eine Tetrazolylgruppe, eine Oxazolylgruppe, eine Isoxazolylgruppe, eine Thiazolylgruppe, eine Isothiazolylgruppe, eine Oxadiazolylgruppe, eine Thiadiazolylgruppe, eine Benzopyrazolylgruppe, eine Benzimidazolylgruppe, eine Indazolylgruppe, eine Benzoxazolylgruppe, eine Isobenzoxazolylgruppe, eine Benzothiazolylgruppe, eine Benzoxadiazolylgruppe, eine Benzothiadiazolylgruppe, eine Benzotriazolylgruppe, eine Diazaindenylgruppe, eine Triazaindenylgruppe, eine 5,6,7,8-Tetrahydroisochinolinylgruppe, oder eine 5,6,7,8-Tetrahydrochinolinylgruppe sind.

**3.** Metallorganische Verbindung nach Anspruch 1 oder 2, wobei $X_{40}$ in $A_4$ C ist und $X_{41}$ und $X_{44}$ jeweils N sind; und/oder wobei $A_5$ durch eine von Formeln A5-1 bis A5-6 dargestellt ist:

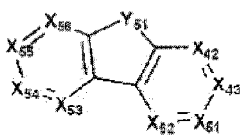
A5-1

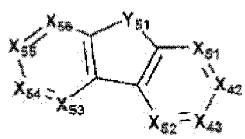
A5-2

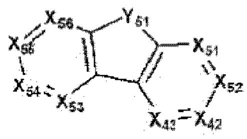
A5-3

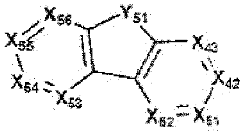

A5-4

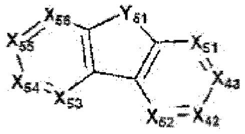

A5-5

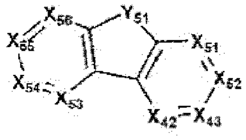

A5-6

wobei, in Formeln A5-1 bis A5-6,

$Y_{51}$ *-O-*', *-S-*', *-N($R_5$)-*', *-C($R_5$)($R_6$)-*', *-Si($R_5$)($R_6$)-*', *-B($R_5$)-*', *-P($R_5$)- *', oder *-P(=O)($R_5$)-*' ist, wobei * und *' jeweils eine Bindungsstelle zu einem benachbarten Atom angeben,
$R_5$ und $R_6$ jeweils unter Bezugnahme auf $R_1$ und $R_2$ in Anspruch 1 zu verstehen sind,
$X_{42}$ und $X_{43}$ jeweils unter Bezugnahme auf $X_{42}$ und $X_{43}$ in Anspruch 1 zu verstehen sind, und
$X_{51}$ bis $X_{56}$ jeweils unabhängig C oder N sind.

**4.** Metallorganische Verbindung nach einem der Ansprüche 1 bis 3, wobei $X_{10}$ N ist und $X_{20}$, $X_{30}$ und $X_{40}$ jeweils C sind; und/oder

wobei
eine Bindung zwischen $M_1$ und $X_{10}$ eine Koordinatenbindung ist,
eine Bindung zwischen $M_1$ und $X_{20}$ eine kovalente Bindung ist,
eine Bindung zwischen $M_1$ und $X_{30}$ eine kovalente Bindung ist, und
eine Bindung zwischen $M_1$ und $X_{40}$ eine Koordinatenbindung ist; und/oder
wobei $T_1$ bis $T_3$ jeweils unabhängig eine Einfachbindung, *-N[($L_1$)$_{a1}$-($R_1$)$_{b1}$]-*', *-C($R_1$)($R_2$)-*', *-Si($R_1$)($R_2$)-*', *-O-*' oder *-S-*' sind, wobei * und *' jeweils eine Bindungsstelle zu einem benachbarten Atom angeben.

**5.** Metallorganische Verbindung nach einem der Ansprüche 1 bis 4, wobei $L_1$ wie folgt ist:

eine Phenylengruppe, eine Pentalenylengruppe, eine Indenylengruppe, eine Naphthylengruppe, eine Azulenylengruppe, eine Heptalenylengruppe, eine Acenaphthylengruppe, eine Fluorenylengruppe, eine Phenalenylengruppe, eine Phenanthrenylengruppe, eine Anthracenylengruppe, eine Fluoranthenylengruppe, eine Triphenylenylengruppe, eine Pyrenylengruppe, eine Chrysenylenylengruppe, eine Naphthacenylengruppe, eine Picenylengruppe, eine Perylenylengruppe oder eine Pentacenylengruppe; oder
eine Phenylengruppe, eine Pentalenylengruppe, eine Indenylengruppe, eine Naphthylengruppe, eine Azulenylengruppe, eine Heptalenylengruppe, eine Acenaphthylengruppe, eine Fluorenylengruppe, eine Phenalenylengruppe, eine Phenanthrenylengruppe, eine Anthracenylengruppe, eine Fluoranthenylengruppe, eine Triphenylenylengruppe, eine Pyrenylengruppe, eine Chrysenylenylengruppe, eine Naphthacenylengruppe, eine Picenylengruppe, eine Perylenylengruppe oder eine Pentacenylengruppe, jeweils substituiert mit mindestens einem von Deuterium, -F, -Cl, -Br, -I, einer Hydroxylgruppe, einer Cyanogruppe, einer Nitrogruppe, einer Aminogruppe, einer Amidinogruppe, einer Hydrazingruppe, einer Hydrazongruppe, einer Carbonsäuregruppe oder einem Salz davon, einer Sulfonsäuregruppe oder einem Salz davon, einer Phosphorsäuregruppe oder einem Salz davon, einer $C_1$- $C_{60}$-Alkylgruppe, einer $C_2$-$C_{60}$-Alkenylgruppe, einer $C_2$-$C_{60}$-Alkinylgruppe, einer $C_1$-$C_{60}$-Alkoxygruppe, einer C3-$C_{10}$-Cycloalkylgruppe, einer $C_3$-$C_{10}$-Cycloalkenylgruppe, einer $C_1$-$C_{10}$-Heterocycloalkylgruppe, einer $C_1$-$C_{10}$-Heterocycloalkenylgruppe, einer $C_6$-$C_{60}$-Arylgruppe, einer $C_6$-$C_{60}$-Aryloxygruppe, einer $C_6$-$C_{60}$-Arylthiogruppe, einer $C_1$-$C_{60}$-Heteroarylgruppe, einer einwertigen nicht-aromatischen kondensierten polycyclischen Gruppe oder einer einwertigen nicht-aromatischen kondensierten heteropolycyclischen Gruppe.

**6.** Metallorganische Verbindung nach einem der Ansprüche 1 bis 5, wobei $R_1$, $R_2$, $R_{10}$, $R_{20}$, $R_{30}$, $R_{40}$ und $R_{50}$ jeweils unabhängig wie folgt sind:

Wasserstoff, Deuterium, -F, -Cl, -Br, -I, eine Hydroxylgruppe, eine Cyanogruppe, eine Nitrogruppe, eine Aminogruppe, eine Amidinogruppe, eine Hydrazingruppe, eine Hydrazongruppe, eine Carbonsäuregruppe oder ein Salz davon, eine Sulfonsäuregruppe oder ein Salz davon, eine Phosphorsäuregruppe oder ein Salz davon, -$SF_5$, eine $C_1$-$C_{20}$-Alkylgruppe oder eine $C_1$-$C_{20}$-Alkoxygruppe;
eine $C_1$-$C_{20}$-Alkylgruppe oder eine $C_1$-$C_{20}$-Alkoxygruppe, jeweils substituiert mit mindestens einem von Deuterium, -F, -Cl, -Br, -I, -$CD_3$, -$CD_2H$, -CDH2, -$CF_3$, -$CF_2H$, - CFH2, einer Hydroxylgruppe, einer Cyanogruppe, einer Nitrogruppe, einer Aminogruppe, einer Amidinogruppe, einer Hydrazingruppe, einer Hydrazongruppe, einer Carbonsäuregruppe oder einem Salz davon, einer Sulfonsäuregruppe oder einem Salz davon, einer

Phosphorsäuregruppe oder einem Salz davon, einer $C_1$-$C_{10}$-Alkylgruppe, einer $C_1$-$C_{10}$-Alkoxygruppe, einer Cyclopentylgruppe, einer Cyclohexylgruppe, einer Cycloheptylgruppe, einer Cyclooctylgruppe, einer Adamantylgruppe, einer Norbornanylgruppe, einer Norbornenylgruppe, einer Cyclopentenylgruppe, einer Cyclohexenylgruppe, einer Cycloheptenylgruppe, einer Phenylgruppe, einer Naphthylgruppe, einer Pyridinylgruppe oder einer Pyrimidinylgruppe;

eine Cyclopentylgruppe, eine Cyclohexylgruppe, eine Cycloheptylgruppe, eine Cyclooctylgruppe, eine Adamantanylgruppe, eine Norbornanylgruppe, eine Norbornenylgruppe, eine Cyclopentenylgruppe, eine Cyclohexenylgruppe, eine Cycloheptenylgruppe, eine Phenylgruppe, eine Naphthylgruppe, eine Fluorenylgruppe, eine Phenanthrenylgruppe, eine Anthracenylgruppe, eine Fluoranthenylgruppe, eine Triphenylenylgruppe, eine Pyrenylgruppe, eine Chrysenylgruppe, eine Pyrrolylgruppe, eine Thiophenylgruppe, eine Furanylgruppe, eine Imidazolylgruppe, eine Pyrazolylgruppe, eine Thiazolylgruppe, eine Isothiazolylgruppe, eine Oxazolylgruppe, eine Isoxazolylgruppe, eine Pyridinylgruppe, eine Pyrazinylgruppe, eine Pyrimidinylgruppe, eine Pyridazinylgruppe, eine Isoindolylgruppe, eine Indolylgruppe, eine Indazolylgruppe, eine Purinylgruppe, eine Chinolinylgruppe, eine Isochinolinylgruppe, eine Benzochinolinylgruppe, eine Chinoxalinylgruppe, eine Chinazolinylgruppe, eine Cinnolinylgruppe, eine Carbazolylgruppe, eine Phenanthrolinylgruppe, eine Benzimidazolylgruppe, eine Benzofuranylgruppe, eine Benzothiophenylgruppe, eine Isobenzothiazolylgruppe, eine Benzoxazolylgruppe, eine Isobenzoxazolylgruppe, eine Triazolylgruppe, eine Tetrazolylgruppe, eine Oxadiazolylgruppe, eine Triazinylgruppe, eine Dibenzofuranylgruppe, eine Dibenzothiophenylgruppe, eine Benzocarbazolylgruppe, eine Dibenzocarbazolylgruppe, eine Imidazopyridinylgruppe oder eine Imidazopyrimidinylgruppe;

eine Cyclopentylgruppe, eine Cyclohexylgruppe, eine Cycloheptylgruppe, eine Cyclooctylgruppe, eine Adamantanylgruppe, eine Norbornanylgruppe, eine Norbornenylgruppe, eine Cyclopentenylgruppe, eine Cyclohexenylgruppe, eine Cycloheptenylgruppe, eine Phenylgruppe, eine Naphthylgruppe, eine Fluorenylgruppe, eine Phenanthrenylgruppe, eine Anthracenylgruppe, eine Fluoranthenylgruppe, eine Triphenylenylgruppe, eine Pyrenylgruppe, eine Chrysenylgruppe, eine Pyrrolylgruppe, eine Thiophenylgruppe, eine Furanylgruppe, eine Imidazolylgruppe, eine Pyrazolylgruppe, eine Thiazolylgruppe, eine Isothiazolylgruppe, eine Oxazolylgruppe, eine Isoxazolylgruppe, eine Pyridinylgruppe, eine Pyrazinylgruppe, eine Pyrimidinylgruppe, eine Pyridazinylgruppe, eine Isoindolylgruppe, eine Indolylgruppe, eine Indazolylgruppe, eine Purinylgruppe, eine Chinolinylgruppe, eine Isochinolinylgruppe, eine Benzochinolinylgruppe, eine Chinoxalinylgruppe, eine Chinazolinylgruppe, eine Cinnolinylgruppe, eine Carbazolylgruppe, eine Phenanthrolinylgruppe, eine Benzimidazolylgruppe, eine Benzofuranylgruppe, eine Benzothiophenylgruppe, eine Isobenzothiazolylgruppe, eine Benzoxazolylgruppe, eine Isobenzoxazolylgruppe, eine Triazolylgruppe, eine Tetrazolylgruppe, eine Oxadiazolylgruppe, eine Triazinylgruppe, eine Dibenzofuranylgruppe, eine Dibenzothiophenylgruppe, eine Benzocarbazolylgruppe, eine Dibenzocarbazolylgruppe, eine Imidazopyridinylgruppe und eine Imidazopyrimidinylgruppe, jeweils substituiert mit mindestens einem von Deuterium, -F, -Cl, -Br, -I, $-CD_3$, $-CD_2H$, $-CDH_2$, - $CF_3$, $-CF_2H$, $-CFH_2$, einer Hydroxylgruppe, einer Cyanogruppe, einer Nitrogruppe, einer Aminogruppe, eine Amidinogruppe, einer Hydrazingruppe, einer Hydrazongruppe, einer Carbonsäuregruppe oder einem Salz davon, einer Sulfonsäuregruppe oder einem Salz davon, einer Phosphorsäuregruppe oder einem Salz davon, einer $C_1$-$C_{10}$-Alkylgruppe, einer $C_1$-$C_{10}$-Alkoxygruppe, einer Cyclopentylgruppe, einer Cyclohexylgruppe, einer Cycloheptylgruppe, einer Cyclooctylgruppe, einer Adamantanylgruppe, einer Norbornanylgruppe, einer Norbornenylgruppe, einer Cyclopentenylgruppe, einer Cyclohexenylgruppe, einer Cycloheptenylgruppe, einer Phenylgruppe, einer Naphthylgruppe, einer Pyridinylgruppe oder einer Pyrimidinylgruppe; oder

$-N(Q_1)(Q_2)$, $-Si(Q_3)(Q_4)(Q_5)$, $-Ge(Q_3)(Q_4)(Q_5)$, $-B(Q_6)(Q_7)$, $-P(Q_8)(Q_9)$ oder - $P(=O)(Q_8)(Q_9)$,

wobei $Q_1$ bis $Q_9$ jeweils unabhängig wie folgt sind:

> $-CH_3$, $-CD_3$, $-CD_2H$, $-CDH_2$, $-CH_2CH_3$, $-CH_2CD_3$, $-CH_2CD_2H$, $-CH_2CDH_2$, $-CHDCH_3$, - $CHDCD_2H$, $-CHDCDH_2$, $-CHDCD_3$, $-CD_2CD_3$, $-CD_2CD_2H$ oder $-CD_2CDH_2$;
>
> eine n-Propylgruppe, eine iso-Propylgruppe, eine n-Butylgruppe, eine iso-Butylgruppe, eine sec-Butylgruppe, eine tert-Butylgruppe, eine n-Pentylgruppe, eine iso-Pentylgruppe, eine sec-Pentylgruppe, eine tert-Pentylgruppe, eine Phenylgruppe oder eine Naphthylgruppe; oder
>
> eine n-Propylgruppe, eine iso-Propylgruppe, eine n-Butylgruppe, eine iso-Butylgruppe, eine sec-Butylgruppe, eine tert-Butylgruppe, eine n-Pentylgruppe, eine iso-Pentylgruppe, eine sec-Pentylgruppe, eine tert-Pentylgruppe, eine Phenylgruppe und eine Naphthylgruppe, jeweils substituiert mit mindestens einem, ausgewählt aus Deuterium, einer $C_1$-$C_{10}$-Alkylgruppe oder einer Phenylgruppe.

**7.** Metallorganische Verbindung nach einem der Ansprüche 1 bis 6, wobei $R_1$, $R_2$, $R_{10}$, $R_{20}$, $R_{30}$, $R_{40}$ und $R_{50}$ jeweils unabhängig Wasserstoff, Deuterium, -F, eine Cyanogruppe, eine Nitrogruppe, $-SF_5$, $-CH_3$, $-CD_3$, $-CD_2H$, $-CDH_2$, $-CF_3$, $-CF_2H$, $-CFH_2$, eine Gruppe, dargestellt durch eine von Formeln 9-1 bis 9-19 oder eine Gruppe, dargestellt durch eine von Formeln 10-1 bis 10-194 sind:

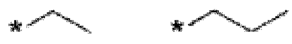
9-1 9-2

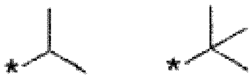
9-3 9-4

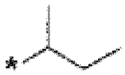
9-5

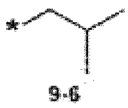
9-6

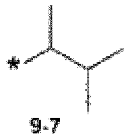
9-7

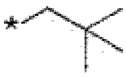
9-8

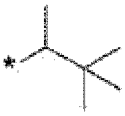
9-9

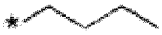
9-10

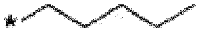
9-11

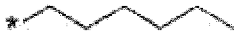
9-12

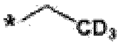
9-13

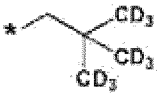
9-14

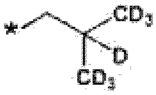
9-15

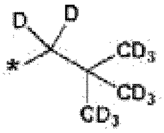
9-16

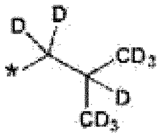
9-17

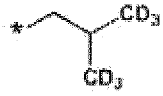
9-18

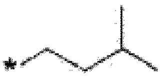
9-19

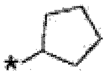
10-1

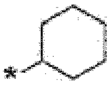
10-2

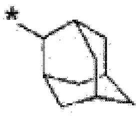
10-3

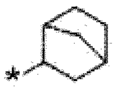
10-4

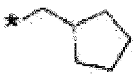
10-5

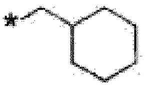
10-6

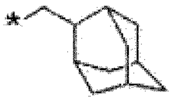
10-7

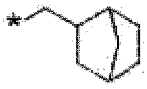
10-8

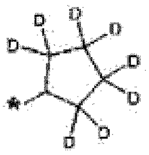
10-9

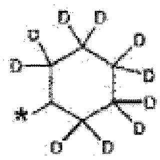
10-10

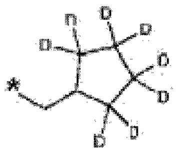
10-11

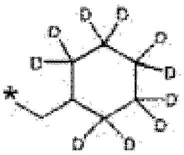
10-12

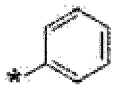
10-13

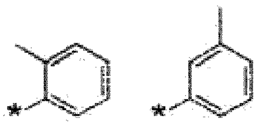
10-14 10-15

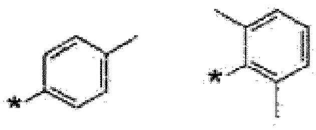
10-16 10-17

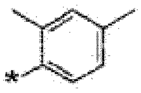
10-18

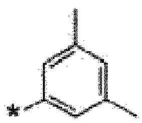
10-19

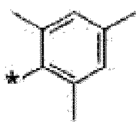
10-20

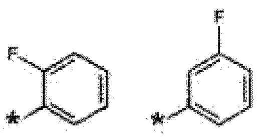
10-21 10-22

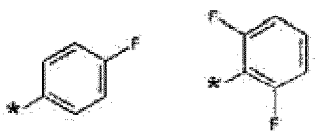
10-23 10-24

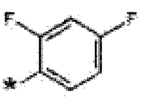
10-25

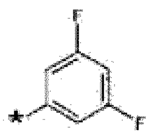
10-26

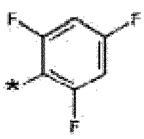
10-27

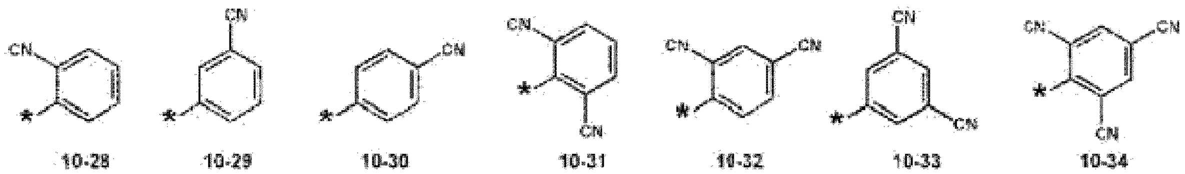

10-28 10-29 10-30 10-31 10-32 10-33 10-34

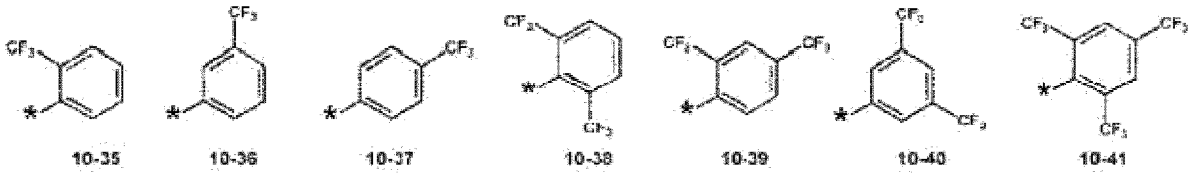

10-35 10-36 10-37 10-38 10-39 10-40 10-41

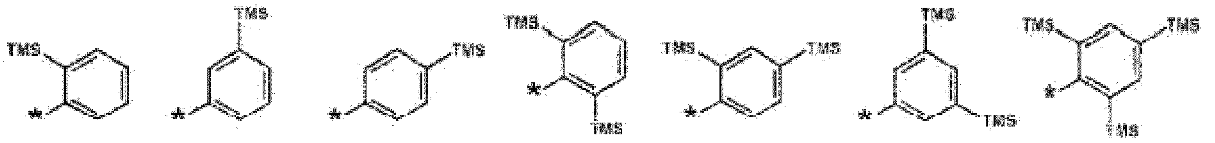

10-42 10-43 10-44 10-45 10-46 10-47 10-48

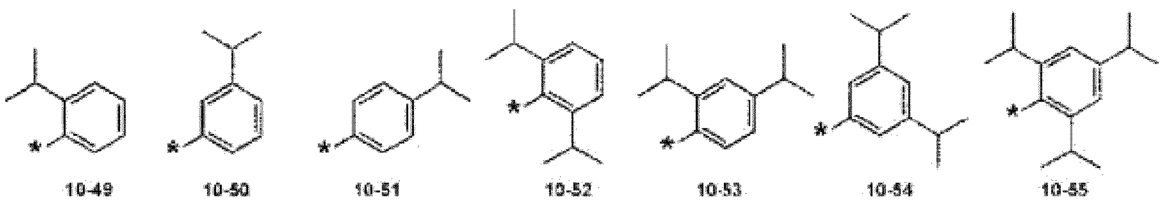

10-49 10-50 10-51 10-52 10-53 10-54 10-55

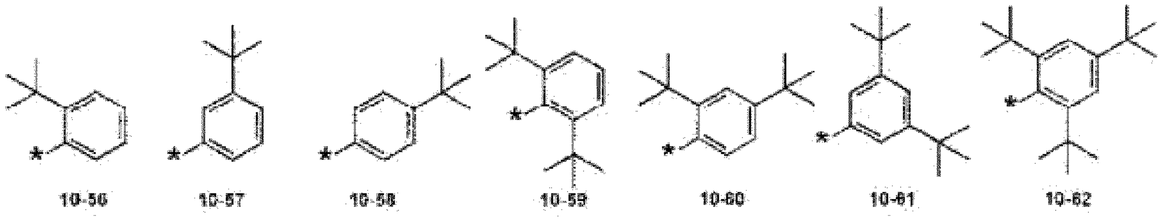

10-56 10-57 10-58 10-59 10-60 10-61 10-62

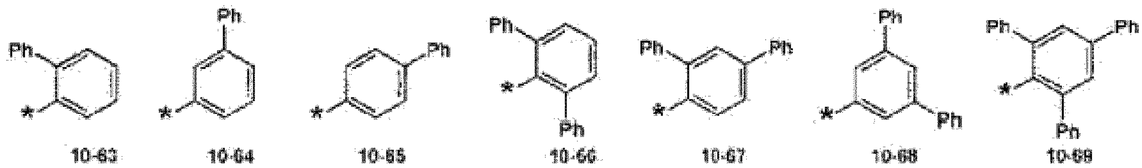

10-63 10-64 10-65 10-66 10-67 10-68 10-69

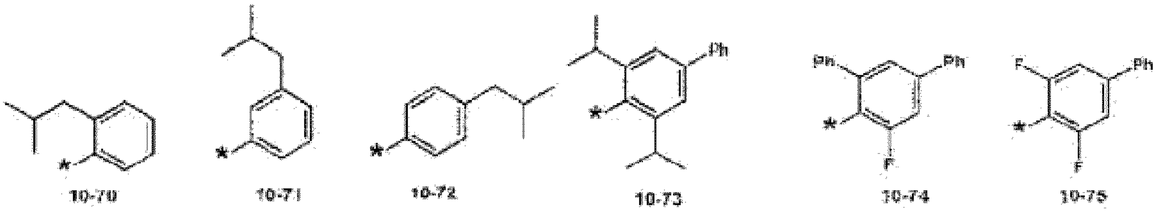

10-70 10-71 10-72 10-73 10-74 10-75

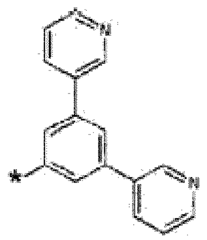
10-76

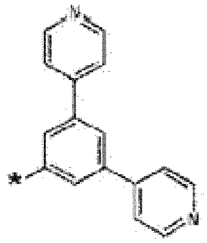
10-77

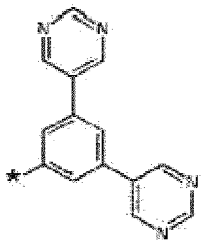
10-78

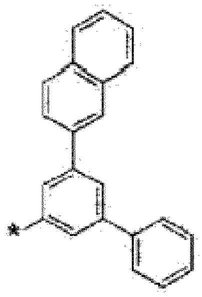
10-79

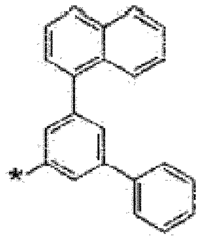
10-80

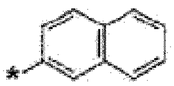
10-81

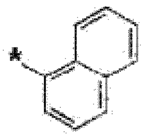
10-82

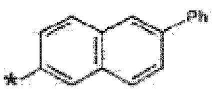
10-83

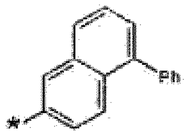
10-84

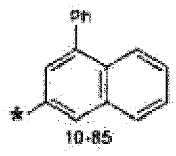
10-85

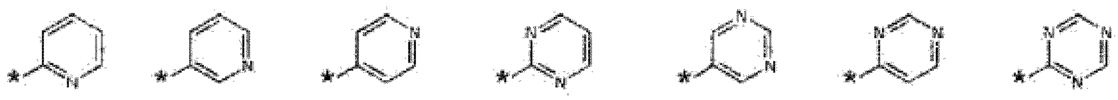
10-86 10-87 10-88 10-89 10-90 10-91 10-92

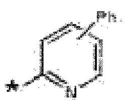
10-93

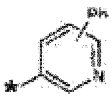
10-94

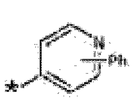
10-95

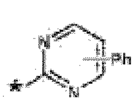
10-96

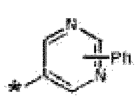
10-97

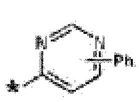
10-98

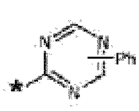
10-99

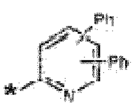
10-100

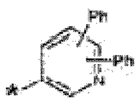
10-101

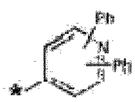
10-102

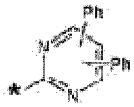
10-103

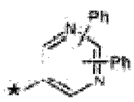
10-104

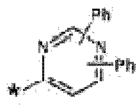
10-105

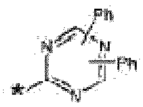
10-106

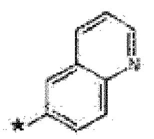
10-107

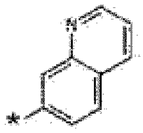
10-108

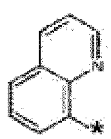
10-109

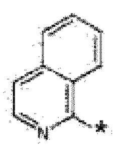
10-110

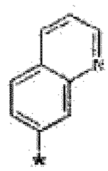
10-111

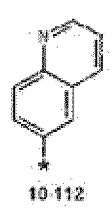
10-112

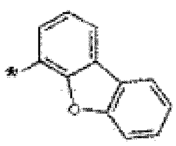
10-113

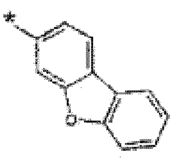
10-114

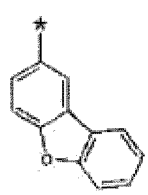
10-115

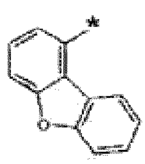
10-116

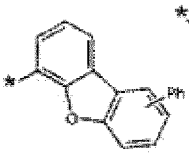
10-117

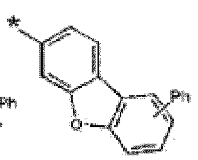
10-118

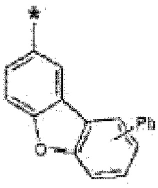
10-119

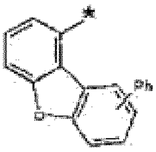
10-120

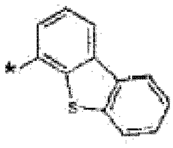
10-121

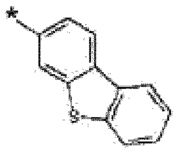
10-122

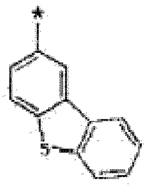
10-123

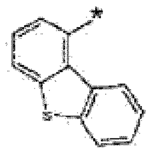
10-124

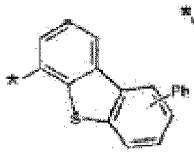
10-125

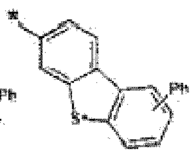
10-126

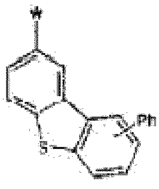
10-127

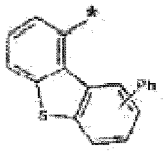
10-128

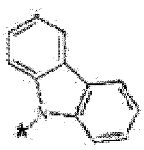
10-129

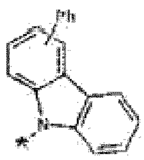
10-130

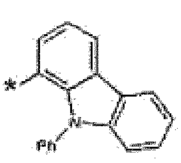
10-131

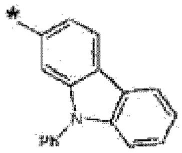
10-132

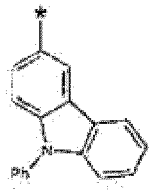
10-133

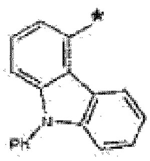
10-134

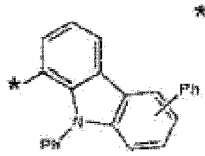
10-135

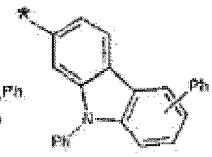
10-136

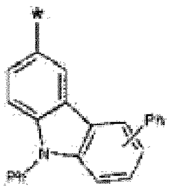
10-137

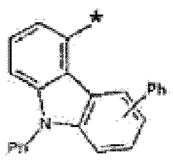
10-138

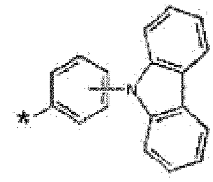
10-139

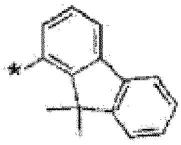
10-140

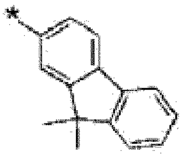
10-141

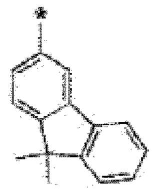
10-142

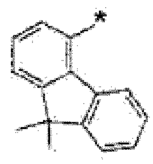
10-143

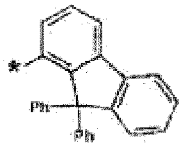
10-144

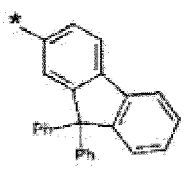
10-145

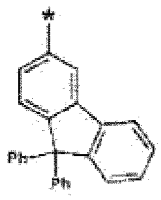
10-146

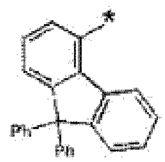
10-147

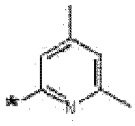
10-148

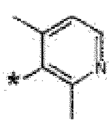
10-149

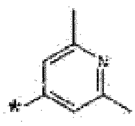
10-150

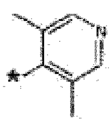
10-151

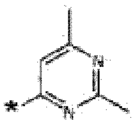
10-152

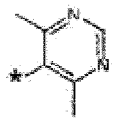
10-153

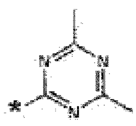
10-154

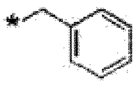
10-155
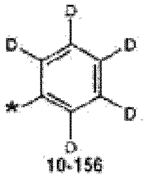
10-156
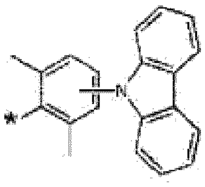
10-157
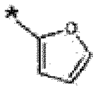
10-158
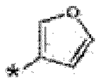
10-159
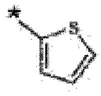
10-160
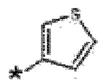
10-161

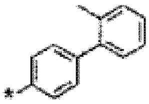
10-162
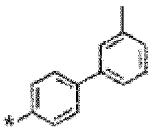
10-163
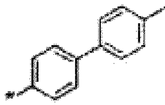
10-164
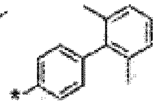
10-165
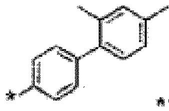
10-166
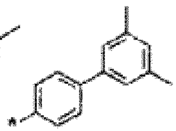
10-167
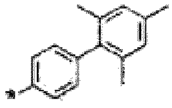
10-168

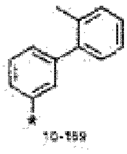
10-169
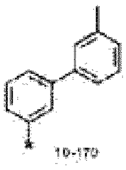
10-170
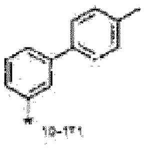
10-171
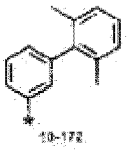
10-172
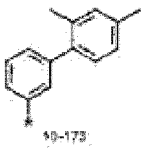
10-173
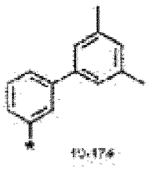
10-174
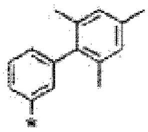
10-175

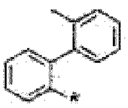
10-176
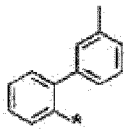
10-177
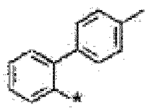
10-178
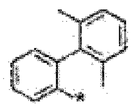
10-179
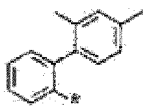
10-180
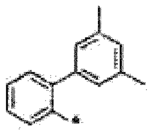
10-181
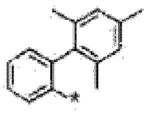
10-182

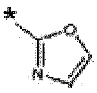
10-183
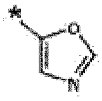
10-184
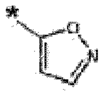
10-185
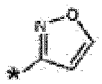
10-186
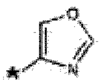
10-187
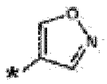
10-188
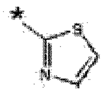
10-189
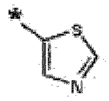
10-190
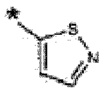
10-191

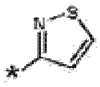
10-192
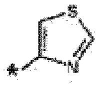
10-193
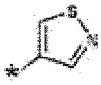
10-194

wobei in Formeln 9-1 bis 9-19 und 10-1 bis 10-194 * eine Bindungsstelle zu einem benachbarten Atom angibt, "Ph" eine Phenylgruppe und "TMS" eine Trimethylsilylgruppe angibt.

**8.** Metallorganische Verbindung nach einem der Ansprüche 1 bis 7, wobei die durch Formel 1 dargestellte metallorganische Verbindung durch eine von Formeln 11-1 bis 11-6 dargestellt ist:

Formel 11-1

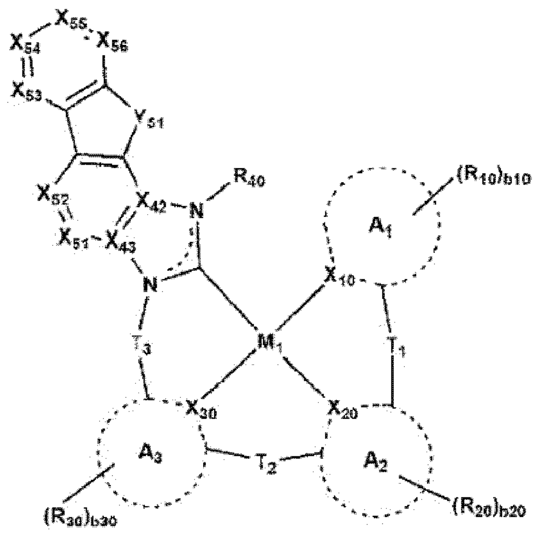

Formel 11-2

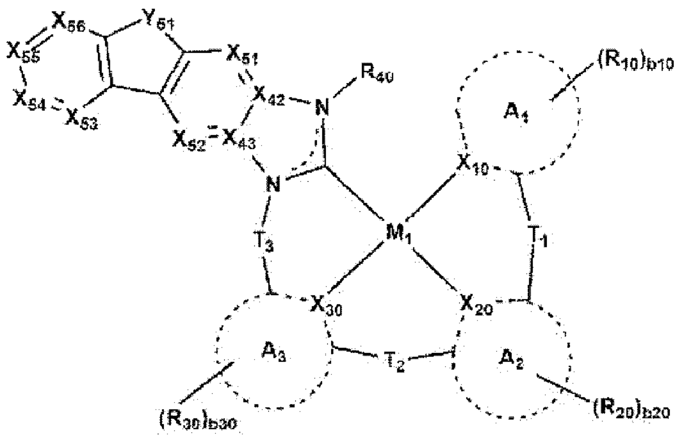

Formel 11-3

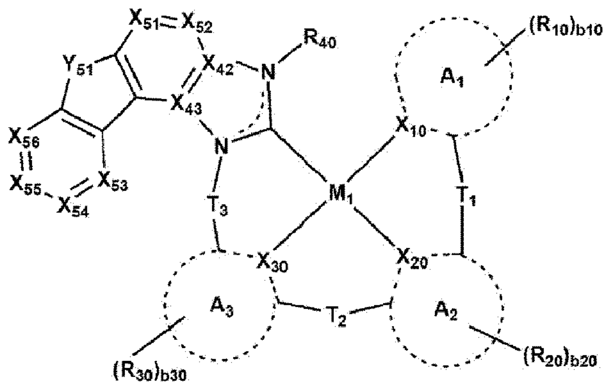

Formel 11-4

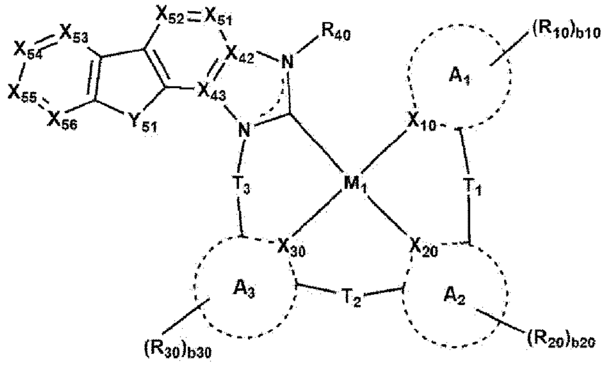

Formel 11-5

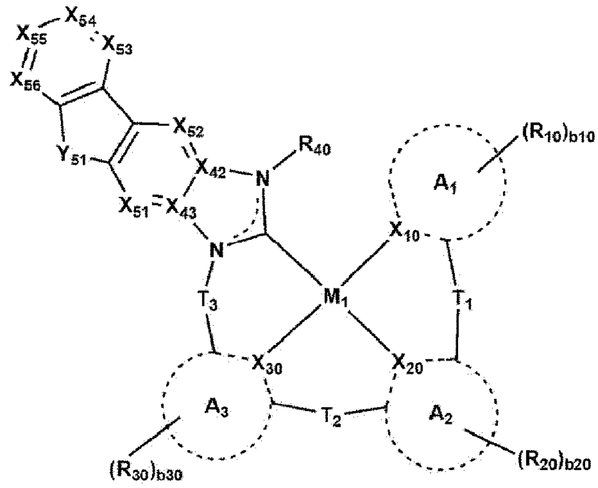

Formel 11-6

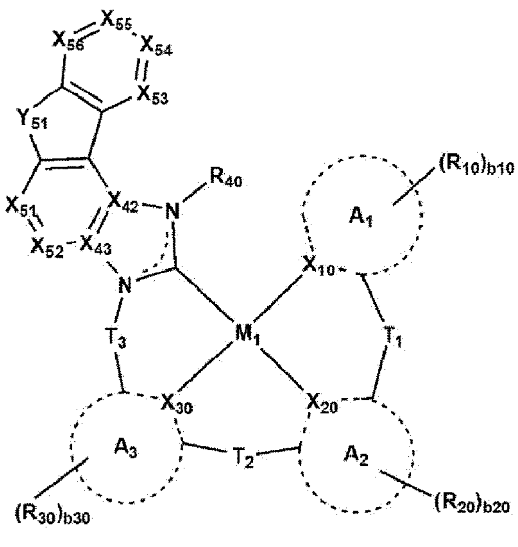

wobei, in Formeln 11-1 bis 11-6,

$M_1$, $A_1$ bis $A_3$, $X_{10}$, $X_{20}$, $X_{30}$, $X_{42}$, $X_{43}$, $T_1$ bis $T_3$, $R_{10}$, $R_{20}$, $R_{30}$, $R_{40}$, $R_{50}$, b10, b20 und b30 jeweils unter Bezugnahme auf die Beschreibungen von Mi, $A_1$ bis $A_3$, $X_{10}$, $X_{20}$, $X_{30}$, $X_{42}$, $X_{43}$, $T_1$ bis $T_3$, $R_{10}$, $R_{20}$, $R_{30}$, $R_{40}$, $R_{50}$, b10, b20 und b30 in Anspruch 1 zu verstehen sind,

$Y_{51}$ *-O-*', *-S-*', *-N($R_5$)-*', *-C($R_5$)($R_6$)-*', *-Si($R_5$)($R_6$)-*', *-B($R_5$)-*', *-P($R_5$)-*' oder *-P(=O)($R_5$)-*' ist, wobei *

und *' jeweils eine Bindungsstelle zu einem benachbarten Atom angeben,
$R_5$ und $R_6$ jeweils unter Bezugnahme auf die Beschreibungen von $R_1$ und $R_2$ in Anspruch 1 zu verstehen sind,
$X_{51}$ C($R_{51}$) oder N ist, $X_{52}$ C($R_{52}$) oder N ist, $X_{53}$ C($R_{53}$) oder N ist, $X_{54}$ C($R_{54}$) oder N ist, $X_{55}$C($R_{55}$) oder N ist, $X_{56}$ C($R_{56}$) oder N ist, und
$R_{51}$ bis $R_{56}$ jeweils unabhängig unter Bezugnahme auf die Beschreibung von $R_{50}$ in Anspruch 1 zu verstehen sind.

**9.** Metallorganische Verbindung nach einem der Ansprüche 1 bis 8, wobei die durch Formel 1 dargestellte metallorganische Verbindung durch eine von Formeln 12-1 bis 12-6 dargestellt ist:

Formel 12-1

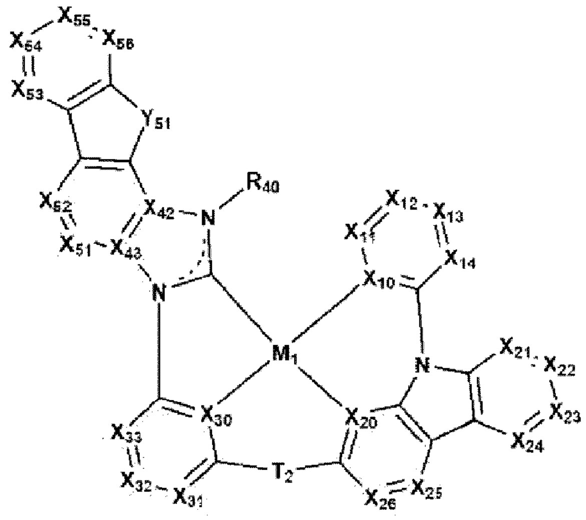

Formel 12-2

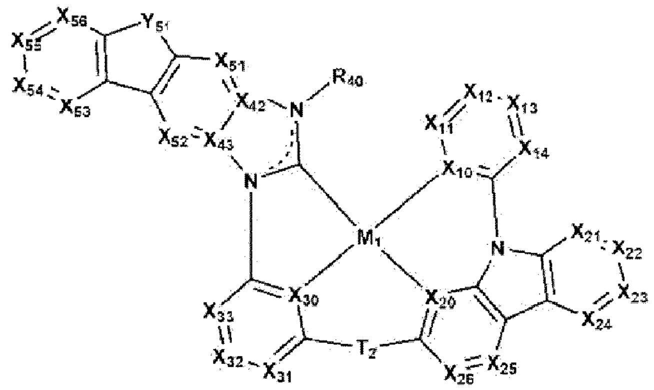

Formel 12-3

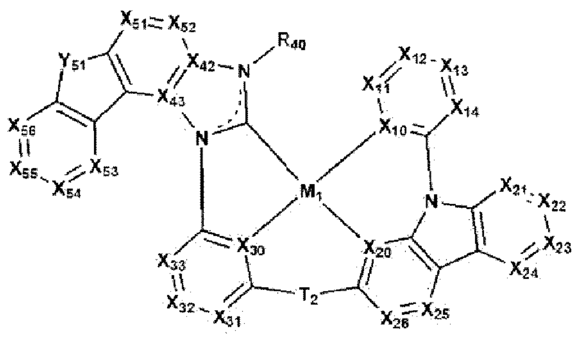

Formel 12-4

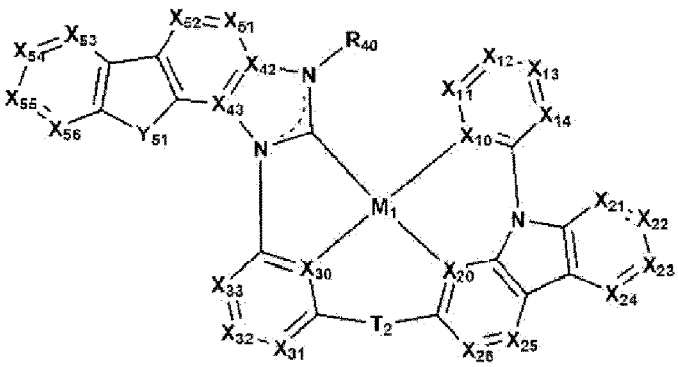

Formel 12-5

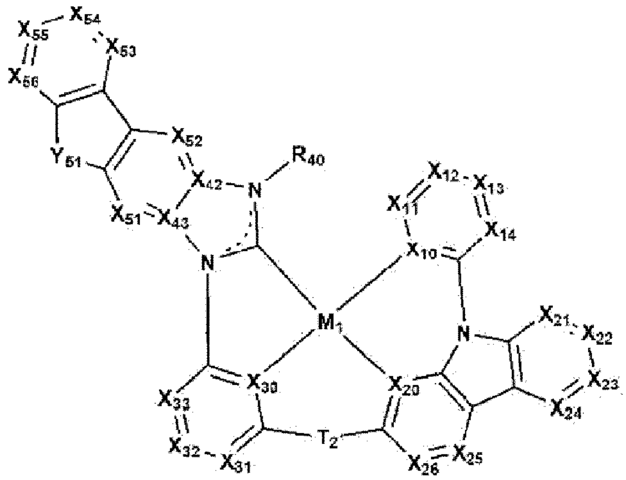

Formel 12-6

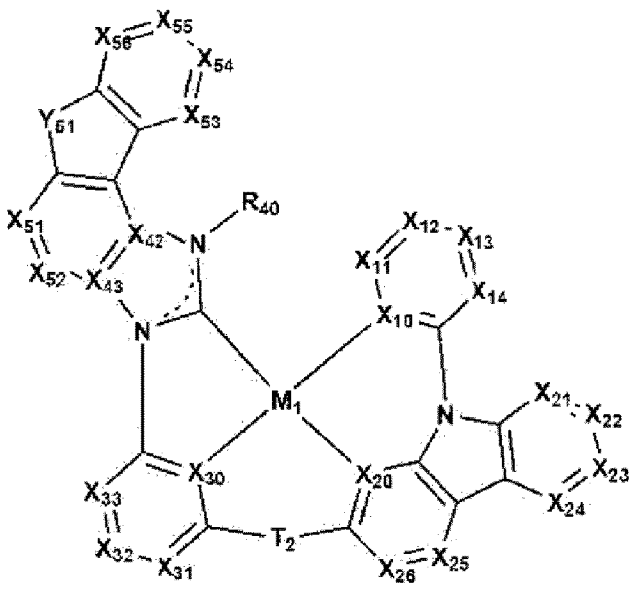

wobei, in Formeln 12-1 bis 12-6,

$M_1$, $X_{10}$, $X_{20}$, $X_{30}$, $X_{42}$, $X_{43}$, $R_{40}$ und $T_2$ jeweils unter Bezugnahme auf $M_1$, $X_{10}$, $X_{20}$, $X_{30}$, $X_{42}$, $X_{43}$, $R_{40}$ und $T_2$ in Anspruch 1 zu verstehen sind,
$Y_{51}$ *-O-*', *-S-*', *-N($R_5$)-*', *-C($R_5$)($R_6$)-*', *-Si($R_5$)($R_6$)-*', *-B($R_5$)-*', *-P($R_5$)-*' oder *-P(=O)($R_5$)-*' ist, wobei * und *' jeweils eine Bindungsstelle zu einem benachbarten Atom angeben,
$R_5$ und $R_6$ jeweils unter Bezugnahme auf $R_1$ und $R_2$ in Anspruch 1 zu verstehen sind,
$X_{11}$ C($R_{11}$) oder N ist, $X_{12}$ C($R_{12}$) oder N ist, $X_{13}$ C($R_{13}$) oder N ist, und $X_{14}$ C($R_{14}$) oder N ist,
$X_{21}$ C($R_{21}$) oder N ist, $X_{22}$C($R_{22}$) oder N ist, $X_{23}$ C($R_{23}$) oder N ist, $X_{24}$ C($R_{24}$) oder N ist, $X_{25}$ C($R_{25}$) oder N ist, und $X_{26}$C($R_{26}$) oder N ist,
$X_{31}$C($R_{31}$) oder N ist, $X_{32}$C($R_{32}$) oder N ist, and $X_{33}$C($R_{33}$) oder N ist,
$X_{51}$C($R_{51}$) oder N ist, $X_{52}$C($R_{52}$) oder N ist, $X_{53}$C($R_{53}$) oder N ist, $X_{54}$ C($R_{54}$) oder N ist, $X_{55}$C($R_{55}$) oder N ist, und $X_{56}$C($R_{56}$) oder N ist,
$R_{11}$ bis $R_{14}$ jeweils unabhängig unter Bezugnahme auf Rio in Anspruch 1 zu verstehen sind,
$R_{21}$ bis $R_{26}$ jeweils unabhängig unter Bezugnahme auf $R_{20}$ in Anspruch 1 zu verstehen sind,
$R_{31}$ bis $R_{33}$ jeweils unabhängig unter Bezugnahme auf $R_{30}$ in Anspruch 1 zu verstehen sind, und
$R_{51}$ bis $R_{56}$ jeweils unabhängig unter Bezugnahme auf $R_{50}$ in Anspruch 1 zu verstehen sind.

**10.** Metallorganische Verbindung nach einem der Ansprüche 1 bis 9, wobei die metallorganische Verbindung eine von Verbindungen 1 bis 72 ist:

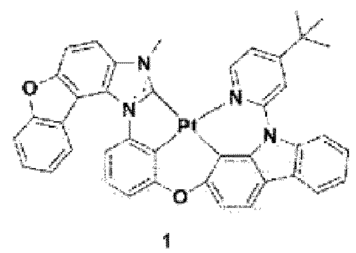

1

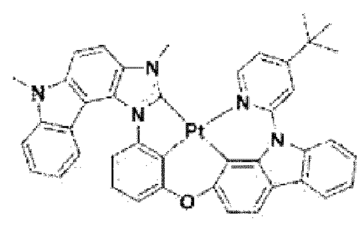

2

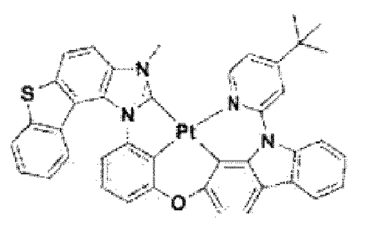

3

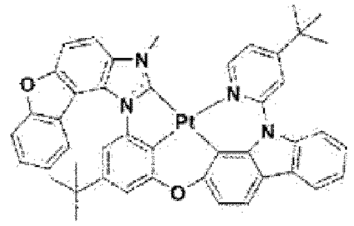
4

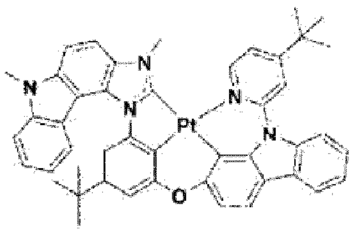
5

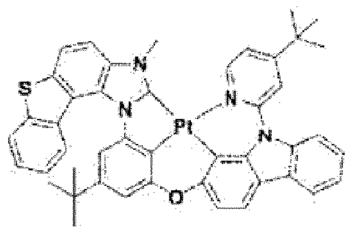
6

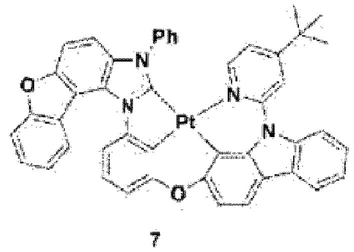
7

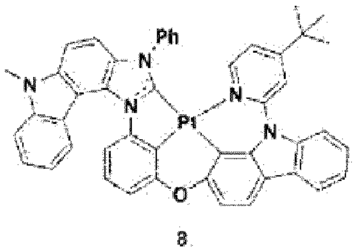
8

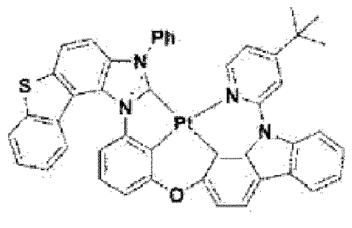
9

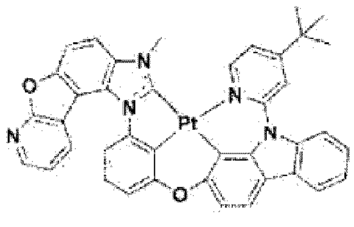
10

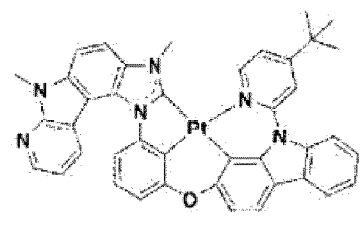
11

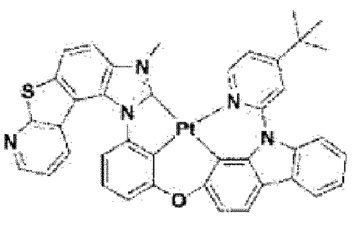
12

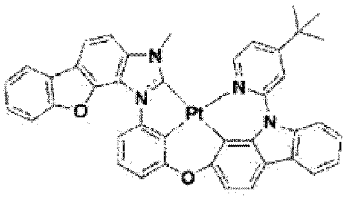
13

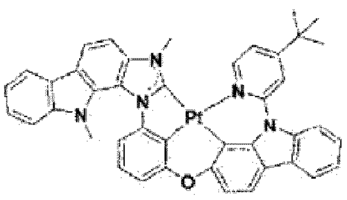
14

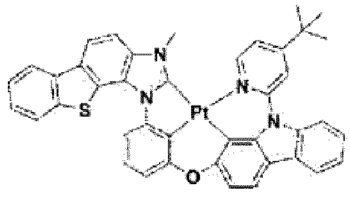
15

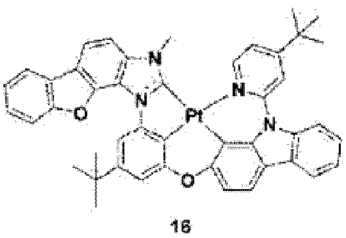
16

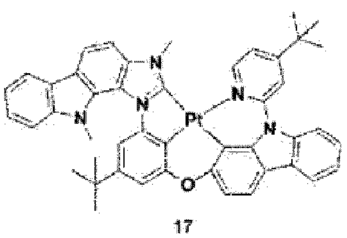
17

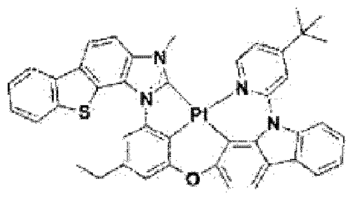
18

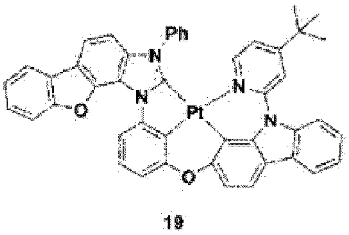
19

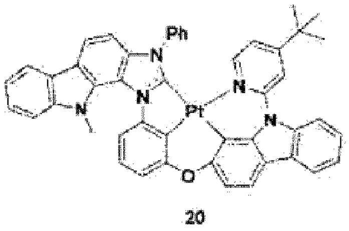
20

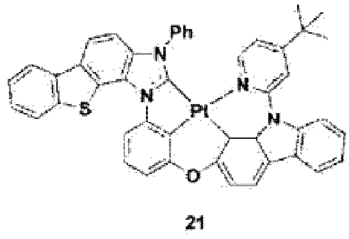
21

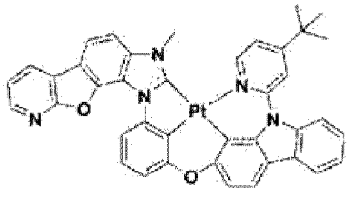

22

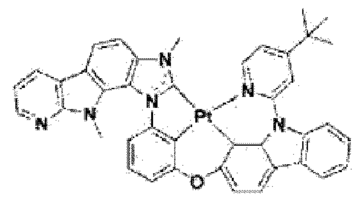

23

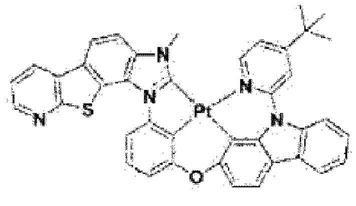

24

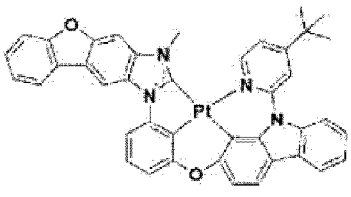

25

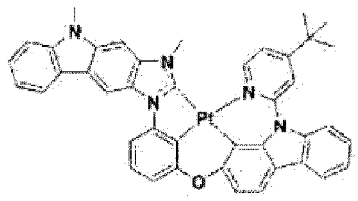

26

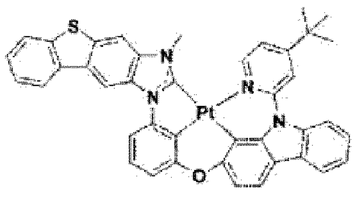

27

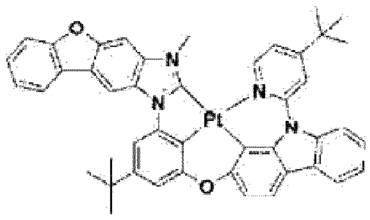

28

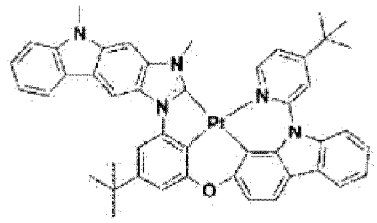

29

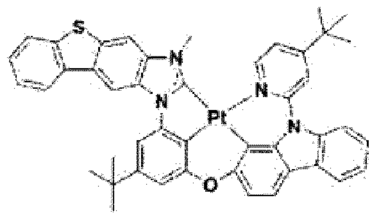

30

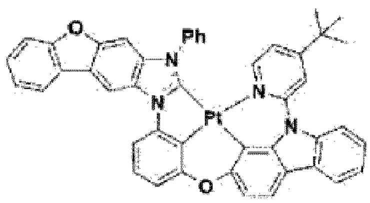

31

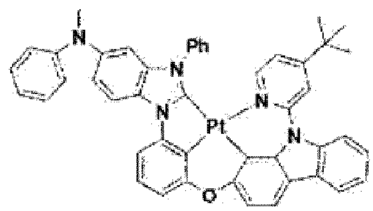

32

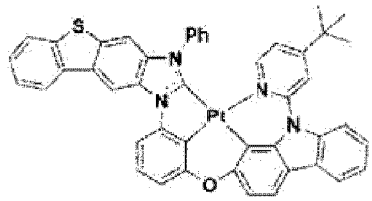

33

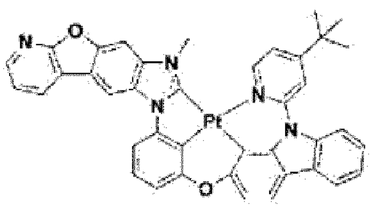

34

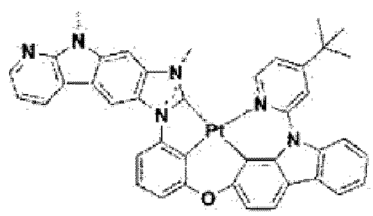

35

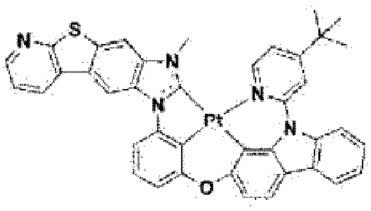

36

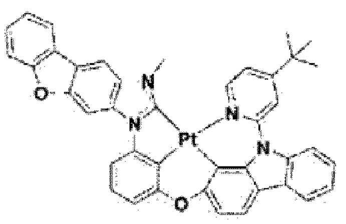

37

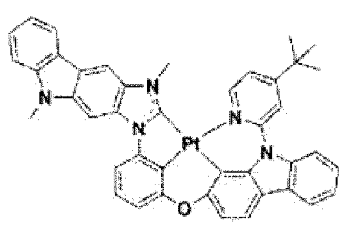

38

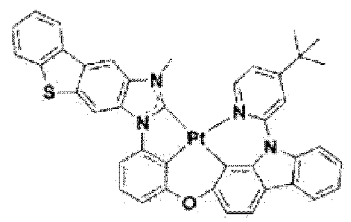

39

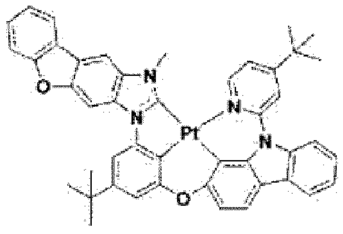

40

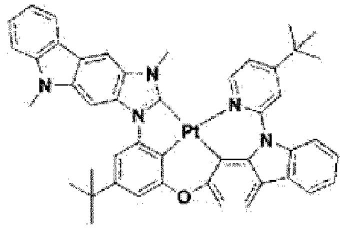

41

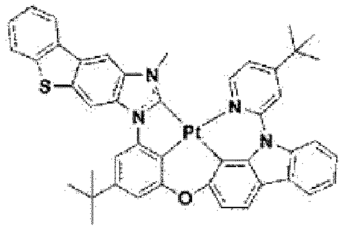

42

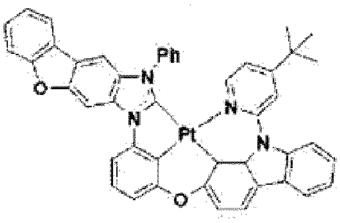

43

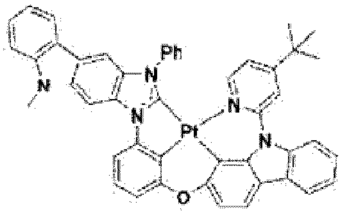

44

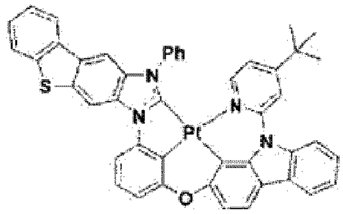

45

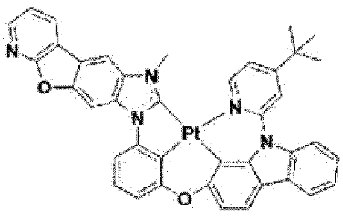

46

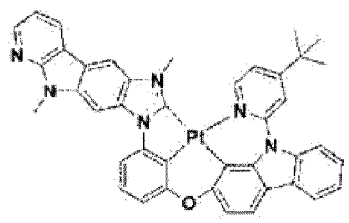

47

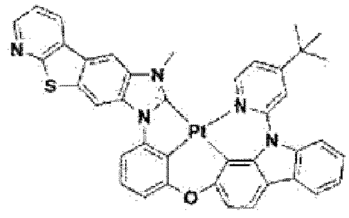

48

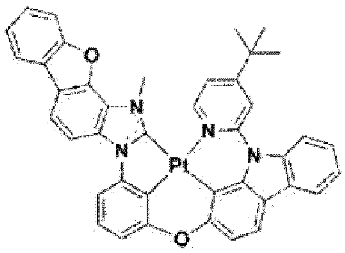

49

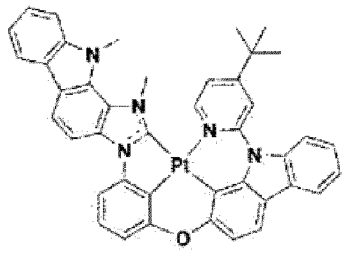

50

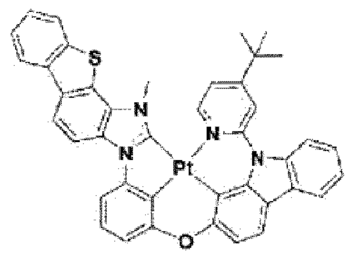

51

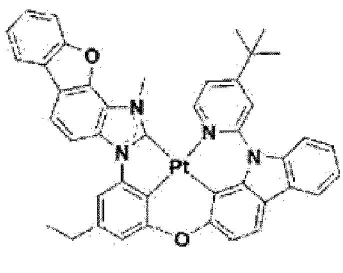

52

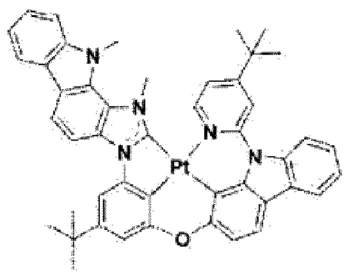

53

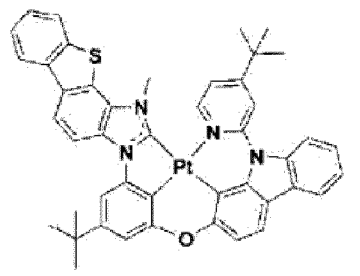

54

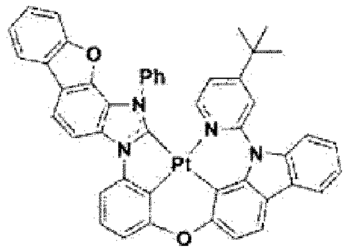
55

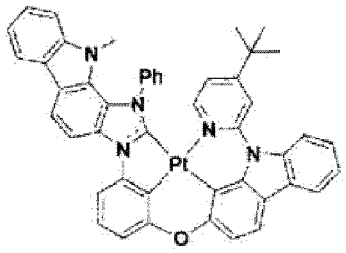
56

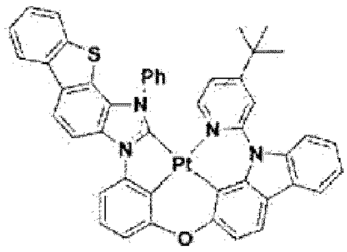
57

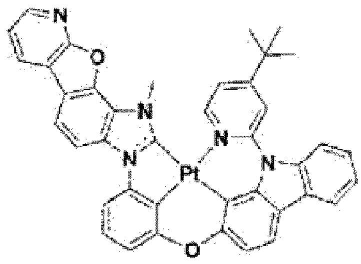
58

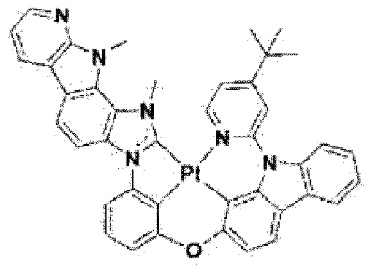
59

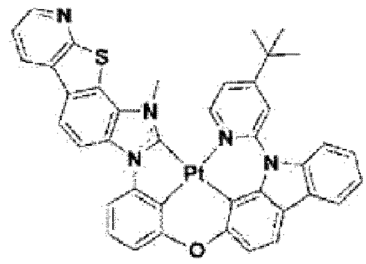
60

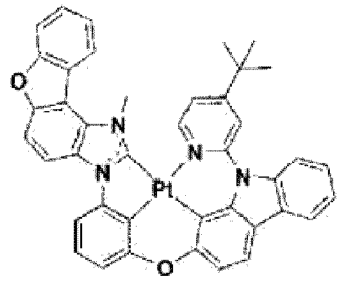
61

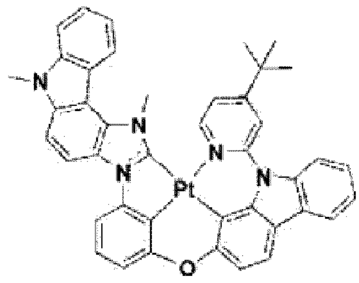
62

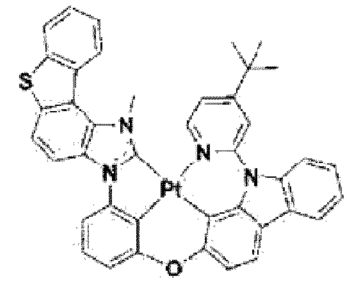
63

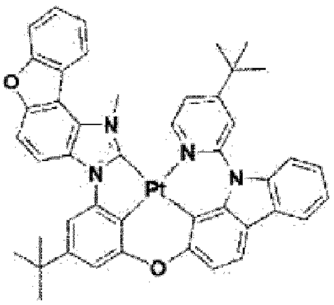
64

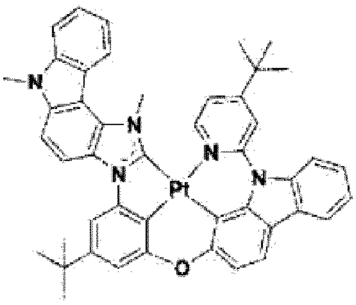
65

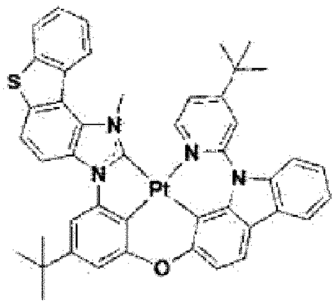
66

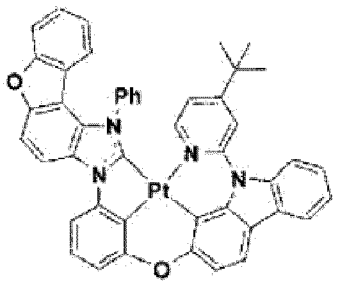
67

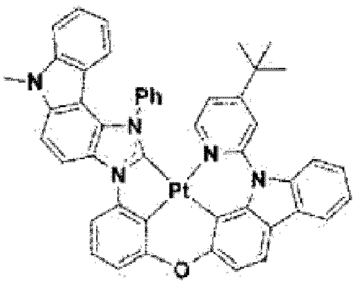
68

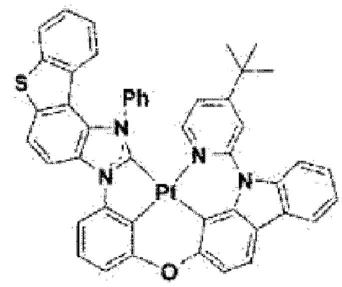
69

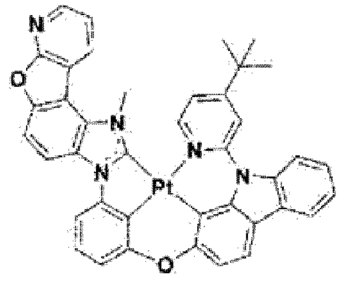

70

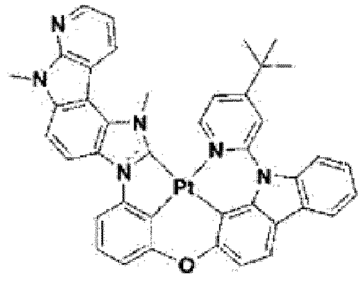

71

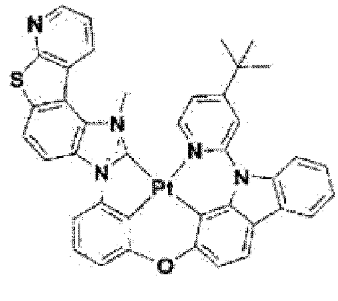

72

**11.** Organische lichtemittierende Vorrichtung, umfassend:

eine erste Elektrode;
eine zweite Elektrode; und
eine organische Schicht, die zwischen der ersten Elektrode und der zweiten Elektrode angeordnet ist, wobei die organische Schicht Folgendes umfasst:

eine Emissionsschicht, und
mindestens eine metallorganische Verbindung nach einem der Ansprüche 1 bis 10.

**12.** Organische lichtemittierende Vorrichtung nach Anspruch 11, wobei

die erste Elektrode eine Anode ist,
die zweite Elektrode eine Kathode ist, und
die organische Schicht einen Lochtransportbereich, angeordnet zwischen der ersten Elektrode und der Emissionsschicht, und einen Elektronentransportbereich, angeordnet zwischen der Emissionsschicht und der zweiten Elektrode, umfasst,
der Lochtransportbereich eine Lochinjektionsschicht, eine Lochtransportschicht, eine Elektronensperrschicht oder eine beliebige Kombination davon umfasst, und
der Elektronentransportbereich eine Lochsperrschicht, eine Elektronentransportschicht, eine Elektroneninjektionsschicht oder eine beliebige Kombination davon umfasst.

**13.** Organische lichtemittierende Vorrichtung nach Anspruch 11 oder 12, wobei die Emissionsschicht die metallorganische Verbindung umfasst.

**14.** Organische lichtemittierende Vorrichtung nach Anspruch 13, wobei die Emissionsschicht ferner einen Wirt umfasst, wobei der Wirt in der Emissionsschicht in einer Menge vorhanden ist, die größer ist als die Menge der metallorganischen Verbindung in der Emissionsschicht; und/oder
wobei die Emissionsschicht blaues Licht mit einer maximalen Emissionswellenlänge in einem Bereich von 430 Nanometern bis 480 Nanometern emittiert.

**15.** Diagnostische Zusammensetzung, umfassend mindestens eine metallorganische Verbindung nach einem der Ansprüche 1 bis 10.

**Revendications**

**1.** Composé organométallique représenté par la formule 1 :

Formule 1

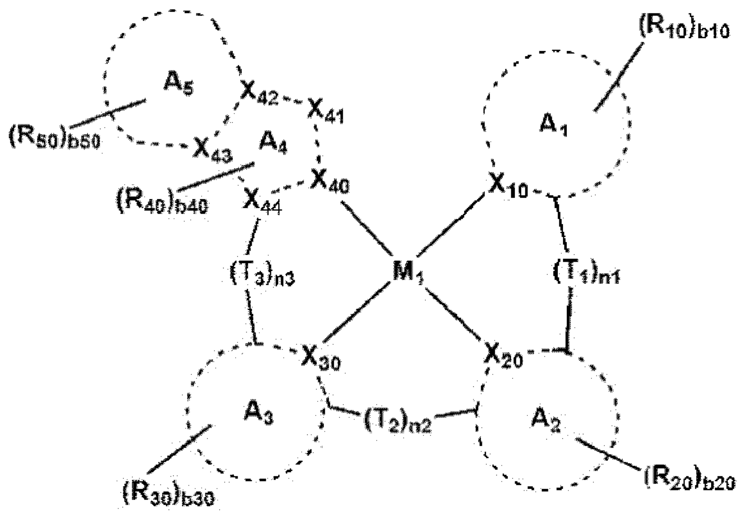

dans lequel, dans la formule 1,

$M_1$ est le béryllium, le magnésium, l'aluminium, le calcium, le titane, le manganèse, le cobalt, le cuivre, le zinc, le gallium, le germanium, le zirconium, le ruthénium, le rhodium, le palladium, l'argent, le rhénium, le platine ou l'or,
$A_1$ à $A_3$ sont chacun indépendamment un groupe carbocyclique en $C_5$ à $C_{30}$ ou un groupe hétérocyclique en $C_1$ à $C_{30}$,
$A_4$ est un groupe hétérocyclique à 5 chaînons,
$A_5$ est un groupe carbocyclique en $C_7$ à $C_{30}$ d'au moins deux cycles comprenant un groupe carbocyclique à 6 chaînons, ou $A_5$ est un groupe hétérocyclique en $C_1$ à $C_{30}$ d'au moins deux cycles comprenant un groupe carbocyclique à 6 chaînons ou un groupe hétérocyclique à 6 chaînons, $X_{10}$, $X_{20}$, $X_{30}$, et $X_{40}$ à $X_{44}$ sont chacun indépendamment C ou N,
$T_1$ et $T_3$ sont chacun indépendamment une simple liaison, *-N[($L_1$)$_{a1}$-($R_1$)b$_1$]-*', *-B($R_1$)-*', *-P($R_1$)-*', *-C($R_1$)($R_2$)-*', *-Si($R_1$)($R_2$)-*', *-Ge($R_1$)($R_2$)-*', *-S-*', *-Se-*', *-O-*', *-C(=O)-*', *-S(=O)-*', *-S(=O)$_2$-*', *-C($R_1$)=C($R_2$)-*', *-C(=S)-*', ou *-C≡C-*',
$T_2$ est une liaison simple, *-N[($L_1$)$_{a1}$-($R_1$)b$_1$]-*', *-P($R_1$)-*', *-C($R_1$)($R_2$)-*', *-Si($R_1$)($R_2$)-*', *-Ge($R_1$)($R_2$)-*', *-S-*', *-Se-*', *-O-*', *-C(=O)-*', *-S(=O)-*', *-S(=O)$_2$-*', *-C($R_1$)=C($R_2$)-*', *-C(=S)-*', ou *-C≡C-*',
* et *' indiquent chacun un site de liaison à un atome adjacent,
n1 à n3 sont chacun indépendamment un nombre entier de 1 à 3,
$L_1$ est une simple liaison, un groupe carbocyclique en $C_5$ à $C_{30}$ substitué ou non substitué, ou un groupe hétérocyclique en $C_1$ à $C_{30}$ substitué ou non substitué,
a1 est un nombre entier de 1 à 3, et lorsque a1 est égal ou supérieur à 2, au moins deux groupes $L_1$ sont identiques ou différents l'un de l'autre,
$R_1$, $R_2$, $R_{10}$, $R_{20}$, $R_{30}$, $R_{40}$, et $R_{50}$ sont chacun indépendamment l'hydrogène, le deutérium, -F, -Cl, - Br, -I, -$SF_5$, un groupe hydroxyle, un groupe cyano, un groupe nitro, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en $C_1$ à $C_{60}$ substitué ou non substitué, un groupe alcényle en $C_2$ à $C_{60}$ substitué ou non substitué, un groupe alcynyle en $C_2$ à $C_{60}$ substitué ou non substitué, un groupe alcoxy en $C_1$ à $C_{60}$ substitué ou non substitué, un groupe cycloalkyle en $C_3$ à $C_{10}$ substitué ou non substitué, un groupe hétérocycloalkyle en $C_1$ à $C_{10}$ substitué ou non substitué, un groupe cycloalcényle en $C_3$ à $C_{10}$ substitué ou non substitué, un groupe hétérocycloalcényle en $C_1$ à $C_{10}$ substitué ou non substitué, un groupe aryle en $C_6$ à $C_{60}$ substitué ou non substitué, un groupe aryloxy en $C_6$ à $C_{60}$ substitué ou non substitué, un groupe arylthio en $C_6$ à $C_{60}$ substitué ou non substitué, un groupe arylalkyle en $C_7$ à $C_{60}$ substitué ou non substitué, un groupe hétéroaryle en $C_1$ à $C_{60}$ substitué ou non substitué, un groupe hétéroaryloxy en $C_1$ à $C_{60}$ substitué ou non substitué, un groupe hétéroarylthio en $C_1$ à $C_{60}$ substitué ou non substitué, un groupe hétéroarylalkyle en $C_2$ à $C_{60}$ substitué ou non substitué, un groupe polycyclique condensé non aromatique monovalent substitué ou non substitué, un groupe hétéropolycyclique condensé non aromatique monovalent substitué ou non substitué, -N($Q_1$)($Q_2$), -Si($Q_3$)($Q_4$)($Q_5$), -Ge($Q_3$)($Q_4$)($Q_5$), -B($Q_6$)($Q_7$), -P($Q_8$)($Q_9$), ou

$-P(=O)(Q_8)(Q_9)$,

au moins deux groupes $R_1$, $R_2$, $R_{10}$, $R_{20}$, $R_{30}$, $R_{40}$, ou $R_{50}$ adjacents sont éventuellement liés ensemble pour former un groupe carbocyclique en $C_5$ à $C_{30}$ substitué ou non substitué ou un groupe hétérocyclique en $C_1$ à $C_{30}$ substitué ou non substitué,

b1 est un nombre entier de 1 à 5, et lorsque b1 est égal ou supérieur à 2, au moins deux groupes $R_1$ sont identiques ou différents l'un de l'autre,

b10, b20, b30, et b50 représentent chacun indépendamment un nombre entier de 1 à 10,

b40 est un nombre entier de 1 à 3 ;

lorsque b 10 est égal ou supérieur à 2, au moins deux groupes $R_{10}$ sont identiques ou différents les uns des autres, lorsque b20 est égal ou supérieur à 2, au moins deux groupes $R_{20}$ sont identiques ou différents les uns des autres, lorsque b30 est égal ou supérieur à 2, au moins deux groupes $R_{30}$ sont identiques ou différents les uns des autres, lorsque b40 est égal ou supérieur à 2, au moins deux groupes $R_{40}$ sont identiques ou différents les uns des autres, lorsque b50 est égal ou supérieur à 2, au moins deux groupes $R_{50}$ sont identiques ou différents les uns des autres, et

au moins un substituant du groupe carbocyclique en $C_5$ à $C_{30}$ substitué, du groupe hétérocyclique en $C_1$ à $C_{30}$ substitué, du groupe alkyle en $C_1$ à $C_{60}$ substitué, du groupe alcényle en $C_2$ à $C_{60}$ substitué, du groupe alcynyle en $C_2$ à $C_{60}$ substitué, du groupe alcoxy en $C_1$ à $C_{60}$ substitué, du groupe cycloalkyle en $C_3$ à $C_{10}$ substitué, du groupe hétérocycloalkyle en $C_1$ à $C_{10}$ substitué, du groupe cycloalcényle en $C_3$ à $C_{10}$ substitué, du groupe hétérocycloalcényle en $C_1$ à $C_{10}$ substitué, du groupe aryle en $C_6$ à $C_{60}$ substitué, du groupe aryloxy en $C_6$ à $C_{60}$ substitué, du groupe arylthio en $C_6$ à $C_{60}$ substitué, du groupe arylalkyle en $C_7$ à $C_{60}$ substitué, du groupe hétéroaryle en $C_1$ à $C_{60}$ substitué, , du groupe hétéroaryloxy en $C_1$ à $C_{60}$ substitué, du groupe hétéroarylthio en $C_1$ à $C_{60}$ substitué, du groupe hétéroarylalkyle en $C_2$ à $C_{60}$ substitué, du groupe polycyclique condensé non aromatique monovalent substitué, ou du groupe hétéropolycyclique condensé non aromatique monovalent substitué est :

le deutérium, -F, -Cl, -Br, -I, $-CD_3$, $-CD_2H$, $-CDH_2$, $-CF_3$, $-CF_2H$, $-CFH_2$, un groupe hydroxyle, un groupe cyano, un groupe nitro, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en $C_1$ à $C_{60}$, un groupe alcényle en $C_2$ à $C_{60}$, un groupe alcynyle en $C_2$ à $C_{60}$, ou un groupe alcoxy en $C_1$ à $C_{60}$ ;

un groupe alkyle en $C_1$ à $C_{60}$, un groupe alcényle en $C_2$ à $C_{60}$, un groupe alcynyle en $C_2$ à $C_{60}$, un groupe alcoxy en $C_1$ à $C_{60}$, chacun substitué par au moins l'un choisi parmi le deutérium, -F, -Cl, -Br, -I, $-CD_3$, $-CD_2H$, $-CDH_2$, $-CF_3$, $-CF_2H$, $-CFH_2$, un groupe hydroxyle, un groupe cyano, un groupe nitro, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe cycloalkyle en $C_3$ à $C_{10}$, un groupe hétérocycloalkyle en $C_1$ à $C_{10}$, un groupe cycloalcényle en $C_3$ à $C_{10}$, un groupe hétérocycloalcényle en $C_1$ à $C_{10}$, un groupe aryle en $C_6$ à $C_{60}$, un groupe aryloxy en $C_6$ à $C_{60}$, un groupe arylthio en $C_6$ à $C_{60}$, un groupe arylalkyle en $C_7$ à $C_{60}$, un groupe hétéroaryle en $C_1$ à $C_{60}$, un groupe hétéroaryloxy en $C_1$ à $C_{60}$, un groupe hétéroarylthio en $C_1$ à $C_{60}$, un groupe hétéroarylalkyle en $C_2$ à $C_{60}$, un groupe polycyclique condensé non aromatique monovalent, un groupe hétéropolycyclique condensé non aromatique monovalent, $-N(Q_{11})(Q_{12})$, - $Si(Q_{13})(Q_{14})(Q_{15})$, $-Ge(Q_{13})(Q_{14})(Q_{15})$, - $B(Q_{16})(Q_{17})$, $-P(Q_{18})(Q_{19})$, OU $-P(=O)(Q_{18})(Q_{19})$ ;

un groupe cycloalkyle en $C_3$ à $C_{10}$, un groupe hétérocycloalkyle en $C_1$ à $C_{10}$, un groupe cycloalcényle en $C_3$ à $C_{10}$, un groupe hétérocycloalcényle en $C_1$ à $C_{10}$, un groupe aryle en $C_6$ à $C_{60}$, un groupe aryloxy en $C_6$ à $C_{60}$, un groupe arylthio en $C_6$ à $C_{60}$, un groupe hétéroaryle en $C_1$ à $C_{60}$, un groupe polycyclique condensé non aromatique monovalent ou un groupe hétéropolycyclique condensé non aromatique monovalent ;

un groupe cycloalkyle en $C_3$ à $C_{10}$, un groupe hétérocycloalkyle en $C_1$ à $C_{10}$, un groupe cycloalcényle en $C_3$ à $C_{10}$, un groupe hétérocycloalcényle en Ci à $C_{10}$, un groupe aryle en $C_6$ à $C_{60}$, un groupe aryloxy en $C_6$ à $C_{60}$, un groupe arylthio en $C_6$ à $C_{60}$, un groupe hétéroaryle en $C_1$ à $C_{60}$, un groupe polycyclique condensé non aromatique monovalent, ou un groupe hétéropolycyclique condensé non aromatique monovalent, chacun substitué par au moins l'un choisi parmi le deutérium, -F, -Cl, -Br, -I, $-CD_3$, $-CD_2H$, $-CDH_2$, $-CF_3$, $-CF_2H$, $-CFH_2$, un groupe hydroxyle, un groupe cyano, un groupe nitro, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en $C_1$ à $C_{60}$, un groupe alcényle en $C_2$ à $C_{60}$, un groupe alcynyle en $C_2$ à $C_{60}$, un groupe alcoxy en $C_1$ à $C_{60}$, un groupe cycloalkyle en $C_3$ à $C_{10}$, un groupe hétérocycloalkyle en $C_1$ à $C_{10}$, un groupe cycloalcényle en $C_3$ à $C_{10}$, un groupe hétérocycloalcényle en $C_1$ à $C_{10}$, un groupe aryle en $C_6$ à $C_{60}$, un groupe aryloxy en

$C_6$ à $C_{60}$, un groupe arylthio en $C_6$ à $C_{60}$, un groupe arylalkyle en $C_7$ à $C_{60}$, un groupe hétéroaryle en $C_1$ à $C_{60}$, un groupe hétéroaryloxy en $C_1$ à $C_{60}$, un groupe hétéroarylthio en $C_1$ à $C_{60}$, un groupe hétéroarylalkyle en $C_2$ à $C_{60}$, un groupe polycyclique condensé non aromatique monovalent, un groupe hétéropolycyclique condensé non aromatique monovalent, $-N(Q_{21})(Q_{22})$, - $Si(Q_{23})(Q_{24})(Q_{25})$, $-Ge(Q_{23})(Q_{24})(Q_{25})$, $-B(Q_{26})(Q_{27})$, $-P(Q_{28})(Q_{29})$, ou $-P(=O)(Q_{28})(Q_{29})$ ; ou $-N(Q_{31})(Q_{32})$, $-Si(Q_{33})(Q_{34})(Q_{35})$, $-Ge(Q_{33})(Q_{34})(Q_{35})$, $-B(Q_{36})(Q_{37})$, $-P(Q_{38})(Q_{39})$, ou $-P(=O)(Q_{38})(Q_{39})$,

dans lequel $Q_1$ à $Q_9$, $Q_{11}$ à $Q_{19}$, $Q_{21}$ à $Q_{29}$ et $Q_{31}$ à $Q_{39}$ sont chacun indépendamment l'hydrogène ; -F ; -Cl ; -Br ; -I ; un groupe hydroxyle, un groupe cyano, un groupe nitro, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en $C_1$ à $C_{60}$, un groupe alcényle en $C_2$ à $C_{60}$ ; un groupe alcynyle en $C_2$ à $C_{60}$ ; un groupe alkoxy en $C_1$ à $C_{60}$ ; un groupe cycloalkyle en $C_3$ à $C_{10}$ ; un groupe hétérocycloalkyle en $C_1$ à $C_{10}$ ; un groupe cycloalcényle en $C_3$ à $C_{10}$ ; un groupe hétérocycloalcényle en $C_1$ à $C_{10}$ un groupe aryle en $C_6$ à $C_{60}$ ; un groupe aryle en $C_6$ à $C_{60}$ substitué par un groupe alkyle en $C_1$ à $C_{60}$, un groupe aryle en $C_6$ à $C_{60}$, ou une combinaison de ceux-ci ; un groupe aryloxy en $C_6$ à $C_{60}$ ; un groupe arylthio en $C_6$ à $C_{60}$ ; un groupe arylalkyle en $C_7$ à $C_{60}$ ; un groupe hétéroaryle en $C_1$ à $C_{60}$ ; un groupe hétéroaryloxy en $C_1$ à $C_{60}$ ; un groupe hétéroarylthio en $C_1$ à $C_{60}$ ; un groupe hétéroarylalkyle en $C_2$ à $C_{60}$ ; un groupe polycyclique condensé non aromatique monovalent ; ou un groupe hétéropolycyclique condensé non aromatique monovalent.

**2.** Composé organométallique selon la revendication 1, dans lequel $M_1$ est Pt, Pd ou Au ; et/ou dans lequel $A_1$ à $A_3$ sont chacun indépendamment un groupe phényle, un groupe naphthyle, un groupe anthracényle, un groupe phénanthrényle, un groupe triphénylényle, un groupe pyrényle, un groupe chrysényle, un groupe cyclopentadiène, un groupe 1,2,3,4-tétrahydronaphtyle, un groupe furanyle, un groupe thiophényle, un groupe silolyle, un groupe indényle, un groupe fluorényle, un groupe indolyle, un grope isoindolyle, un groupe carbazolyle, un groupe benzocarbazolyle, un groupe dibenzocarbazolyle, un groupe benzofuranyle, un groupe dibenzofuranyle, un groupe benzothiophényle, un groupe benzosilolyle, un groupe dibenzosilolyle, un groupe azafluorényle, un groupe azacarbazolyle, un groupe azadibenzofuranyle, un groupe azadibenzothiophényle, un groupe azadibenzosilolyle, un groupe pyridinyle, un groupe imidazopyridinyle, un groupe pyrimidinyle, un groupe imidazopyrimidinyle, un groupe pyrazinyle, un groupe pyridazinyle, un groupe triazinyle, un groupe purinyle, un groupe quinolinyle, un groupe benzoquinolinyle, un groupe isoquinolinyle, un groupe quinoxalinyle, un groupe quinazolinyle, un groupe cinnolinyle, un groupe phénanthrolinyle, un groupe pyrrolyle, un groupe pyrazolyle, un groupe imidazolyle, un groupe triazolyle, un groupe tétrazolyle, un groupe oxazolyle, un groupe isoxazolyle, un groupe thiazolyle, un groupe isothiazolyle, un groupe oxadiazolyle, un groupe thiadiazolyle, un groupe benzopyrazolyle, un groupe benzimidazolyle, un groupe indazolyle, un groupe benzoxazolyle, un groupe isobenzoxazolyle, un groupe benzothiazolyle, un groupe benzoxadiazolyle, un groupe benzothiadiazolyle, un groupe benzotriazolyle, un groupe diazaindenyle, un groupe triazaindényle, un groupe 5,6,7,8-tétrahydroisoquinolinyle, ou un groupe 5,6,7,8-tétrahydroquinolinyle.

**3.** Composé organométallique selon la revendication 1 ou 2, dans lequel $X_{40}$ dans $A_4$ est C, et $X_{41}$ et $X_{44}$ sont chacun N ; et/ou dans lequel $A_5$ est représenté par l'une quelconque des formules A5-1 à A5-6 :

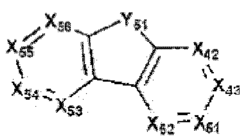
A5-1

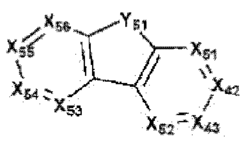
A5-2

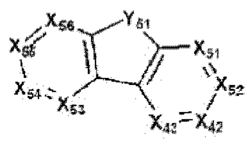
A5-3

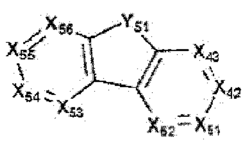
A5-4

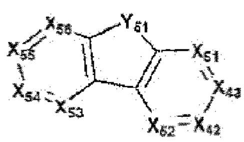
A5-5

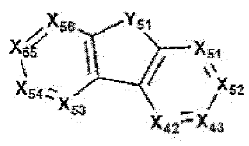
A5-6

dans lequel, dans les formules A5-1 à A5-6,

$Y_{51}$ est *-O-*', *-S-*', *-N($R_5$)-*', *-C($R_5$)($R_6$)-*', *-Si($R_5$)($R_6$)-*', *-B($R_5$)-*', *-P($R_5$)-*' ou *-P(=O)($R_5$)-*', dans

lequel * et *' indiquent chacun un site de liaison à un atome adjacent,
$R_5$ et $R_6$ s'entendent respectivement en référence à $R_1$ et $R_2$ dans la revendication 1,
$X_{42}$ et $X_{43}$ s'entendent respectivement en référence à $X_{42}$ et $X_{43}$ dans la revendication 1, et
$X_{51}$ à $X_{56}$ sont chacun indépendamment C ou N.

**4.** Composé organométallique selon l'une quelconque des revendications 1 à 3, dans lequel $X_{10}$ est N, et $X_{20}$, $X_{30}$ et $X_{40}$ sont chacun C ; et/ou

dans lequel
une liaison entre $M_1$ et $X_{10}$ est une liaison coordonnée,
une liaison entre $M_1$ et $X_{20}$ est une liaison covalente,
une liaison entre $M_1$ et $X_{30}$ est une liaison covalente, et
une liaison entre $M_1$ et $X_{40}$ est une liaison coordonnée ; et/ou
dans lequel $T_1$ à $T_3$ sont chacun indépendamment une simple liaison, *-N[$(L_1)_{a1}$-$(R_1)b_1$]-*', *-C($R_1$)($R_2$)-*', *-Si($R_1$)($R_2$)-*', *-O-*', ou *-S-*', dans lequel * et *' indiquent chacun un site de liaison à un atome adjacent.

**5.** Composé organométallique selon l'une quelconque des revendications 1 à 4, dans lequel $L_1$ est :

un groupe phénylène, un groupe pentalénylène, un groupe indénylène, un groupe naphtylène, un groupe azulénylène, un groupe heptalénylène, un groupe acénaphtylène, un groupe fluorénylène, un groupe phénalénylène, un groupe phénanthrénylène, un groupe anthracénylène, un groupe fluoranthénylène, un groupe triphénylénylène, un groupe pyrénylène, un groupe chrysénylénylène, un groupe naphtacénylène, un groupe picénylène, un groupe pérylénylène ou un groupe pentacénylène ; ou
un groupe phénylène, un groupe pentalénylène, un groupe indénylène, un groupe naphtylène, un groupe azulénylène, un groupe heptalénylène, un groupe acénaphtylène, un groupe fluorénylène, un groupe phénalénylène, un groupe phénanthrénylène, un groupe anthracénylène, un groupe fluoranthénylène, un groupe triphénylénylène, un groupe pyrénylène, un groupe chrysénylénylène, un groupe naphthacénylène, un groupe picénylène, un groupe pérylénylène, ou un groupe pentacénylène, chacun substitué par au moins l'un parmi le deuterium, -F, -Cl, -Br, -I, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en $C_1$ à $C_{60}$, un groupe alcényle en $C_2$ à $C_{60}$, un groupe alcynyle en $C_2$ à $C_{60}$, un groupe alkoxy en $C_1$ à $C_{60}$, un groupe cycloalkyle en $C_3$ à $C_{10}$, un groupe cycloalcényle en $C_3$ à $C_{10}$, un groupe hétérocycloalkyle en Ci à Cio, un groupe hétérocycloalcényle en $C_1$ à Cio, un groupe aryle en $C_6$ à $C_{60}$, un groupe aryloxy en $C_6$ à $C_{60}$, un groupe arylthio en $C_6$ à $C_{60}$, un groupe hétéroaryle en $C_1$ à $C_{60}$, un groupe polycyclique condensé non aromatique monovalent, ou un groupe hétéropolycyclique condensé non aromatique monovalent.

**6.** Composé organométallique selon l'une quelconque des revendications 1 à 5, dans lequel Ri, $R_2$, $R_{10}$, $R_{20}$, $R_{30}$, $R_{40}$, et $R_{50}$ sont chacun indépendamment :

hydrogène, deutérium, -F, -Cl, -Br, -I, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, $-SF_5$, un groupe alkyle en $C_1$ à $C_{20}$, ou un groupe alcoxy en $C_1$ à $C_{20}$ ;
un groupe alkyle en $C_1$ à $C_{20}$ ou un groupe alcoxy en $C_1$ à $C_{20}$, chacun substitué par au moins un choisi parmi le deutérium, -F, -Cl, -Br, -I, $-CD_3$, $-CD_2H$, $-CDH_2$, $-CF_3$, $-CF_2H$, $-CFH_2$, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en $C_1$ à $C_{10}$, un groupe alkoxy en $C_1$ à $C_{10}$, un groupe cyclopentyle, un groupe cyclohexyle, un groupe cycloheptyle, un groupe cyclooctyle, un groupe adamantanyle, un groupe norbornanyle, un groupe norbornényle, un groupe cyclopentényle, un cyclohexényle, un groupe cycloheptényle, un groupe phényle, un groupe naphtyle, un groupe pyridinyle ou un groupe pyrimidinyle ;
un groupe cyclopentyle, un groupe cyclohexyle, un groupe cycloheptyle, un groupe cyclooctyle, un groupe adamantanyle, un groupe norbornanyle, un groupe norbornényle, un groupe cyclopentényle, un groupe cyclohexényle, un groupe cycloheptényle, un groupe phényle, un groupe naphtyle, un groupe fluorényle, un groupe phénanthrényle, un groupe anthracényle, un groupe fluoranthényle, un groupe triphénylényle, un groupe pyrényle, un groupe chrysényle, un groupe pyrrolyle, un groupe thiophényle, un groupe furanyle, un groupe imidazolyle, un groupe pyrazolyle, un groupe thiazolyle, un groupe isothiazolyle, un groupe oxazolyle, un groupe

isoxazolyle, un groupe pyridinyle, un groupe pyrazinyle, un groupe pyrimidinyle, un groupe pyridazinyle, un groupe isoindolyle, un groupe indolyle, un groupe indazolyle, un groupe purinyle, un groupe quinolinyle, un groupe isoquinolinyle, un groupe benzoquinolinyle, un groupe quinoxalinyle, un groupe quinazolinyle, un groupe cinnolinyle, un groupe carbazolyle, un groupe phénanthrolinyle, un groupe benzimidazolyle, un groupe benzofuranyle, un groupe benzothiophényle, un groupe isobenzothiazolyle, un groupe benzoxazolyle, un groupe isobenzoxazolyle, un groupe triazolyle, un groupe tétrazolyle, un groupe oxadiazolyle, un groupe triazinyle, un groupe dibenzofuranyle, un groupe dibenzothiophényle, un groupe benzocarbazolyle, un groupe dibenzocarbazolyle, un groupe imidazopyridinyle , ou un groupe imidazopyrimidinyle ;

un groupe cyclopentyle, un groupe cyclohéxyle, un groupe cyclohéptyle, un groupe cyclooctyle, un groupe adamantanyle, un groupe norbornanyle, un groupe norbornényle, un groupe cyclopentényle, un groupe cyclohexényle, un groupe cycloheptényle, un groupe phényle, un groupe naphthyle, un groupe fluorényle, un groupe phénanthrényle, un groupe anthracényle, un groupe fluoranthényle, un groupe triphénylényle, un groupe pyrényle, un groupe chrysényle, un groupe pyrrolyle, un groupe thiophényle, un groupe furanyle, un groupe imidazolyle, un groupe pyrazolyle, un groupe thiazolyle, un groupe isothiazolyle, un groupe oxazolyle, un groupe isoxazolyle, un groupe pyridinyle, un groupe pyrazinyle, un groupe pyrimidinyle, un groupe pyridazinyle, un groupe isoindolyle, un groupe indolyle, un groupe indazolyle, un groupe purinyle, un groupe quinolinyle, un groupe isoquinolinyle, un groupe benzoquinolinyle, un groupe quinoxalinyle, un groupe quinazolinyle, un groupe cinnolinyle, un groupe carbazolyle, un groupe phenanthrolinyle, un groupe benzimidazolyle, un groupe benzofuranyle, un groupe benzothiophéyle, un groupe isobenzothiazolyle, un groupe benzoxazolyle, un groupe isobenzoxazolyle, un groupe triazolyle, un groupe térazolyle, un groupe oxadiazolyle, un groupe triazinyle, un groupe dibenzofuranyle, un groupe dibenzothiophéyle, un groupe benzocarbazolyle, un groupe dibenzocarbazolyle, un groupe imidazopyridinyle, ou un groupe imidazopyrimidinyle, chacun substitué par au moins l'un du deuterium, -F, -Cl, -Br, -I, $-CD_3$,

$-CD_2H$, $-CDH_2$, $-CF_3$, $-CF_2H$, $-CFH_2$, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe akyle en $C_1$ à $C_{10}$, un groupe alkoxy en $C_1$ à $C_{10}$, un groupe cyclopentyle, un groupe cyclohexyle, un groupe cycloheptyle, un groupe cyclooctyle, un groupe adamantanyle, un groupe norbornanyle, un groupe norbornényle, un groupe cyclopentényle, un groupe cyclohexényle, un groupe cycloheptényle, un groupe phényle, un groupe naphthyle, un groupe pyridinyle, ou un groupe pyrimidinyle ; ou

$-N(Q_1)(Q_2)$, $-Si(Q_3)(Q_4)(Q_5)$, $-Ge(Q_3)(Q_4)(Q_5)$, $-B(Q_6)(Q_7)$, $-P(Q_8)(Q_9)$, ou $-P(=O)(Q_8)(Q_9)$, dans lequel $Q_1$ à $Q_9$ sont chacun indépendamment :

$-CH_3$, $-CD_3$, $-CD_2H$, $-CDH_2$, $-CH_2CH_3$, $-CH_2CD_3$, $-CH_2CD_2H$, $-CH_2CDH_2$, $-CHDCH_3$, $-CHDCD_2H$, $-CHDCDH_2$, $-CHDCD_3$, $-CD_2CD_3$, $-CD_2CD_2H$, ou $-CD_2CDH_2$ ;

un groupe n-propyle, un groupe iso-propyle, un groupe n-butyle, un groupe iso-butyle, un groupe sec-butyle, un groupe tert-butyle, un groupe n-pentyle, un groupe iso-pentyle, un groupe sec-pentyle, un groupe tert-pentyle, un groupe phényle ou un groupe naphtyle ; ou

un groupe n-propyle, un groupe iso-propyle, un groupe n-butyle, un groupe iso-butyle, un groupe sec-butyle, un groupe tert-butyle, un groupe n-pentyle, un groupe iso-pentyle, un groupe sec-pentyle, un groupe tert-pentyle, un groupe phényle et un groupe naphtyle, chacun substitué par au moins un choisi parmi le deutérium, un groupe alkyle en $C_1$ à $C_{10}$ ou un groupe phényle.

**7.** Composé organométallique selon l'une quelconque des revendications 1 à 6, dans lequel $R_1$, $R_2$, $R_{10}$, $R_{20}$, $R_{30}$, $R_{40}$, et $R_{50}$ sont chacun indépendamment hydrogène, deutérium, -F, un groupe cyano, un groupe nitro, $-SF_5$, $-CH_3$, $-CD_3$, $-CD_2H$, $-CDH_2$, $-CF_3$, $-CF_2H$, $-CFH_2$, un groupe représenté par l'une quelconque des formules 9-1 à 9-19, ou un groupe représenté par l'une quelconque des formules 10-1 à 10-194 :

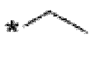
9-1

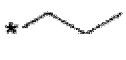
9-2

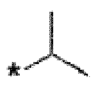
9-3

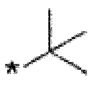
9-4

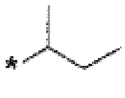
9-5

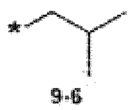
9-6

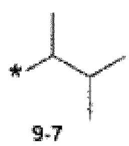
9-7

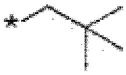
9-8
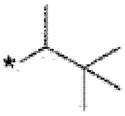
9-9
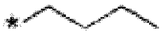
9-10
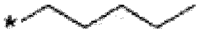
9-11
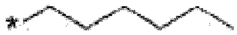
9-12

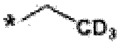
9-13
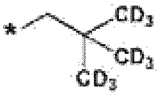
9-14
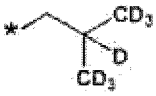
9-15
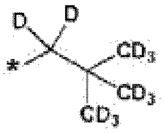
9-16
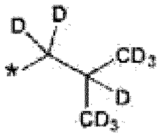
9-17
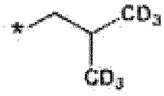
9-18

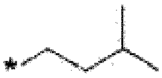
9-19

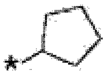
10-1
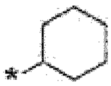
10-2
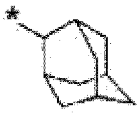
10-3
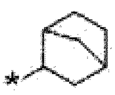
10-4
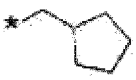
10-5
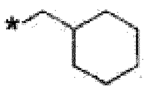
10-6
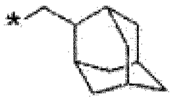
10-7

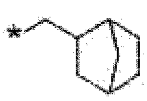
10-8
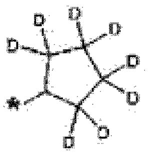
10-9
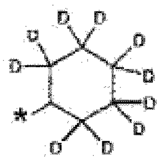
10-10
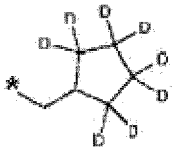
10-11
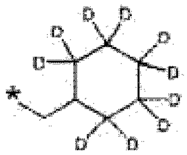
10-12
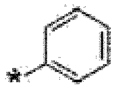
10-13

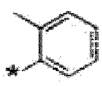
10-14
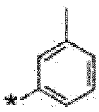
10-15
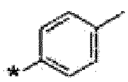
10-16
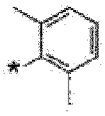
10-17
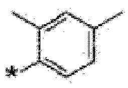
10-18
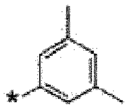
10-19
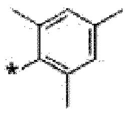
10-20

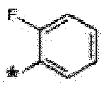
10-21
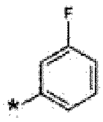
10-22
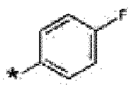
10-23
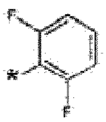
10-24
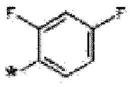
10-25
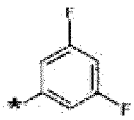
10-26
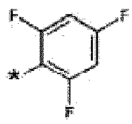
10-27

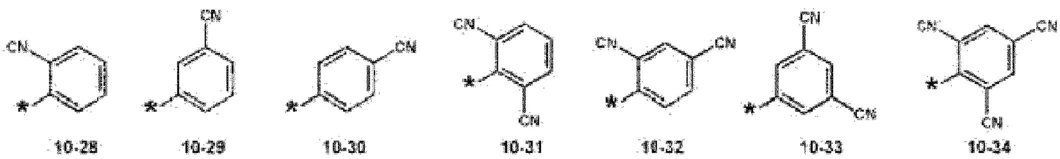
10-28 10-29 10-30 10-31 10-32 10-33 10-34

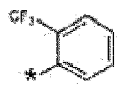 10-35 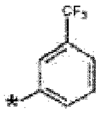 10-36 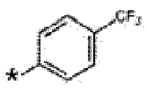 10-37 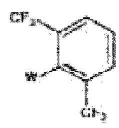 10-38 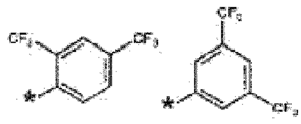 10-39 10-40 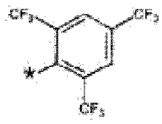 10-41

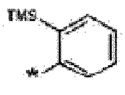 10-42 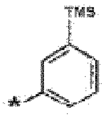 10-43 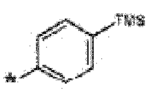 10-44 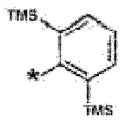 10-45 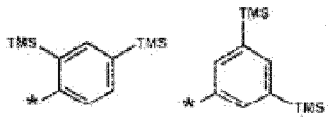 10-46 10-47 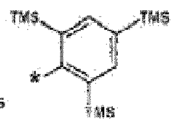 10-48

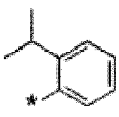 10-49 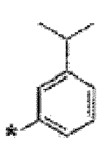 10-50 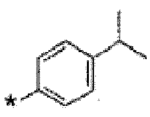 10-51 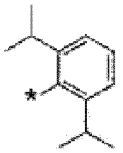 10-52 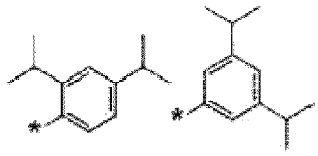 10-53 10-54 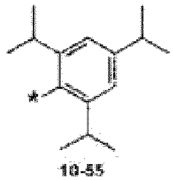 10-55

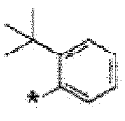 10-56 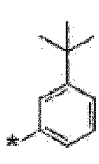 10-57 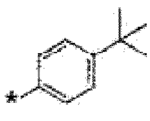 10-58 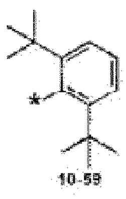 10-59 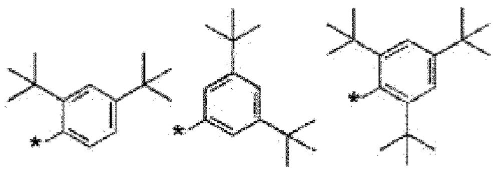 10-60 10-61 10-62

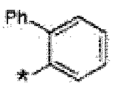 10-63 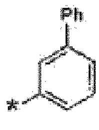 10-64 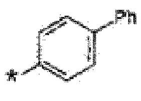 10-65 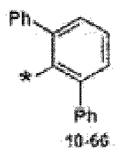 10-66 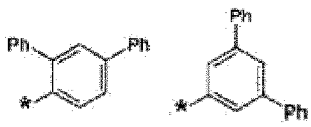 10-67 10-68 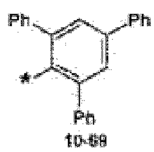 10-69

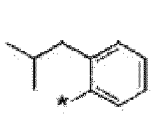 10-70 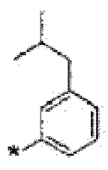 10-71 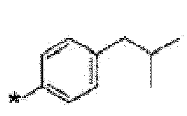 10-72 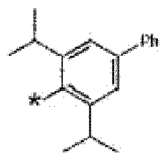 10-73 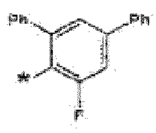 10-74 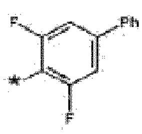 10-75

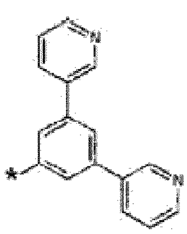 10-76 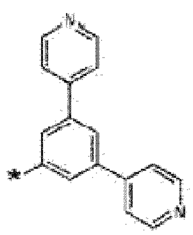 10-77 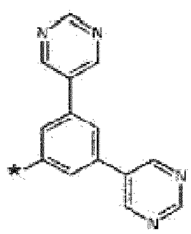 10-78 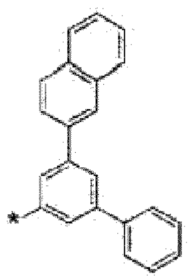 10-79 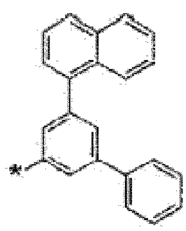 10-80

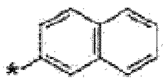
10-81
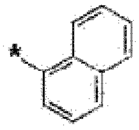
10-82
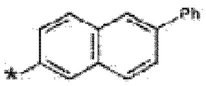
10-83
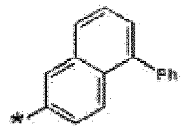
10-84
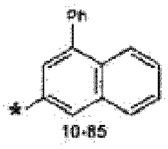
10-85

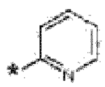
10-86
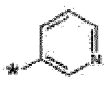
10-87
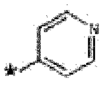
10-88
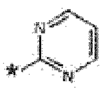
10-89
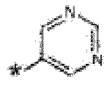
10-90
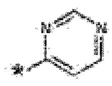
10-91
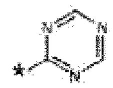
10-92

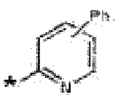
10-93
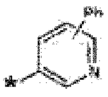
10-94
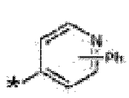
10-95
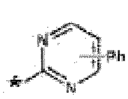
10-96
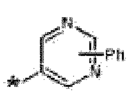
10-97
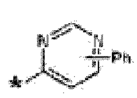
10-98
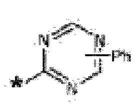
10-99

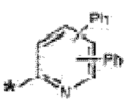
10-100
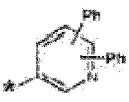
10-101
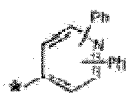
10-102
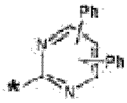
10-103
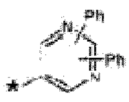
10-104
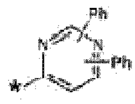
10-105
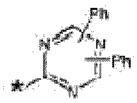
10-106

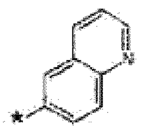
10-107
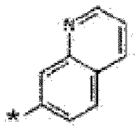
10-108
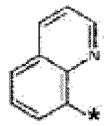
10-109
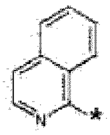
10-110
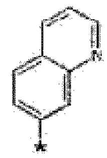
10-111
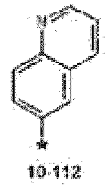
10-112

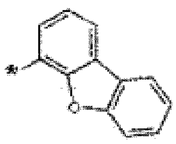
10-113
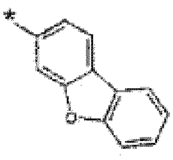
10-114
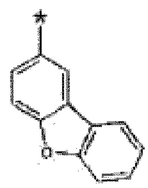
10-115
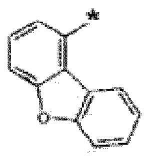
10-116
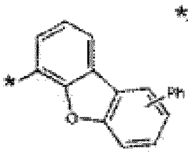
10-117
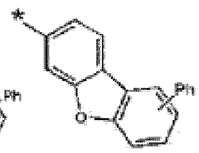
10-118

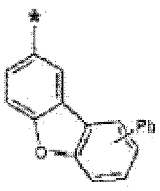
10-119
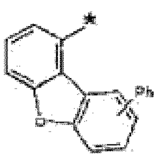
10-120
10-121
10-122
10-123
10-124

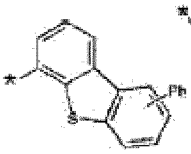
10-125
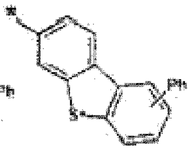
10-126
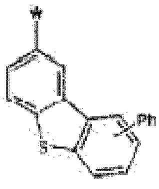
10-127
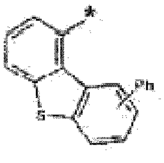
10-128
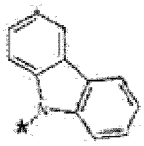
10-129
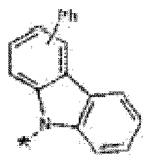
10-130

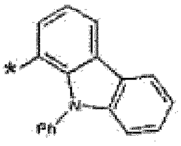
10-131
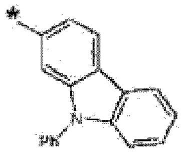
10-132
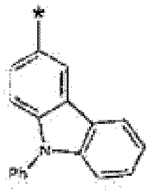
10-133
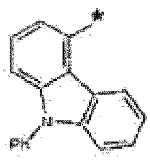
10-134
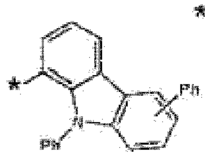
10-135
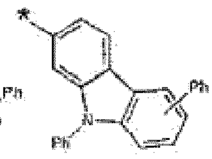
10-136

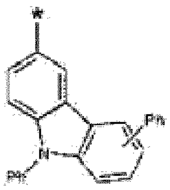
10-137
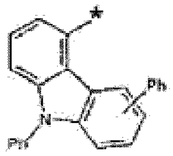
10-138
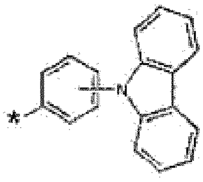
10-139
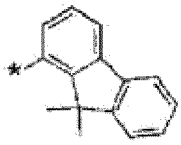
10-140
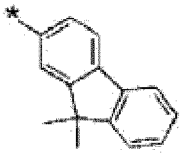
10-141
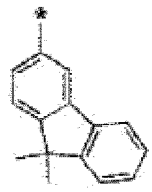
10-142

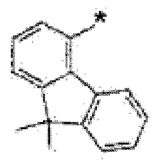
10-143
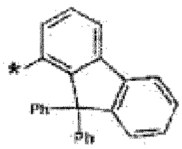
10-144
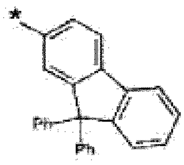
10-145
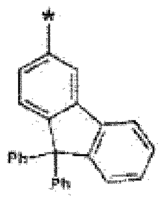
10-146
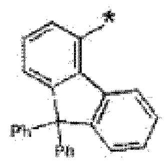
10-147

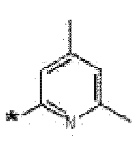
10-148
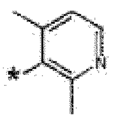
10-149
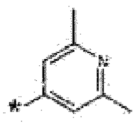
10-150
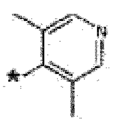
10-151
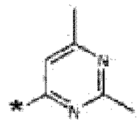
10-152
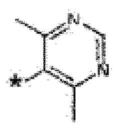
10-153
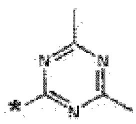
10-154

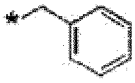
10-155
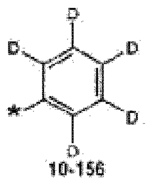
10-156
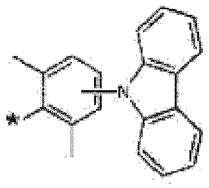
10-157
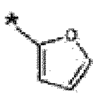
10-158
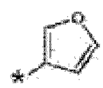
10-159
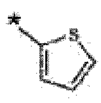
10-160
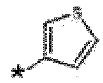
10-161

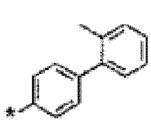
10-162
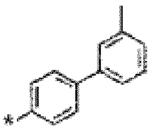
10-163
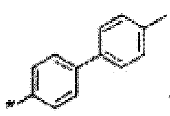
10-164
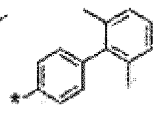
10-165
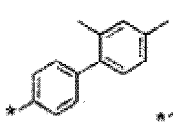
10-166
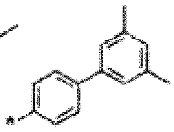
10-167
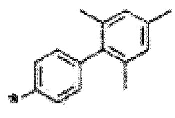
10-168

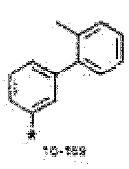
10-169
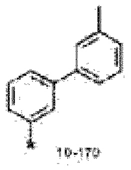
10-170
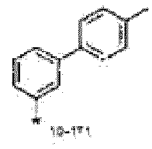
10-171
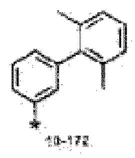
10-172
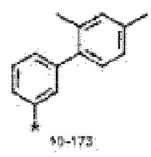
10-173
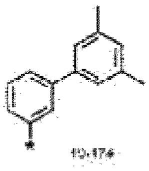
10-174
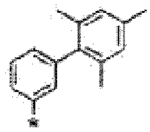
10-175

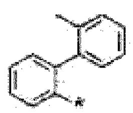
10-176
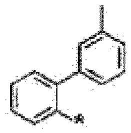
10-177
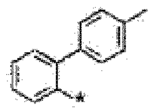
10-178
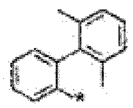
10-179
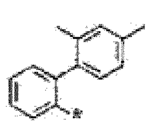
10-180
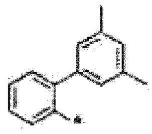
10-181
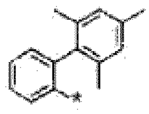
10-182

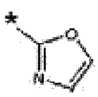
10-183
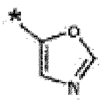
10-184
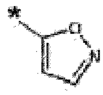
10-185
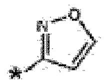
10-186
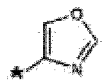
10-187
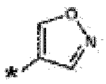
10-188
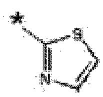
10-189
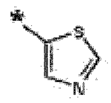
10-190
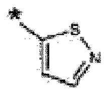
10-191

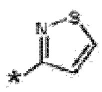
10-192
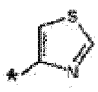
10-193
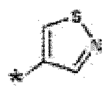
10-194

dans lequel, dans les formules 9-1 à 9-19 et 10-1 à 10-194, * indique un site de liaison à un atome adjacent, « Ph » indique un groupe phényle et « TMS » indique un groupe triméthylsilyle.

**8.** Composé organométallique selon l'une quelconque des revendications 1 à 7, dans lequel le composé organométallique représenté par la formule 1 est représenté par l'une quelconque des formules 11-1 à 11-6 :

Formule 11-1

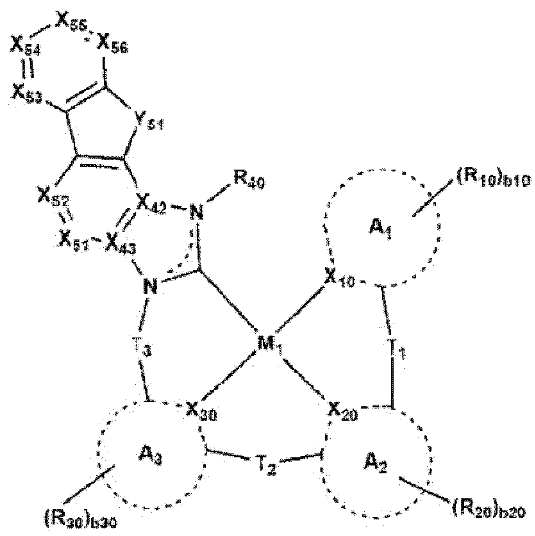

Formule 11-2

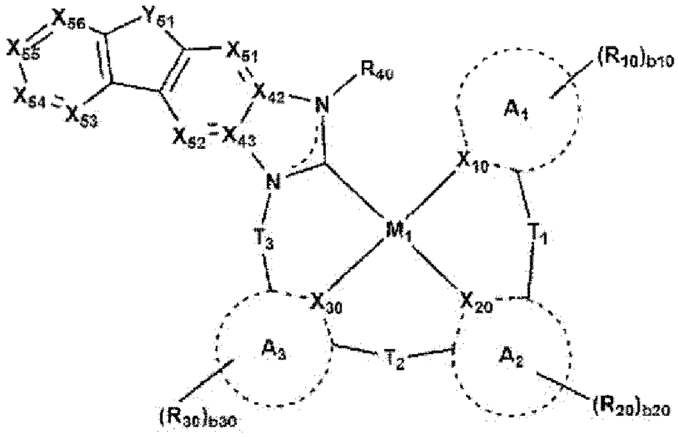

Formule 11-3

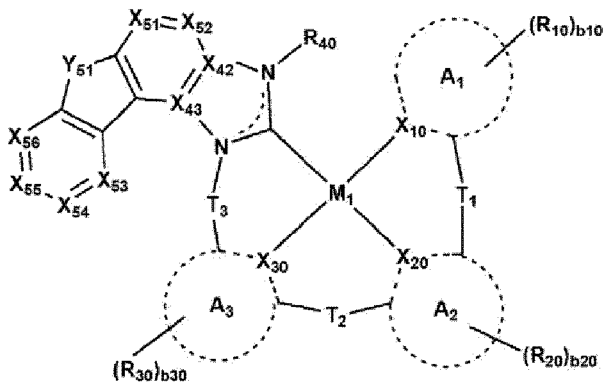

Formule 11-4

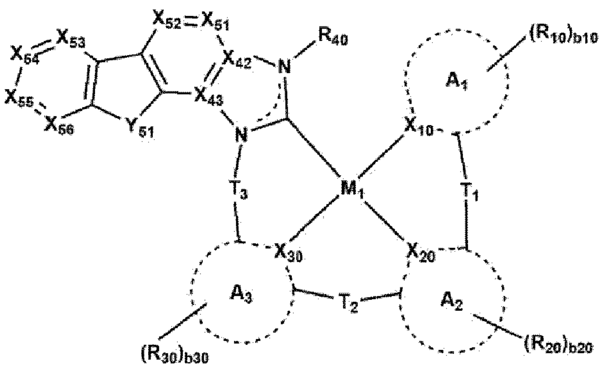

Formule 11-5

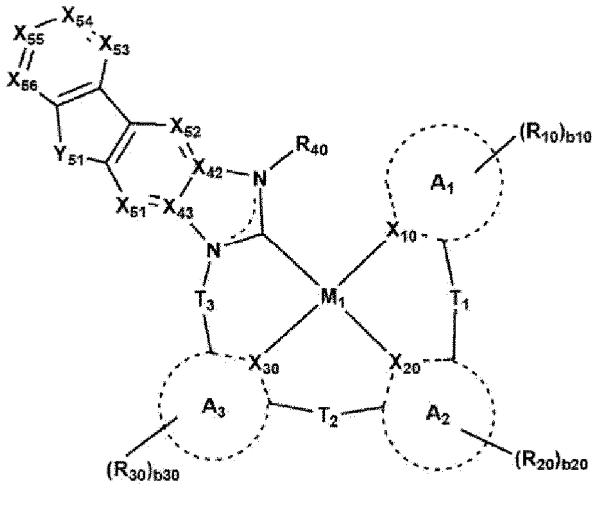

Formule 11-6

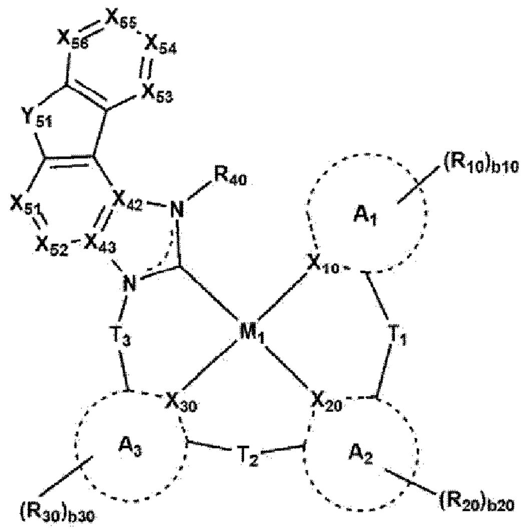

dans lequel, dans les formules 11-1 à 11-6,

$M_1$, $A_1$ à $A_3$, $X_{10}$, $X_{20}$, $X_{30}$, $X_{42}$, $X_{43}$, $T_1$ à $T_3$, $R_{10}$, $R_{20}$, $R_{30}$, $R_{40}$, $R_{50}$, b10, b20, et b30 s'entendent respectivement en référence aux descriptions de $M_1$, .$A_1$ à $A_3$, $X_{10}$, $X_{20}$, $X_{30}$, $X_{42}$, $X_{43}$, $T_1$ à $T_3$, $R_{10}$, $R_{20}$, $R_{30}$, $R_{40}$, $R_{50}$, b10, b20, et b30 dans la revendication 1,

$Y_{51}$ est *-O-*', *-S-*', *-N($R_5$)-*', *-C($R_5$)($R_6$)-*', *-Si($R_5$)($R_6$)-*', *-B($R_5$)-*', *-P($R_5$)-*', ou *-P(=O)($R_5$)-*', dans lequel * et *' indiquent chacun un site de liaison à un atome adjacent,

$R_5$ et $R_6$ s'entendent respectivement en référence aux descriptions de $R_1$ et $R_2$ dans la revendication 1,

$X_{51}$ est C($R_{51}$) ou N, $X_{52}$ est C($R_{52}$) ou N, $X_{53}$ est C($R_{53}$) ou N, $X_{54}$ est C($R_{54}$) ou N, $X_{55}$ est C($R_{55}$) ou N, $X_{56}$ est C($R_{56}$) ou N, et

$R_{51}$ à $R_{56}$ s'entendent chacun indépendamment en référence à la description de $R_{50}$ dans la revendication 1.

**9.** Composé organométallique selon l'une quelconque des revendications 1 à 8, dans lequel le composé organométallique représenté par la formule 1 est représenté par l'une quelconque des formules 12-1 à 12-6 :

Formule 12-1

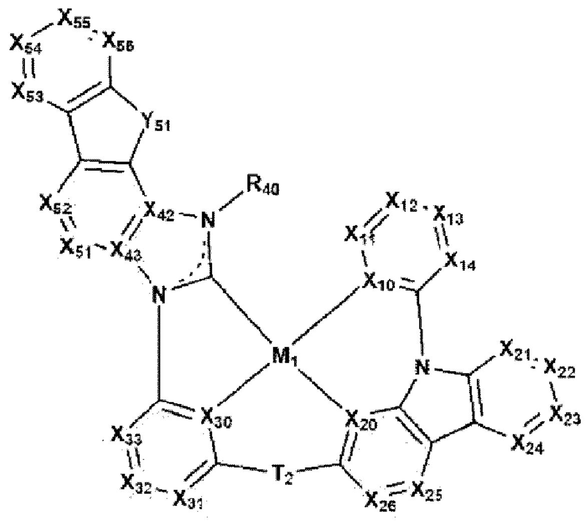

Formule 12-2

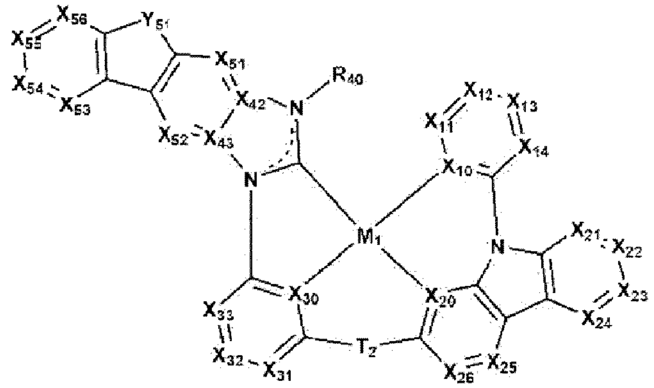

Formule 12-3

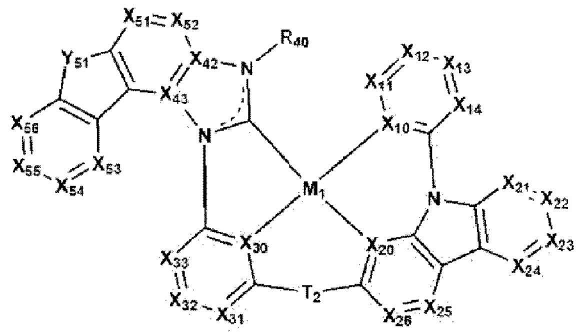

Formule 12-4

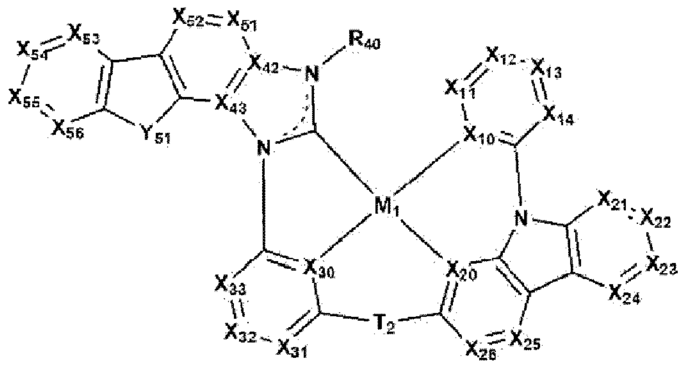

Formule 12-5

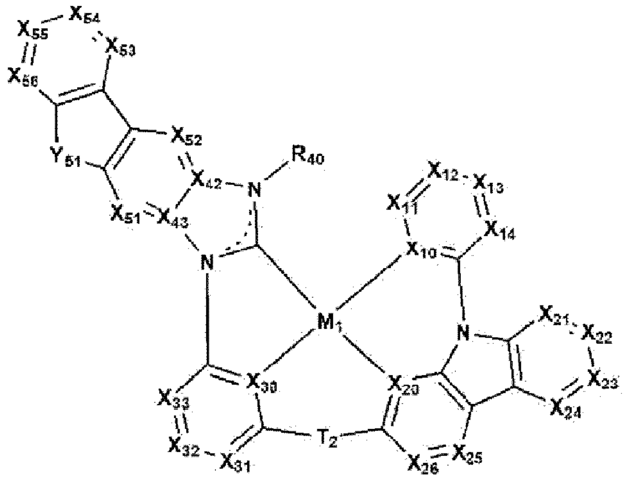

Formule 12-6

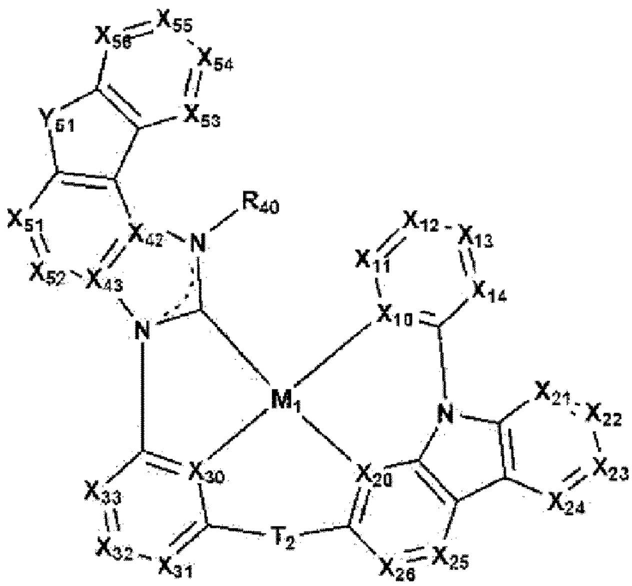

dans lequel, dans les formules 12-1 à 12-6,

$M_1$, $X_{10}$, $X_{20}$, $X_{30}$, $X_{42}$, $X_{43}$, $R_{40}$, et $T_2$ s'entendent respectivement en référence à $M_1$, $X_{10}$, $X_{20}$, $X_{30}$, $X_{42}$, $X_{43}$, $R_{40}$, et $T_2$ dans la revendication 1,

$Y_{51}$ est *-O-*', *-S-*', *-N($R_5$)-*', *-C($R_5$)($R_6$)-*', *-Si($R_5$)($R_6$)-*', *-B($R_5$)-*', *-P($R_5$)-*' ou *-P(=O)($R_5$)-*', dans lequel * et *' indiquent chacun un site de liaison à un atome adjacent,

$R_5$ et $R_6$ s'entendent respectivement en référence à $R_1$ et $R_2$ dans la revendication 1,

$X_{11}$ est C($R_{11}$) ou N, $X_{12}$ est C($R_{12}$) ou N, $X_{13}$ est C($R_{13}$) ou N, et $X_{14}$ est C($R_{14}$) ou N,

$X_{21}$ est C($R_{21}$) ou N, $X_{22}$ est C($R_{22}$) ou N, $X_{23}$ est C($R_{23}$) ou N, $X_{24}$ est C($R_{24}$) ou N, $X_{25}$ est C($R_{25}$) ou N, et $X_{26}$ est C($R_{26}$) ou N,

$X_{31}$ est C($R_{31}$) ou N, $X_{32}$ est C($R_{32}$) ou N, et $X_{33}$ est C($R_{33}$) ou N,

$X_{51}$ est C($R_{51}$) ou N, $X_{52}$ est C($R_{52}$) ou N, $X_{53}$ est C($R_{53}$) ou N, $X_{54}$ est C($R_{54}$) ou N, $X_{55}$ est C($R_{55}$) ou N, et $X_{56}$ est C($R_{56}$) ou N,

$R_{11}$ à $R_{14}$ sont chacun indépendamment compris en référence à $R_{10}$ dans la revendication 1,

$R_{21}$ à $R_{26}$ sont chacun indépendamment compris en référence à $R_{20}$ dans la revendication 1,

$R_{31}$ à $R_{33}$ sont chacun indépendamment compris en référence à $R_{30}$ dans la revendication 1, et

$R_{51}$ à $R_{56}$ sont chacun indépendamment compris en référence à $R_{50}$ dans la revendication 1.

**10.** Composé organométallique selon l'une quelconque des revendications 1 à 9, dans lequel le composé organométallique est l'un quelconque des composés 1 à 72 :

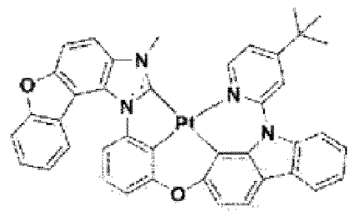

1

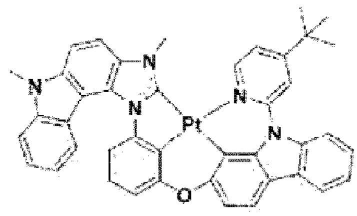

2

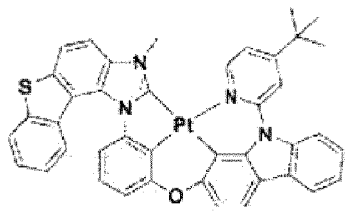

3

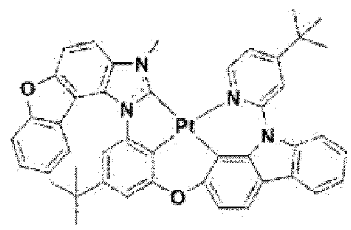

4

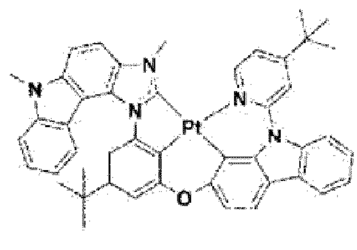

5

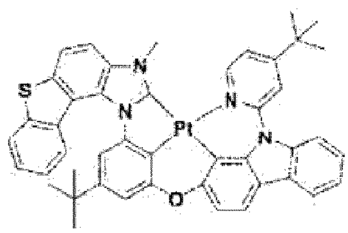

6

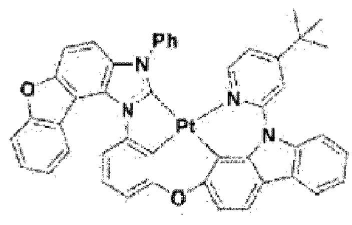

7

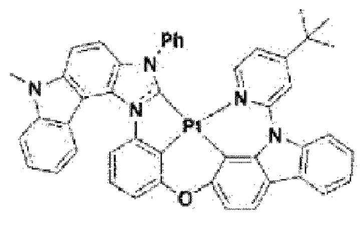

8

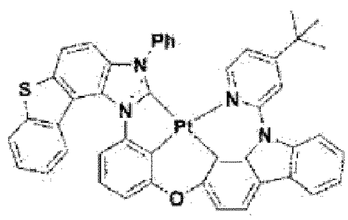

9

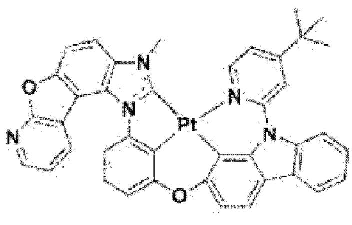

10

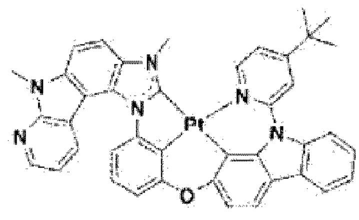

11

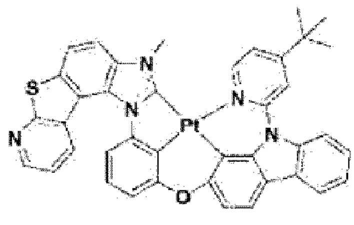

12

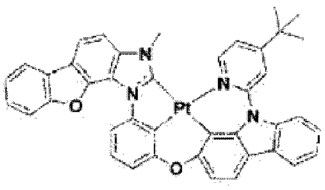

13

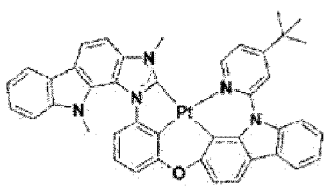

14

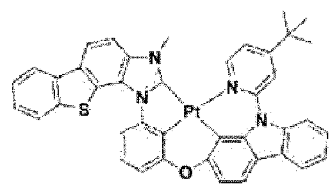

15

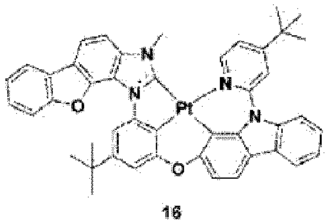

16

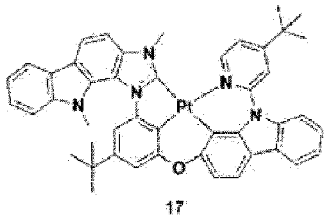

17

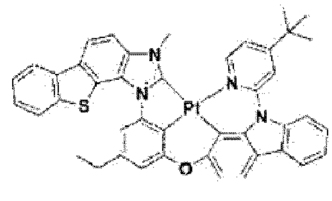

18

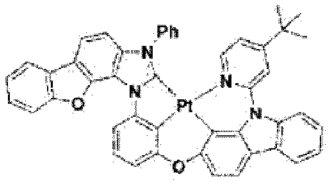

19

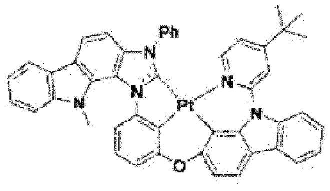

20

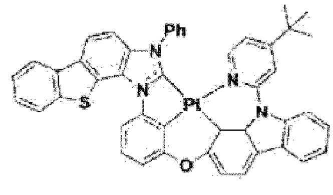

21

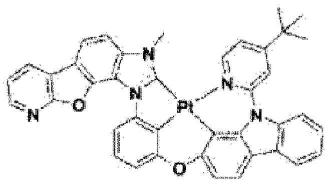

22

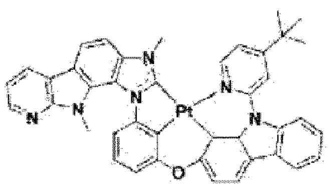

23

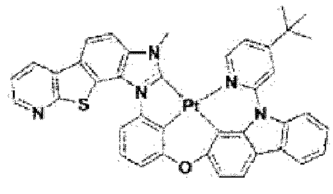

24

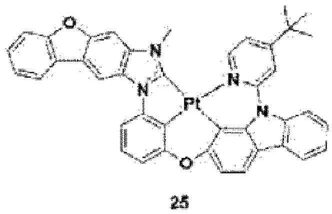

25

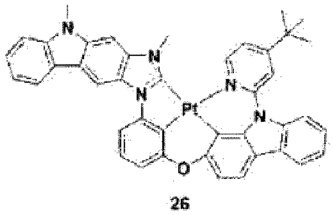

26

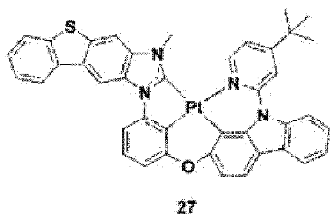

27

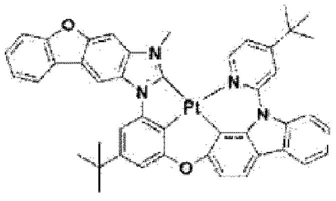

28

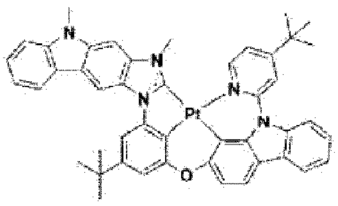

29

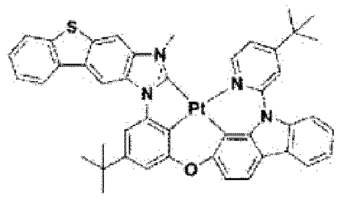

30

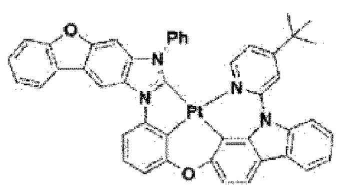

31

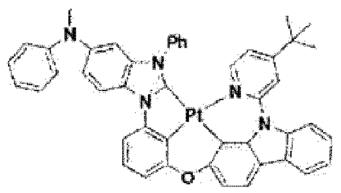

32

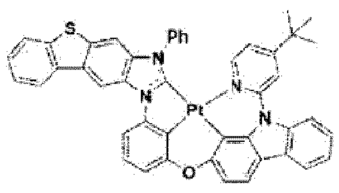

33

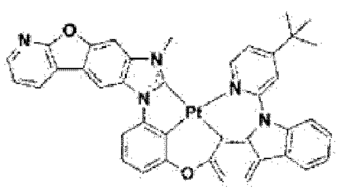

34

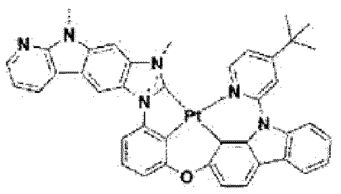

35

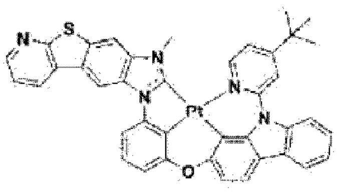

36

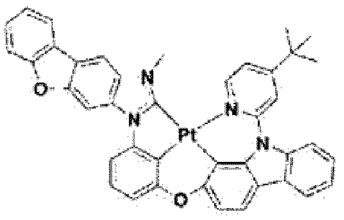

37

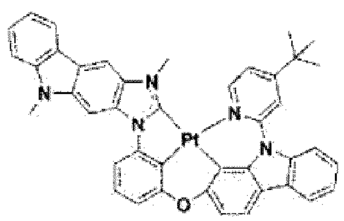

38

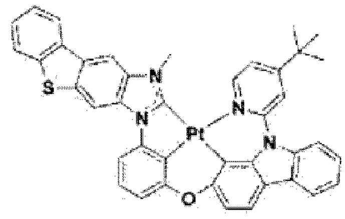

39

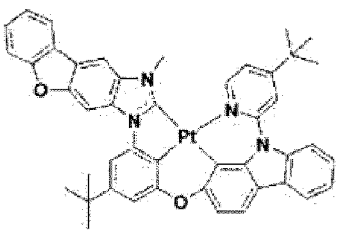

40

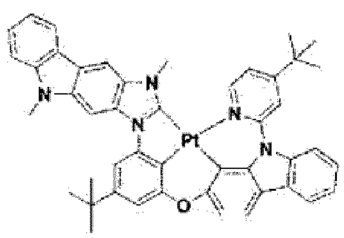

41

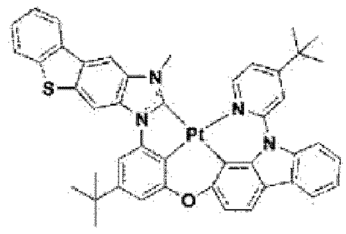

42

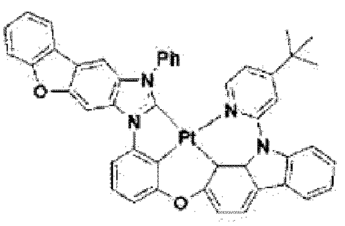

43

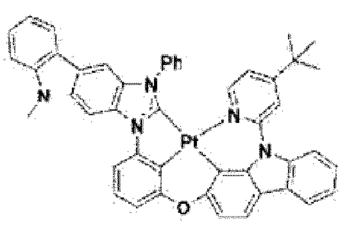

44

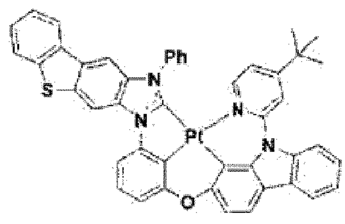

45

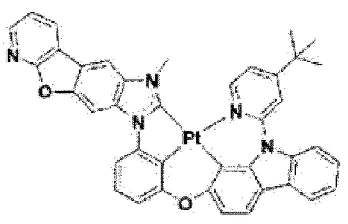

46

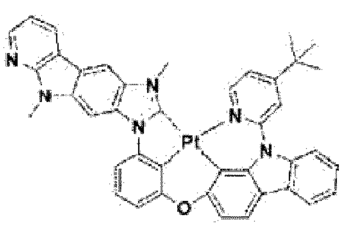

47

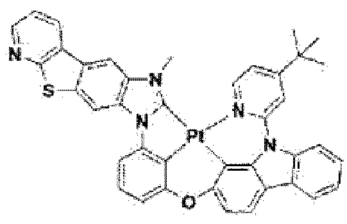

48

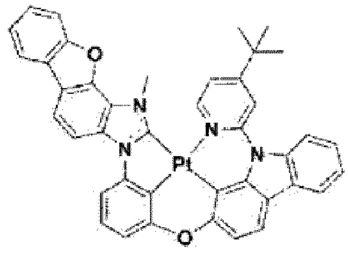

49

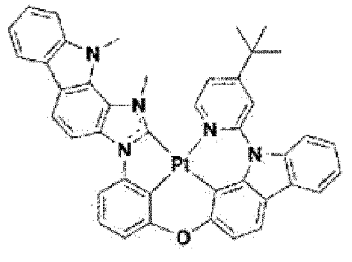

50

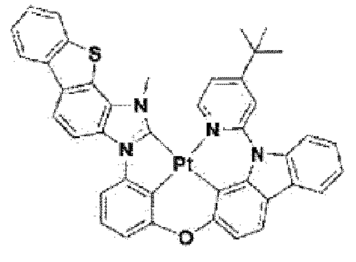

51

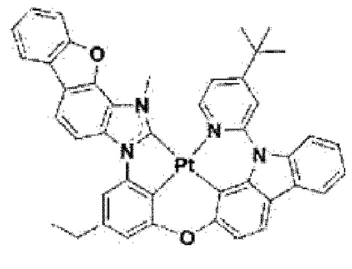

52

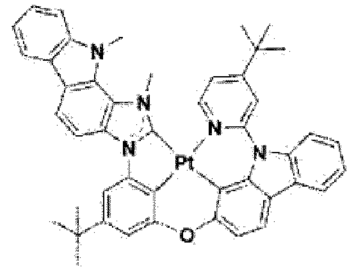

53

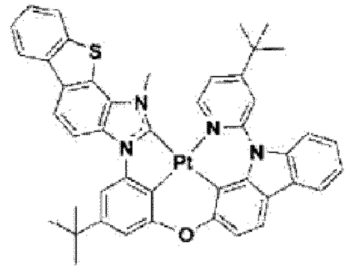

54

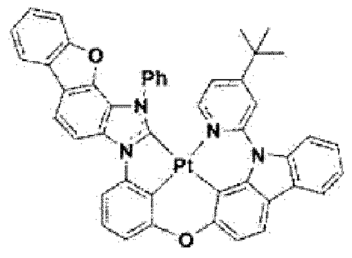

55

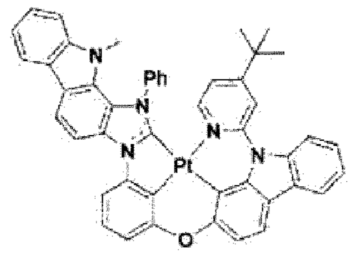

56

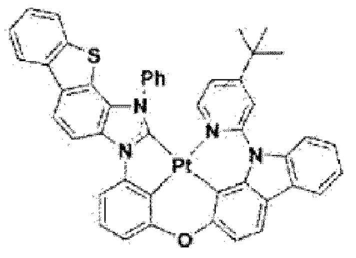

57

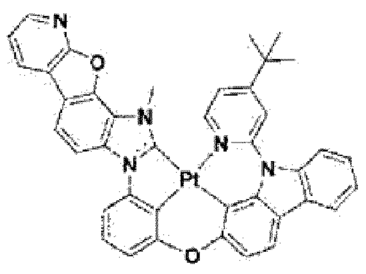

58

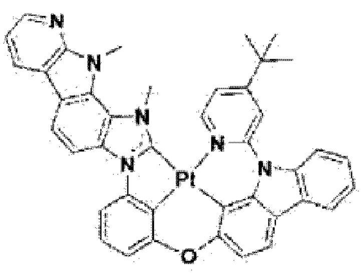

59

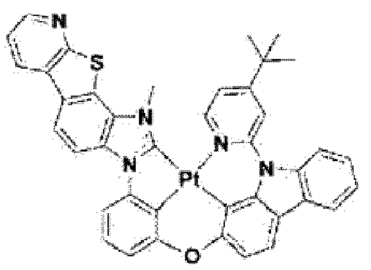

60

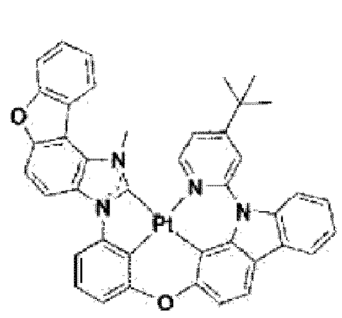

61

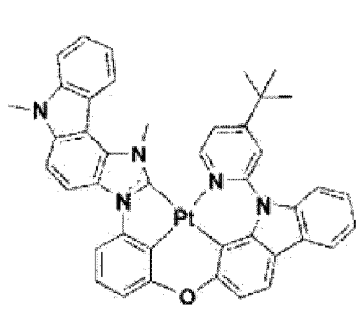

62

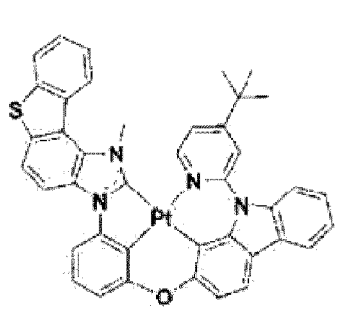

63

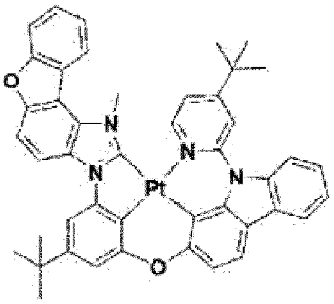

64

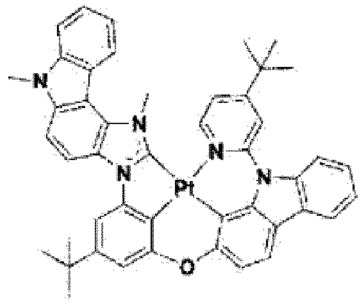

65

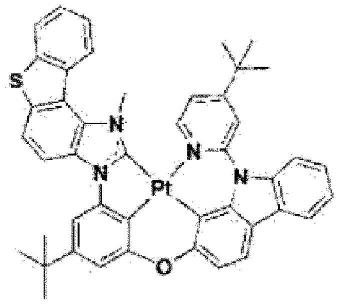

66

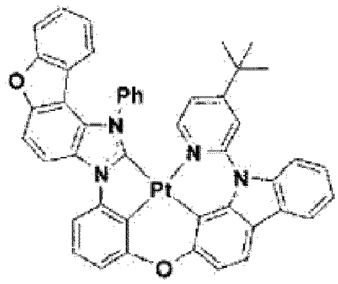

67

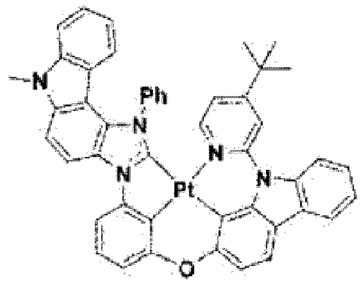

68

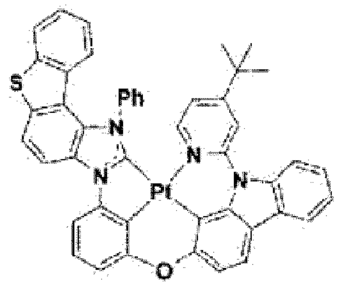

69

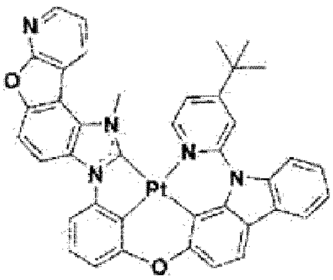

70

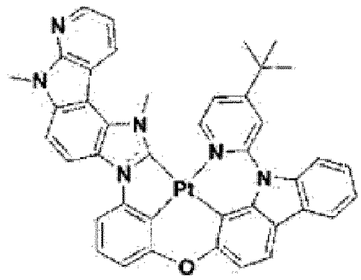

71

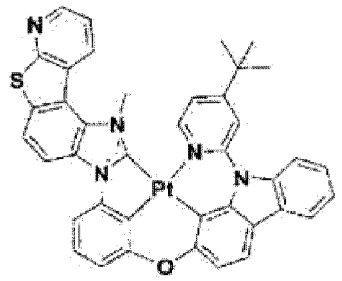

72

**11.** Dispositif électroluminescent organique, comprenant :

une première électrode ;
une seconde électrode ; et
une couche organique disposée entre la première électrode et la seconde électrode, dans lequel la couche organique comprend :

une couche d'émission, et
au moins un composé organométallique selon l'une quelconque des revendications 1 à 10.

**12.** Dispositif électroluminescent organique selon la revendication 11, dans lequel :

la première électrode est une anode,
la seconde électrode est une cathode, et
la couche organique comprend une région de transport de trous disposée entre la première électrode et la couche d'émission et une région de transport d'électrons disposée entre la couche d'émission et la seconde électrode,
la région de transport de trous comprend une couche d'injection de trous, une couche de transport de trous, une couche de blocage d'électrons, ou toute combinaison de celles-ci, et
la région de transport d'électrons comprend une couche de blocage de trous, une couche de transport d'électrons, une couche d'injection d'électrons ou toute combinaison de celles-ci.

**13.** Dispositif électroluminescent organique selon la revendication 11 ou 12, dans lequel la couche d'émission comprend le composé organométallique.

**14.** Dispositif électroluminescent organique selon la revendication 13, dans lequel la couche d'émission comprend en outre un hôte, dans lequel l'hôte est présent dans la couche d'émission en une quantité supérieure à une quantité du composé organométallique dans la couche d'émission ; et/ou
dans lequel la couche d'émission émet une lumière bleue ayant une longueur d'onde d'émission maximale dans une plage de 430 nanomètres à 480 nanomètres.

**15.** Composition de diagnostic comprenant au moins l'un des composés organométalliques de l'une quelconque des revendications 1 à 10.

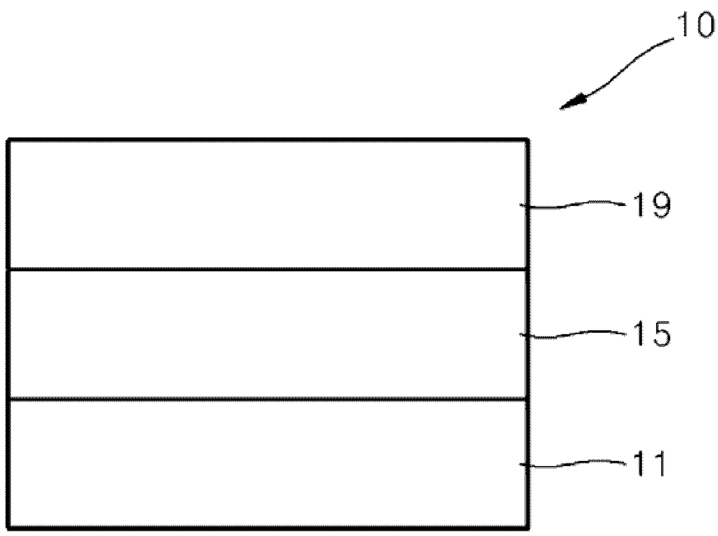
10
19
15
11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2019036042 A1